(19) **Europäisches Patentamt / European Patent Office / Office européen des brevets**

(11) **EP 3 248 018 B1**

(12) # EUROPEAN PATENT SPECIFICATION

(45) Date of publication and mention
of the grant of the patent:
**08.01.2020 Bulletin 2020/02**

(51) Int Cl.:
*G01N 35/10* (2006.01)     *G01N 35/08* (2006.01)
*B01L 3/02* (2006.01)

(21) Application number: **16740872.3**

(22) Date of filing: **22.01.2016**

(86) International application number:
**PCT/US2016/014612**

(87) International publication number:
**WO 2016/118915 (28.07.2016 Gazette 2016/30)**

(54) **DEVICES AND SYSTEMS FOR MOLECULAR BARCODING OF NUCLEIC ACID TARGETS IN SINGLE CELLS**

VORRICHTUNGEN UND SYSTEME ZUM MOLEKULAREN BARCODING VON
NUKLEINSÄURETARGETS IN EINZELZELLEN

DISPOSITIFS ET SYSTÈMES PERMETTANT LE CODAGE MOLÉCULAIRE À BARRES D'ACIDES
NUCLÉIQUES CIBLES DANS DES CELLULES INDIVIDUELLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.01.2015 US 201562106680 P
26.02.2015 US 201562121361 P
11.09.2015 US 201562217274 P**

(43) Date of publication of application:
**29.11.2017 Bulletin 2017/48**

(73) Proprietor: **Becton, Dickinson and Company
Franklin Lakes, NJ 07417-1880 (US)**

(72) Inventors:
• **LI, Sixing
  Menlo Park, CA 94025 (US)**
• **HUANG, Xiaohua, Chen
  Mountain View, CA 94043 (US)**
• **METZLER, Kimberly, R.
  San Francisco, CA 94121 (US)**
• **WALCZAK, Elisabeth, Marie
  Menlo Park, CA 94025 (US)**
• **FAN, Christina
  San Jose, CA 95138 (US)**

• **FODOR, Stephen, P., A.
  Palo Alto, CA 94301 (US)**
• **FU, Glenn
  Dublin, CA 94568 (US)**
• **FACER, Geoff
  Redwood City, CA 94061 (US)**
• **STERN, David
  Mountain View, CA 94043 (US)**
• **LAI, Janice
  Mountain View, CA 94041 (US)**
• **CHANEY, Ari
  Palm Beach Gardens, FL 33418 (US)**
• **SPUHLER, Philipp
  Menlo Park, CA 94025 (US)**

(74) Representative: **Carpmaels & Ransford LLP
One Southampton Row
London WC1B 5HA (GB)**

(56) References cited:
**WO-A1-2008/147428     WO-A1-2013/148525
US-A1- 2006 002 824     US-A1- 2006 040 297
US-A1- 2010 120 630     US-A1- 2010 300 895
US-A1- 2012 045 844     US-A1- 2014 155 274
US-A1- 2014 309 945**

EP 3 248 018 B1

**Description**

**BACKGROUND**

The ability to detect and quantify specific nucleic acid and protein molecules in individual cells is critical for understanding the role of cellular diversity in development, health, and disease. Flow cytometry has become a standard technology for high-throughput detection of protein markers on single cells and has been widely adopted in basic research and clinical diagnostics. In contrast, nucleic acid measurements such as mRNA expression are typically conducted on bulk samples, obscuring the contributions from individual cells. In order to characterize the complexity of cellular systems, it is highly desirable to develop methods, devices, and systems for monitoring the expression of a large number of genes across many thousands of cells.

US2010/0300895 discloses an apparatus and methods for performing electrochemical reactions. US2006/0040297 discloses an apparatus and method for performing rapid DNA sequencing, such as genomic sequencing. WO2008/147428 discloses methods and systems for providing a cell support system. WO2013/148525 discloses flow cells for high density array chips.

**SUMMARY**

The invention provides a device comprising: a substrate comprising at least 100 microwells, wherein each microwell has a volume ranging from about 1,000 $\mu$m$^3$ to about 786,000 $\mu$m$^3$, and a plurality of beads, wherein a plurality of the at least 100 microwells each contain a single bead, and wherein the ratio of the average diameter of the microwells to the diameter of the beads ranges from about 1.2 to about 1.8; and a flow cell in fluid communication with the substrate; and a pipette tip interface for loading or removing samples, assay reagents, bead suspensions, or waste from the device; and a valve that prevents fluid flow within the device unless a pipette tip is inserted into the conical feature of the pipette tip interface. The invention also provides a system comprising: the device of the invention; and a flow controller; wherein the flow controller is configured to control the delivery of fluids.
The invention also provides a kit comprising the device of the invention.
The invention also provides a method for loading one or more cell samples into the microwells of the device of the invention, the method comprising: a) injecting air into the flow cell in fluid communication with the substrate comprising at least 100 microwells; b) injecting a cell sample into the flow cell; and c) injecting air into the flow cell.

Disclosed herein are devices, systems, and kits for determining the number of target molecules in a sample. In some embodiments, the devices of the present disclosure comprise: a) a substrate comprising: i) at least 100 microwells, wherein each microwell has a volume ranging from about 1,000 $\mu$m$^3$ to about 786,000 $\mu$m$^3$, and ii) a plurality of beads, wherein a plurality of the at least 100 microwells each contain a single bead, and wherein the ratio of the average diameter of the microwells to the diameter of the beads ranges from about 1.2 to about 1.8; and b) a flow cell in fluid communication with the substrate.

In some embodiments, the devices of the present disclosure comprise: a) a substrate comprising at least 100 microwells, wherein each microwell has a volume ranging from about 1,000 $\mu$m$^3$ to about 786,000 $\mu$m$^3$, and wherein a surface of the at least 100 microwells is coated with a surface coating to improve wettability; and b) a flow cell in fluid communication with the substrate.

In some embodiments, the devices of the present disclosure comprise: a) a substrate comprising at least 100 microwells, wherein each microwell has a volume ranging from about 1,000 $\mu$m$^3$ to about 786,000 $\mu$m$^3$, a non-circular cross-section in the plane of the substrate, and an aspect ratio of average diameter to depth ranges from about 0.1 to 2; and b) a flow cell in fluid communication with the substrate.

In some embodiments, the devices of the present disclosure comprise: a) a substrate comprising at least 100 microwells, wherein each microwell has a volume ranging from about 1,000 $\mu$m$^3$ to about 786,000 $\mu$m$^3$, and a positive draft angle ranging from about 1 degree to about 15 degrees; and b) a flow cell in fluid communication with the substrate.

In some embodiments, the devices of the present disclosure comprise: a) a substrate comprising at least 100 microwells, wherein each microwell has a volume ranging from about 1,000 $\mu$m$^3$ to about 786,000 $\mu$m$^3$, and wherein the substrate further comprises surface features that surround each microwell or straddle the surface between each microwell of the at least 100 microwells; and b) a flow cell in fluid communication with the substrate.

In some embodiments, the devices of the present disclosure comprise: a) a substrate comprising at least 100 microwells, wherein each microwell has a volume ranging from about 1,000 $\mu$m$^3$ to about 786,000 $\mu$m$^3$; b) a flow cell in fluid communication with the substrate; and c) at least one inlet port and at least one outlet port, wherein the at least one inlet port and at least one outlet port are in fluid communication with the flow cell via fluid channels having axes that form an angle relative to the plane of the substrate, and wherein the at least one inlet port and at least one outlet port are capable of directing a flow of a fluid through the flow cell, thereby contacting the microwells with the fluid.

In some embodiments, the devices further comprise at least one inlet port and at least one outlet port, wherein the at least one inlet port and at least one outlet port are capable of directing a flow of a fluid through the flow cell, thereby contacting the microwells with the fluid. In some embodiments, the coefficient of variation for microwell volume is less than 5%. In some embodiments, the substrate comprises from 1,000 to 5,000,000 microwells. In some embodiments, the substrate comprises from 10,000 to 200,000 microwells. In some embodiments, each microwell has a volume ranging from about 21,000 $\mu m^3$ to about 170,000 $\mu m^3$. In some embodiments, each microwell has a volume of about 144,000 $\mu m^3$. In some embodiments, the microwells have a non-circular cross-section in the plane of the substrate. In some embodiments, the aspect ratio of average diameter to depth for the at least 100 microwells ranges from about 0.1 to 2. In some embodiments, the aspect ratio of average diameter to depth for the at least 100 microwells is about 0.9. In some embodiments, the dimensions of the at least 100 microwells are chosen so that each microwell may contain at most one bead. In some embodiments, the ratio of the average diameter of the microwells to the diameter of the beads is about 1.5. In some embodiments, the side walls of the microwells have a positive draft angle of about 1 to 15 degrees. In some embodiments, the side walls of the microwells have a positive draft angle of about 3 to 7 degrees. In some embodiments, the substrate further comprises surface features that surround each microwell or straddle the surface between microwells of the at least 100 microwells. In some embodiments, the substrate further comprises surface features that surround each microwell or straddle the surface between microwells of the at least 100 microwells, wherein the surface features are selected from the group consisting of rounded, domed, ridged, and peaked surface features, or any combination thereof. In some embodiments, the percentage of the at least 100 microwells that contain a single bead is at least about 10%. In some embodiments, the percentage of the at least 100 microwells that contain a single bead is at least about 50%. In some embodiments, the percentage of the at least 100 microwells that contain a single cell is between about 0.01% and about 15%. In some embodiments, the percentage of the at least 100 microwells that contain a single cell is between about 1% and about 11%. In some embodiments, a surface of the at least 100 microwells is coated with polyethylene glycol (PEG), poly-Hema, pluronic acid F68, pluronic acid F108, polysorbate 20, silicon dioxide (SiO2), or any combination thereof. In some embodiments, a surface of the at least 100 microwells comprises a plasma-treated surface. In some embodiments, the at least one inlet port and at least one outlet port are in fluid communication with the flow cell via fluid channels having axes that form an angle relative to the plane of the substrate. In some embodiments, the at least one inlet port and at least one outlet port are in fluid communication with the flow cell via fluid channels having axes that form an angle relative to the plane of the substrate, and wherein the angles for the fluid channels communicating with the inlet port and the outlet port are the same. In some embodiments, the at least one inlet port and at least one outlet port are in fluid communication with the flow cell via fluid channels having axes that form an angle relative to the plane of the substrate, and wherein the angles for the fluid channels communicating with the inlet port and the outlet port are different. In some embodiments, the angle between the axes of the fluid channels and the plane of the substrate is between about 15 degrees and about 75 degrees. In some embodiments, at least one of the angles between the axes of the fluid channels and the plane of the substrate is about 45 degrees. In some embodiments, the substrate is fabricated from a material selected from the group consisting of silicon, fused-silica, glass, a polymer, a metal, an elastomer, polydimethylsiloxane, agarose, and a hydrogel, or any combination thereof. In some embodiments, the flow cell comprises the substrate. In some embodiments, the flow cell can be detached from the substrate. In some embodiments, the flow cell is fabricated from a material selected from the group consisting of silicon, fused-silica, glass, polydimethylsiloxane (PDMS; elastomer), polymethylmethacrylate (PMMA), polycarbonate (PC), polypropylene (PP), polyethylene (PE), high density polyethylene (HDPE), polyimide, cyclic olefin polymers (COP), cyclic olefin copolymers (COC), polyethylene terephthalate (PET), epoxy resin, and metal, or any combination of these materials. In some embodiments, the substrate or flow cell further comprises a transparent window for optical imaging of the at least 100 microwells. In some embodiments, the devices further comprise one or more sample reservoirs, reagent reservoirs, waste reservoirs, or any combination thereof. In some embodiments, one or more reagent reservoirs are pre-loaded with a bead suspension or an assay reagent. In some embodiments, the devices further comprise a pipette tip interface for loading or removing samples, assay reagents, bead suspensions, or waste from the device. In some embodiments, the pipette tip interface comprises a conical feature that mates to a pipette tip to form a fluid connection with the inlet port or outlet port. In some embodiments, the conical feature is comprised of a compliant material that forms a substantially leak-proof seal with the pipette tip. In some embodiments, the compliant material is polydimethylsiloxane (PDMS), polybutadiene, polyisoprene, polyurethane, or any combination thereof. In some embodiments, the devices further comprising a valve that prevents fluid flow within the device unless a pipette tip is inserted into the conical feature of the pipette tip interface. In some embodiments, each single bead of a plurality of beads contained within the at least 100 microwells comprises a plurality of tethered stochastic labels capable of attaching to a target nucleic acid molecule in a stochastic manner. In some embodiments, each stochastic label in the plurality of tethered stochastic labels comprises a cell label that is identical for all of the stochastic labels attached to that bead, but is different for stochastic labels attached to different beads. In some embodiments, the plurality of tethered stochastic labels attached to a single bead further comprises a diverse set of molecular labels. In some embodiments, each stochastic label in the plurality of tethered stochastic labels further comprises a target nucleic acid molecule binding region. In some embodiments, each stochastic label in the plurality of tethered stochastic labels further comprises a universal primer sequence.

In some embodiments, the target nucleic acid molecule binding regions of the plurality of stochastic labels tethered to a bead comprise a sequence selected from the group consisting of a gene-specific sequence, an oligo-dT sequence, and a random multimer, or any combination thereof. In some embodiments, the device constitutes a consumable component of an instrument system configured to perform automated, stochastic labeling assays on a plurality of single cells.

Also disclosed herein are systems comprising: a) a device comprising: i) a substrate comprising at least 100 microwells, wherein each microwell has a volume ranging from about 1,000 $\mu m^3$ to about 786,000 $\mu m^3$, and wherein the substrate further comprises surface features that surround each microwell or straddle the surface between microwells of the at least 100 microwells; ii) a flow cell in fluid communication with the substrate; and iii) at least one inlet port and at least one outlet port, wherein the at least one inlet port and at least one outlet port are capable of directing a flow of a fluid through the flow cell, thereby contacting the microwells with the fluid; and b) a flow controller; wherein the flow controller is configured to control the delivery of fluids.

In some embodiments, the systems of the present disclosure comprise: a) a device comprising: i) a substrate comprising at least 100 microwells, wherein each microwell has a volume ranging from about 1,000 $\mu m^3$ to about 786,000 $\mu m^3$, a non-circular cross-section in the plane of the substrate, and an aspect ratio of average diameter to depth that ranges from about 0.1 to 2; ii) a flow cell in fluid communication with the substrate; and iii) at least one inlet port and at least one outlet port, wherein the at least one inlet port and at least one outlet port are capable of directing a flow of a fluid through the flow cell, thereby contacting the microwells with the fluid; and b) a flow controller; wherein the flow controller is configured to control the delivery of fluids.

In some embodiments, the systems further comprise fluids wherein the fluids comprise cell samples, bead suspensions, assay reagents, or any combination thereof. In some embodiments, the device is a removable, consumable component of the system. In some embodiments, cell samples and bead suspensions are dispensed or injected directly into the device by the user. In some embodiments, beads and assay reagents other than cell samples are preloaded in the device. In some embodiments, the flow controller is configured to intersperse fluid injections into the flow cell with air injections. In some embodiments, the systems further comprise a distribution mechanism for enhancing the uniform distribution of cells and beads across the at least 100 microwells, wherein the distribution mechanism performs an action selected from the group consisting of rocking, shaking, swirling, recirculating flow, low frequency agitation, and high frequency agitation, or any combination thereof. In some embodiments, the systems further comprise a cell lysis mechanism that uses a high frequency piezoelectric transducer for sonicating the cells. In some embodiments, the systems further comprise a temperature controller for maintaining a user-specified temperature, or for ramping temperature between two or more specified temperatures over two or more specified time intervals. In some embodiments, the systems further comprise a magnetic field controller for creating magnetic field gradients used in eluting beads from the at least 100 microwells or for transporting beads through the device. In some embodiments, the systems further comprise an imaging system configured to capture and process images of all or a portion of the at least 100 microwells, wherein the imaging system further comprises an illumination subsystem, an imaging subsystem, and a processor. In some embodiments, the imaging system is configured to perform bright-field, dark-field, fluorescence, or quantitative phase imaging. In some embodiments, the imaging system is configured to provide real-time image analysis capability, and wherein the real-time image analysis is used to control a distribution mechanism for enhancing the uniform distribution of cells or beads across the at least 100 microwells so that a predetermined cell or bead distribution is achieved. In some embodiments, the predetermined distribution of cells and beads is one in which at least 10% of the microwells contain both a single cell and a single bead. In some embodiments, the predetermined distribution of cells and beads is one in which at least 25% of the microwells contain both a single cell and a single bead. In some embodiments, the systems further comprise a selection mechanism, wherein information derived from the processed images is used to identify a subset of cells exhibiting one or more specified characteristics, and the selection mechanism is configured to either include or exclude the subset of cells from subsequent data analysis. In some embodiments, the selection mechanism comprises physical removal of beads co-localized with cells of the identified subset of cells from the at least 100 microwells. In some embodiments, the selection mechanism comprises physical entrapment of beads co-localized with cells of the identified subset of cells in the at least 100 microwells. In some embodiments, the selection mechanism comprises use of dual-encoded beads wherein each individual bead is both optically-encoded and encoded by an attached oligonucleotide cellular label, and sequencing of the cellular labels attached to beads co-localized with cells of the identified subset of cells generates a list of sequence data to be included or excluded from further analysis. In some embodiments, the flow controller is configured to deliver a first test compound to the at least 100 microwells at a first time, and to deliver a cell lysis reagent to the at least 100 microwells at a second time. In some embodiments, the first time and the second time are the same. In some embodiments, the one or more specified characteristics are selected from the group consisting of cell size, cell shape, live cells, dead cells, a specified range of intracellular pH, a specified range of membrane potential, a specified level of intracellular calcium, the presence of one or more specified cell surface markers, and the expression of one or more specified genetic markers. In some embodiments, the cell samples comprise patient samples and the assay results are used by a healthcare provider for diagnosis of a disease or to make informed healthcare treatment decisions. In some embodiments, the viscosity of a fluid used in the system is adjusted to be between about 1.2x and 10x that of water in order to reduce the rate of diffusion of substances between microwells. In some embodiments, the viscosity of a fluid used to distribute cells or beads into the at least 100 microwells of the device is adjusted to be between about 1.2x and 10x that of water. In some embodiments, the density of a fluid used to distribute cells or beads into the at least 100 microwells of the device is adjusted to be between about 0.8x and 1.25x that of the cells or beads. In some embodiments, the viscosity of a fluid used to retrieve beads from the at least 100 microwells of the device is adjusted to be between about 1.2x and 10x that of water. In some embodiments, the density of a fluid used to retrieve beads from the at least 100 microwells of the device is adjusted to be between about 0.8x and 1.25x that of the beads.

Disclosed herein is software residing in a computer readable medium programmed to perform one or more of the following sequence data analysis steps: a) decoding or demultiplexing of sample barcode, cell barcode, molecular barcode, and target sequence data; b) automated clustering of cellular labels to compensate for amplification or sequencing errors, wherein the sequence data is collected for a library of stochastically-labeled target oligonucleotide molecules; c) alignment of sequence data with known reference sequences; d) determining the number of reads per gene per cell, and the number of unique transcript molecules per gene per cell; e) statistical analysis to predict confidence intervals for determinations of the number of transcript molecules per gene per cell; and f) statistical analysis to cluster cells according to gene expression data or to identify subpopulations of rare cells.

Disclosed herein is software residing in a computer readable medium programmed to perform the following image processing and instrument control steps: a) detection of microwells in one or more images of a plurality of microwells; b) detection of microwells containing single cells in one or more images of a plurality of microwells; c) detection of microwells containing two or more cells in one or more images of a plurality of microwells, and determining the number of cells in each of the detected microwells; d) detection of microwells containing single beads in one or more images of a plurality of microwells; e) detection of microwells containing two or more beads in one or more images of a plurality of microwells, and determining the number of beads in each of the detected microwells; f) determining the number of microwells containing single cells after performing step (b); g) determining the number of microwells containing single beads after performing step (d); and h) determining the number of microwells containing single cells and single beads after performing steps (b) and (d), wherein the numbers determined in steps (f) - (h) are used to control an apparatus configured to distribute cells and beads across a plurality of microwells.

Disclosed herein is software residing in a computer readable medium programmed to perform the following image processing and instrument control steps: a) detection of a subset of cells exhibiting one or more specified characteristics in one or more images of a plurality of microwells, wherein a fraction of the microwells contain cells; b) determining the locations of the microwells which contain the subset of cells; and c) using the locations determined in step (b) to control a selection apparatus configured to exclude the subset of cells from subsequent sequence data analysis.

In some embodiments, the selection apparatus of step c) is configured to include only the subset of cells in subsequent sequence data analysis. In some embodiments, the one or more images of a plurality of microwells are images selected from the group consisting of bright-field images, dark-field images, fluorescence images, luminescence images, and phosphorescence images. In some embodiments, the software further comprises the use of one or more algorithms selected from the group consisting of the Canny edge detection method, the Canny-Deriche edge detection method, the Sobel operator method, a first-order gradient detection method, a second order differential edge detection method, a phase coherence edge detection method, an intensity thresholding method, an intensity clustering method, an intensity histogram-based method, the generalized Hough transform, the circular Hough transform, the Fourier transform, the fast Fourier transform, wavelet analysis, and auto-correlation analysis. In some embodiments, the one or more specified characteristics are selected from the group consisting of cell size, cell shape, live cells, dead cells, a specified range of intracellular pH, a specified range of membrane potential, a specified level of intracellular calcium, the presence of one or more specified cell surface markers, and the expression of one or more specified genetic markers.

Disclosed herein are kits comprising: a) a device comprising: i) a substrate, wherein the substrate further comprises: 1) at least 100 microwells, wherein each microwell has a volume ranging from about 1,000 $\mu m^3$ to about 786,000 $\mu m^3$; and 2) a plurality of beads, wherein a plurality of the at least 100 microwells each contain a single bead, and wherein the ratio of the average diameter of the microwells to the diameter of the beads ranges from about 1.2 to about 1.8; and ii) a flow cell in fluid communication with the substrate.

Disclosed herein are kits comprising a) a device comprising: i) a substrate comprising at least 100 microwells, wherein each microwell has a volume ranging from about 1,000 $\mu m^3$ to about 786,000 $\mu m^3$, and wherein a surface of the at least 100 microwells is coated with a surface coating to improve wettability; and ii) a flow cell in fluid communication with the substrate.

Disclosed herein are kits comprising: a) a device comprising: i) a substrate comprising at least 100 microwells, wherein each microwell has a volume ranging from about 1,000 $\mu m^3$ to about 786,000 $\mu m^3$, a non-circular cross-section in the plane of the substrate, and an aspect ratio of average diameter to depth of about 0.9; and ii) a flow cell in fluid communication with the substrate.

Disclosed herein are kits comprising: a) a device comprising: i) a substrate comprising at least 100 microwells, wherein each microwell has a volume ranging from about 1,000 $\mu m^3$ to about 786,000 $\mu m^3$, and a positive draft angle of between about 1 degree and 15 degrees; and ii) a flow cell in fluid communication with the substrate.

Disclosed herein are kits comprising: a) a device comprising: i) a substrate comprising at least 100 microwells, wherein each microwell has a volume ranging from about 1,000 $\mu$m$^3$ to about 786,000 $\mu$m$^3$, and wherein the substrate further comprises surface features that surround each microwell or straddle the surface between microwells of the at least 100 microwells; and ii) a flow cell in fluid communication with the substrate.

In some embodiments, the device further comprises at least one inlet port and at least one outlet port, wherein the at least one inlet port and at least one outlet port are capable of directing a flow of a fluid through the flow cell, thereby contacting the microwells with the fluid. In some embodiments, a plurality of the at least 100 microwells contain a single bead. In some embodiments, the percentage of the at least 100 microwells that contain a single bead is at least about 10%. In some embodiments, the percentage of the at least 100 microwells that contain a single bead is at least about 50%. In some embodiments, the percentage of the at least 100 microwells that contain a single bead is at least about 80%. In some embodiments, each single bead in the plurality of beads contained in microwells comprises a plurality of tethered stochastic labels capable of attaching to a target nucleic acid molecule in a stochastic manner. In some embodiments, the kit further comprises reagents for performing a reverse transcription reaction. In some embodiments, the kit further comprises reagents for performing a nucleic acid amplification reaction. In some embodiments, the kit further comprises reagents for performing one or more target-specific nucleic acid amplification reactions. In some embodiments, the kit further comprises a cell lysis buffer or hybridization buffer. In some embodiments, a surface of the at least 100 microwells is coated with polyehtylene glycol (PEG), poly-Hema, pluronic acid F68, pluronic acid F108, polysorbate 20, silicon dioxide (SiO2), or any combination thereof. In some embodiments, a surface of the at least 100 microwells comprises a plasma-treated surface. In some embodiments, the substrate further comprises surface features that surround each microwell or straddle the surface between each microwell of the at least 100 microwells. In some embodiments, the surface features are selected from the group consisting of rounded, domed, ridged, and peaked surface features, or any combination thereof.

Discslosed herein are methods for determining a number of occurrences of a target nucleic acid molecule in single cells from a selected subpopulation of cells, the methods comprising: a) capturing single cells and single beads in a plurality of microwells, wherein a single bead comprises a plurality of tethered stochastic labels, and wherein the plurality of tethered stochastic label further comprises: i) a bead-specific cellular label; ii) a diverse set of molecular labels; and ii) a plurality of target binding regions capable of hybridizing with nucleic acid molecules, wherein a subset of the target binding regions attached to a bead are specific for a set of one or more nucleic acid markers which define the subpopulation; b) hybridizing target nucleic acid molecules and nucleic acid markers released from single cells with the plurality of target binding regions tethered to single beads in a stochastic manner; c) performing an extension reaction to create: i) a plurality of molecular conjugates each comprising a stochastic label and a portion of a complementary sequence of the target nucleic acid molecule; and ii) a plurality of molecular conjugates each comprising a stochastic label and a portion of a complementary sequence of a nucleic acid marker; d) amplifying and sequencing the molecular conjugates; e) generating a list of cellular labels associated with the set of one or more nucleic acid markers that define the subpopulation; and f) determining the number of occurrences of the target molecule in single cells of the subpopulation of cells.

In some embodiments, the method is multiplexed. In some embodiments, the list of cellular labels associated with the set of one or more nucleic acid markers that define the subpopulation is used to exclude data from the subpopulation of cells from further sequence data analysis. In some embodiments, the target nucleic acid molecules are RNA molecules. In some embodiments, the target nucleic acid molecules are mRNA molecules. In some embodiments, the plurality of tethered stochastic labels further comprises a universal primer sequence. In some embodiments, the plurality of target binding regions of the plurality of stochastic labels tethered to a bead comprise a mixture of sequences selected from the group consisting of gene-specific sequences, oligo-dT sequences, and random multimer sequences, or any combination thereof.

Disclosed herein are methods for loading one or more cell samples into the microwells of the device of any one of claims 1 to 6, the method comprising: a) injecting air into the flow cell in fluid communication with the substrate comprising at least 100 microwells; b) injecting a cell sample into the flow cell; and c) injecting air into the flow cell.

In some embodiments, the methods further comprise injecting a buffer or bead suspension into the flow cell following step c). In some embodiments, the one or more cell samples each comprise one or more single cells. In some embodiments, the one or more cell samples comprise cells of at least two different cell types. In some embodiments, the one or more cell samples comprise immune cells. In some embodiments, the bead suspension comprises a plurality of beads, and each individual bead comprises a plurality of oligonucleotides attached to bead. In some embodiments, the plurality of oligonucleotides attached to each individual bead comprises a diverse set of molecular labels. In some embodiments, the plurality of oligonucleotides attached to each individual bead comprises a cellular label that is the same for all oligonucleotides attached to the individual bead, and wherein the cellular labels of the pluralities of oligonucleotides attached to different beads are different from each other. In some embodiments, the plurality of oligonucleotides attached to each individual bead comprises a target binding region. In some embodiments, the plurality of oligonucleotides attached to each individual bead comprises a universal primer binding sequence. In some embodiments, the injecting steps displace the contents of the flow cell. In some embodiments, the one or more cell samples and the beads are dispersed at least 10% more uniformly across the at least 100 microwells compared to loading without injecting air. In some embodiments, the one or more cell samples and the beads are dispersed at least 30% more uniformly across the at least 100 microwells compared to loading without injecting air. In some embodiments, the one or more cell samples and the beads are dispersed at least 60% more uniformly across the at least 100 microwells compared to loading without injecting air. In some embodiments, the steps of injecting air do not remove the contents of the at least 100 microwells. In some embodiments, the steps of injecting air comprise injecting air at a rate between about 0.08 ml per second to about 1.8 ml per second. In some embodiments, the steps of injecting air comprise injecting air at a pressure between about 0.01 and about 0.25 atm. In some embodiments, the steps of injecting air comprise generating a uniform environment for the flow of liquid through the flow cell. In some embodiments, the method further comprises adjusting the viscosity of the buffer used for the cell sample or bead suspension to improve the uniformity of distribution of the cells or beads into the microwells. In some embodiments, the viscosity of the buffer is adjusted to between about 1.2 times that of water and about 10 times that of water. In some embodiments, the method further comprises adjusting the density of the buffer used for the one or more cell samples or bead suspension to improve the uniformity of distribution of the cells or beads into the microwells. In some embodiments, the density of the buffer used for injection of beads is adjusted to between about 0.8 times and about 0.99 times the density of the beads.

Disclosed herein are methods for retrieving beads from the microwells of a microwell array, the methods comprising: a) loading the microwell array with beads; b) exposing the microwell array to a magnetic field; c) flowing a buffer over the microwell array; and d) retrieving the beads from the microwell array using a magnetic field.

In some embodiments, the loading comprises loading the beads such that at least 90% of the microwells of the microwell array contain a single bead. In some embodiments, the loading comprises loading the beads such that at least 95% of the microwells of the microwell array contain a single bead. In some embodiments, the loading comprises loading the beads such that about 100% of the microwells of the microwell array contain a single bead. In some embodiments, the loading is substantially uniform across the microwell array. In some embodiments, each bead comprises a plurality of attached oligonucleotides. In some embodiments, the plurality of attached oligonucleotides for a give bead comprise a diverse set of molecular labels. In some embodiments, the plurality of attached oligonucleotides for a given bead comprises a cellular label that is the same for all oligonucleotides attached to the bead. In some embodiments, the pluralities of attached oligonucleotides for different beads comprise different cellular labels. In some embodiments, the plurality of attached oligonucleotides for each bead comprises a target binding region. In some embodiments, the plurality of attached oligonucleotides for each bead comprises a universal primer binding sequence. In some embodiments, the magnetic field is generated using a magnet. In some embodiments, the exposing step comprises moving the magnetic field across the microwell array at a rate of between 0.01 millimeter per second and 10 millimeters per second. In some embodiments, the exposing step comprises moving the magnetic field across the microwell array at a rate of about 0.5 millimeters per second. In some embodiments, the exposing step comprises exposing the microwell array to a magnetic field having field lines that form a non-perpendicular angle relative to the plane of the substrate. In some embodiments, the field lines form an angle of between 45 degrees and 80 degrees relative to the plane of the substrate. In some embodiments, the exposing step comprises drawing the beads into the wells of the microwell array. In some embodiments, the buffer comprises a lysis buffer. In some embodiments, the buffer comprises a wash buffer. In some embodiments, the flowing step does not substantially remove the beads from the microwells of the microwell array. In some embodiments, the flowing step moves beads into the microwells of the microwell array. In some embodiments, the retrieving step comprises retrieving the beads with a magnet. In some embodiments, the retrieving step retrieves at least 85% of the beads from the microwell array. In some embodiments, the retrieving step retrieves at least 95% of the beads from the microwell array. In some embodiments, the method further comprises adjusting the viscosity of the buffer to improve the efficiency of bead retrieval. In some embodiments, the viscosity of the buffer is adjusted to between about 1.2 times that of water and about 10 times that of water. In some embodiments, the method further comprises adjusting the density of the buffer to improve the efficiency of bead retrieval. In some embodiments, the density of the buffer is adjusted to between about 0.8 times and about 0.99 times the density of the beads.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIG. 1 illustrates the structure of the oligonucleotides attached to the beads, and the assay workflow for stochastic labeling and molecular indexing of target oligonucleotides from single cells.

FIGS. 2A-B illustrate the loading of cells and beads into microwell arrays. FIG. 2A shows micrographs of a microwell array after loading with a dilute cell suspension (upper) and after loading with a bead suspension (lower). FIG. 2B shows magnified views of two microwells that contain both a single cell and a single bead.

FIGS. 3A-C illustrate one embodiment of the disclosed methods, devices, and systems for performing massively-parallel, stochastic barcoding of the RNA content of thousands of single cells. FIG. 3A depicts single cells are trapped in microwells along with beads comprising libraries of tethered stochastic labels (one cell and one bead per well). FIG. 3B depicts principal component analysis of stochastic barcoding data for human peripheral blood mononuclear cells. FIG. 3C depicts principal component analysis of stochastic barcoding data for a rare cell population.

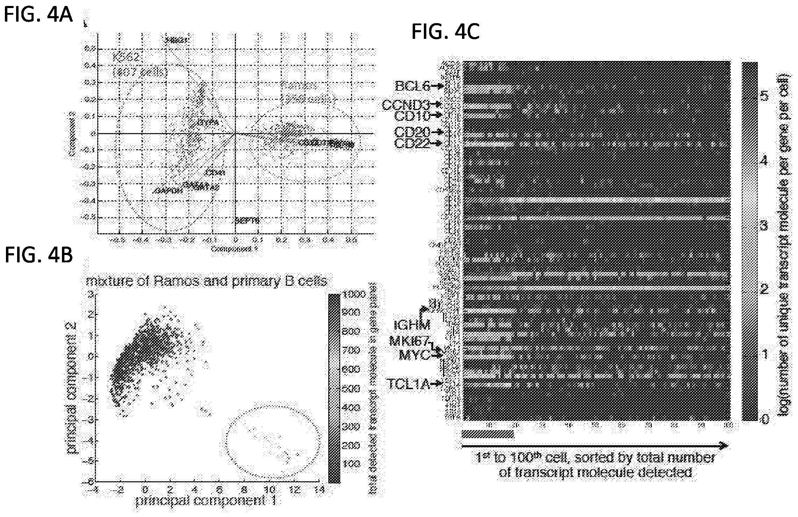

FIGS. 4A-C illustrate clustering of data for single cells in controlled mixtures containing two distinct cell types. FIG. 4A shows clustering of data for a mixture of K562 and Ramos cells by principal component analysis of the expression data for 12 genes. The biplot shows two distinct clusters, with one cluster expressing Ramos specific genes (CD74, CD79a, IGJ, TCL1A, SEPT9, CD27) and the other expressing K562 specific genes (CD41, GYPA, GATA1, GATA2, HBG1) separated by the first principal component (horizontal). The second principal component (vertical) indicates the variability in fetal hemoglobin (HBG1) within the K562 cells. FIG. 4B shows a principal component analysis of a mixture containing a small percentage of Ramos cells in a background of primary B cells from a healthy individual using a panel of 111 genes. The color of each data point indicates the total number of unique transcript molecules detected across the entire gene panel. A set of 18 cells (circled) out of 1,198 cells displays a distinct gene expression profile with much higher transcription levels. FIG. 4C is a heatmap showing expression levels for each gene in the top 100 cells in the sample of FIG. 4B, ranked by the total number of transcript molecules detected in the gene panel. The top 18 cells, indicated by the horizontal red bar, expressed preferentially a set of genes known to be associated with follicular lymphoma, as indicated by arrows.

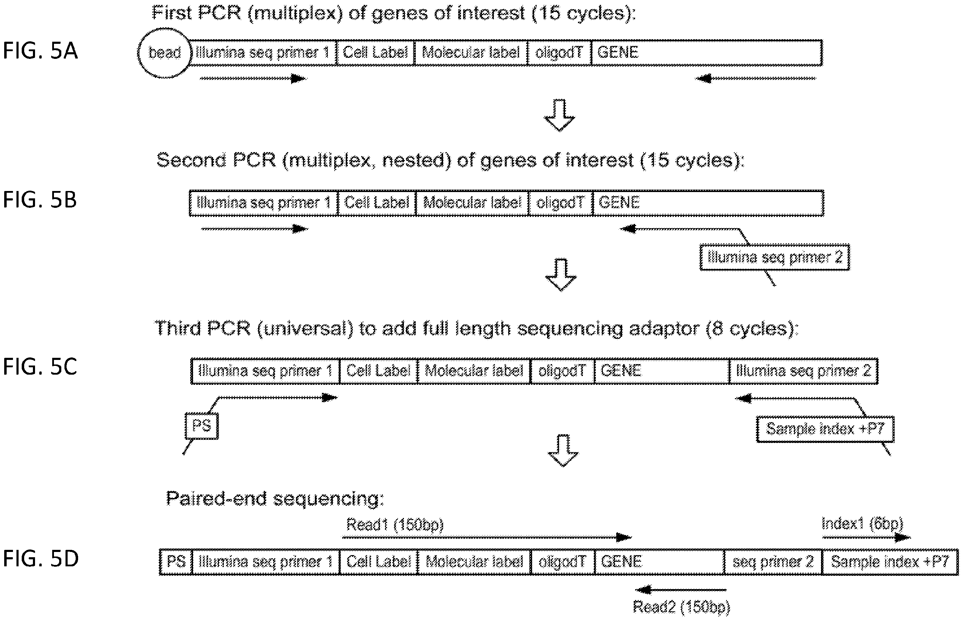

FIGS. 5A-D illustrate one embodiment of an amplification and sequencing scheme for use in methods of the present disclosure. FIG. 5A illustrates multiplexed PCR amplification of molecular barcodes associated with specific genes of interest. FIG 5B illustrates multiplexed, nested PCR amplification of the previous amplification product that introduces a second sequencing primer into the amplified, barcoded molecules. FIG. 5C illustrates a third PCR amplification reaction that is used to add full length sequencing adaptor sequences as well as an optional sample index. FIG. 5D illustrates paired end sequencing of the amplified molecular barcode - gene sequence conjugates.

FIG. 6 provides a schematic illustration of a microwell containing a single bead and a single cell.

FIGS. 7A-B show micrographs of a microwell array and the modified micropillars used to fabricate the microwell array using a micromolding process. FIG. 7A shows a micrograph of a microwell array having domed ridges between adjacent microwells. FIG. 7B shows a micrograph of the side-view of modified micropillars having curved surfaces that may be used to create domed ridges between micromolded wells.

FIGS. 8A-B show micrographs of microwell arrays molded with a micropillar master before and after subjecting the micropillar array to a reflow process. FIG. 8A shows a microwell array having flat substrate surfaces between wells, as fabricated using a micropillar master before subjecting it to a reflow process. FIG. 8B shows a microwell array having domed ridges between wells, as fabricated with a micropillar master that had been subjected to a reflow process.

FIGS. 9A-B illustrate a mechanical fixture within which microwell array substrates may be clamped, thereby forming a reaction chamber or well into which samples and reagents may be pipetted for performing multiplexed, single cell stochastic labeling or molecular barcoding experiments. FIG. 9A: exploded view showing the upper and lower parts of the fixture and an elastomeric gasket for forming a leak-proof seal with the microwell array substrate. FIG. 9B: exploded side-view of the fixture.

FIG. 10 illustrates a mechanical fixture which creates two reaction chambers or wells when a microwell array substrate is clamped within the fixture.

FIG. 11 illustrates a hinged mechanical fixture for facilitating single cell stochastic labeling and molecular barcoding assays when performed manually. The substrate comprising the microwell array is clamped between an elastomeric gasket and the fixture to create a reaction well into which cell samples, bead samples, and other assay reagents may be pipetted.

FIG. 12 illustrates a flow cell which incorporated a microwell array and an optically transparent window for use in imaging all or a portion of the microwell array. FIG. 12 inset: micrographs of microwell arrays in which a subset of microwells contain cells and/or beads.

FIG. 13A illustrates examples of different fluidic layer designs for use in constructing a flow cell.

FIGS. 13B-C provide schematic illustrations of a flow cell design that incorporates tapered, slanted inlet and outlet fluid channels in fluid communication with a chamber that comprises a microwell array. FIG. 13B: top view. FIG. 13C: cross-sectional side view.

FIG. 14A depicts an exploded view of a cartridge assembly that includes a flow cell comprising an interface layer, a fluidic layer, and a substrate.

FIG. 14B depicts a partially-exploded view of a cartridge assembly in which the layers forming the flow cell (*e.g.* an interface layer, a fluidic layer, and a substrate) have been bonded.

FIG. 15 shows an exemplary cartridge design.

FIG. 16 shows an exploded view of an exemplary cartridge design.

FIG. 17 depicts an exploded view of an exemplary flow cell design.

FIG. 18 depicts an exemplary flowcell design.

FIG. 19A illustrates a 3D printed flow cell part that incorporates a sealing ring as a design feature.

FIG. 19B illustrates a flow cell part fabricated from a polymer material.

FIG. 20A illustrates one approach to cartridge assembly in which multiple parts are mechanically clamped together.

FIG. 20B illustrates one approach to cartridge assembly that comprises both bonded and/or consumable parts and additional parts that are mechanically clamped together to form the complete assembly.

FIG. 20C illustrates a pre-assembled, consumable cartridge.

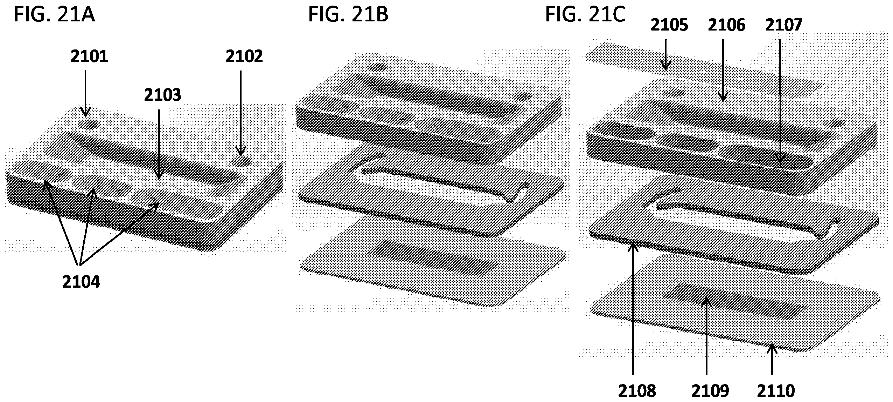

FIGS. 21A-C depict one embodiment of a cartridge within which a microwell array is packaged. FIG. 21A: A view of the assembled cartridge showing inlet and outlet ports, a relief for bringing a retrieval magnet into close proximity with the microwell array, and onboard reagent reservoirs. FIG. 21B: A partially exploded view of the cartridge assembly. FIG. 21C: An exploded view of the cartridge illustrating (from bottom to top) the microwell array substrate, an injection molded plastic bonded to the substrate and cartridge body that defines the flow cell or array chamber, a cartridge body that defines sample, reagent, or waste reservoirs that may contain pre-loaded assay reagents or store spent reagents, and a cover for sealing the reagent and waste reservoirs.

FIGS. 22A-B depict another embodiment of a cartridge within which a microwell array is packaged. FIG. 22A: A view of the assembled cartridge. FIG. 22B: An exploded view of the cartridge assembly illustrating (from bottom to top) the microwell array substrate, a fluidic layer (e.g., part of the flow cell) that defines the flow cell or array chamber, and a cartridge body that defines sample, reagent, or waste reservoirs. In this embodiment, the reservoirs are not visible externally.

FIG. 23A illustrates components of a flow cell or cartridge assembly comprising 4 fluid ports which may optionally incorporate inlet and outlet fluid port connectors, e.g. threaded connectors, Luer lock connectors, Luer slip or "slip tip" connectors, press fit connectors, and the like.

FIG. 23B illustrates components of a flow cell or cartridge assembly comprising fluid ports designed to accommodate conventional micropipettes for convenient introduction of fluids.

FIG. 24A illustrates components of a flow cell or cartridge assembly comprising fluid ports designed to accommodate conventional micropipettes for convenient introduction of fluids.

FIG. 24B illustrates a flow cell or cartridge assembly in which the angled inlet(s) designed to accommodate conventional micropipettes may incorporate rigid or compliant features to form a substantially leak-proof seal with the micropipette tip.

FIG. 24C illustrates a flow cell or cartridge assembly in which the angled inlet(s) designed to accommodate conventional micropipettes may incorporate features such as O-rings to form a substantially leak-proof seal with the micropipette tip.

FIG. 25A illustrates a cartridge design that comprises a modular pipette tip interface which can be modified independently from the rest of the cartridge design.

FIG. 25B illustrates a modular pipette tip interface for incorporation into cartridge designs.

FIG. 26 illustrates a cartridge assembly that incorporates a syringe connector and check valve(s), as well as a pipette tip interface and a sample collection tube that stores beads after their retrieval from the microwell array. There can be two inlets and one outlet. The cartridge incorporates external valves connected to a tube-in inlet and a tube-out outlet, and a pipette interface on the second inlet.

FIG. 27 illustrates a variation of the cartridge design shown in FIG. 26.

FIG. 28 illustrates a cartridge design that incorporates Upchurch Nanoport™ connectors (Upchurch Scientific, Division of IDEX Health and Science, Oak Harbor, WA) to create low dead volume connections between external tubing and the cartridge. The cartridge design also incorporates syringe connectors, check valves, and optional pipette tip interfaces.

FIG. 29A depicts an exploded view of a cartridge design that incorporates fluid ports (with pipette interface), fluid channels, fluid reservoirs, and check valves adjacent to all or some of the fluid ports or fluid reservoirs. The view shows an exploded view of the cartridge and depicts four cylinders (red) that represent valves, an interface layer (top, green), a fluidic layer (middle, blue) that forms the fluidic path of the flow-cell, and the substrate layer (bottom, gray) that incorporates the micro-wells array and which also is the base of the cartridge.

FIG. 29B depicts an assembled view of a cartridge design that incorporates fluid ports (with pipette interface), fluid channels, fluid reservoirs, a pipette interface on the input and the outlet, a reservoir on the inlet and the outlet, and a valve that gates each inlet and outlet and check valves adjacent to all or some of the fluid ports or fluid reservoirs.

FIG. 30 provides a schematic illustration of an instrument system for performing multiplexed, single cell stochastic labeling or molecular barcoding assay. The instrument system may provide a variety of control and analysis capabilities, and may be packaged as individual modules or as a fully integrated system. Microwell arrays may be integrated with flow cells that are either a fixed component of the system or are removable, or may be packaged within removable cartridges that further comprise pre-loaded assay reagent reservoirs and other functionality.

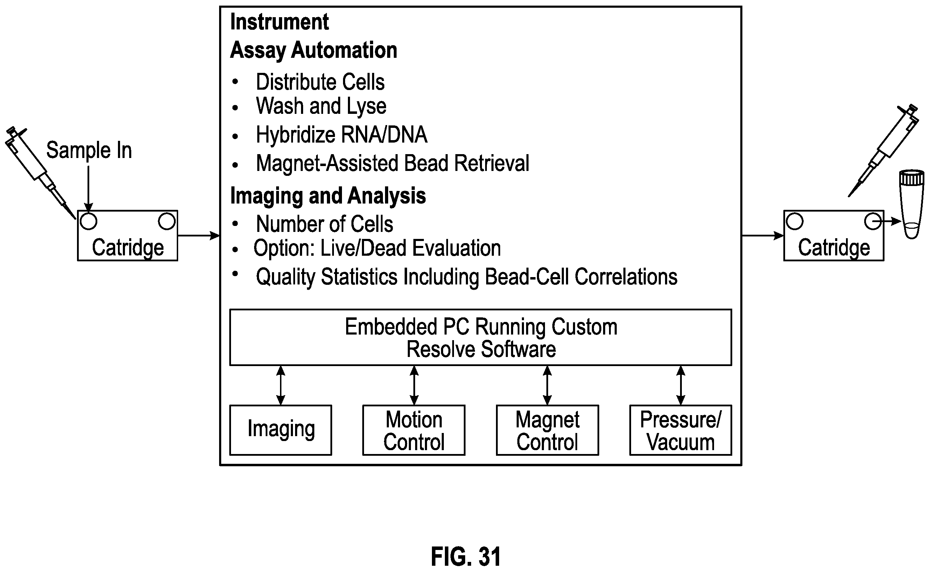

FIG. 31 provides a block diagram for an instrument system that performs multiplexed, single cell stochastic labeling or molecular barcoding assay. The instrument system may provide a variety of control and analysis capabilities, and may be packaged as individual modules or as a fully integrated system. The control computer may be embedded as shown, or operatively connected via a cable or wireless interface. Microwell arrays may be integrated with flow cells that are either a fixed component of the system or are removable, or may be packaged within removable, consumable cartridges that further comprise pre-loaded assay reagent reservoirs and other functionality.

FIG. 32 illustrates one embodiment of a process flow diagram for a cartridge and instrument system where cell samples, bead suspensions, and other assay reagents are loaded into a consumable cartridge by the user and inserted into the instrument. All process steps not listed under the "User" heading are performed by the instrument in a semi-automated or fully-automated fashion.

FIG. 33 illustrates another embodiment of a process flow diagram for a cartridge and instrument system where cell samples and bead suspensions are loaded into a consumable cartridge by the user and inserted into the instrument. Other assay reagents are pre-loaded in the cartridge and/or stored in the instrument. All process steps not listed under the "User" heading are performed by the instrument in a semi-automated or fully-automated fashion.

FIG. 34 illustrates another embodiment of a process flow diagram for a cartridge and instrument system where bead suspensions and other assay reagents are pre-loaded into a consumable cartridge and/or stored in the instrument. Cell samples are loaded into the cartridge by the user and inserted into the instrument. All process steps not listed under the "User" heading are performed by the instrument in a semi-automated or automated fashion.

FIG. 35 illustrates yet another embodiment of a process flow diagram for a cartridge and instrument system where beads and other assay reagents are pre-loaded into a consumable cartridge, and where the beads are pre-distributed amongst the microwells of a microwell array contained within the cartridge. Cell samples are loaded into the cartridge by the user and inserted into the instrument. All process steps not listed under the "User" heading are performed by the instrument in a semi-automated or automated fashion.

FIG. 36 illustrates one embodiment of a computer system or processor for providing instrument control and data analysis capabilities for the assay system presently disclosed.

FIG. 37 shows a block diagram illustrating one example of a computer system architecture that can be used in connection with example embodiments of the assay systems of the present disclosure.

FIG. 38 depicts a diagram showing a network with a plurality of computer systems, cell phones, personal data assistants, and Network Attached Storage (NAS), that can be used with example embodiments of the assay systems of the present disclosure.

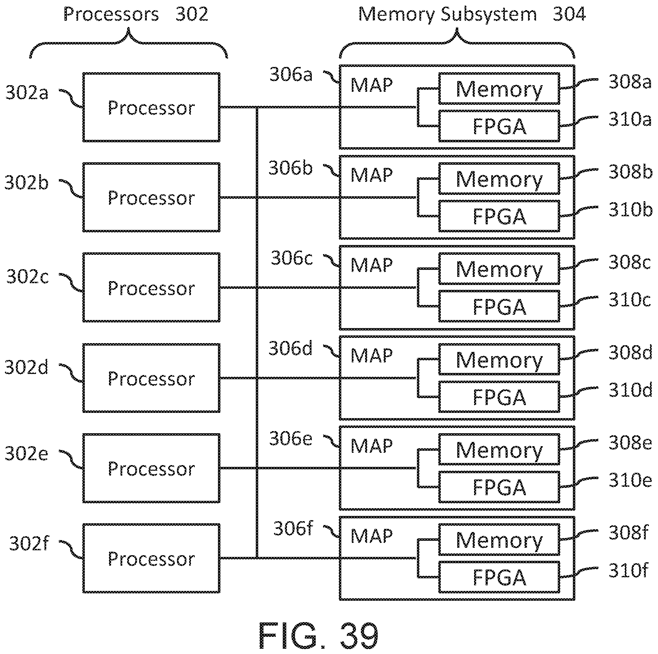

FIG. 39 depicts a block diagram of a multiprocessor computer system that can be used with example embodiments of the assay systems of the present disclosure.

FIG. 40 illustrates one embodiment of an optical design for an illumination system for use in the disclosed systems for performing multiplexed, single cell stochastic labeling and molecular barcoding assays.

FIG. 41 shows data from a cell settling study in which the percentage of wells in a microwell array containing Ramos cells was determined using automated image analysis software as a function of time following the filling of a flow cell such as that illustrated in FIG. 12 with the cell suspension.

FIG. 42 shows data from cell distribution studies in which the number of microwells containing a specified number of cells is plotted versus the number of cells per well for data collected at different times following the filling of a flow cell such as that illustrated in FIG. 12 with a Ramos cell suspension.

FIG. 43 shows a comparison of cell distribution data with the predictions of Poisson statistics. The observed number of microwells containing a specified number of cells is plotted versus the number of cells per well, along with the value calculated from the Poisson distribution.

FIG. 44 shows data from a bead settling study in which the percentage of wells in a microwell array containing beads was determined using automated image analysis software as a function of time following the filling of a flow cell such as that illustrated in FIG. 12 with the bead suspension.

FIGS. 45A-C illustrate the workflow for image processing and automated cell counting.

FIG. 46 shows an example of the output results for the image processing and automated cell counting algorithms disclosed herein.

FIGS. 47A shows a bright-filed image of a microwell array containing cells.

FIG. 47B shows a fluorescence image corresponding to the field of view shown in FIG. 47A, where the cells have been pre-loaded with calcein.

FIG. 47C shows an overlay of the images shown in FIGS. 47A and 47B.

FIG. 48 illustrates the steps used for microwell detection, bead identification, and cell identification in the image processing and automated cell counting algorithms described herein.

FIG. 49A shows a bright-field image of a microwell array where some of the microwells contain beads or cells.

FIG. 49B illustrates the result of performing image processing using edge detection and the Hough circle transformation to identify the microwells in the bright-field image shown in FIG. 49A.

FIG. 49C illustrates a binary mask created using the processed image of FIG. 49B, in which areas outside of the wells are set to "black" (*i.e.* a value of "0") and areas inside of the wells are set to "white" (*i.e.* a value of "1").

FIGS. 50A-C illustrates use of a binary mask to eliminate some parts of the image and focus subsequent image processing and analysis on the regions inside of the wells to identify beads and cells.

FIGS. 51A-C shows an example of the output results obtained using the image processing steps illustrated in FIGS. 49A-C and 50A-C.

FIGS. 52A-C show micrographs of a microwell array with a draft angle on the sidewall fabricated from Norland Optical Adhesive 63 (NOA63). FIG. 52A: top view. FIG. 52B: bottom view. FIG. 52C: cross-sectional view. The well-top diameter is larger than the well-bottom diameter.

FIGS. 53A-C show micrographs of a microwell array with a negative draft angle fabricated from cyclic olefin copolymer (COC). FIG. 53A: top view. FIG. 53B: bottom view. FIG. 53C: cross-sectional view. The well-top diameter is smaller the well-bottom diameter.

FIG. 54 shows examples of data for bead capture and retrieval efficiency for microwells of different diameter (50 $\mu$m depth, 60 $\mu$m pitch) fabricated from COC. The wells shown have a positive draft angle.

FIGS. 55A-D show examples of data for bead capture efficiency (FIGS. 55A and 55C) and bead doublet percentage (FIGS. 55B and 55D) for microwells of different diameter fabricated from NOA63 or COC (well depth = 40 $\mu$m for the optical adhesive wells, and 50 $\mu$m for the COC wells).

FIG. 56 shows examples of data for bead capture and retrieval from microwells of different diameter fabricated from NOA63 or COC (well depth = 40 $\mu$m for the optical adhesive wells, and 50 $\mu$m for the COC wells).

FIG. 57 shows micrographs of COC microwells from sectioned PDMS pre-treated with poly-HEMA.

FIGS. 58A illustrates a flow cell that encloses the microwell array and includes a tapered inlet and outlet.

FIG. 58B illustrates the steps of the loading and retrieval process: (i) loading, (ii) magnetic field-enhanced entrapment, (iii) wash, and (iv) magnetic field-based retrieval.

FIGS. 59A-D shows micrographs of a microwell array after each of the steps outlined in FIG. 58B. The percentage of wells containing a bead are indicated below each image.

FIG. 60 shows examples of data generated using automated image processing and analysis as described to quantify the number of microwells containing beads after each process step as a function of position on the substrate.

FIG. 61 shows an example of data for studies of bead loading and retrieval after different process steps, including the lysis step, as a function of position on the microwell substrate.

FIGS. 62A-B illustrate two non-limiting examples of flow cell design. FIG. 62A illustrates a single inlet - single outlet flow cell design. FIG. 62B illustrates a branched inlet flow cell design.

FIG. 63 shows a flow cell image overlaid with bead fill efficiency data for the single inlet-single outlet flow cell design.

FIG. 64 shows a flow cell image overlaid with bead fill efficiency data for the branched inlet flow cell design.

FIG. 65 shows a flow cell image overlaid with bead fill efficiency data for the single inlet-single outlet flow cell design, where air injections were used between liquid fill steps.

FIG. 66 summarizes data from bead fill efficiency studies.

FIG. 67 provides a high level overview of the assay workflow for single cell, stochastic labeling.

FIG. 68 summarizes data from a proof-of-concept study to demonstrate cell loading, bead loading, and bead retrieval from a microwell array enclosed in a flow cell.

FIG. 69 summarizes data from a proof-of-concept study to demonstrate cell loading, bead loading, and bead retrieval from a microwell array enclosed in a flow cell.

FIG. 70A shows examples of bead loading data as a function of position along the flow cell for a 1 mm thick flow cell at different steps in the assay procedure.

FIG. 70B shows examples of bead loading data as a function of position along the flow cell for a 2 mm thick flow cell at different steps in the assay procedure.

FIG. 71A shows examples of data for the percentage of beads lost during the lysis step or retrieved at the end of the process as a function of position along a flow cell of 1 mm depth.

FIG. 71B shows examples of data for the percentage of beads lost during the lysis step or retrieved at the end of the process as a function of position along a flow cell of 1 mm depth.

FIGS. 72A-F show examples of data for cell loading as a function of position along the flow cell at different steps in the assay procedure.

FIG. 73 shows data for the percentage of wells containing beads as a function of position along the length of the flow cell at various stages of the assay process.

FIG. 74 shows examples of data for the percentage of wells containing beads (averaged over the entire set of microwells) after each process step.

FIGS. 75A-C show data for cell loading after the initial cell loading and settling step (FIG. 75A), after the initial bead loading an settling step (FIG. 75B), and after the beads are subsequently pulled down with the magnet (FIG. 75C).

FIG. 76 provides a summary of cell loading and bead loading data at different steps in the process.

FIGS. 77A-B show examples of data comparing bead loading at different steps in the assay procedure when using lysis buffer with and without DTT added.

FIGS. 78A-B show examples of data comparing the impact of magnetic field-assisted bead distribution on bead loading at different steps in the assay procedure when the lysis buffer includes DTT.

FIG. 79A shows examples of data for the percentage of wells containing beads after the bead loading step and after bead retrieval.

FIG. 79B provides a table of analysis statistics for cell loading and bead loading in an individual experiment.

FIG. 80A shows a heat map for the bead fill rate (% of wells with beads) as a function of position within the flow cell after the bead loading step.

FIG. 80B shows a heat map for the bead fill rate (% of wells with beads) as a function of position within the flow cell after the bead retrieval step.

FIG. 80C shows a heat map of the bead retrieval percentage (1 - beads remaining/beads loaded)*100%) as a function of position with the flow cell after the bead retrieval step.

FIG. 81 shows a micrograph of an exemplary microwell array with rounded surfaces between the wells.

FIGS. 82A-B show a comparison of the standard ("cells first") and alternate ("beads first") assay workflows.

FIGS. 83A-B show several variations of standard and alternate assay workflows that have been evaluated.

FIGS. 84A-B show a comparison of bead loading efficiency for each step in the "beads first" and "cells first" assay workflows, respectively.

FIG. 85 shows a composite image of the flow cell comprising an microwell array. The rectangle in the lower right-hand corner of the composite image shows a region of increased flow impedance in an outlet branch of the flow-cell.

FIGS. 86A-C show heat maps of single bead loading efficiency as a function of position within the flow cell for different steps of the "beads first" assay workflow.

FIGS. 87A-F show a comparison of the cell capture efficiency for the "beads first" (FIGS. 87A-C) and "cells first" (FIGS. 87D-F) assay workflows.

FIGS. 88A-B show plots of the frequency of an important assay performance metric, *i.e.* the percentage of cells associated with a single bead within the microwell array.

FIGS. 89A-D show examples of data for an important assay performance metric, *i.e.* the percentage of micro wells containing a single bead prior to the cell lysis step.

FIGS. 90A-B show plots of bead loading efficiency versus the number of cells in the microwell for the "bead first" (FIG. 90A) and "cells first" (FIG. 90B) assay workflows, respectively.

FIG. 91 illustrates a design for a pipette tip interface that utilizes a friction fit seal between a pipette tip and a cartridge as well as a duckbill valve on the cartridge outlet.

FIG. 92 shows a cross-sectional side view of a pipette tip interface that utilizes a friction fit seal between a pipette tip and a cartridge as well as a duckbill valve on the cartridge outlet.

FIG. 93 shows an alternate pipette tip interface having a molded compliant gasket and an x-fragm dispense valve.

FIG. 94 shows a summary comparison of two pipette tip interface designs.

FIGS. 95A-B illustrate the steps of the standard "beads first" assay workflow (FIG. 95A) and the modified, Ficoll-based assay workflow (FIG. 95B).

FIGS 96A-B show examples of data illustrating the improved bead loading efficiency achieved using Ficoll-based bead washing steps rather than an air displacement technique with $SiO_2$-coated microwell substrates.

FIGS. 97A-F show examples of data for cell capture efficiency achieved using a Ficoll-based assay workflow.

FIG. 98 summarizes the estimated density and viscosity values of magnetic beads, cells, and solutions used in a Ficoll-based assay workflow.

## DETAILED DESCRIPTION

Disclosed herein are methods, compositions, devices, systems, and kits for molecular barcoding of a plurality of target molecules in single cells.

*Overview of stochastic labeling for molecular barcoding and counting:*

The methodology underlying the methods, devices, and systems of the present disclosure utilizes a recursive Poisson strategy to implement single cell, molecular barcoding assays for large numbers of individual cells. For example, molecular targets from individual cells can be stochastically labeled with a cellular label (also referred to as a cellular index, barcode, or tag) and a molecular label (also referred to as a molecular index, barcode, or tag) by randomly associating individual cells with individual beads, wherein each individual bead comprises a plurality of attached stochastic labels. The stochastic labels attached to a given bead can be used to randomly label protein or nucleic acid targets from an associated cell. In some embodiments, single cells are randomly distributed into a plurality of microwells (e.g. a microwell array). A combinatorial library of beads, each comprising a plurality of tethered stochastic labels, is also randomly distributed into the plurality of microwells so that a subset of the microwells contains both a single cell and a single bead. In some embodiments the beads are deposited prior to depositing the cells. In other embodiments the beads are deposited after depositing the cells. The stochastic labels comprising the cellular and molecular barcodes may further comprise a target recognition region that is capable of attaching to or hybridizing with molecular targets, for example, nucleic acid molecules. The target molecules can be released from each cell, for example by lysing the cell, and then attached to or hybridized with the stochastic labels on a corresponding bead. In some embodiments the target molecules are released from the cells by cleavage, e.g. enzymatic cleavage. In some embodiments, e.g., when the target molecules are mRNA molecules, the beads are retrieved from the microwells following hybridization of the mRNA target molecules to the stochastic labels, and pooled prior to performing reverse transcription, amplification, and sequencing reactions.

In some embodiments, the plurality of stochastic labels attached to a given bead comprises a cellular label that is identical for all of the stochastic labels attached to the bead, while the cellular labels for the pluralities of stochastic labels attached to different beads are different. In some embodiments, the plurality of stochastic labels attached to a given bead comprises a diverse set of molecular labels selected from a set comprising a specified number of unique molecular label sequences. In some embodiments, the plurality of stochastic labels attached to a given bead may comprise the same target recognition region. In some embodiments, the plurality of stochastic labels attached to a given bead may comprise two or more different target recognition regions.

In some embodiments, the bead library has a cellular label diversity (*i.e.* a number of unique cellular label sequences) that is at least one or two orders of magnitude higher than the number of cells to be labeled, such that the probability that each cell is paired with a unique cell barcode is very high. For example, the probability that each cell is paired with a unique cell barcode may be greater than 80%, greater than 90%, greater than 95%, greater than 99%, greater than 99.9%, greater than 99.99%, or greater than 99.999%.

In some embodiments, the molecular label diversity (*i.e.* the number of unique molecular label sequences) for the plurality of stochastic labels attached to a bead is at least one or two orders of magnitude higher than the estimated number of occurrences of a target molecule species to be labeled, such that the probability that each occurrence of a target molecule (*e.g.* an mRNA molecule) within a cell becomes uniquely labeled is also very high. For example, the probability that each occurrence of a target molecule is paired with a unique molecular barcode may be greater than 80%, greater than 90%, greater than 95%, greater than 99%, greater than 99.9%, greater than 99.99%, or greater than 99.999%. In these embodiments, the number of occurrences of a target molecule species in each cell may be counted (or estimated) by determining the number of unique molecular label sequences that are attached to the target molecule sequence. In many embodiments, the determining step may be performed through sequencing of an amplified library of labeled target molecules (or their complementary sequences).

In some embodiments, the molecular label diversity for the plurality of stochastic labels attached to a bead is comparable to or low compared to the estimated number of occurrences of a target molecule species to be labeled, such that there is a significant probability that the multiple occurrences of a given type of target molecule will be labeled by more than one copy of a given molecular label. In these embodiments, the number of target molecules in each cell may be calculated from the number of unique molecular label sequences attached to the target molecule sequence with the use of Poisson statistics.

In many embodiments, the target molecules of interest are mRNA molecules expressed within a single cell. Since cDNA copies of all or a portion of the polyadenylated mRNA molecules in each cell are covalently archived on the surface of a corresponding bead, any selection of gene transcripts may be subsequently analyzed. A digital gene expression profile for each cell may be reconstructed when the barcoded transcripts are sequenced and assigned to the cell of origin (based on the cellular label identified) and counted (based on the number of unique molecular labels identified). An exemplary description of the assay methodology can be found in Fan, et al., "Combinatorial Labeling of Single Cells for Gene Expression Cytometry", Science 347(6222):628; and Science 347(6222):1258367.

*Assay targets, samples, components, & methods:*

*Target molecules:* Suitable target molecules for analysis by the disclosed methods, devices, and systems include oligonucleotide molecules, DNA molecules, RNA molecules, mRNA molecules, microRNA molecules, tRNA molecules, and the like. In some embodiments target molecules can be peptides or proteins. The target molecules can be antibody heavy and light polypeptide chains, and/or receptor polypeptide chains (*e.g.,* the alpha and beta chains of the T cell receptor).

*Cell samples:* Suitable samples for analysis by the disclosed methods, devices, and systems include any sample comprising a plurality of cells, for example, cell cultures, blood samples, tissue samples in which the extracellular matrix has been digested or dissolved to release individual cells into suspension, and the like. The plurality of cells may be derived from a single sample, or from two or more samples that have been combined, and may comprise a plurality of cells of the same type, or a plurality of cells of mixed type.

In some embodiments, either cells, sub-cellular structures, or other nucleic acid containing particles may comprise suitable samples. For example, in some embodiments, the samples may comprise cellular organelles (*e.g.* mitochondria, nuclei, exosomes, *etc.*), liposomes, cell clusters, or multicellular organisms, and the like.

In some embodiments, the cells are normal cells, for example, human cells in different stages of development, or human cells from different organs or tissue types (e.g. white blood cells, red blood cells, platelets, epithelial cells, endothelial cells, neurons, glial cells, fibroblasts, skeletal muscle cells, smooth muscle cells, gametes, or cells from the heart, lungs, brain, liver, kidney, spleen, pancreas, thymus, bladder, stomach, colon, small intestine). In some embodiments, the cells may be undifferentiated human stem cells, or human stem cells that have been induced to differentiate. In some embodiments, the cells may be fetal human cells. The fetal human cells may be obtained from a mother pregnant with the fetus.

In some embodiments, the cells are rare cells. A rare cell may be, for example, a circulating tumor cell (CTC), circulating epithelial cell, circulating endothelial cell, circulating endometrial cell, circulating stem cell, stem cell, undifferentiated stem cell, cancer stem cell, bone marrow cell, progenitor cell, foam cell, mesenchymal cell, trophoblast, immune system cell (host or graft), cellular fragment, cellular organelle (e.g. mitochondria or nuclei), pathogen infected cell, and the like.

Circulating tumor cells can be cancer cells. CTCs can be CD45-. CTCs may express cytokeratins such as 8, 18, and/or 19, or be cytokeratin negative. CTCs can be cancer stem cells and/or cells undergoing epithelial to mesenchymal transition (EMT). A CTC can be a metastatic cell.

In some embodiments, the sample comprises an immune cell. An immune cell can include, for example, T cell, B cell, lymphoid stem cell, myeloid progenitor cell, lymphocyte, granulocyte, B-cell progenitor, T cell progenitor, Natural Killer cell, Tc cell, The cell, plasma cell, memory cell, neutrophil, eosinophil, basophil, mast cell, monocyte, dendritic cell and/or macrophage, or any combination thereof.

A cell can be a T cell. A T cell can be a T cell clone, which can refer to T cells derived from a single T cell or those having identical TCRs. A T cell can be part of a T cell line which can include T cell clones and mixed populations of T cells with different TCRs all of which may recognize the same target *(e.g.,* an antigen, a tumor, or a virus). T cells can be obtained from a number of sources, including peripheral blood mononuclear cells, bone marrow, lymph node tissue, spleen tissue, and tumors. T cells can be obtained from a unit of blood collected from a subject, such as an individual or patient, using Ficoll separation techniques. Cells from the circulating blood of an individual can be obtained by apheresis or leukapheresis. The apheresis product can comprise lymphocytes, including T cells, monocytes, granulocytes, B cells, other nucleated white blood cells, red blood cells, and platelets. The cells can be washed and resuspended in media to isolate the cell of interest.

T cells can be isolated from peripheral blood lymphocytes by lysing the red blood cells and depleting the monocytes, for example, by centrifugation through a PERCOLL™ gradient. A specific subpopulation of T cells, such as CD28+, CD4+, CDC, CD45RA+, and CD45RO+ T cells, can be further isolated by positive or negative selection techniques. For example, T cells can be isolated by incubation with anti-CD3/anti-CD28 (*i.e.*, 3×28)-conjugated beads, such as DYNABEADS® M-450 CD3/CD28 T, or XCYTE DYNABEADS™ for a time period sufficient for positive selection of the desired T cells. Immune cells *(e.g.,* T cells and B cells) can be antigen specific (*e.g.*, specific for a tumor.

In some embodiments, the cell can be an antigen-presenting cell (APC), such as a B cell, an activated B cell from a lymph node, a lymphoblastoid cell, a resting B-cell, or a neoplastic B cell, *e.g.* from a lymphoma. An APC can refer to a B-cell or a follicular dendritic cell expressing at least one of the BCRC proteins on its surface.

In some embodiments, the cells are non-human cells, for example, other types of mammalian cells (e.g. mouse, rat, pig, dog, cow, or horse). In some embodiments, the cells are other types of animal or plant cells. In other embodiments, the cells may be any prokaryotic or eukaryotic cells.

In some embodiments the cells are sorted prior to associating a cell with a bead. For example the cells can be sorted by fluorescence-activated cell sorting or magnetic-activated cell sorting, or more generally by flow cytometry. The cells may be filtered by size. In some instances a retentate contains the cells to be associated with the beads. In some instances the flow through contains the cells to be associated with the beads.

When loading cells, the concentration of the cell suspension (*i.e.* the number of cells per mL) is usually adjusted so that the probability of having more than one cell settle into a given micro well is very small. Typically, the concentration of the cell suspension will be adjusted so that the volume of cell suspension used to load, *e.g.* a microwell array, contains approximately one-tenth the number of cells as the number of wells in the microwell array. The probability that more than one cell settles into a given microwell is governed by Poisson statistics.

*Stochastic label chemical structure:* In some embodiments of the disclosed methods, the stochastic labels (also referred to as barcodes, tags, or indexes) used for single cell molecular barcoding studies comprise oligonucleotides, for example, oligodeoxyribonucleotides (DNA), oligoribonucleotides (RNA), peptide nucleic acid (PNA) polymers, 2'-O-methyl-substituted RNA, locked nucleic acid (LNA) polymers, bridged nucleic acid (BNA) polymers, and the like.

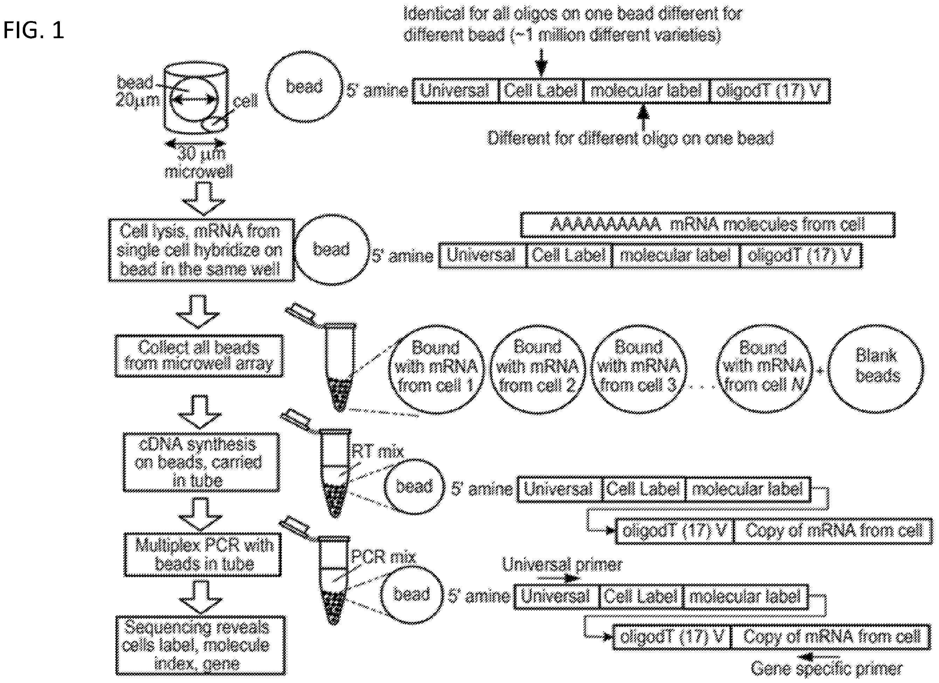

*Stochastic labels may be attached to solid supports:* In many embodiments, the stochastic labels used in the disclosed methods may be tethered to beads, for example to synthesis resin beads, or other solid supports as will be described in more detail below. As used herein, the phrases "tethered", "attached", and "immobilized" are used interchangeably, and may refer to covalent or non-covalent means for attaching stochastic labels to solid supports such as beads. One non-limiting example of stochastic label structure is illustrated schematically in FIG. 1. In this embodiment, the stochastic labels comprise a plurality of 5'-amine modified oligonucleotides attached to a bead. The oligonucleotides comprise a 5' amine group, a universal primer, a cellular label, a molecular label, and a target binding region. In some embodiments, the oligonucleotides may optionally further comprise one or more additional labels, *e.g.* a sample label for use in labeling all cells from a given sample when two or more samples are processed simultaneously. In some embodiments, the stochastic label oligonucleotide sequences may be attached to a solid support at their 5' end. In some embodiments, the stochastic label oligonucleotide sequences may be attached to a solid support at their 3' end.

*Stochastic labels may comprise one or more universal labels:* In some embodiments, the stochastic labels used in the disclosed methods, compositions, devices, kits, and systems may comprise one or more universal labels. In some embodiments, the one or more universal labels may be the same for all oligonucleotides in the set of oligonucleotides attached to a given bead. In some embodiments, the one or more universal labels may be the same for all oligonucleotides attached to a plurality of beads. In some embodiments, a universal label may comprise a nucleic acid sequence that is capable of hybridizing to a sequencing primer. Sequencing primers may be used for sequencing oligonucleotides comprising a universal label. Sequencing primers (*e.g.*, universal sequencing primers) may comprise sequencing primers associated with high-throughput sequencing platforms. In some embodiments, a universal label may comprise a nucleic acid sequence that is capable of hybridizing to a PCR primer. In some embodiments, the universal label may comprise a nucleic acid sequence that is capable of hybridizing to a sequencing primer and a PCR primer. The nucleic acid sequence of the universal label that is capable of hybridizing to a sequencing or PCR primer may be referred to as a primer binding site. A universal label may comprise a sequence that may be used to initiate transcription of the oligonucleotide. A universal label may comprise a sequence that may be used for extension of the oligonucleotide or a region within the oligonucleotide. A universal label may be at least about 1, 2, 3, 4, 5, 10, 15, 20, 25, 30, 35, 40, 45, 50 or more nucleotides in length. A universal label may comprise at least about 10 nucleotides. A universal label may be at most about 1, 2, 3, 4, 5, 10, 15, 20, 25, 30, 35, 40, 45, 50 or more nucleotides in length. In some embodiments, a cleavable linker or modified nucleotide may be part of the universal label sequence to enable stochastic label oligonucleotides to be cleaved off from the solid support.

*Stochastic labels may comprise a cellular label:* In some embodiments, stochastic labels may comprise a cellular label, *e.g.* a nucleic acid sequence that provides information for determining which target nucleic acid originated from which cell. In many embodiments, the cellular label is identical for all oligonucleotides attached to a given bead or solid support, but different for different beads or solid supports. In some embodiments, at least 60%, 70%, 80%, 85%, 90%, 95%, 97%, 99% or 100% of the oligonucleotides on the same solid support may comprise the same cellular label. In some embodiments, at least 60% of the oligonucleotides on the same solid support may comprise the same cellular label. In some embodiment, at least 95% of the oligonucleotides on the same solid support may comprise the same cellular label. In some embodiments, there may be as many as $10^3$ or more unique cellular label sequences represented in a plurality of beads or solid supports. In some embodiments, there may be as many as $10^4$ or more unique cellular label sequences represented in a plurality of beads or solid supports. In some embodiments, there may be as many as $10^5$ or more unique cellular label sequences represented in a plurality of beads or solid supports. In some embodiments, there may be as many as $10^6$ or more unique cellular label sequences represented in a plurality of beads or solid supports. A cellular label may be at least about 1, 2, 3, 4, 5, 10, 15, 20, 25, 30, 35, 40, 45, 50 or more nucleotides in length. A cellular label may be at most about 300, 200, 100, 90, 80, 70, 60, 50, 40, 30, 20, 15, 12, 10, 9, 8, 7, 6, 5, 4 or fewer nucleotides in length. A cellular label may comprise between about 5 to about 200 nucleotides. A cellular label may comprise between about 10 to about 150 nucleotides. A cellular label may comprise between about 20 to about 125 nucleotides in length.

*Cellular labels may comprise error correction codes:* In some embodiments, the cellular label may further comprise a unique set of nucleic acid sub-sequences of defined length, *e.g.* 7 nucleotides each (equivalent to the number of bits used in some Hamming error correction codes), which are designed to provide error correction capability. In some embodiments, the set of error correction sub-sequences comprise 7 nucleotide sequences designed such that any pairwise combination of sequences in the set exhibits a defined "genetic distance" (or number of mismatched bases), for example, a set of error correction sub-sequences may be designed to exhibit a genetic distance of 3 nucleotides. In this case, review of the error correction sequences in the set of sequence data for labeled target nucleic acid molecules (described more fully below) may allow one to detect or correct amplification or sequencing errors. In some embodiments, the length of the nucleic acid sub-sequences used for creating error correction codes may vary, for example, they may be 3 nucleotides, 7 nucleotides, 15 nucleotides, or 31 nucleotides in length. In some embodiments, nucleic acid sub-sequences of other lengths may be used for creating error correction codes.

*Cellular labels may comprise two or more subunits:* In some embodiments, the cellular label may comprise an assembly of two or more subunits (also referred to as subparts or components) that are assembled (or synthesized) in a combinatorial split-pool fashion to create a large number of unique cellular label sequences in a minimal number of assembly or synthesis steps. For example, three rounds of split-pool synthesis performed using a set of 6 unique cellular label subunits will yield $6^3$ = 216 unique cellular label sequences, where each cellular label comprises an assembly of three subunits. More generally, a cellular label sequence comprising M subunits that have been assembled in a combinatorial split-pool fashion using a set of N unique subunits at each step will yield $N^M$ unique combinations. In some embodiments, where the stochastic labels are oligonucleotides, the cellular subunit sequences may be assembled through the use of polymerase extension or ligation reactions. In some embodiments, one or more linker sequences may be used to facilitate the assembly of the cellular label sequence subunits.

*Stochastic labels may comprise a molecular label.* A molecular label may comprise a nucleic acid sequence that provides information for identifying the specific type of target nucleic acid species hybridized to the oligonucleotide. A molecular label may comprise a nucleic acid sequence that provides a counter for the specific occurrence of the target nucleic acid species hybridized to the oligonucleotide. In many embodiments, a diverse set of molecular labels are attached to a given bead. In some embodiments, there may be as many as $10^6$ or more unique molecular label sequences attached to a given bead. In some embodiments, there may be as many as $10^5$ or more unique molecular label sequences attached to a given bead. In some embodiments, there may be as many as $10^4$ or more unique molecular label sequences attached to a given bead. In some embodiments, there may be as many as $10^3$ or more unique molecular label sequences attached to a given bead. In some embodiments, there may be as many as $10^2$ or more unique molecular label sequences attached to a given bead. A molecular label may be at least about 1, 2, 3, 4, 5, 10, 15, 20, 25, 30, 35, 40, 45, 50 or more nucleotides in length. A molecular label may be at most about 300, 200, 100, 90, 80, 70, 60, 50, 40, 30, 20, 15, 12, 10, 9, 8, 7, 6, 5, 4 or fewer nucleotides in length.

*Stochastic labels may comprise a target binding region:* In some embodiments, the target binding regions may comprise a nucleic acid sequence that hybridizes specifically to a target nucleic acid (*e.g.*, a cellular nucleic acid to be analyzed), for example to a specific gene sequence. In some embodiments, a target binding region may comprise a nucleic acid sequence that may attach (*e.g.*, hybridize) to a specific location of a specific target nucleic acid. In some embodiments, the target binding region may comprise a nucleic acid sequence that is capable of specific hybridization to a restriction site overhang (e.g. an EcoRI sticky-end overhang). The stochastic label may then ligate to any nucleic acid molecule comprising a sequence complementary to the restriction site overhang. In some embodiments, a target binding region may comprise a non-specific target nucleic acid sequence. A non-specific target nucleic acid sequence may refer to a sequence that may bind to multiple target nucleic acids, independent of the specific sequence of the target nucleic acid. For example, target binding region may comprise a random multimer sequence, or an oligo-dT sequence that hybridizes to the poly-A tail on mRNA molecules (FIG. 1). A random multimer sequence can be, for example, a random dimer, trimer, quatramer, pentamer, hexamer, septamer, octamer, nonamer, decamer, or higher multimer sequence of any length. In some embodiments, a target binding region may be at least about 5, 10, 15, 20, 25, 30, 35, 40, 45, 50 or more nucleotides in length. In some embodiments, a target binding region may be at most about 5, 10, 15, 20, 25, 30, 35, 40, 45, 50 or more nucleotides in length. A target binding region may comprise any number of nucleotides within this range, for example, about a target binding region may be about 18 nucleotides in length.

In some embodiments, the target binding region is the same for all oligonucleotides attached to a given bead. In some embodiments, the target binding regions for the plurality of oligonucleotides attached to a given bead may comprise two or more different target binding sequences. For example, in some embodiments, the target binding regions for the plurality of oligonucleotides attached to a given bead may comprise a mixture of oligo-dT sequences and copies of a single target specific sequence. In some embodiments, the target binding regions for the plurality of oligonucleotides attached to a given bead may comprise a mixture of an oligo-dT sequence and copies of two different target specific sequences. In some embodiments, the target binding regions for the plurality of oligonucleotides attached to a given bead may comprise a mixture of an oligo-dT sequence and copies of three different target specific sequences. In general, the target binding regions for the plurality of oligonucleotides attached to a given bead may comprise a mixture of between one and one hundred, or more, different target binding sequences, including, but not limited to, target specific sequences, random multimer sequences, sequences capable of specific hybridization to a restriction site overhang, or oligo-dT sequences, in any combination of sequences and in any combination of relative proportions.

*Stochastic labels tethered to beads:* In some embodiments, the stochastic labels disclosed herein may be attached to solid supports such as beads. As used herein, the terms "tethered", "attached", and "immobilized" are used interchangeably, and may refer to covalent or non-covalent means for attaching stochastic labels to solid supports such as beads. In some embodiments, the stochastic labels may be immobilized within a small reaction volume, e.g. attached to a surface in a well or microwell, or to a different form of solid support rather than attached to a bead.

Pre-synthesized stochastic labels may be attached to beads or other solid supports through any of a variety of immobilization techniques involving functional group pairs on the solid support and the oligonucleotide. In some embodiments, the oligonucleotide functional group and the solid support functional group are individually selected from the group consisting of biotin, streptavidin, primary amine(s), carboxyl(s), hydroxyl(s), aldehyde(s), ketone(s), and any combination thereof. A stochastic label oligonucleotide may be tethered to a solid support, for example, by coupling (*e.g.* using 1-Ethyl-3-(3-dimethylaminopropyl) carbodiimide) a 5' amino group on the oligonucleotide to the carboxyl group of the functionalized solid support. Residual non-coupled oligonucleotides may be removed from the reaction mixture by performing multiple rinse steps. In some embodiments, the stochastic label oligonucleotides and solid support are attached indirectly via linker molecules (e.g. short, functionalized hydrocarbon molecules or polyethylene oxide molecules) using similar attachment chemistries. In some embodiments, the linkers may be cleavable linkers, e.g. acid-labile linkers or photo-cleavable linkers.

In some embodiment, stochastic labels are synthesized on solid supports such as synthesis resin beads using any of a number of solid-phase oligonucleotide synthesis techniques known to those of skill in the art. In some embodiments, single nucleotides may be coupled in step-wise fashion to the growing, tethered oligonucleotide. In some embodiments, a short, pre-synthesized sequence (or block) of several oligonucleotides may be coupled to the growing, tethered oligonucleotide. In some embodiments, oligonucleotides may be synthesized by interspersing step-wise or block coupling reactions with one or more rounds of split-pool synthesis, in which the total pool of synthesis beads is divided into a number of individual smaller pools which are then each subjected to a different coupling reaction, followed by recombination and mixing of the individual pools to randomize the growing oligonucleotide sequence across the total pool of beads. Split-pool synthesis is an example of a combinatorial synthesis process in which a maximum number of chemical compounds are synthesized using a minimum number of chemical coupling steps. The potential diversity of the compound library thus created is determined by the number of unique building blocks (e.g. nucleotides) available for each coupling step, and the number of coupling steps used to create the library. For example, a split-pool synthesis comprising 10 rounds of coupling using 4 different nucleotides at each step will yield $4^{10} = 1,048,576$ unique nucleotide sequences. In some embodiments, split-pool synthesis may be performed using enzymatic methods such as polymerase extension or ligation reactions rather than chemical coupling. For example, in each round of a split-pool polymerase extension reaction, the 3' ends of the stochastic label oligonucleotides tethered to beads in a given pool may be hybridized with the 5'ends of a set of semi-random primers, e.g. primers having a structure of 5'-$(M)_k$-$(X)_i$-$(N)_j$-3', where $(X)_i$ is a random sequence of nucleotides that is i nucleotides long (the set of primers comprising all possible combinations of $(X)_i$), $(N)_j$ is a specific nucleotide (or series of j nucleotides), and $(M)_k$ is a specific nucleotide (or series of k nucleotides), wherein a different deoxyribonucleotide triphosphate (dNTP) is added to each pool and incorporated into the tethered oligonucleotides by the polymerase.

In some embodiments, the number of oligonucleotides conjugated to or synthesized on a solid support such as a bead may comprise 100 or more oligonucleotide molecules. In some embodiments, the solid support may comprise 1,000 or more oligonucleotide molecules. In some embodiments, the solid support may comprise 10,000 or more oligonucleotide molecules. In some embodiments, the solid support may comprise 100,000 or more oligonucleotides. In some embodiments, the solid support may comprise 1,000,000 or more oligonucleotides.

In some embodiments, the number of oligonucleotides conjugated to or synthesized on a solid support such as a bead may be 1, 2, 3, 4, 5, 6, 7, 8, 9, or 10-fold more than the number of target nucleic acids in a cell. In some embodiments, the number of oligonucleotides conjugated to or synthesized on a solid support such as a bead may be 100-fold more than the number of target nucleic acids in a cell. In some embodiments, the number of oligonucleotides conjugated to or synthesized on a solid support such as a bead may be 1,000-fold more than the number of target nucleic acids in a cell. In some instances, at least 10, 20, 30, 40, 50, 60, 70, 80, 90 or 100% of the oligonucleotides are bound by a target nucleic acid. In some instances, at most 10, 20, 30, 40, 50, 60, 70, 80, 90 or 100% of the oligonucleotides are bound by a target nucleic acid. In some instances, at least 1, 2, 3, 4, 5, 6, 7, 8, 9, 10, 20, 30, 40, 50, 60, 70, 80, 90 or 100 or more different target nucleic acids are captured by the oligonucleotides on a solid support. In some instances, at most 1, 2, 3, 4, 5, 6, 7, 8, 9, 10, 20, 30, 40, 50, 60, 70, 80, 90 or 100 or more different target nucleic acids are captured by the oligonucleotides on a solid support.

In some embodiments of the disclosed methods, devices, and systems, the plurality of stochastic labels tethered to a given set of beads may be designed to focus the downstream analysis of sequence data on selected subpopulations of cells. In some embodiments, the plurality of stochastic labels tethered to a set of beads may be designed to exclude selected subpopulations of cells from the downstream analysis of sequence data. An example of a suitable approach for implementing these embodiments would be the inclusion of a subset of tethered labels attached to each bead that comprise one or more target-specific binding regions, where the one or more nucleic acid targets (*e.g.* nucleic acid markers, genetic markers) are chosen to define the subset of cells to be included in or excluded from further analysis at the sequence data analysis stage. The tethered label molecules of the subset would each comprise the cellular label (the same sequence for all labels attached to a given bead), a molecular label (*e.g.* a single, unique sequence selected at random from a diverse set of molecular label or barcode sequences included in the subset of tethered label molecules attached to a given bead), and a target-specific binding region, as well as one or more additional primer sequences, sample label sequences, *etc*., as described above. A set of nucleic acid targets (nucleic acid markers or genetic markers), *e.g.* mRNA targets, may be chosen to identify, for example, cells undergoing apoptosis (*e.g.* by monitoring Bax, Bcl-2, caspase-3, and caspase-7 expression, or expression of other genes potentially involved in apoptosis, or combinations thereof), rapid proliferation (*e.g.* by monitoring CKS1B, CCNB2, CDC2, DLG7, BUB3, MAD2L1, DLG7, PLK4, KIF2C, MKI67, BRRN1, NUSAP1, ASPM, or KLF7 expression, or expression of other genes potentially involved in cell proliferation, or combinations thereof), or any other subpopulation of cells that can be defined on the basis of nucleic acid markers. Analysis of sequence data generated by performing the stochastic labeling or molecular barcoding assay then provides a list of the cellular barcodes associated with the specified subpopulation of cells so that further analysis may be focused on the selected subpopulation of cells, or the selected subpopulation of cells may be excluded from further analysis.

*Beads & other encoded solid supports.* Any of a variety of different beads may be used as solid supports for attaching pre-synthesized stochastic label oligonucleotides or for in situ solid-phase synthesis of stochastic label oligonucleotides. A bead may encompass any type of solid, porous, or hollow sphere, ball, bearing, cylinder, or other similar configuration composed of plastic, ceramic, metal, or polymeric material onto which a nucleic acid may be immobilized (*e.g.*, covalently or non-covalently). A bead may comprise a discrete particle that may be spherical (*e.g.*, microspheres) or have a non-spherical or irregular shape, such as cubic, cuboid, pyramidal, cylindrical, conical, oblong, or disc-shaped, and the like. Beads may comprise a variety of materials including, but not limited to, paramagnetic materials (*e.g.* magnesium, molybdenum, lithium, and tantalum), superparamagnetic materials (*e.g.* ferrite ($Fe_3O_4$; magnetite) nanoparticles), ferromagnetic materials (e.g. iron, nickel, cobalt, some alloys thereof, and some rare earth metal compounds), ceramic, plastic, glass, polystyrene, silica, methylstyrene, acrylic polymers, titanium, latex, sepharose, agarose, hydrogel, polymer, cellulose, nylon, and any combination thereof. A bead may refer to any three dimensional structure that may provide an increased surface area for immobilization of biological particles and macromolecules, such as DNA and RNA.

In general, the diameter of the beads may range from about 1 $\mu$m to about 100 $\mu$m, or larger. In some embodiments, the diameter of the beads may be at least 1 $\mu$m, at least 5$\mu$m, at least 10$\mu$m, at least 20$\mu$m, at least 25$\mu$m, at least 30$\mu$m, at least 35$\mu$m, at least 40$\mu$m, at least 45$\mu$m, at least 50$\mu$m, at least 60 $\mu$m, at least 70 $\mu$m, at least 80 $\mu$m, at least 90 $\mu$m, or at least 100 $\mu$m. In some embodiment, the diameter of the beads may be at most 100 $\mu$m, at most 90 $\mu$m, at most 80 $\mu$m, at most 70 $\mu$m, at most 60 $\mu$m, at most 50 $\mu$m, at most 45 $\mu$m, at most 40 $\mu$m, at most 35 $\mu$m, at most 30 $\mu$m, at most 25 $\mu$m, at most 20 $\mu$m, at most 15 $\mu$m, at most 10 $\mu$m, at most 5 $\mu$m, or at most 1 $\mu$m. The diameter of the beads may have any value within this range, for example, beads may have a diameter in the range of about 20 to 50 $\mu$m. In some embodiments, beads may have a diameter of about 33 $\mu$m. In some embodiments, it may be desirable to use the smallest beads possible (*i.e.* that are compatible with the synthesis process used to create the plurality of stochastic labels attached to the beads and with other assay and device requirements), as larger beads will tend to settle faster than smaller beads of the same density, and therefore may result in less uniform distributions of beads across the microwell array. For spherical beads, settling velocity may be calculated using Stokes' Law:

$$V = \frac{2Ga^2(\rho_1 - \rho_2)}{9\eta}$$

where V = settling velocity (cm/sec), G = the acceleration due to gravity (cm/sec$^2$), a = bead radius (cm), $\rho_1$ = density of the bead (g/cm$^3$), $\rho_2$ = density of suspending media (g/cm$^3$), and $\eta$ = coefficient of viscosity (poise; g/cm-sec). Thus, a 50 $\mu$m diameter bead can settle 25-times faster than a 10 $\mu$m diameter bead of the same density.

A bead may be attached to, positioned within, or embedded into one or more supports. For example, a bead may be attached to a gel or hydrogel. A bead may be attached to a matrix. A bead may be embedded into a matrix. A bead may be attached to a polymer. A bead may be embedded into a polymer. The spatial position of a bead within the support (*e.g.*, gel, matrix, scaffold, or polymer) may be identified using the oligonucleotide present on the bead which serves as a location address.

Examples of beads include, but are not limited to, streptavidin beads, agarose beads, magnetic beads, Dynabeads®, MACS® microbeads, antibody conjugated beads (*e.g.*, anti-immunoglobulin microbead), protein A conjugated beads, protein G conjugated beads, protein A/G conjugated beads, protein L conjugated beads, oligo-dT conjugated beads, silica beads, silica-like beads, anti-biotin microbead, anti-fluorochrome microbead, and BcMag™ Carboxy-Terminated Magnetic Beads.

A bead may be associated with (*e.g.* impregnated with) quantum dots or fluorescent dyes to make it fluorescent in one fluorescence optical channel or multiple optical channels. A bead may be associated with iron oxide or chromium oxide to make it paramagnetic or ferromagnetic. Also, beads may be non-spherical in shape. A flatter, disc-like bead may be used in some embodiments. In some embodiments the disc-like bead may substantially occlude the well volume in some orientations but permit cells to move freely into the well in other orientations. This large disc-like bead is beneficial for achieving two different functions, the first being confinement of the cell and materials from within the cell after lysis, and the second being the ability for a cell to be readily loaded during the assay. The orientation may also be controlled during operation, including automatically by the instrument, using an applied magnetic field. Other bead shapes, used in combination with other well shapes (which may be different from simple cylinders), can be used to improve the efficiency and speed of cartridge, of bead and cell loading, and of performing the assay.

*Dual-encoded beads for stochastic labeling and molecular barcoding:* In some embodiments of the stochastic labeling and molecular barcoding methods described above, it may be desirable that beads associated with individual cells exhibiting a predefined set of properties, or beads associated with more than one cell, be removed from further processing, or that the sequence data arising from said beads be used to focus the downstream analysis of sequence data. In some embodiments, the sequence data arising from said beads may be excluded from any further sequence data analysis. In some embodiments, further analysis of sequence data may include only that sequence data arising from said beads. In some embodiments, this may be achieved through the use of optically-encoded beads in a dual encoding scheme, e.g. where individual beads are uniquely identified both by an optical code (*e.g.* by impregnating the beads with a spectrally-distinct set of fluorophores, quantum dots, Raman tags, up-converting phosphors, and the like; or by synthesis of an attached optical code through the use of solid-phase split-pool synthesis methodologies and a set of spectrally-distinct fluorescent building blocks) as well as a nucleic acid sequence (*e.g.* the cellular label) that is incorporated into the plurality of tethered stochastic labels attached to a given bead. Beads co-localized with cells exhibiting a set of predefined properties, or with more than one cell, would each be identified based on their optical code, and the sequence data arising from said beads would be subsequently identified by the corresponding cellular label sequence, thereby generating a list of sequence data to be included or excluded from further analysis. Individual cells or sub-populations of cells that exhibit a predefined set of characteristics, e.g. that express a particular cell surface receptor (marker) or set of cell surface receptors, may be identified through any of a variety of suitable techniques, e.g. through immunohistochemical staining of individual cells in a microwell array format using fluorescently-labeled antibodies directed towards the cell surface markers and fluorescence imaging techniques, or through the use of flow-cytometry and fluorescence-activated cell-sorting methods. In some embodiments, if the location of each cell label sequence in a two-dimensional space can be identified and recorded, the assay may provide spatial information for single cell gene expression, and may be particularly useful for analyzing gene expression in, for example, the thin tissue sections routinely collected for pathological studies.

*Dual encoding using array address codes:* In some embodiments, dual encoding schemes may be implemented by use of pre-deposited array address codes *(e.g.* nucleic acid barcodes that code for the location of a specific well in the array) instead of optically-encoded beads to implement dual encoding schemes. In some embodiments, array address codes may be deposited in wells using ink-jet printing techniques, microarray spotting techniques, dip-pen nanolithography techniques, and the like. In some embodiments, the array address codes may be non-specifically adsorbed to one or more inner surfaces of the microwells. In some embodiments, the array address codes may be covalently attached to one or more inner surfaces of the microwells. In some embodiments, the array address codes may be synthesized in situ by means of solid phase synthesis techniques, wherein one or more inner surfaces of the microwells are used as a solid support. In embodiments where the array address codes are covalently attached to one or more inner surfaces of the microwells, the attachment may comprise the use of cleavable linkers, e.g. acid-labile, base-labile, or photocleavable linkers, so that the array address codes may be released when desired and allowed to hybridize with a subset of the tethered stochastic labels attached to a bead. In some embodiments, the array address codes may be used in combination with the plurality of stochastic labels attached to a bead that comprises a cellular label. In some embodiments, the array address codes may be used instead of a plurality of stochastic labels attached to a bead, and may themselves comprise a cellular label, a molecular label, and one or more primer or adapter sequences. The array address codes may be used in similar fashion to that described above for optically-encoded beads in identifying subsets of cells to be included or excluded from downstream sequence data analysis.

*Stimulation of cells:* In some embodiments, cells may be contacted with an activating agent, physical or chemical stimulus, or test compound at known, adjustable times prior to performing cell lysis and subsequent assay steps. In this manner, the assay may be used to measure time-dependent or transient changes in stimulus-induced gene expression, for example, or the time-dependent dose-response curve for a drug candidate. Examples of cell activating agents include, but are not limited to, T-cell activating agents such as pharmacological agents (e.g. phorbol 12-myristate 13-acetate), anti-CD3/TCR or anti-Thy-1 monoclonal antibodies, enterotoxins and lectins. Examples of physical and chemical stimuli include, but are not limited to, temperature jumps, pH changes, ionic strength changes, and the like.

*Cell lysis:* Following the random distribution of cells and bead-based stochastic labels, such that an individual cell and individual bead are confined together within a small reaction volume, *e.g.* a well or microwell, the cells can be lysed to liberate the target molecules (FIG. 1). Cell lysis may be accomplished by any of a variety of means, for example, by chemical or biochemical means, by osmotic shock, or by means of thermal lysis, mechanical lysis, or optical lysis. In some embodiments, for example, cells may be lysed by addition of a cell lysis buffer comprising a detergent (*e.g.* SDS, Li dodecyl sulfate, Triton X-100, Tween-20, or NP-40), an organic solvent (*e.g.* methanol or acetone), or digestive enzymes (*e.g.* proteinase K, pepsin, or trypsin), or any combination thereof.

*Attachment of stochastic labels to target nucleic acid molecules:* Following lysis of the cells and release of nucleic acid molecules therefrom, the nucleic acid molecules may randomly attach to the stochastic label oligonucleotides of the co-localized bead. In some embodiments, attachment may comprise hybridization of a label's target recognition region to a complementary portion of the target nucleic acid molecule (FIG. 1). The assay conditions used for hybridization (*e.g.* buffer pH, ionic strength, temperature, *etc.*) are chosen to promote formation of specific, stable hybrids, as is well known to those of skill in the art.

In some embodiments, attachment may further comprise ligation of a label's target recognition region and a portion of the target nucleic acid molecule. For example, in some embodiments, the target binding region may comprise a nucleic acid sequence that is capable of specific hybridization to a restriction site overhang (*e.g.* an EcoRI sticky-end overhang). In some embodiments, the assay procedure further comprises treating the target nucleic acids with a restriction enzyme (*e.g.* EcoRI) to create a restriction site overhang. The stochastic label may then be ligated to any nucleic acid molecule comprising a sequence complementary to the restriction site overhang. A ligase *(e.g.,* T4 DNA ligase) may be used to join the two oligonucleotide fragments.

In some embodiments of the disclosed methods, the labeled target nucleic acid molecules from a plurality of cells (or a plurality of samples) are subsequently pooled, for example by retrieving beads to which the stochastically-labeled nucleic acid molecules are attached (FIG. 1). In some embodiments, the distribution and/or retrieval of bead-based collections of attached nucleic acid molecules may be implemented by use of magnetic beads and an externally-applied magnetic field. Once the stochastically-labeled nucleic acid molecules have been pooled, all further processing may proceed in a single reaction vessel. In some embodiments, further processing (*e.g.* reverse transcription reactions (or other nucleic acid extension reactions) and amplification reactions) may be performed within the microwells, that is, without first pooling the labeled target nucleic acid molecules from a plurality of cells.

*Reverse transcription:* In some embodiments of the disclosed methods, a reverse transcription reaction is performed (FIG. 1) to create a stochastic label - target nucleic acid conjugate (*e.g.* a covalently-linked molecular complex or molecular conjugate) comprising the stochastic label and a complementary sequence of all or a portion of the target nucleic acid (*i.e.* a labeled cDNA molecule). Reverse transcription may be performed using any of a variety of techniques known to those of skill in the art. Reverse transcription of the labeled-RNA molecule may occur by the addition of a reverse transcription primer along with the reverse transcriptase. In some embodiments, the reverse transcription primer is an oligo-dT primer, a random hexanucleotide primer, or a target-specific oligonucleotide primer. Generally, oligo-dT primers are 12-18 nucleotides in length and bind to the endogenous poly-A tail at the 3' end of mammalian mRNA. Random hexa-nucleotide primers may bind to mRNA at a variety of complementary sites. Target-specific oligonucleotide primers typically selectively prime the mRNA of interest.

*Amplification:* In some embodiments of the disclosed methods, one or more nucleic acid amplification reactions may be performed to create multiple copies of the labeled target nucleic acid molecules (FIGS. 1 and 5A-D). Amplification may be performed in a multiplexed manner, wherein multiple target nucleic acid sequences are amplified simultaneously. The amplification reaction may be used to add sequencing adaptors to the nucleic acid molecules. The amplification reactions may comprise amplifying at least a portion of a sample label, if present. The amplification reactions may comprise amplifying at least a portion of the cellular and or molecular label. The amplification reactions may comprise amplifying at least a portion of a sample tag, a cellular label, a molecular label, a target nucleic acid, or a combination thereof. The amplification reactions may comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids. The method may further comprise conducting one or more cDNA synthesis reactions to produce one or more cDNA copies of sample label-tagged nucleic acids, cellular label-tagged nucleic acids, or molecular label-tagged nucleic acids.

In some embodiments, amplification may be performed using a polymerase chain reaction (PCR). As used herein, PCR may refer to a reaction for the *in vitro* amplification of specific DNA sequences by the simultaneous primer extension of complementary strands of DNA. As used herein, PCR may encompass derivative forms of the reaction, including but not limited to, RT-PCR, real-time PCR, nested PCR, quantitative PCR, multiplexed PCR, digital PCR, and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM).

In some instances, the methods disclosed herein further comprise conducting a polymerase chain reaction on the labeled nucleic acid (*e.g.*, labeled-RNA, labeled-DNA, labeled-cDNA) to produce a labeled-amplicon. The labeled-amplicon may be double-stranded molecule. The double-stranded molecule may comprise a double-stranded RNA molecule, a double-stranded DNA molecule, or a RNA molecule hybridized to a DNA molecule. One or both of the strands of the double-stranded molecule may comprise a sample label, a cellular label, or a molecular label. Alternatively, the labeled-amplicon is a single-stranded molecule. The single-stranded molecule may comprise DNA, RNA, or a combination thereof. The nucleic acids may comprise synthetic or altered nucleic acids.

In some embodiment, amplification may comprise use of one or more non-natural nucleotides. Non-natural nucleotides may comprise photolabile or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides may be added to one or more cycles of an amplification reaction. The addition of the non-natural nucleotides may be used to identify products as specific cycles or time points in the amplification reaction.

Conducting the one or more amplification reactions may comprise the use of one or more primers. The one or more primers may comprise one or more oligonucleotides. The one or more oligonucleotides may comprise at least about 7-9 nucleotides. The one or more oligonucleotides may comprise less than 12-15 nucleotides. The one or more primers may anneal to at least a portion of the plurality of labeled nucleic acids. The one or more primers may anneal to the 3' end or 5' end of the plurality of labeled nucleic acids. The one or more primers may anneal to an internal region of the plurality of labeled nucleic acids. The internal region may be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers may comprise a fixed panel of primers. The one or more primers may comprise at least one or more custom primers. The one or more primers may comprise at least one or more control primers. The one or more primers may comprise at least one or more housekeeping gene primers. The one or more primers may comprise a universal primer. The universal primer may anneal to a universal primer binding site. The one or more custom primers may anneal to a first sample label, a second sample label, a cellular label, a molecular label, a target nucleic acid, or a combination thereof. The one or more primers may comprise a universal primer and a custom primer. The custom primer may be designed to amplify one or more target nucleic acids. The target nucleic acids may comprise a subset of the total nucleic acids in one or more samples. The target nucleic acids may comprise a subset of the total labeled nucleic acids in one or more samples. The one or more primers may comprise at least 96 or more custom primers. The one or more primers may comprise at least 960 or more custom primers. The one or more primers may comprise at least 9600 or more custom primers. The one or more custom primers may anneal to two or more different labeled nucleic acids. The two or more different labeled nucleic acids may correspond to one or more genes.

FIGS. 5A-D illustrates one embodiment of an amplification scheme for use in methods of the present disclosure. The first PCR reaction amplifies molecules attached to the bead using a gene specific primer and a primer against the universal Illumina sequencing primer 1 sequence (FIG. 5A). The second PCR reaction amplifies the first PCR products using a nested gene specific primer flanked by Illumina sequencing primer 2 sequence, and a primer against the universal Illumina sequencing primer 1 sequence (FIG. 5B). The third PCR reaction adds P5 and P7 and sample index to turn PCR products into an Illumina sequencing library (FIG. 5C). Sequencing using 150bp x 2 sequencing reveals the cell label and molecular index on read 1, the gene on read 2, and the sample index on index 1 read (FIG. 5D).

In some embodiments, the downstream analysis of sequence data may be focused on selected subpopulations of cells by performing an amplification reaction using one or more target-specific primers, wherein the one or more target-specific primers are capable of specific hybridization with, for example, one or more genes or gene products that define a subpopulation of cells. A set of nucleic acid targets (nucleic acid markers or genetic markers), *e.g.* mRNA targets, may be chosen to identify, for example, cells undergoing apoptosis (*e.g.* by monitoring Bax, Bcl-2, caspase-3, and caspase-7 expression, or expression of other genes potentially involved in apoptosis, or combinations thereof), rapid proliferation (*e.g.* by monitoring CKS1B, CCNB2, CDC2, DLG7, BUB3, MAD2L1, DLG7, PLK4, KIF2C, MKI67, BRRN1, NUSAP1, ASPM, or KLF7 expression, or expression of other genes potentially involved in cell proliferation, or combinations thereof), or any other subpopulation of cells that can be defined on the basis of nucleic acid markers. A multiplexed amplification reaction performed using the one or more target-specific primers is used to create multiple copies of the labeled target nucleic acid molecules attached to beads, which may then be sequenced to generate a list of cells comprising the one of more specified target nucleic acid molecules.

*Sequencing:* determining the number of different labeled nucleic acids may comprise determining the sequence of the labeled nucleic acid or any product thereof (e.g. labeled-amplicons, labeled-cDNA molecules). In some instances, an amplified target nucleic acid may be subjected to sequencing (FIGS. 1 and 5A-D). Determining the sequence of the labeled nucleic acid or any product thereof may comprise conducting a sequencing reaction to determine the sequence of at least a portion of a sample label, a cellular label, a molecular label, at least a portion of the labeled target nucleic acid, a complement thereof, a reverse complement thereof, or any combination thereof.

Determination of the sequence of a nucleic acid (*e.g.* amplified nucleic acid, labeled nucleic acid, cDNA copy of a labeled nucleic acid, *etc.*) may be performed using variety of sequencing methods including, but not limited to, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads, wobble sequencing, multiplex sequencing, polymerized colony (POLONY) sequencing; nanogrid rolling circle sequencing (ROLONY), allele-specific oligo ligation assays (*e.g.*, oligo ligation assay (OLA), single template molecule OLA using a ligated linear probe and a rolling circle amplification (RCA) readout, ligated padlock probes, or single template molecule OLA using a ligated circular padlock probe and a rolling circle amplification (RCA) readout), and the like.

In some instances, determining the sequence of the labeled nucleic acid or any product thereof comprises paired-end sequencing, nanopore sequencing, high-throughput sequencing, shotgun sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of the labeled nucleic acid or any product thereof may be determined by electron microscopy or a chemical-sensitive field effect transistor (chemFET) array.

High-throughput sequencing methods, such as cyclic array sequencing using platforms such as Roche 454, Illumina Solexa, ABI-SOLiD, ION Torrent, Complete Genomics, Pacific Bioscience, Helicos, or the Polonator platform, may also be utilized. In some embodiment, sequencing may comprise MiSeq sequencing. In some embodiment, sequencing may comprise HiSeq sequencing.

In some embodiments, the labeled nucleic acids comprise nucleic acids representing from about 0.01% of the genes of an organism's genome to about 100% of the genes of an organism's genome. For example, about 0.01% of the genes of an organism's genome to about 100% of the genes of an organism's genome can be sequenced using a target complimentary region comprising a plurality of multimers by capturing the genes containing a complimentary sequence from the sample. In some embodiments, the labeled nucleic acids comprise nucleic acids representing from about 0.01% of the transcripts of an organism's transcriptome to about 100% of the transcripts of an organism's transcriptome. For example, about 0.501% of the transcripts of an organism's transcriptome to about 100% of the transcripts of an organism's transcriptome can be sequenced using a target complimentary region comprising a poly-T tail by capturing the mRNAs from the sample.

Sequencing may comprise sequencing at least about 10, 20, 30, 40, 50, 60, 70, 80, 90, 100 or more nucleotides or base pairs of the labeled nucleic acid. In some instances, sequencing comprises sequencing at least about 200, 300, 400, 500, 600, 700, 800, 900, 1,000 or more nucleotides or base pairs of the labeled nucleic acid. In other instances, sequencing comprises sequencing at least about 1,500; 2,000; 3,000; 4,000; 5,000; 6,000; 7,000; 8,000; 9,000; or 10,000 or more nucleotides or base pairs of the labeled nucleic acid.

Sequencing may comprise at least about 200, 300, 400, 500, 600, 700, 800, 900, 1,000 or more sequencing reads per run. In some instances, sequencing comprises sequencing at least about 1,500; 2,000; 3,000; 4,000; 5,000; 6,000; 7,000; 8,000; 9,000; or 10,000 or more sequencing reads per run. Sequencing may comprise less than or equal to about 1,600,000,000 sequencing reads per run. Sequencing may comprise less than or equal to about 200,000,000 reads per run.

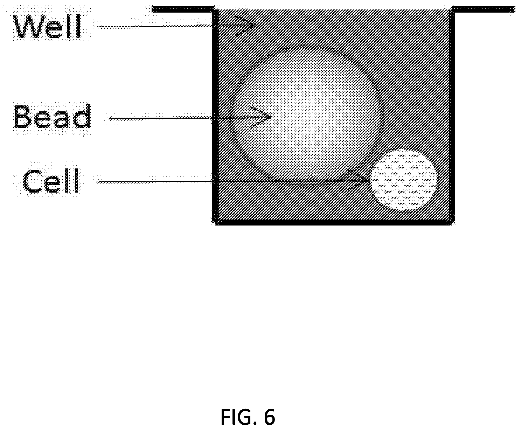

*Microwells used for entrapment:* As described above, in some embodiments microwells are used to entrap single cells and beads (one bead per cell) within a small reaction chamber of defined volume (FIG. 6A). Each bead comprises a library of oligonucleotide probes for use in stochastic labeling and digital counting of nucleic acid targets (*e.g.* the entire complement of cellular mRNA molecules) which are released upon lysis of the cell.

In some embodiments of the disclosed methods, devices, and systems, a plurality of microwells that are randomly distributed across a substrate are used. In some embodiments, the plurality of microwells are distributed across a substrate in an ordered pattern, *e.g.* an ordered array. In some embodiments, the plurality of microwells are distributed across a substrate in a random pattern, *e.g.* a random array. In one embodiment of the present disclosure, the plurality of microwells (*e.g.* a microwell array) is a consumable component of the assay system. In other embodiments, the plurality of microwells (*e.g.* a microwell array) may be reusable. In either case, they may be configured for use as a stand-alone device for performing assays manually, or they may be configured to comprise a fixed or removable component of an instrument system that provides for full or partial automation of the assay procedure.

In some embodiments of the disclosed methods, bead-based libraries of stochastic labels (e.g. oligonucleotide probes) are deposited in the microwells as part of the assay procedure. In some embodiments, the beads may be preloaded into the microwells and provided to the user as part of, for example, a kit for performing stochastic labeling and digital counting of nucleic acid targets. In some embodiments, two mated microwell arrays may be provided, one preloaded with beads which are held in place by a first magnet and the other for use by the user in loading individual cells. Following distribution of cells into the second microwell array, the two arrays may be placed face-to-face and the first magnet removed while a second magnet is used to draw the beads from the first array down into the corresponding microwells of the second array, thereby ensuring that the beads rest above the cells in the second microwell array and thus minimizing diffusional loss of target molecules following cell lysis, while maximizing efficient attachment of target molecules to the stochastic labels on the bead. Any of a variety of bead loading and retrieval processes may be used, as described in more detail below.

*Microwell geometries and dimensions:* The microwells can be fabricated in a variety of shapes and sizes. Microwell geometries and dimensions may impact cell loading and bead loading/retrieval efficiencies, and in general are chosen to minimize the chances of loading more than one bead per well. Appropriate well geometries include, but are not limited to, cylindrical, elliptical, cubic, conical, hemispherical, rectangular, or polyhedral (*e.g.*, three dimensional geometries comprised of several planar faces, for example, rectangular cuboid, hexagonal columns, octagonal columns, inverted triangular pyramids, inverted square pyramids, inverted pentagonal pyramids, inverted hexagonal pyramids, or inverted truncated pyramids). In some embodiments, non-cylindrical microwells, *e.g.* wells having an elliptical or square footprint, may offer advantages in terms of being able to accommodate larger cells. In some embodiments, the upper and/or lower edges of the well walls may be rounded to avoid sharp corners and thereby decrease electrostatic forces that may arise at sharp edges or points due to concentration of electrostatic fields. Thus, use of rounded off corners may improve the ability to retrieve beads from the microwells.

In some embodiments, the side walls of the microwells may have a non-zero draft angle (*i.e.* the angle between the side-wall and a vertical axis that is perpendicular to the plane of the microwell substrate). In other words, the walls of the microwell may be slanted rather than vertical, and a positive draft angle gives rise to a larger opening at the top of the well. In some embodiments, the walls may be slanted positively or negatively by at least 1 degree, at least 2 degrees, at least 3 degrees, at least 4 degrees, at least 5 degrees, at least 6 degrees, at least 7 degrees, at least 8 degrees, at least 9 degrees, at least 10 degrees, at least 11 degrees, at least 12 degrees, at least 13 degrees, at least 14 degrees, or at least 15 or more degrees. In some embodiments, the walls may be slanted positively or negatively by at most 1 degree, at most 2 degrees, at most 3 degrees, at most 4 degrees, at most 5 degrees, at most 6 degrees, at most 7 degrees, at most 8 degrees, at most 9 degrees, at most 10 degrees, at most 11 degrees, at most 12 degrees, at most 13 degrees, at most 15 degrees, or at most 15 or more degrees. In these embodiments, therefore, the top of the microwell can have a different diameter (or average diameter) than the bottom of the microwell. In preferred embodiments, the walls are slanted by a positive draft angle in the range of about 3 to about 7 degrees.

In some embodiments, the microwells may comprise a shape that combines two or more geometries. For example, in one embodiment it may be partly cylindrical, with the remainder having the shape of an inverted cone. In another embodiment, it may include two side-by-side cylinders, one of larger diameter (*e.g.* that corresponds roughly to the diameter of the beads) than the other (*e.g.* that corresponds roughly to the diameter of the cells), that are connected by a vertical channel or slot (that is, parallel to the cylinder axes) that extends the full length (depth) of the cylinders. In general, an open end of the microwells will be located at an upper surface of the substrate that comprises the plurality of microwells, but in some embodiments the openings may be located at a lower surface of the substrate. In some embodiments, the openings may be located on a side of the substrate, *e.g.*, that is neither an upper or lower surface, for example, if a substantially flat, planar substrate is oriented with one long axis in the vertical direction. In general, the closed end (or bottom) of the microwells will be flat, but curved surfaces (*e.g.*, convex or concave) are also possible. In general, the shape (and size) of the microwells will be determined based on the types of cells or beads to be trapped within the microwells.

Microwell dimensions may be characterized in terms of the average diameter and depth of the well. As used herein, the average diameter of the microwell refers to the largest circle that can be inscribed within the planar cross-section of the microwell geometry. In one embodiment of the present disclosure, the average diameter of the microwells may range from about 1-fold to about 10-fold the diameter of the cells or beads to be trapped within the microwells. In other embodiments, the average microwell diameter is at least 1-fold, at least 1.5-fold, at least 2-fold, at least 3-fold, at least 4-fold, at least 5-fold, or at least 10-fold the diameter of the cells or beads to be trapped within the microwells. In yet other embodiments, the average microwell diameter is at most 10-fold, at most 5-fold, at most 4-fold, at most 3-fold, at most 2-fold, at most 1.5-fold, or at most 1-fold the diameter of the cells or beads to be trapped within the microwells. In one embodiment, the average microwell diameter is about 2.5-fold the diameter of the cells or beads to be trapped within the microwells. Those of skill in the art will appreciate that the average diameter of the microwells may fall within any range bounded by any of these values (*e.g.* from about 1.2-fold to about 3.5-fold the diameter of the cells or beads to be trapped within the microwells). In some embodiments, the average diameter for each microwell in the plurality of microwells may be the same. In other embodiments, the average diameters for the individual microwells of the plurality of microwells may be different.

Alternatively, the diameter of the microwells can be specified in terms of absolute dimensions. In one embodiment of the present disclosure, the average diameter of the microwells may range from about 5 $\mu$m to about 100 $\mu$m. In other embodiments, the average microwell diameter is at least 5 $\mu$m, at least 10 $\mu$m, at least 15 $\mu$m, at least 20 $\mu$m, at least 25 $\mu$m, at least 30 $\mu$m, at least 35 $\mu$m, at least 40 $\mu$m, at least 45 $\mu$m, at least 50 $\mu$m, at least 60 $\mu$m, at least 70 $\mu$m, at least 80 $\mu$m, at least 90 $\mu$m, or at least 100 $\mu$m. In yet other embodiments, the average microwell diameter is at most

100 μm, at most 90 μm, at most 80 μm, at most 70 μm, at most 60 μm, at most 50 μm, at most 45 μm, at most 40 μm, at most 35 μm, at most 30 μm, at most 25 μm, at most 20 μm, at most 15 μm, at most 10 μm, or at most 5 μm. In one embodiment, the average microwell diameter is about 50 μm. Those of skill in the art will appreciate that the average diameter of the microwells may fall within any range bounded by any of these values (e.g. from about 34 μm to about 64 μm).

The microwell depth may be chosen to provide efficient trapping of cells and beads. The microwell depth may be chosen to provide efficient exchange of assay buffers and other reagents contained within the wells. The ratio of microwell depth to average diameter (*i.e.* the aspect ratio) may be chosen such that once a cell and bead settle inside a microwell, they will not be displaced by fluid motion above the microwell. The dimensions of the microwell may be chosen such that the microwell has sufficient space to accommodate a bead and a cell of various sizes without being dislodged by fluid motion above the microwell. In one embodiment of the present disclosure, the depth of the microwells may range from about 1-fold to about 10-fold the diameter of the cells or beads to be trapped within the microwells. In other embodiments, the microwell depth is at least 1-fold, at least 1.5-fold, at least 2-fold, at least 3-fold, at least 4-fold, at least 5-fold, or at least 10-fold the diameter of the cells or beads to be trapped within the microwells. In yet other embodiments, the microwell depth is at most 10-fold, at most 5-fold, at most 4-fold, at most 3-fold, at most 2-fold, at most 1.5-fold, or at most 1-fold the diameter of the cells or beads to be trapped within the microwells. In one embodiment, the microwell depth is about 2.5-fold the diameter of the cells or beads to be trapped within the microwells. Those of skill in the art will appreciate that the microwell depth may fall within any range bounded by any of these values (*e.g.* from about 1.2-fold to about 3.5-fold the diameter of the cells or beads to be trapped within the microwells).

For microwells of cylindrical geometry, suitable ratios of bead diameter-to-microwell diameter may range from about 0.1 to about 1.0. In some embodiments, the bead diameter-to-microwell diameter ratio is at least 0.1, at least 0.2, at least 0.3, at least 0.4, at least 0.5, at least 0.6, at least 0.7, at least 0.9, at least 0.95, or at least 0.99. In some embodiments, the bead diameter-to-microwell diameter ratio is at most 0.99, at most 0.95, at most 0.9, at most 0.8, at most 0.7, at most 0.6, at most 0.5, at most 0.4, at most 0.3, at most 0.2, or at most 0.1. In some embodiments, the bead diameter-to-microwell diameter ratio may have a value of 0.67. Those of skill in the art will recognize that the bead diameter-to-microwell diameter ratio may have any value within this range, for example, about 075.

For microwells of cylindrical geometry, suitable well depth-to-diameter aspect ratios may range from about 0.1 to about 2. In some embodiments, the well depth-to-diameter aspect ratio is at least 0.1, at least 0.2, at least 0.3, at least 0.4, at least 0.5, at least 0.6, at least 0.7, at least 0.9, at least 0.95, at least 1.0, or at least 1.05, at least 1.1, at least 1.2, at least 1.3, at least 1.4, at least 1.5, at least 1.6, at least 1.7, at least 1.8, at least 1.9, or at least 2.0. In some embodiments, the well depth-to-diameter aspect ratio is at most 2, at most 1.9, at most 1.8, at most 1.7, at most 1.6, at most 1.5, at most 1.4, at most 1.3, at most 1.2, at most 1.1, at most 1.1, at most 1.05, at most 1.0, at most 0.95, at most 0.9, at most 0.8, at most 0.7, at most 0.6, at most 0.5, at most 0.4, at most 0.3, at most 0.2, or at most 0.1. In some embodiments, the well depth-to-diameter aspect ratio may have a value of 0.9. Those of skill in the art will recognize that the well depth-to-diameter aspect ratio may have any value within this range, for example, about 0.94.

Alternatively, the depth of the microwells can be specified in terms of absolute dimensions. In one embodiment of the present disclosure, the depth of the microwells may range from about 5 μm to about 100 μm. In other embodiments, the microwell depth is at least 5 μm, at least 10 μm, at least 20 μm, at least 25 μm, at least 30 μm, at least 35 μm, at least 40 μm, at least 50 μm, at least 60 μm, at least 70 μm, at least 80 μm, at least 90 μm, or at least 100 μm. In yet other embodiments, the microwell depth is at most 100 μm, at most 90 μm, at most 80 μm, at most 70 μm, at most 60 μm, at most 50 μm, at most 40 μm, at most 35 μm, at most 30 μm, at most 25 μm, at most 20 μm, at most 10 μm, or at most 5 μm. In one embodiment, the microwell depth is about 30 μm. Those of skill in the art will appreciate that the microwell depth may fall within any range bounded by any of these values (e.g. from about 24 μm to about 36 μm).

The volumes of the microwells used in the methods, devices, and systems of the present disclosure may range from about 200 $\mu m^3$ to about 800,000 $\mu m^3$. In some embodiments, the micro well volume is at least 200 $\mu m^3$, at least 500 $\mu m^3$, at least 1,000 $\mu m^3$, at least 10,000 $\mu m^3$, at least 25,000 $\mu m^3$, at least 50,000 $\mu m^3$, at least 100,000 $\mu m^3$, at least 200,000 $\mu m^3$, at least 300,000 $\mu m^3$, at least 400,000 $\mu m^3$, at least 500,000 $\mu m^3$, at least 600,000 $\mu m^3$, at least 700,000 $\mu m^3$, or at least 800,000 $\mu m^3$. In other embodiments, the microwell volume is at most 800,000 $\mu m^3$, at most 700,000 $\mu m^3$, at most 600,000 $\mu m^3$, 500,000 $\mu m^3$, at most 400,000 $\mu m^3$, at most 300,000 $\mu m^3$, at most 200,000 $\mu m^3$, at most 100,000 $\mu m^3$, at most 50,000 $\mu m^3$, at most 25,000 $\mu m^3$, at most 10,000 $\mu m^3$, at most 1,000 $\mu m^3$, at most 500 $\mu m^3$, or at most 200 $\mu m^3$. In one embodiment, the microwell volume is about 119,000 $\mu m^3$. Those of skill in the art will appreciate that the microwell volume may fall within any range bounded by any of these values (*e.g.* from about 18,000 $\mu m^3$ to about 350,000 $\mu m^3$).

The volumes of the microwells used in the methods, devices, and systems of the present disclosure may be further characterized in terms of the variation in volume from one microwell to another. In some embodiments, the coefficient of variation (expressed as a percentage) for microwell volume may range from about 1% to about 10%. In some embodiments, the coefficient of variation for microwell volume may be at least 1%, at least 2%, at least 3%, at least 4%, at least 5%, at least 6%, at least 7%, at least 8%, at least 9%, or at least 10%. In some embodiments, the coefficient of variation for microwell volume may be at most 10%, at most 9%, at most 8%, at most 7%, at most 6%, at most 5%, at most 4%, at most 3%, at most 2%, or at most 1%. The coefficient of variation for microwell volume may have any value within a range encompassed by these values, for example between about 1.5% and about 6.5%. In some embodiments, the coefficient of variation of microwell volume may be about 2.5%.

The ratio of the volume of the microwells to the surface area of the beads (or to the surface area of a solid support to which stochastic label oligonucleotides may be attached) used in the methods, devices, kits, and systems of the present disclosure may range from about 2.5 to about 2,500 (in units of micrometers). In some embodiments, the ratio is at least 2.5, at least 5, at least 10, at least 100, at least 500, at least 750, at least 1,000, at least 1,250, at least 1,500, at least 1,750, at least 2,000, at least 2,250, or at least 2,500. In other embodiments, the ratio is at most 2,500, at most 2,250, at most 2,000, at most 1,750, at most 1,500, at most 1,250, at most 1,000, at most 750, at most 500, at most 100, at most 10, at most 5, or at most 2.5. In one embodiment, the ratio is about 67.5. Those of skill in the art will appreciate that the ratio of microwell volume to the surface area of the bead (or solid support used for immobilization) may fall within any range bounded by any of these values (*e.g.* from about 30 to about 120).

*Random and ordered arrays of microwells:* In some embodiments, the wells of the plurality of microwells may be arranged in a one dimensional, two dimensional, or three dimensional array, where three dimensional arrays may be achieved, for example, by stacking a series of two or more two dimensional arrays (that is, by stacking two or more substrates comprising micro well arrays). In general, the pattern and spacing between wells is chosen to optimize the efficiency of trapping a single cell and single bead in each well, as well as to maximize the number of wells per unit area of the substrate. The microwells may be distributed according to a variety of random or non-random patterns, for example, they may be distributed entirely randomly across the surface of the substrate, or they may be arranged in a square grid, rectangular grid, hexagonal grid, circular pattern, or the like.

In some embodiments of the present disclosure, the center-to-center distance (or "pitch", or "spacing") between wells may vary from about 15 $\mu$m to about 75 $\mu$m. In other embodiments, the spacing between wells is at least 15 $\mu$m, at least 20 $\mu$m, at least 25 $\mu$m, at least 30 $\mu$m, at least 35 $\mu$m, at least 40 $\mu$m, at least 45 $\mu$m, at least 50 $\mu$m, at least 55 $\mu$m, at least 60 $\mu$m, at least 65 $\mu$m, at least 70 $\mu$m, or at least 75 $\mu$m. In yet other embodiments, the microwell spacing is at most 75 $\mu$m, at most 70 $\mu$m, at most 65 $\mu$m, at most 60 $\mu$m, at most 55 $\mu$m, at most 50 $\mu$m, at most 45 $\mu$m, at most 40 $\mu$m, at most 35 $\mu$m, at most 30 $\mu$m, at most 25 $\mu$m, at most 20 $\mu$m, or at most 15 $\mu$m. In one embodiment, the microwell spacing is about 55 $\mu$m. Those of skill in the art will appreciate that the microwell spacing may fall within any range bounded by any of these values (e.g. from about 18 $\mu$m to about 72 $\mu$m).

The total number of wells in the plurality of microwells is determined by the pattern and spacing of the wells and the overall dimensions of the substrate. In one embodiment of the present disclosure, the number of microwells in the array may range from about 10 to about 5,000,000 or more. In other embodiments, the number of microwells in the array is at least 10, at least 96, at least 384, at least 1,536, at least 2,500, at least 5,000, at least 10,000, at least 25,000, at least 50,000, at least 75,000, at least 100,000, at least 200,000, at least 300,000, at least 400,000, at least 500,000, at least 600,000, at least 700,000, at least 800,000, at least 900,000, at least 1,000,000, at least 2,500,000, or at least 5,000,000. In yet other embodiments, the number of microwells in the array is at most 5,000,000, at most 2,500,000, at most 1,000,000, at most 900,000, at most 800,000, at most 700,000, at most 600,000, at most 500,000, at most 400,000, at most 300,000, at most 200,000, at most 100,000, at most 75,000, at most 50,000, at most 25,000, at most 10,000, at most 5,000, at most 2,400, at most 1,536, at most 384, at most 96 wells, or at most 10 wells. In one embodiment, the number of microwells in the plurality of microwells is about 96. In one embodiment, the number of microwells in the plurality of microwells is about 10,000. In yet another embodiment, the number of microwells is about 150,000. Those of skill in the art will appreciate that the number of microwells in the plurality of microwells may fall within any range bounded by any of these values (e.g. about 144,000 microwells).

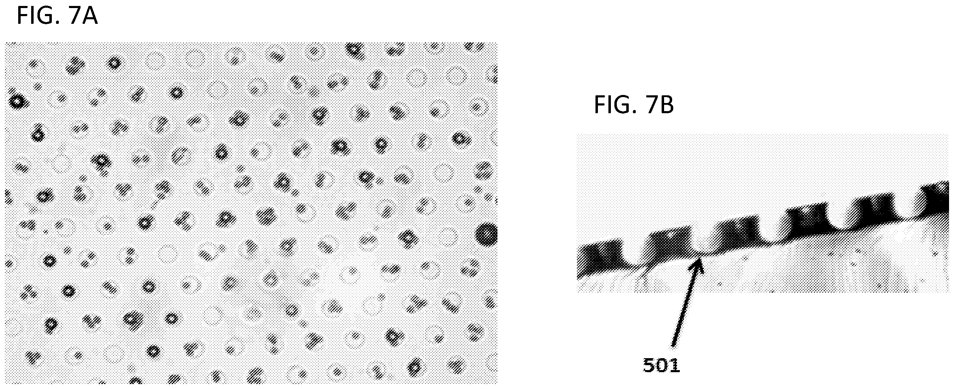

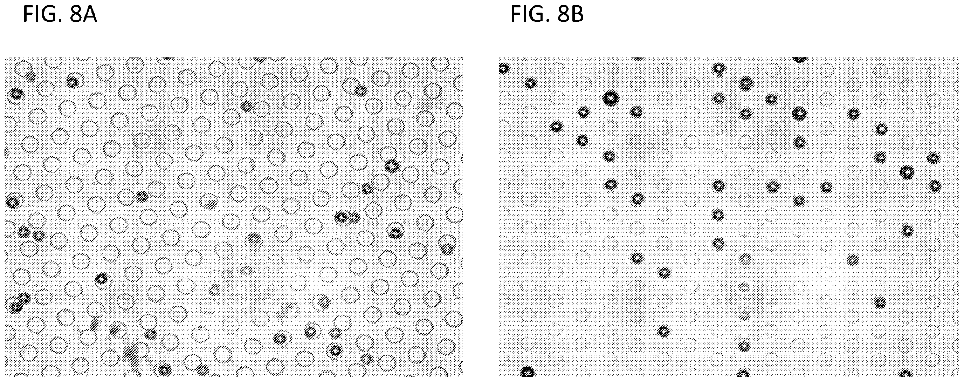

*Microwell substrate surface features:* In some embodiments, the plurality of microwells may comprise surface features between the microwells that are designed to help guide cells and beads into the wells or prevent them from settling on the substrate surfaces between wells. Examples of suitable surface features include, but are not limited to, rounded, domed, ridged, or peaked surface features that encircle the wells or straddle the surface between wells. FIGS. 7A-B and 8A-B show micrographs of microwell arrays in which the wells are separated by domed ridges (FIG. 7B, **501**) to minimize the number of cells or beads that settle on the surfaces between wells.

*Microwell fabrication:* Microwells may be fabricated using any of a number of fabrication techniques known to those of skill in the art. Examples of fabrication methods that may be used include, but are not limited to, bulk micromachining techniques such as photolithography and wet chemical etching, plasma etching, or deep reactive ion etching; micro-molding and micro-embossing; laser micromachining; 3D printing or other direct write fabrication processes using curable materials; and similar techniques.

Microwells may be fabricated from any of a number of substrate materials known to those of skill in the art, where the choice of material typically depends on the choice of fabrication technique, and vice versa. Examples of suitable materials include, but are not limited to, silicon, fused-silica, glass, polymers (*e.g.* agarose, gelatin, hydrogels, polydimethylsiloxane (PDMS; elastomer), polymethylmethacrylate (PMMA), polycarbonate (PC), polystyrene (PS), polypropylene (PP), polyethylene (PE), high density polyethylene (HDPE), polyimide, cyclic olefin polymers (COP), cyclic olefin copolymers (COC), polyethylene terephthalate (PET), optical adhesives (*e.g.* Norland Optical Adhesive 63), epoxy resins, thiol-ene based resins, metals or metal films (*e.g.* aluminum, stainless steel, copper, nickel, chromium, and titanium), and the like. Typically, a hydrophilic material is desirable for fabrication of the microwell arrays (*e.g.* to enhance wettability and minimize non-specific binding of cells and other biological material), but hydrophobic materials that can be treated or coated (*e.g.* by oxygen plasma treatment, or grafting of a polyethylene oxide surface layer) can also be used. The use of porous, hydrophilic materials for the fabrication of the microwell array may be desirable in order to facilitate capillary wicking/venting of entrapped air bubbles in the device. In some embodiments, the microwells are fabricated from a single material. In other embodiments, microwells may comprise two or more different materials that have been bonded together or mechanically joined.

Microwells may be fabricated using substrates of any of a variety of sizes and shapes. In some embodiments, for example, the shape (or footprint) of the substrate within which microwells are fabricated may be square, rectangular, circular, or irregular in shape. In some embodiments, the footprint of the microwell substrate will be similar to that of a microtiter plate. In some embodiments, the footprint of the microwell substrate will be similar to that of standard microscope slides, *e.g.* about 75 mm long x 25 mm wide (about 3" long x 1" wide), or about 75 mm long x 50 mm wide (about 3" long x 2" wide).

In some embodiments, the thickness of the substrate within which the microwells are fabricated may range from about 0.1 mm thick to about 10 mm thick, or more. In some embodiments, the thickness of the microwell substrate may be at least 0.1 mm thick, at least 0.5 mm thick, at least 1 mm thick, at least 2 mm thick, at least 3 mm thick, at least 4 mm thick, at least 5 mm thick, at least 6 mm thick, at least 7 mm thick, at least 8 mm thick, at least 9 mm thick, or at least 10 mm thick. In some embodiments, the thickness of the microwell substrate may be at most 10 mm thick, at most 9 mm thick, at most 8 mm thick, at most 7 mm thick, at most 6 mm thick, at most 5 mm thick, at most 4 mm thick, at most 3 mm thick, at most 2 mm thick, at most 1 mm thick, at most 0.5 mm thick, or at most 0.1 mm thick. In some embodiments, the thickness of the microwell substrate will be about 1 mm thick. As will be apparent to those of skill in the art, the thickness of the microwell substrate may be any value within these ranges, for example, the thickness of the microwell substrate may be between about 0.2 mm and about 9.5 mm.

In some embodiments, the substrate in which microwells are fabricated may itself comprise a three-dimensional geometry, for example, the substrate may be spherical, cylindrical, elliptical, conical, hemispherical, cubic, rectangular, or polyhedral, or may have an irregular three-dimensional geometry.

*Microwell and substrate coatings:* A variety of surface treatments and surface modification techniques may be used to alter the properties of microwell surfaces, for example, to improve the wettability of and/or reduce adherence of beads and cells to microwell or substrate surfaces. Examples of suitable surface treatments include, but are not limited to, oxygen plasma treatments to render hydrophobic material surfaces more hydrophilic, the use of wet or dry etching techniques to smooth (or roughen) glass and silicon surfaces, and adsorption (*e.g.* nonspecific adsorption or electrostatic adsorption) or covalent grafting of polyethylene oxide, polyethylene glycol (PEG), poly-L-lysine-PEG, or other polymer layers (*e.g.* a pluronic polyol, poly(2-hydroxyethyl methacrylate) (pHEMA or poly-HEMA)), polysorbates (*e.g.,* tween 20, tween 80, tween 60), or proteins (*e.g.* bovine serum albumin), to substrate surfaces to render them either more hydrophilic (or more hydrophobic in some cases) and less prone to fouling through non-specific adsorption of biomolecules and cells. In some embodiments, the application of surface coatings may be used to render substrate surfaces both non-toxic and non-sticky to cells. In some embodiments, the application of surface coating or modification techniques may be used to neutralize charged surfaces. In some embodiments, the application of surface coating or modification techniques may be used to add charge to otherwise neutral surfaces. In some embodiments, silane reactions may be used to graft chemically-reactive functional groups to otherwise inert silicon and glass surfaces, *etc.* Photodeprotection techniques can be used to selectively activate chemically-reactive functional groups at specific locations in the microwell structure, for example, the selective addition or activation of chemically-reactive functional groups such as primary amines or carboxyl groups on the inner walls of the microwells may be used to covalently couple oligonucleotide probes, peptides, proteins, or other biomolecules to the walls of the microwells. In general, the choice of surface treatment or surface modification utilized will depend both on the type of surface property that is desired and on the type of material from which the microwells are made.

Pluronic® polyols are block copolymer surfactants based on ethylene oxide and propylene oxide that may provide advantages in terms improved wettability. Examples of commercially-available Pluronic® products include, bujt are not limited to, Pluronic® 10R5, Pluronic® 17R2, Pluronic® 17R4, Pluronic® 25R2, Pluronic® 25R4, Pluronic® 31R1, Pluronic® F 108 Cast Solid Surfactant, Pluronic® F 108 NF, Pluronic® F 108 Pastille, Pluronic® F 108NF Prill Poloxamer 338, Pluronic® F 127 NF, Pluronic® F 127 NF 500 BHT Prill, Pluronic® F 127 NF Prill Poloxamer 407, Pluronic® F 38, Pluronic® F 38 Pastille, Pluronic® F 68, Pluronic® F 68 LF Pastille, Pluronic® F 68 NF, Pluronic® F 68 NF Prill Poloxamer 188, Pluronic® F 68 Pastille, Pluronic® F 77, Pluronic® F 77 Micropastille, Pluronic® F 87, Pluronic® F 87 NF, Pluronic® F 87 NF Prill Poloxamer 237, Pluronic® F 88, Pluronic® F 88 Pastille, Pluronic® FT L 61, Pluronic® L 10, Pluronic® L 101, Pluronic® L 121, Pluronic® L 31, Pluronic® L 35, Pluronic® L 43, Pluronic® L 61, Pluronic® L 62, Pluronic® L 62 LF, Pluronic® L 62D, Pluronic® L 64, Pluronic® L 81, Pluronic® L 92, Pluronic® L44 NF INH surfactant Poloxamer 124, Pluronic® N 3, Pluronic® P 103, Pluronic® P 104, Pluronic® P 105, Pluronic® P 123 Surfactant, Pluronic® P 65, Pluronic® P 84, and Pluronic® P 85, available from BASF (Florham Park, NJ).

Examples of zitterionic detergents that may be advantageous for improving wettability and/or reducing adherence of beads and cells to microwell or substrate surfaces when included in assay buffers include, but are not limited to, 1-Dodecanoyl-sn-glycero-3-phosphocholine, 3-(4-tert-Butyl-1-pyridinio)-1-propanesulfonate, 3-(N,N-Dimethylmyristylammonio)propanesulfonate, 3-(N,N-Dimethylmyristylammonio)propanesulfonate, 3-(N,N-Dimethylmyristylammonio)propanesulfonate, 3-(N,N-Dimethyloctadecylammonio)propanesulfonate, 3-(N,N-Dimethyloctylammonio)propanesulfonate inner salt, 3-(N,N-Dimethylpalmitylammonio)propanesulfonate, 3-(1-Pyridinio)-1-propanesulfonate, 3-(Benzyldimethylammonio)propanesulfonate BioXtra, 3-(Decyldimethylammonio)-propane-sulfonate inner salt zwitterionic detergent, 3-(N,N-Dimethyloctylammonio)propanesulfonate, 3-[N,N-Dimethyl(3-palmitoylaminopropyl)ammonio]-propanesulfonate, L-α-Lysophosphatidylcholine from Glycine max (soybean), L-α-Lysophosphatidylcholine from bovine brain, L-α-Lysophosphatidylcholine from egg yolk, L-α-Lysophosphatidylcholine from soybean, ASB-14, ASB-C80, C7BzO, CHAPS, CHAPS hydrate, CHAPS hydrate BioReagent, CHAPS hydrate BioXtra, CHAPSO, CHAPSO BioXtra, DDMAB, Dimethylethylammoniumpropane sulfonate, EMPIGEN® BB detergent, Miltefosine hydrate, Miltefosine, N,N-Dimethyldodecylamine N-oxide solution BioUltra, N,N-Dimethyldodecylamine N-oxide, N-Dodecyl-N,N-dimethyl-3-ammonio-1-propanesulfonate, N-Dodecyl-N,N-dimethyl-3-ammonio-1-propanesulfonate, O-(Decylphosphoryl)choline, O-(Octylphosphoryl)choline, Poly(maleic anhydride-alt-1-decene), 3-(dimethylamino)-1-propylamine derivative BioReagent, Poly(maleic anhydride-alt-1-tetradecene), 3-(dimethylamino)-1-propylamine derivative BioReagent, Sodium 2,3-dimercaptopropanesulfonate monohydrate, and Surfactin from Bacillus subtilis, available from Sigma-Aldrich (St. Louis, MO).

Examples of other detergents that may be advantageous for improving wettability and/or reducing adherence of beads and cells to microwell or substrate surfaces when included in assay buffers include, but are not limited to anionic, cationic, and non-ionic detergents. Nonionic detergents include poly(oxyethylene) ethers and related polymers (e.g. Brij®, TWEEN®, TRITON®, TRITON X-100 and IGEPAL® CA-630), bile salts, and glycosidic detergents.

*Sealing of microwells:* In some embodiments, it may be advantageous to seal the openings of microwells during, for example, cell lysis steps, to prevent cross hybridization of target nucleic acid between adjacent microwells. A microwell (or plurality of microwells) may be sealed or capped using, for example, a flexible membrane or sheet of solid material (*i.e.* a plate or platten) that clamps against the surface of the microwell substrate, or a suitable bead, where the diameter of the bead is larger than the diameter of the microwell. A seal formed using a flexible membrane or sheet of solid material can comprise, for example, inorganic nanopore membranes (*e.g.,* aluminum oxides), dialysis membranes, glass slides, coverslips, elastomeric films (*e.g.* PDMS), or hydrophilic polymer films (*e.g.,* a polymer film coated with a thin film of agarose that has been hydrated with lysis buffer).

In some embodiments, microwells (or wells) may be sealed by displacing the fluid above the wells with an immiscible fluid or material that undergoes a phase change upon an appropriate physical or chemical stimulus. Examples of suitable fluids and materials include, but are not limited to, mineral oil, waxes, polymer solutions, optical adhesives (*e.g.* Norland 63 or 81) that respond to UV light, and pluronic polyol solutions which may undergo solution-gel phase transitions with $T_m$s near room temperature depending on the concentration and molecular weight of the polymer.

Beads used for capping the microwells may comprise, for example, cross-linked dextran beads (*e.g.,* Sephadex). Cross-linked dextran can range from about 10 micrometers to about 80 micrometers. The cross-linked dextran beads used for capping can be from 20 micrometers to about 50 micrometers. In some embodiments, the beads may be, for example, at least about 10, 20, 30, 40, 50, 60, 70, 80 or 90% larger than the diameter of the microwells. Alternatively, the beads used for capping may be at most about 10, 20, 30, 40, 50, 60, 70, 80 or 90% larger than the diameter of the microwells.

In some embodiments, the seal or cap may allow buffer to pass into and out of the microwells, while preventing macromolecules (*e.g.,* nucleic acids) from migrating out of the well. In some embodiments, a macromolecule of at least about 1, 2, 3, 4, 5, 6, 7, 8, 9, 10, 12, 13, 14, 15, 16, 17, 18, 19, or 20 or more nucleotides may be blocked from migrating into or out of the microwells by the seal or cap. In some embodiments, a macromolecule of at most about 1, 2, 3, 4, 5, 6, 7, 8, 9, 10, 12, 13, 14, 15, 16, 17, 18, 19, or 20 or more nucleotides may be blocked from migrating into or out of the microwells by the seal or cap.

*Alternatives to microwells:* In some embodiments, individual cells and beads may be compartmentalized using alternatives to microwells, for example, a single bead and single cell could be confined within a single droplet in an emulsion (*e.g.* in a droplet digital microfluidic system). Alternatively, cells could potentially be confined within porous beads that themselves comprise the plurality of tethered stochastic labels. As will be understood by those of skill in the art, individual cells and beads may be compartmentalized in any type of container, microcontainer, reaction chamber, reaction vessel, or the like. Thus in some embodiments, single cell, stochastic labeling or molecular barcoding assays may be performed without the use of microwells. In some embodiments, single cell, stochastic labeling or molecular barcoding assays may be performed without the use of any physical container, *e.g.* by embedding cells and beads in close proximity to each other within a polymer layer or gel layer to create a diffusional barrier between different cell/bead pairs.

*Distribution of beads within microwells:* In some embodiments, the beads comprising libraries of tethered stochastic labels may be distributed amongst a plurality of microwells as part of the assay procedure. In some embodiments, the beads may be pre-loaded in a plurality of microwells as part of the manufacturing process for either flow cells or cartridges that incorporate a substrate comprising a plurality of microwells. In some embodiments, the percentage of microwells that contain a single bead may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single bead.

*Distribution of cells within microwells:* In many embodiments, cells may be distributed amongst a plurality of microwells as part of the assay procedure. In some embodiments, the percentage of microwells that contain a single cell may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single cell. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single cell.

*Microwells containing both a single cell and a single bead:* In many embodiments, cells and beads may be distributed amongst a plurality of microwells such that a fraction of the microwells contain both a single cell and a single bead. In some embodiments, the percentage of microwells that contain both a single cell and a single bead may be between about 1% and about 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain both a single cell and a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain both a single cell and a single bead.

*Mechanical fixtures comprising substrates with microwells:*

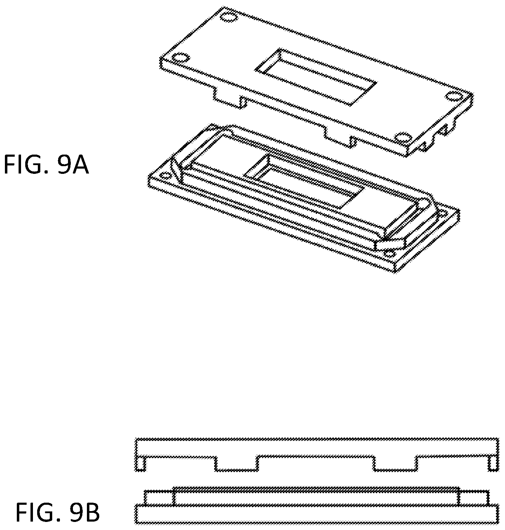

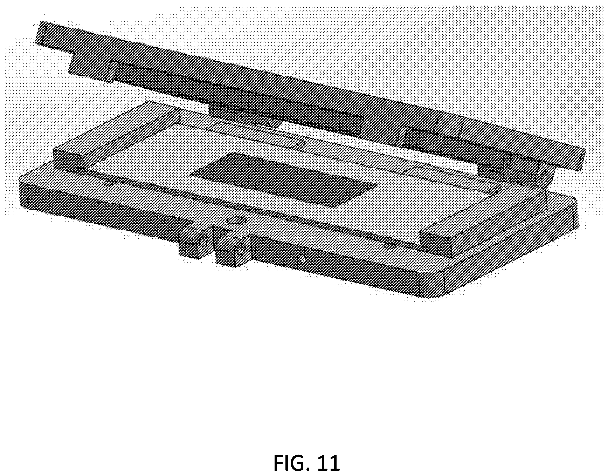

When performing multiplexed, single cell stochastic labeling or molecular barcoding assays manually, it may be convenient to mount the microwell substrate in a mechanical fixture (FIGS. 9A-B, 10, 11) to create a reaction chamber that facilitates the pipetting or dispensing of cell suspensions and assay reagents onto the substrate. In the example illustrated in FIG. 11, the hinged fixture accepts a microwell array fabricated on a 1 mm thick substrate, and provides mechanical support in the form of a silicone gasket to confine the assay reagents to a reaction chamber that is 16 mm wide x 35 mm long x approximately 1 mm deep, thereby enabling the use of 800 ul to 1 ml of cell suspension and bead suspension (comprising bead-based stochastic labels) to perform the assay.

The fixture may consist, for example, of rigid, machined top and bottom plates *(e.g.* aluminum) and a compressible *(e.g.* silicone, polydimethylsiloxane) gasket for creating the walls of the chamber or well. Design features include: (i) chamfered aperture edges and clearance for rotating microscope objectives in and out of position as needed (for viewing the microwell array at different magnifications), (ii) controlled compression of the silicone gasket to ensure uniform, repeatable formation of a leak-proof seal with the microwell array substrate, (iii) captive fasteners for convenient operation, (iv) a locating clamp mechanism for secure and repeatable positioning of the array, and (v) convenient opening for removal of the array during rinse steps.

The top and bottom plates may be fabricated using any of a variety of techniques *(e.g.* conventional machining, CNC machining, injection molding, 3D printing, *etc.)* using a variety of materials *(e.g.* aluminum, anodized aluminum, stainless steel, teflon, polymethylmethacrylate (PMMA), polycarbonate (PC), or similar rigid polymer materials).

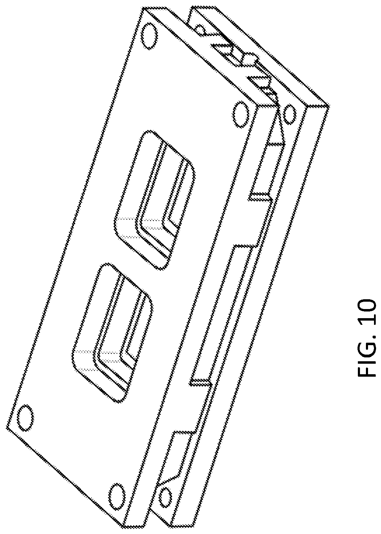

The top and bottom plates and silicone (polydimethylsiloxane; PDMS) gasket may be configured to create multiple chambers (see FIG. 10) in order to run controls and experiments (or replicate experiments) in parallel. The gasket may be molded from PDMS or similar elastomeric material using, for example, a Teflon mold that includes draft angles for the vertical gasket walls to provide for good release characteristics. Alternatively, molds may be machined from aluminum or other materials *(e.g.* black delrin, polyetherimide (ultem), *etc.),* and coated with a material such as Teflon if necessary to provide for good release characteristics. The gasket mold designs may be inverted, *i.e.* so that the top surface of the molded part *(i.e.* the surface at the interface with a glass slide or silicon wafer used to cover the mold during casting) becomes the surface for creating a seal with the microwell substrate during use, thereby avoiding potential problems with mold surface roughness and surface contamination in creating a smooth gasket surface (to ensure a leak-proof seal with the substrate), and also providing for a flexible choice of substrate materials and the option of pre-assembly by using the microwell substrate as a base during casting. The gasket mold designs may also include force focusing ridges at the boundaries of the well areas, *i.e.* the central mesa(s) in the mold (which form the well(s)) have raised ridges at the locations which become the perimeter of the well(s), so that a cover placed on top of the mold after filling rests on a small contact area at the precise location where good edge profile is critical for forming a leak-proof seal between the gasket and substrate during use.

*Devices & instrument systems:*

The present disclosure also includes devices, instrument systems, and consumables to support the automation of multiplexed, single cell stochastic labeling and molecular barcoding assays. Such systems may include consumable cartridges that incorporate microwells integrated with flow cells, as well as the instrumentation necessary to provide control and analysis functionality such as (i) fluidics control, (ii) cell or bead distribution and collection mechanisms, (iii) cell lysis mechanisms, (iv) magnetic field control, (v) temperature control, (vi) imaging capability, and (vii) image processing. In some embodiments, the input for the system comprises a cell sample and the output comprises a bead suspension comprising beads having attached oligonucleotides that incorporate sample labels, cell labels, or molecular labels. In other embodiments, the instrument system may include additional functionality, such as thermal cycling capability for performing PCR amplification, in which case the input for the system comprises a cell sample and the output comprises an oligonucleotide library resulting from amplification of the oligonucleotides incorporating sample labels, cell labels, or molecular labels that were originally attached to beads. In yet other embodiments, the system may also include sequencing capability, with or without the need for oligonucleotide amplification, in which case the input for the system is a cell sample and the output comprises a dataset further comprising the sequences of all sample labels, cell labels, or molecular labels associated with the target sequences of interest.

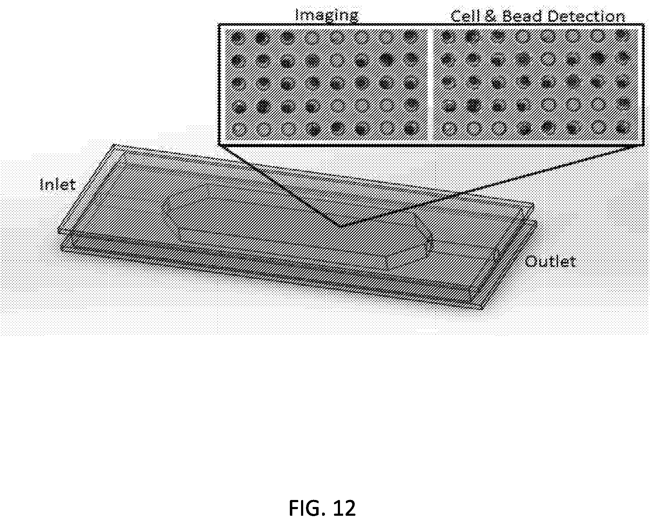

*Flow cells:* In many embodiments of the automated assay system, the microwell substrate will be packaged within a flow cell (FIG. 12) that provides for convenient interfacing with the rest of the fluid handling system and facilitate the exchange of fluids, *e.g.* cell and bead suspensions, lysis buffers, rinse buffers, *etc.*, that are delivered to the microwells. In many embodiments, the flow cell may be designed to facilitate uniform distribution of cells and beads across the plurality of microwells. Design features may include: (i) one or more inlet ports for introducing cell samples, bead suspensions, or other assay reagents, (ii) one or more microwell chambers designed to provide for uniform filling and efficient fluid-exchange while minimizing back eddies or dead zones, and (iii) one or more outlet ports for delivery of fluids to a sample collection point or a waste reservoir. In some embodiments, the design of the flow cell may include a plurality of microwell chambers that interface with a plurality of microwell arrays on a single substrate, or with a plurality of microwell array substrates, such that one or more different cell samples may be processed in parallel. In some embodiments, the design of the flow cell, *e.g.* the layout of the fluid channels and chambers, may be adjusted so that different patterns of microwells (*i.e.* configurable microarray patterns) are accessed by fluids in a given design.

In some embodiments, the design of the flow cell may further include features for creating uniform flow velocity profiles, *i.e.* "plug flow", across the width of the microwell chamber to provide for more uniform delivery of cells and beads to the microwells, for example, by using a porous barrier located near the chamber inlet and upstream of the microwells as a "flow diffuser", or by dividing each microwell chamber into several subsections that collectively cover the same total array area, but through which the divided inlet fluid stream flows in parallel.

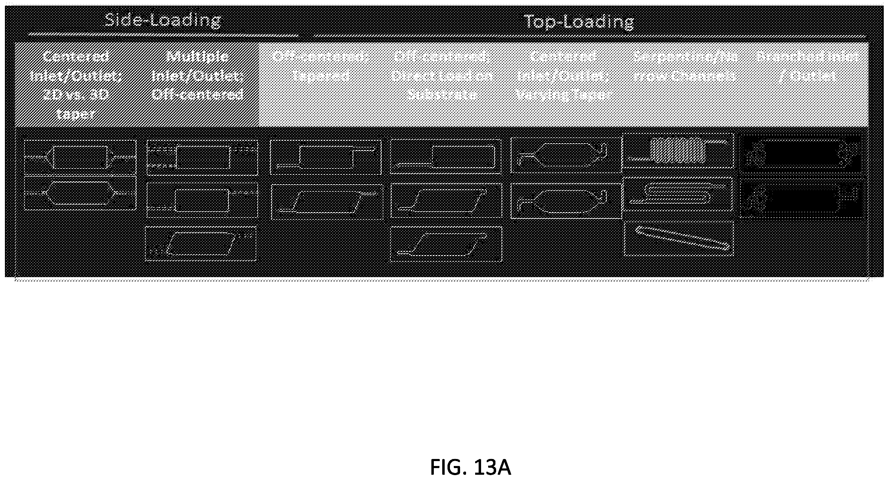

Non-limiting examples of flow cell designs are illustrated in FIG. 13A, and include designs having centered inlets and outlets wherein the inlets and outlets are tapered in either two or three dimensions; designs having inlets and outlets that are off-center and wherein the inlets and outlets are tapered in either two or three dimensions; and designs that have multiple inlets and/or outlets, wherein some or all of the inlets and outlets may be off-center. In some embodiments, inlets may also serve as outlets to facilitate ease of design and assembly as well as providing symmetry-related improvements in bead retrieval efficiency.

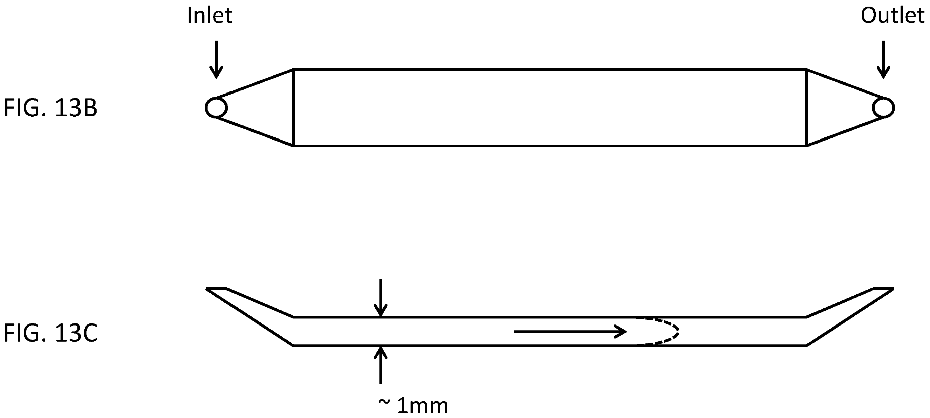

FIGS. 13B-C schematically illustrate an alternative flow cell design in which inlet and outlet ports connect to a microwell array chamber via tapered, slanted inlet and outlet fluid channels. FIG. 13B illustrates a top view of the flow cell design. FIG. 13C illustrates a cross-sectional side view of the flow cell, in which the inlet and outlet channels each form an angle relative to the plane of the microwell substrate. In some embodiments, the vertical height (*i.e.* the depth) of the microwell array chamber is typically on the order of approximately 1 mm (but may take any value within the ranges specifed elsewhere in this disclosure), and therefore permits parabolic flow velocity profiles when pressure is used to drive fluid flow (FIG. 13C). The use of parabolic flow in combination with interspersed air injections, as described elsewhere in this disclosure, allows the liquid/air interface to sweep the surface of the microwell substrate between fluid injections, and may provide advantages in terms of improving cell and/or bead loading efficiencies. In some embodiments, the angles formed by the inlet and outlet channels may be the same. In some embodiments, the angles formed by the inlet and outlet channels may be different. In some embodiments, the angle formed by the inlet and/or outlet fluid channels may range from about 15 to about 75 degrees relative to the plane of the microwell substrate. In some embodiments, the angle may be at least 15 degrees, at least 20 degrees, at least 25 degrees, at least 30 degrees, at least 35 degrees, at least 40 degrees, at least 45 degrees, at least 50 degrees, at least 55 degrees, at least 60 degrees, at least 65 degrees, at least 70 degrees, or at least 75 or more degrees. In some embodiments, the angle may be at most 75 degrees, at most 70 degrees, at most 65 degrees, at most 60 degrees, at most 55 degrees, at most 50 degrees, at most 45 degrees, at most 40 degrees, at most 35 degrees, at most 30 degrees, at most 25 degrees, at most 20 degrees, or at most 15 degrees. In some embodiments, the angle formed by the inlet and/or outlet fluid channels may range from about 30 to about 60 degrees relative to the plane of the microwell substrate. In some embodiments, the angle formed by the inlet and/or outlet fluid channels may be about 45 degrees.

In some embodiments, the flow cell may constitute a fixed component of the instrument system. In some embodiments, the flow cell may be removable from the instrument system. In some embodiments, the flow cell may be a single use device. In other embodiments, the flow cell may be a multi-use device.

*Flow cell fluid channel dimensions:* In general, the dimensions of fluid channels and microwell chamber(s) in flow cell designs may be optimized to (i) provide uniform delivery of cells and beads to the microwells, and (ii) to minimize sample and reagent consumption. In some embodiments, the width of fluid channels or microwell chambers will be between 50 $\mu$m and 20 mm. In other embodiments, the width of fluid channels or microwell chambers may be at least 50 $\mu$m, at least 100 $\mu$m, at least 200 $\mu$m, at least 300 $\mu$m, at least 400 $\mu$m, at least 500 $\mu$m, at least 750 $\mu$m, at least 1 mm, at least 2.5 mm, at least 5 mm, at least 10 mm, at least 20 mm, at least 50 mm, at least 100 mm, or at least 150 mm. In yet other embodiments, the width of fluid channels or microwell chambers may be at most 150 mm, at most 100 mm, at most 50 mm, at most 20 mm, at most 10 mm, at most 5 mm, at most 2.5 mm, at most 1 mm, at most 750 $\mu$m, at most 500 $\mu$m, at most 400 $\mu$m, at most 300 $\mu$m, at most 200 $\mu$m, at most 100 $\mu$m, or at most 50 $\mu$m. In one embodiment, the width of fluid channels is about 2 mm. Those of skill in the art will appreciate that the width of the fluid channels or microwell chambers may fall within any range bounded by any of these values (*e.g.* from about 250 $\mu$m to about 10 mm).

In some embodiments, the depth of the fluid channels will be between 50 $\mu$m and 5 mm. In other embodiments, the depth of fluid channels may be at least 50 $\mu$m, at least 100 $\mu$m, at least 200 $\mu$m, at least 300 $\mu$m, at least 400 $\mu$m, at least 500 $\mu$m, at least 750 $\mu$m, at least 1 mm, at least 1.25 mm, at least 1.5 mm, at least 1.75 mm, at least 2 mm, at least 2.25 mm, at least 2.5 mm, at least 2.75 mm, at least 3 mm, at least 4 mm, or at least 5 mm. In yet other embodiments, the depth of fluid channels may be at most 5 mm, at most 4 mm, at most 3 mm, at most 2.75 mm, at most 2.5 mm, at most 2.25 mm, at most 2 mm, at most 1.75 mm, at most 1.5 mm, at most 1.25 mm, at most 1 mm, at most 750 $\mu$m, at most 500 $\mu$m, at most 400 $\mu$m, at most 300 $\mu$m, at most 200 $\mu$m, at most 100 $\mu$m, or at most 50 $\mu$m. In one embodiment, the depth of the fluid channels is about 1 mm. Those of skill in the art will appreciate that the depth of the fluid channels may fall within any range bounded by any of these values (*e.g.* from about 800 $\mu$m to about 3 mm).

*Flow cell fabrication:* The flow cell can be fabricated as a separate part and subsequently either mechanically clamped against or permanently bonded to the microwell array substrate. Examples of suitable fabrication techniques include conventional machining, CNC machining, injection molding, 3D printing, alignment and lamination of one or more layers of laser or die-cut polymer films, or any of a number of microfabrication techniques such as photolithography and wet chemical etching, dry etching, deep reactive ion etching, or laser micromachining. In some embodiments, the fluidic layer is 3D printed from an elastomeric material.

Flow cells may be fabricated using a variety of materials known to those of skill in the art. In general, the choice of material used will depend on the choice of fabrication technique used, and vice versa. Examples of suitable materials include, but are not limited to, silicon, fused-silica, glass, any of a variety of polymers, e.g. polydimethylsiloxane (PDMS; elastomer), polymethylmethacrylate (PMMA), polycarbonate (PC), polystyrene (PS), polypropylene (PP), polyethylene (PE), high density polyethylene (HDPE), polyimide, cyclic olefin polymers (COP), cyclic olefin copolymers (COC), polyethylene terephthalate (PET), optical adhesive (NOA), epoxy resins, metals (*e.g.* aluminum, stainless steel, copper, nickel, chromium, and titanium), a non-stick material such as teflon (PTFE), or a combination of these materials. In some embodiments, the different layers in a flow cell comprising multiple layers may be fabricated from different materials, *e.g.* the fluid channel layer may be fabricated from an elastomeric material while the microwell substrate and cover plate (top) may be fabricated from glass or another suitable material. In some embodiments, the material(s) chosen for fabrication of flow cells will be ethanol compatible such that priming the fluidic device with ethanol or ethanol:water solutions may be used to facilitate surface wetting and removal of trapped air from the device.

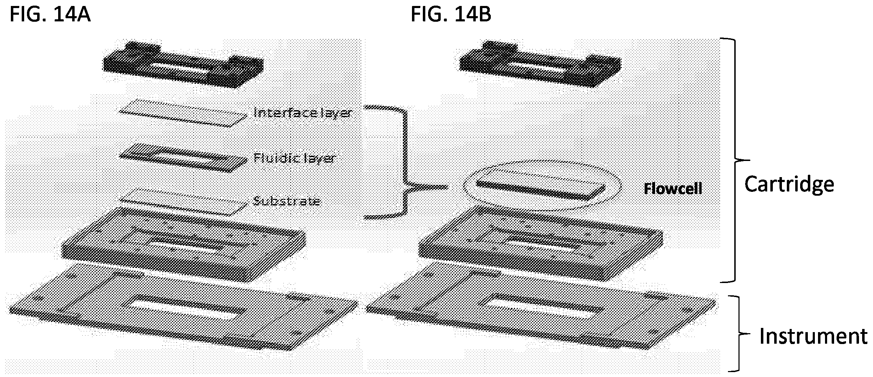

In some embodiments, the flow cell may comprise a three layer structure that includes the microwell array substrate, a fluid channel layer (fluidics layer), and a cover plate (*i.e.* a top or interface layer), whereby the volume of the microwell array chamber is determined by the cross-sectional area of the microwell array chamber and the thickness of the fluid channel layer (see FIGS. 14A-B). In some embodiments, the flow cell - microwell array substrate assembly may comprise two layers, three layers, four layers, five layers, or more than five layers.

As indicated above, in some embodiments the thickness of the fluid channel layer will determine the depth of the fluid channels and microwell array chamber, and will influence the total volume of the microwell array chamber. In some embodiments, the thickness of the fluid channel layer will be between 50 $\mu$m and 3 mm. In other embodiments, the thickness of the fluid channel layer may be at least 50 $\mu$m, at least 100 $\mu$m, at least 200 $\mu$m, at least 300 $\mu$m, at least 400 $\mu$m, at least 500 $\mu$m, at least 750 $\mu$m, at least 1 mm, at least 1.25 mm, at least 1.5 mm, at least 1.75 mm, at least 2 mm, at least 2.25 mm, at least 2.5 mm, at least 2.75 mm, or at least 3 mm. In yet other embodiments, the thickness of the fluid channel layer may be at most 3 mm, at most 2.75 mm, at most 2.5 mm, at most 2.25 mm, at most 2 mm, at most 1.75 mm, at most 1.5 mm, at most 1.25 mm, at most 1 mm, at most 750 $\mu$m, at most 500 $\mu$m, at most 400 $\mu$m, at most 300 $\mu$m, at most 200 $\mu$m, at most 100 $\mu$m, or at most 50 $\mu$m. In one embodiment, the thickness of the fluid channel layer is about 0.8 mm. In another embodiment, the thickness of the fluid channel layer is about 1.2 mm, 2 mm, or 3 mm. Those of skill in the art will appreciate that the depth of the fluid channels may fall within any range bounded by any of these values (e.g. from about 800 $\mu$m to about 1.6 mm).

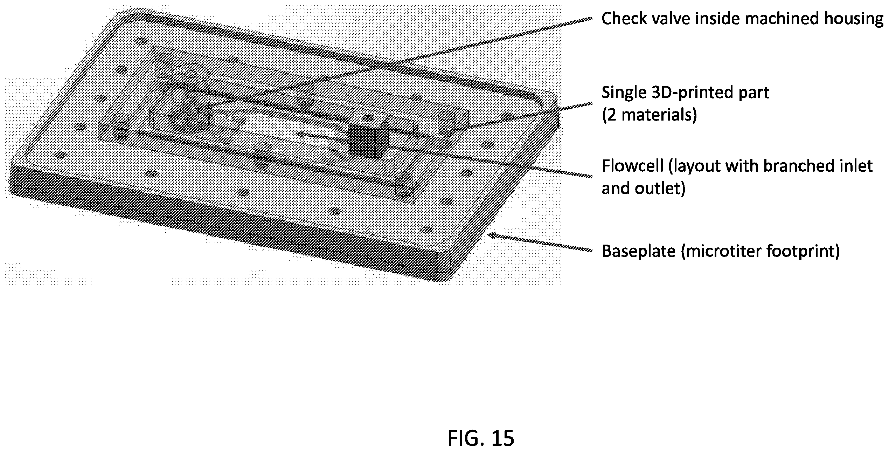

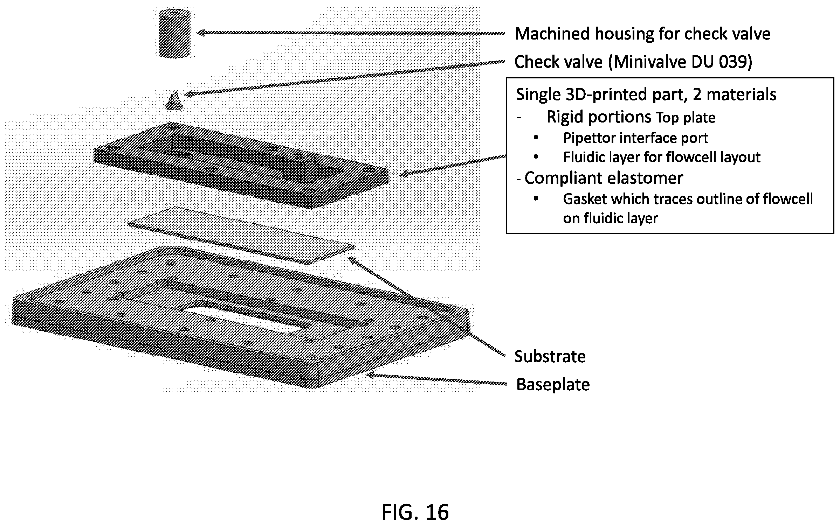

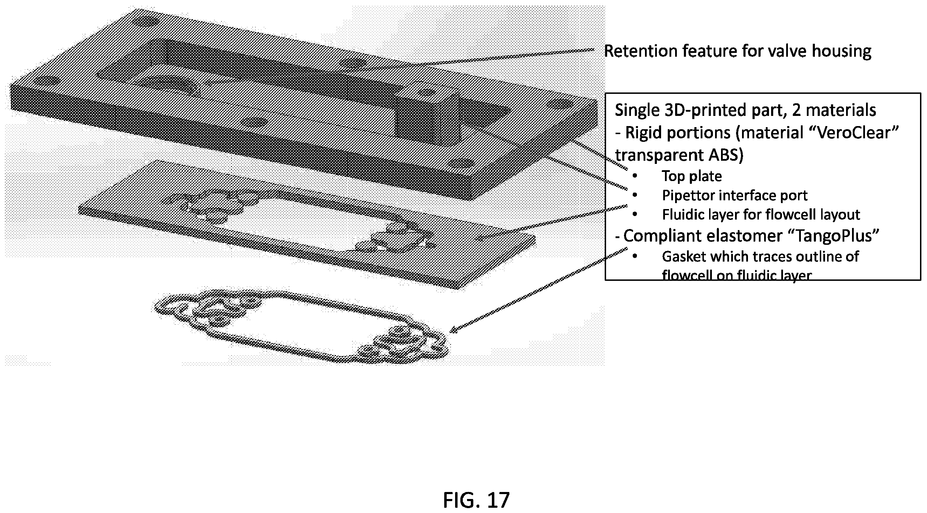

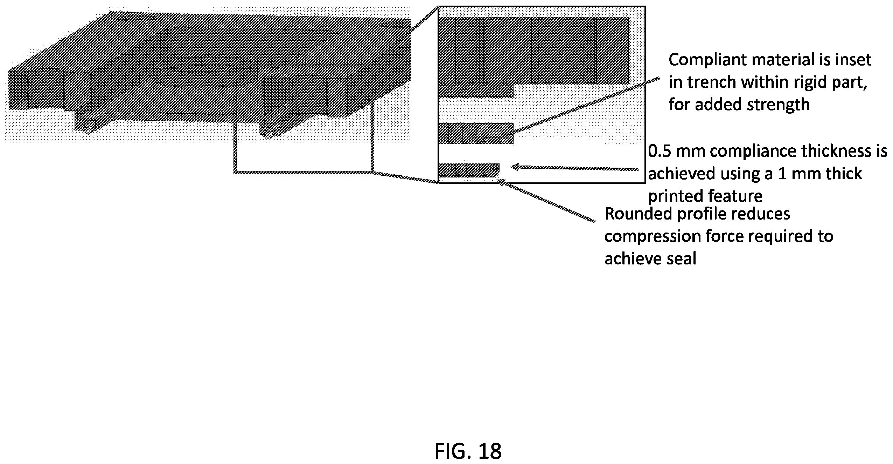

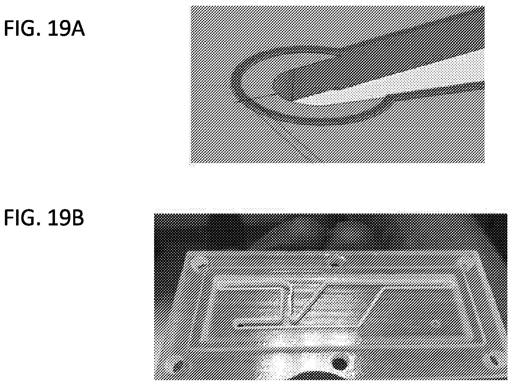

In some embodiments, multiple layers may be fabricated in a single integrated part, e.g. 3D printing of an integrated flow cell and gasket part as shown in FIGS. 15-18. In cases where the 3D printed part is not sufficiently flat to achieve good bonding with a substrate or cover plate, design features such as a sealing ring (also 3D printed) may be incorporated (see FIG. 19A). In some embodiments, more than one material may be used in 3D printing a single part or assembly, for example, the fluidic layer may be printed from a low-adhesion polymer, and directly integrated with a printed sealing gasket made from an elastomeric material to prevent leaks (FIG. 19B).

Once the flow cell part has been fabricated it may be attached to the microwell substrate mechanically, e.g. by clamping it against the microwell substrate (with or without the use of a gasket), or it may be bonded directly to the microwell substrate using any of a variety of techniques (depending on the choice of materials used) known to those of skill in the art, for example, through the use of anodic bonding, thermal bonding, or any of a variety of adhesives or adhesive films, including epoxy-based, acrylic-based, silicone-based, UV curable, polyurethane-based, or cyanoacrylate-based adhesives.

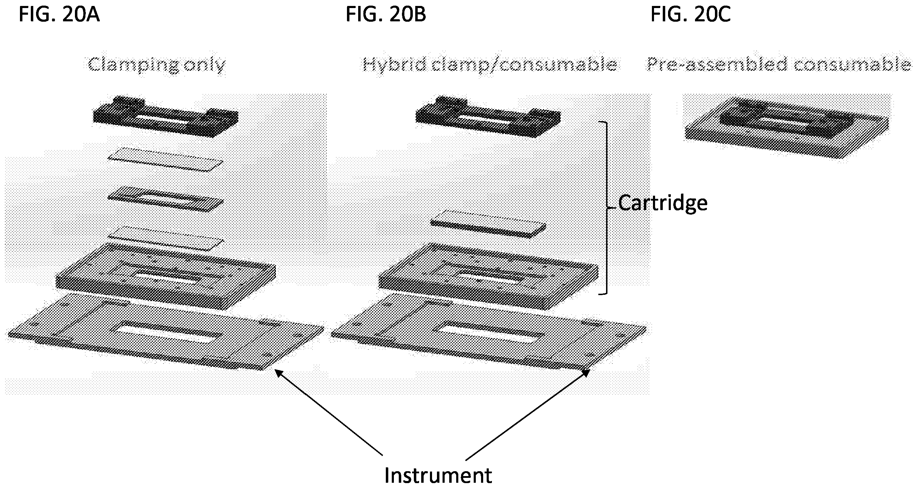

Cartridges: In many embodiments of the automated assay system, the microwell substrate, with or without an attached flow cell, will be packaged within a consumable cartridge that interfaces with the instrument system and which may incorporate additional functionality. FIGS. 14A-B and 20A-C illustrate a variety of approaches for incorporating flow cells (or microwell array substrates) into cartridge assemblies, where the approaches range from mechanical clamping of a stack of individual parts, to mechanical clamping of individual parts and pre-assembled components, to fully-assembled consumable cartridges that provide ease-of-use for end-users of the automated assay system. Design features of cartridges may include (i) one or more inlet ports for creating fluid connections with the instrument or manually introducing cell samples, bead suspensions, or other assay reagents into the cartridge, (ii) one or more bypass channels, i.e. for self-metering of cell samples and bead suspensions, to avoid overfilling or back flow, (iii) one or more integrated microwell substrate / flow cell assemblies, or one or more chambers within which the microarray substrate(s) are positioned, (iv) integrated miniature pumps or other fluid actuation mechanisms for controlling fluid flow through the device, (v) integrated miniature valves (or other containment mechanisms) for compartmentalizing pre-loaded reagents (for example, bead suspensions)or controlling fluid flow through the device, (vi) one or more vents for providing an escape path for trapped air, (vii) one or more sample and reagent waste reservoirs, (viii) one or more outlet ports for creating fluid connections with the instrument or providing a processed sample collection point, (ix) mechanical interface features for reproducibly positioning the removable, consumable cartridge with respect to the instrument system, and for providing access so that external magnets can be brought into close proximity with the microwells, (x) integrated temperature control components or a thermal interface for providing good thermal contact with the instrument system, (xi) optical alignment marks for determining the position of the cartridge or wells within the instrument, and (xii) optical interface features, e.g. a transparent window, for use in optical interrogation of the microwells. In some embodiments, the cartridge is designed to process more than one sample in parallel. In some embodiments of the device, the cartridge may further comprise one or more removable sample collection tube(s) or chamber(s) that are suitable for use in downstream assay procedures or for use in interfacing with stand-alone PCR thermal cyclers or sequencing instruments. In some embodiments of the device, the cartridge itself is suitable for interfacing with stand-alone PCR thermal cyclers or sequencing instruments. The term "cartridge" as used in this disclosure is meant to include any assembly of parts which contains the sample and beads during performance of the assay.

In some embodiments, the design of the cartridge may include a plurality of flow cells or individual microwell chambers, each comprising a plurality of microwells, such that a plurality of cell samples may be processed in parallel. In some embodiments, a plurality of microwell chambers within the cartridge may be used to divide a single cell sample into aliquots which are each analyzed separately. In some embodiments, a plurality of microwell chambers within the cartridge may be used to divide a single cell sample into aliquots, some of which may be used as controls, and some of which may be treated with an activating agent, stimulus, or test compound prior to performing the molecular barcoding assay.

FIGS. 21A-C illustrate one embodiment of a cartridge designed to include onboard reagent reservoirs. The cartridge is comprised of a microwell pattern 809 fabricated in substrate **2110,** a fluidic layer **2108,** a cartridge body **2106** comprising one or more onboard reagent reservoirs **2107,** and one or more reagent reservoir seals **2105,** which may optionally include vents, as shown in the exploded assembly view on the right. The assembled cartridge is shown on the left, which illustrates inlet **2101** and outlet **2102** ports, a relief **2103** or providing access by a retrieval magnet, and the sealed reservoirs **2104,** which may be visible if sealed with a transparent or semi-transparent seal or cover.

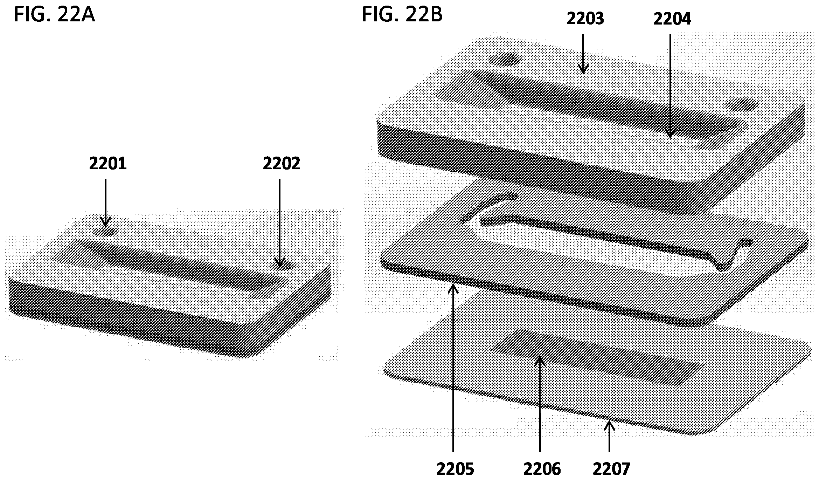

FIGS. 22A-B illustrate another embodiment of a cartridge designed to include onboard reagent reservoirs. In this embodiment, the reagent reservoirs are hidden from view by virtue of being completely enclosed by the cartridge body. The cartridge is comprised of a microwell pattern **2206** fabricated in substrate **2207,** a fluidic layer **2205,** and a cartridge body **2203** comprising one or more onboard reagent reservoirs and a relief 904 for providing access by a retrieval magnet, as shown in the exploded assembly view on the right. The assembled cartridge is shown on the left, which illustrates inlet **2201** and outlet **2202** ports, as well as the relief for providing access by a retrieval magnet.

*Cartridge fluid channel dimensions:* In general, the dimensions of fluid channels and the microwell chamber(s) in cartridge designs will be optimized to (i) provide uniform delivery of cells and beads to the microwells, and (ii) to minimize sample and reagent consumption. In some embodiments, the width of fluid channels will be between 50 um and 20 mm. In other embodiments, the width of fluid channels or microwell chambers may be at least 50 $\mu$m, at least 100 $\mu$m, at least 200 $\mu$m, at least 300 $\mu$m, at least 400 $\mu$m, at least 500 $\mu$m, at least 750 $\mu$m, at least 1 mm, at least 2.5 mm, at least 5 mm, at least 10 mm, or at least 20 mm. In yet other embodiments, the width of fluid channels or microwell chambers may at most 20 mm, at most 10 mm, at most 5 mm, at most 2.5 mm, at most 1 mm, at most 750 $\mu$m, at most 500 $\mu$m, at most 400 $\mu$m, at most 300 $\mu$m, at most 200 $\mu$m, at most 100 $\mu$m, or at most 50 $\mu$m. In one embodiment, the width of fluid channels or microwell chambers is about 2 mm. Those of skill in the art will appreciate that the width of the fluid channels or microwell chambers may fall within any range bounded by any of these values (*e.g.* from about 250 $\mu$m to about 10 mm).

In some embodiments, the depth of the fluid channels in cartridge designs will be between 50 $\mu$m and 4 mm. In other embodiments, the depth of fluid channels may be at least 50 $\mu$m, at least 100 $\mu$m, at least 200 $\mu$m, at least 300 $\mu$m, at least 400 $\mu$m, at least 500 $\mu$m, at least 750 $\mu$m, at least 1 mm, at least 1.25 mm, at least 1.5 mm, at least 1.75 mm, at least 2 mm, at least 3 mm, at least 4 mm, or at least 5 mm. In yet other embodiments, the depth of fluid channels may be at most 5 mm, at most 4 mm, at most 3 mm, at most 2 mm, at most 1.75 mm, at most 1.5 mm, at most 1.25 mm, at most 1 mm, at most 750 $\mu$m, at most 500 $\mu$m, at most 400 $\mu$m, at most 300 $\mu$m, at most 200 $\mu$m, at most 100 $\mu$m, or at most 50 $\mu$m. In one embodiment, the depth of the fluid channels is about 1 mm. Those of skill in the art will appreciate that the depth of the fluid channels may fall within any range bounded by any of these values (*e.g.* from about 800 $\mu$m to about 3 mm).

*Cartridge fabrication:* Cartridges may be fabricated using a variety of techniques and materials known to those of skill in the art. In general, the cartridges will be fabricated as a series of separate component parts (FIGS. 21A-C and 22A-B) and subsequently assembled using any of a number of mechanical assembly or bonding techniques. Examples of suitable fabrication techniques include, but are not limited to, conventional machining, CNC machining, injection molding, thermoforming, and 3D printing. Once the cartridge components have been fabricated they may be mechanically assembled using screws, clips, and the like, or permanently bonded using any of a variety of techniques (depending on the choice of materials used), for example, through the use of thermal bonding/welding or any of a variety of adhesives or adhesive films, including epoxy-based, acrylic-based, silicone-based, UV curable, polyurethane-based, or cyanoacrylate-based adhesives.

Cartridge components may be fabricated using any of a number of suitable materials, including but not limited to silicon, fused-silica, glass, any of a variety of polymers, e.g. polydimethylsiloxane (PDMS; elastomer), polymethylmethacrylate (PMMA), polycarbonate (PC), polystyrene (PS), polypropylene (PP), polyethylene (PE), high density polyethylene (HDPE), polyimide, cyclic olefin polymers (COP), cyclic olefin copolymers (COC), polyethylene terephthalate (PET), epoxy resins, non-stick materials such as teflon (PTFE), metals (*e.g.* aluminum, stainless steel, copper, nickel, chromium, and titanium), or any combination thereof. In some embodiments, the material(s) chosen for fabrication of flow cells will be ethanol compatible such that priming the fluidic device with ethanol or ethanol:water solutions may be used to facilitate surface wetting and removal of trapped air from the device.

*Cartridge diffusion barriers:* In some embodiments of the device, the flow cell or cartridge may further comprise components that are designed to create physical or chemical barriers that prevent diffusion of (or increase pathlengths and diffusion times for) large molecules in order to minimize cross-contamination between microwells. Examples of such barriers include, but are not limited to, a pattern of serpentine channels used for delivery of cells and beads to the microwells, a retractable platen or deformable membrane that is pressed into contact with the surface of the microwell substrate during lysis or incubation steps, the use of larger beads, *e.g.* Sephadex beads as described previously, to block the openings of the microwells, or the release of an immiscible, hydrophobic fluid from a reservoir within the cartridge during lysis or incubation steps, to effectively separate and compartmentalize each microwell in the substrate. In some embodiments, additives may be used to adjust the viscosity of the assay buffer in order to reduce the rate of diffusion of substances between microwells. Examples of buffer additives that may be used to adjust buffer viscosity include, but are not limited to sucrose, polyethylene glycol (PEG), Ficoll, glycerin, glycerol, dextran sulfate, histopaque, bovine serum albumin or other proteins, and the like.

*Cartridges comprising pumps:* As indicated above, in some embodiments the cartridge may include integrated miniature pumps or other fluid actuation mechanisms for control of fluid flow through the device. Examples of suitable miniature pumps or fluid actuation mechanisms include, but are not limited to, electromechanically- or pneumatically-actuated miniature syringe or plunger mechanisms, membrane diaphragm pumps actuated pneumatically or by an external piston, pneumatically-actuated reagent pouches or bladders, or electro-osmotic pumps.

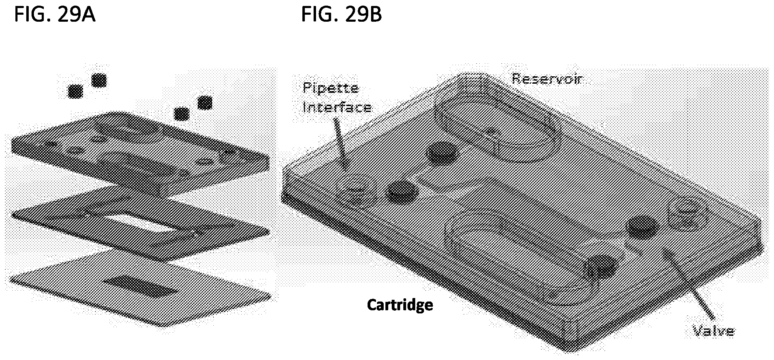

*Cartridges comprising valves:* As described above, in some embodiments the cartridge may include miniature valves for compartmentalizing pre-loaded reagents or controlling fluid flow through the device. Examples of suitable miniature valves include, but are not limited to, one-shot "valves" fabricated using wax or polymer plugs that can be melted or dissolved, or polymer membranes that can be punctured; pinch valves constructed using a deformable membrane or tube and pneumatic, magnetic, electromagnetic, or electromechanical (solenoid) actuation, one-way valves constructed using deformable membrane flaps, and miniature gate valves. In some embodiments, all of the inlets and outlets of the cartridge may include integrated check valves for controlling the directionality of fluid flow, as indicated in FIGS. 29A-B. In some embodiments, the integrated check valves may comprise miniature "duckbill" molded silicone check valves, for example, the Minivalve DU039.005 (Minivalve, Inc., Cleveland, OH).

*Cartridges comprising vents:* As indicated above, in some embodiments the cartridge may include vents for providing an escape path for trapped air. Vents may be constructed according to a variety of techniques known to those of skill in the art, for example, using a porous plug of polydimethylsiloxane (PDMS) or other hydrophobic material that allows for capillary wicking of air but blocks penetration by water.

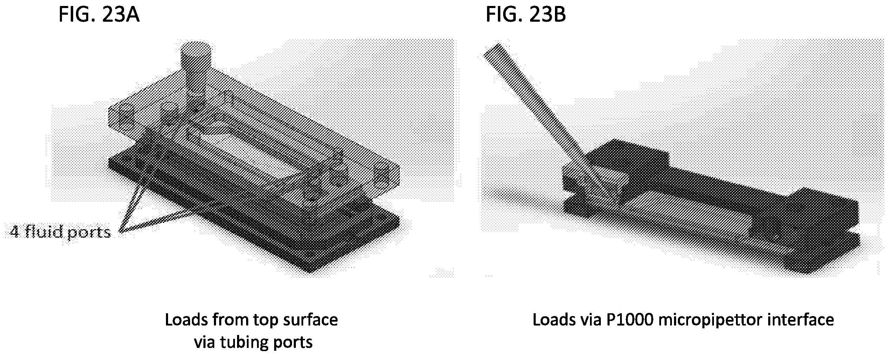

*Cartridge connections:* As described above, the inlet and outlet features of the cartridge may be designed to provide convenient and leak-proof fluid connections with the instrument, or may serve as open reservoirs for manual pipetting of samples and reagents into or out of the cartridge. Examples of convenient mechanical designs for the inlet and outlet port connectors include, but are not limited to, threaded connectors, Luer lock connectors, Luer slip or "slip tip" connectors, press fit connectors, tubing connectors, and the like (FIG. 23A).

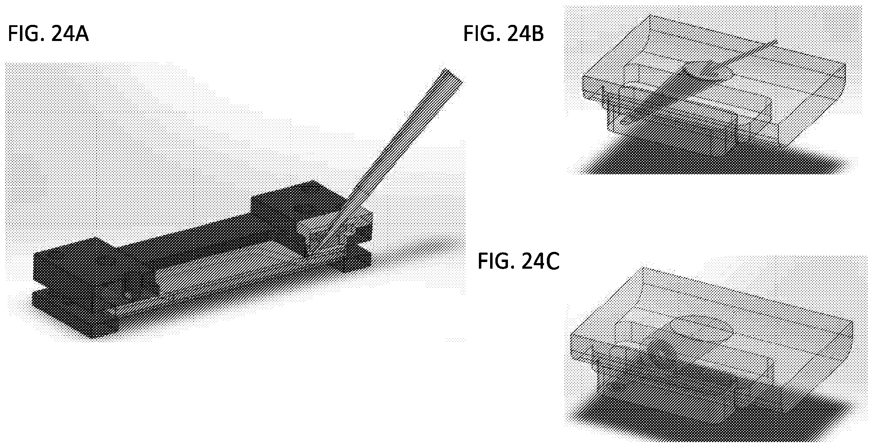

*Cartridge micropipette interface:* Examples of micropipettor interfaces (or "pipette tip interfaces") for facilitating the introduction of fluids into the flow cell device or cartridge using standard micropipettors are illustrated in FIG. 23B and FIGS. 24A-C. The interfaces are designed to provide a reliable seal between a pipette tip and the flow cell device or cartridge, thereby minimizing dead-volume and introduction of air bubbles, and providing for consistent filling using manual or automated pipettes (*e.g.* 1 ml and 5 ml Eppendorf pipette tips, or 5 ml Tecan pipette tips). The pipette tip interface also minimizes the number of transfer steps required to completely exchange fluids, and additionally, simplifies fluid handling by eliminating fluid flow into and out of the cartridge when a pipette tip is not inserted into the inlet port.

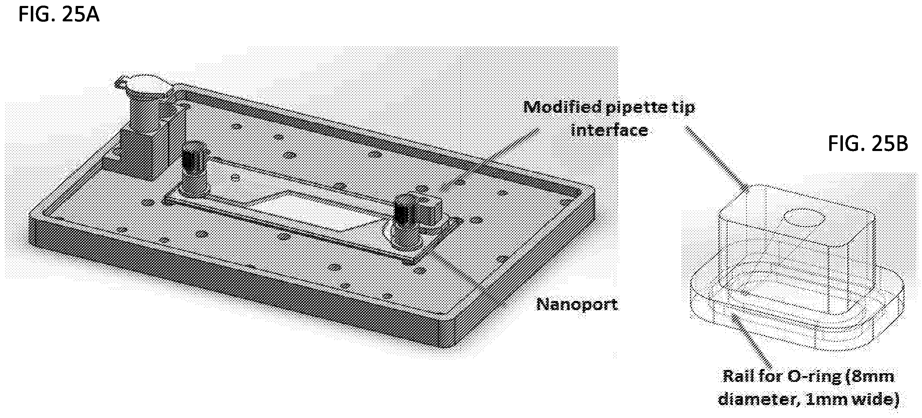

In some embodiments, the pipette tip interface comprises a straight or vertical feature that may be used to avoid handedness differences in users. In some embodiments, the pipette tip interface comprises an angled, conical feature that mates with or conforms to the shape of a pipette tip. In some embodiments, pre-cut pipette tips may be glued in place within angled conical inlet(s) to reduce contact with ethanol during priming steps. In some embodiments, the pipette tip interface comprises a conical feature that mates to a pipette tip to form a low dead-volume connection with an inlet port or outlet port of a flow cell device or cartridge. In some embodiments, the conical features or angled inlet(s) may incorporate features such as O-rings (FIG. 24B) or other rigid or compliant internal features (FIG. 24C) against which the pipette tip mates to form a substantially leak-proof seal. In some embodiments, the conical features or angled inlets are comprised of a compliant material that forms a substantially leak-proof seal with the pipette tip. In some embodiments, the compliant material is polydimethylsiloxane (PDMS), polyisoprene, polybutadiene, or polyurethane. In some embodiments, the pipette tip interface comprises a modular unit which can be modified independently from the rest of the cartridge design (FIG. 25B).

*Cartridge connector caps:* In some embodiments, the inlet and outlet ports of the cartridge may further comprise caps, spring-loaded covers or closures, or polymer membranes that may be opened or punctured when the cartridge is positioned in the instrument, and which serve to prevent contamination of internal cartridge surfaces during storage or which prevent fluids from spilling when the cartridge is removed from the instrument.

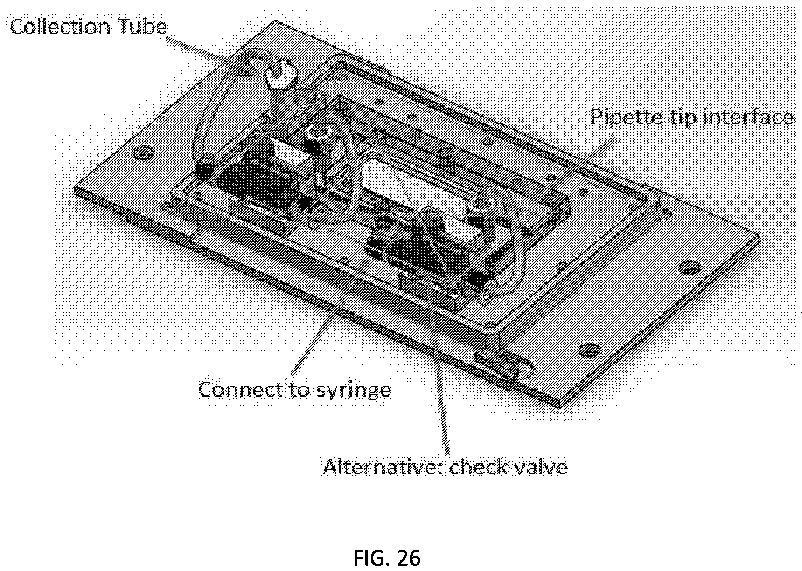

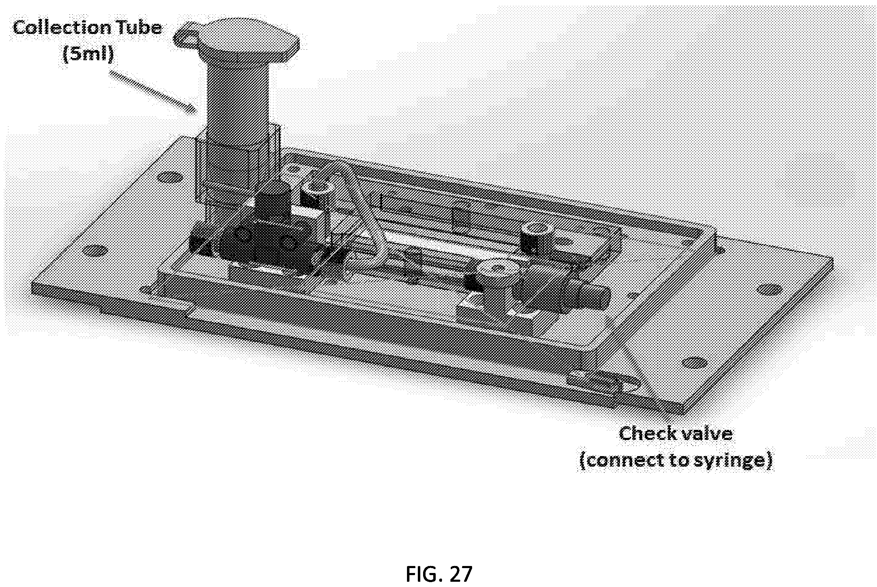

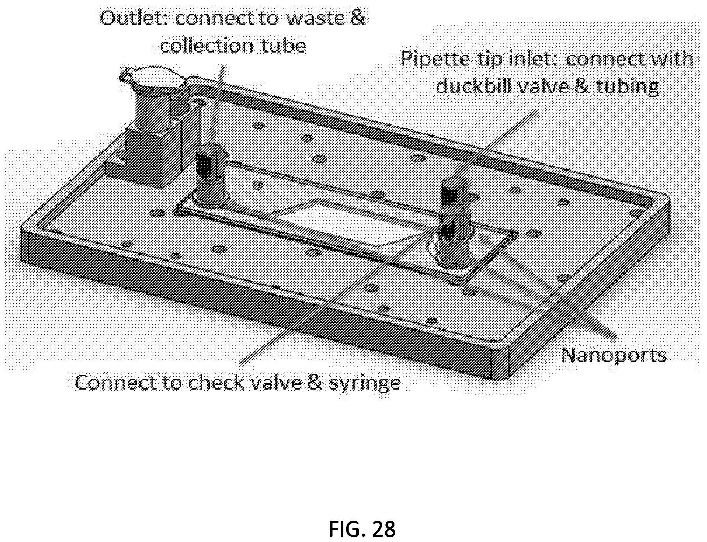

*Removable sample collection tubes:* As indicated above, in some embodiments the one or more outlet ports of the cartridge may further comprise removable sample collection tubes or chambers that are suitable for use in downstream assay procedures or for interfacing with stand-alone PCR thermal cyclers or sequencing instruments. FIGS. 25-28 illustrate different cartridge designs that incorporate sample collection tubes. FIG. 26 illustrates a cartridge assembly that incorporates a Luer lock syringe connector and check valve, as well as a pipette tip interface and a sample collection tube that stores beads after their retrieval from the microwell array. FIG. 27 ilustrates a variation of the cartridge design shown in FIG. 26. FIGS. 25 and 28 illustrate cartridge assemblies that incorporate Upchurch Nanoport™ connectors (Upchurch Scientific, Division of IDEX Health and Science, Oak Harbor, WA) to create low dead volume connections between external tubing and the cartridge. The cartridge designs also incorporate syringe connectors, check valves, and optional pipette tip interfaces.

Any of a variety of different sample collection tubes may be incorporated into the design, *e.g.* a 5 ml sample collection tube as shown, or sample collection tubes of smaller volume, *e.g.* 1.5 ml. In general, the volume of the sample collection tube may range from about 10 $\mu$l to about 5 ml. In some embodiments, the volume of the one or more sample collection tubes may be at least 10 $\mu$l, at least 25 $\mu$l, at least 50 $\mu$l, at least 75 $\mu$l, at least 100 $\mu$l, at least 250 $\mu$l, as least 500 $\mu$l, at least 750 $\mu$l, at least 1 ml, at least 2 ml, at least 3 ml, at least 4 ml, or at least 5 ml. In some embodiments, the volume of the one or more sample collection tubes may be at most 5 ml, at most 4 ml, at most 3 ml, at most 2 ml, at most 1 ml, at most 750 μl, at most 500 μl, at most 250 μl, at most 100 μl, at most 75 μl, at most 50 μl, at most 25 μl, or at most 10 μl. Those of skill in the art will recognize that the volume of the one or more sample collection tubes may vary independently of one another and may have volumes of any value within the above specified range of volumes, for example, about 900 μl.

*Cartridge mechanical interface:* In general, the mechanical interface features of the cartridge provide for easily removable but highly precise and repeatable positioning of the cartridge relative to the instrument system. Suitable mechanical interface features include, but are not limited to, alignment pins, alignment guides, mechanical stops, and the like. In some embodiments, the mechanical design features will include relief features for bringing external apparatus, *e.g.* magnets or optical components, into close proximity with the microwell chamber (FIG. 21A, FIG. 22B).

*Cartridge temperature control or thermal interface:* In some embodiments, the cartridge will also include temperature control components or thermal interface features for mating to external temperature control modules. Examples of suitable temperature control elements include, but are not limited to, resistive heating elements, miniature infrared-emitting light sources, Peltier heating or cooling devices, heat sinks, thermistors, thermocouples, and the like. Thermal interface features will typically be fabricated from materials that are good thermal conductors (*e.g.* copper, gold, silver, *etc.*) and will typically comprise one or more flat surfaces capable of making good thermal contact with external heating blocks or cooling blocks.

*Cartridge optical interface:* In many embodiments, the cartridge will include optical interface features for use in optical imaging or spectroscopic interrogation of the microwell array. Typically, the cartridge will include an optically transparent window, *e.g.* the microwell substrate itself or the side of the flow cell or microwell chamber that is opposite the microwell substrate, fabricated from a material that meets the spectral requirements for the imaging or spectroscopic technique used to probe the microwells. Examples of suitable optical window materials include, but are not limited to, glass, fused-silica, polymethylmethacrylate (PMMA), polycarbonate (PC), cyclic olefin polymers (COP), or cyclic olefin copolymers (COC).

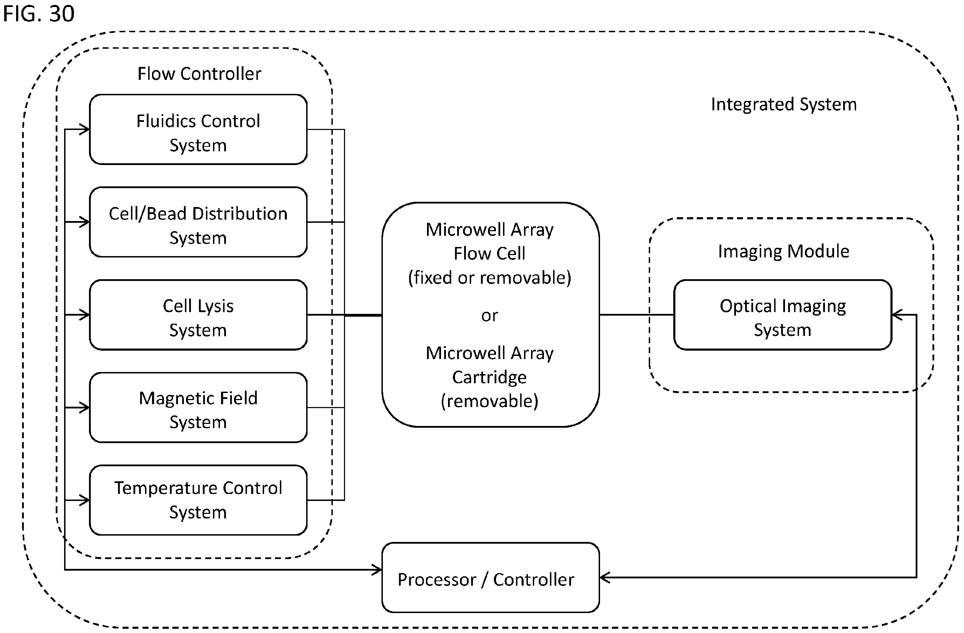

*Instrument modules & systems:* The present disclosure also includes instrument modules and systems for use in the automation of multiplexed, single cell stochastic labeling or molecular barcoding assays. As indicated above, these instruments may provide control and analysis functionality such as (i) fluidics control, (ii) cell or bead distribution and collection mechanisms, (iii) cell lysis mechanisms, (iv) magnetic field control, (v) temperature control, (vi) imaging capability, and (vii) image processing. In some embodiments, the instrument system may comprise one or more modules (illustrated schematically in FIG. 30), where each module provides one or more specific functional feature sets to the system. In other embodiments, the instrument system may be packaged such that all system functionality resides within one or more packages (FIG. 30; inner set of dashed lines) or within the same package (FIG. 30; outer dashed line). As indicated above, in some embodiments, the system may comprise additional functional units, either as integrated components or as modular components of the system, that expand the functional capabilities of the system to include PCR amplification (or other types of oligonucleotide amplification techniques) and oligonucleotide sequencing.

FIG. 31 illustrates one non-limiting example of the instrument system configuration. In FIG. 31, the user pipettes a cell sample into the inlet port or sample well of a removable cartridge that is preloaded with all other assay reagents, inserts the cartridge into the instrument system for processing, and collects the output (*e.g.* a bead suspension comprising libraries of labeled oligonucleotides) from an outlet port or well of the cartridge. The instrument system automates the assay steps, including distribution of cells into the microwells, distribution of beads from an onboard reagent well (if not already pre-loaded into the microwells), rinse steps, cell lysis steps, hybridization steps for RNA or DNA targets, and magnet-assisted bead retrieval. In some embodiments, the instrument system further comprises imaging and analysis capability, and real-time feedback and control of some assay steps, for example cell and bead distribution steps to ensure optimal coverage of the microwell pattern while minimizing the number of wells that contain more than one cell or more than one bead. In some embodiments, the imaging system provides for optical monitoring of the cells in microwells to identify specified subsets of cells, *e.g.* dead cells, cells exhibiting specific cell surface markers, *etc.,* and then provides feedback used to enable mechanisms for physically removing, trapping, or destroying selected cells (or co-localized beads) so that they may be included or excluded from downstream analysis of nucleic acid sequence data. In many embodiments, the instrument system includes an embedded computer or processor (although a peripheral computer or processor may be used in some embodiments) that runs software for controlling and coordinating the activities of imaging, motion control, magnetic control, fluidics control (*e.g.* application of pressure or vacuum to fluid lines), and other functional subsystems.

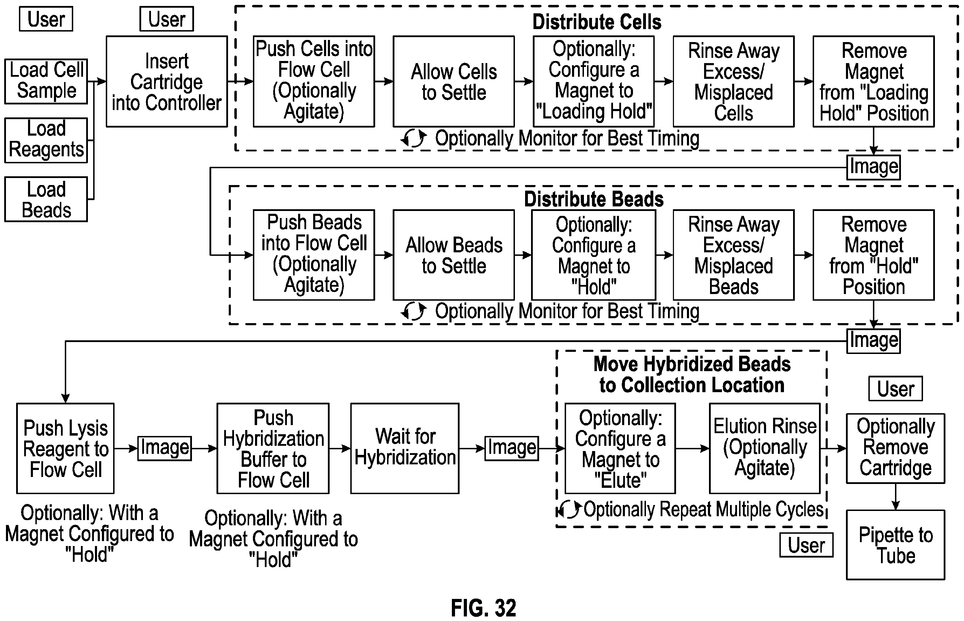

*Automated assay process steps & workflow:* FIGS. 32-35 provide schematic illustrations of different embodiments for the process steps performed by the user and automated assay system.

FIG. 32 illustrates one embodiment of the automated assay workflow in which a consumable assay cartridge containing no beads or other reagents is provided to the user. The user loads beads and reagents into the assay cartridge (including a lysis buffer and a hybridization buffer) at same time as the cell sample. The instrument system automates the steps of distributing cells and beads into the microwells. In some embodiments, the imaging system and real-time image processing and analysis is used to monitor the cell and bead distribution processes and feedback is used to adjust process steps accordingly, *e.g.* by prolonging or repeating some steps, by activating alternative cell or bead distribution mechanisms, and the like. The instrument system then performs cell lysis and hybridization steps in sequence, followed by magnetic field-assisted retrieval of the beads from the microwells. At the end of the process, the output sample (*e.g.* a bead suspension comprising libraries of labeled oligonucleotides) is collected from a port on the assay cartridge. In some embodiments of the assay cartridge design, the outlet port of the assay cartridge may comprise a snap-off tube for convenience.

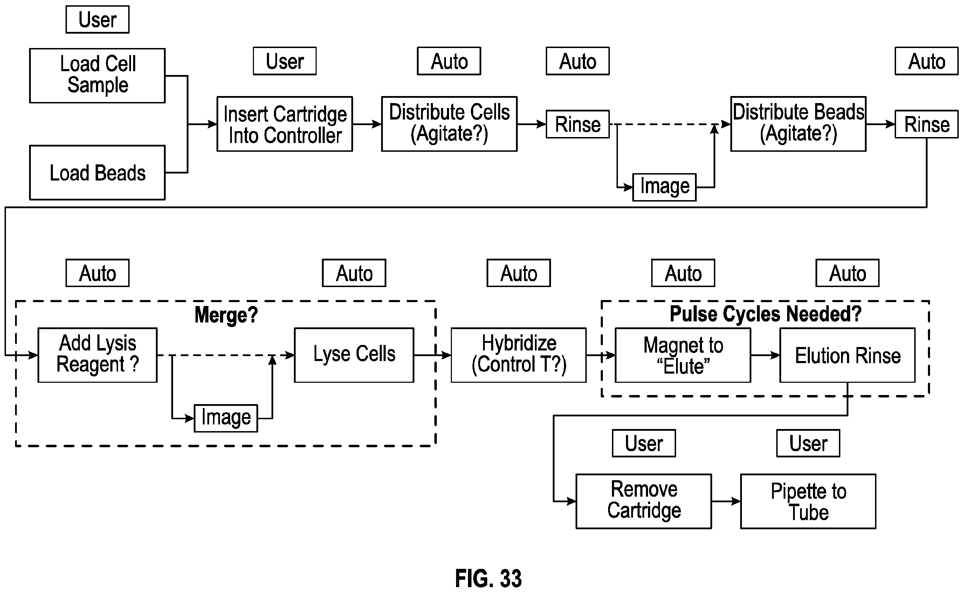

FIG. 33 illustrates another embodiment of the automated assay workflow in which a consumable assay cartridge that comprises pre-loaded assay reagents is supplied to the user. The user loads cell samples and beads into the cartridge, and the instrument system performs the tasks of distributing the cells and the beads across a plurality of microwells, as well as additional assay steps as outlined above. In some embodiments, the cells may be distributed into microwells first, followed by distribution of beads. In other embodiments, the beads may be distributed first, followed by distribution of cells.

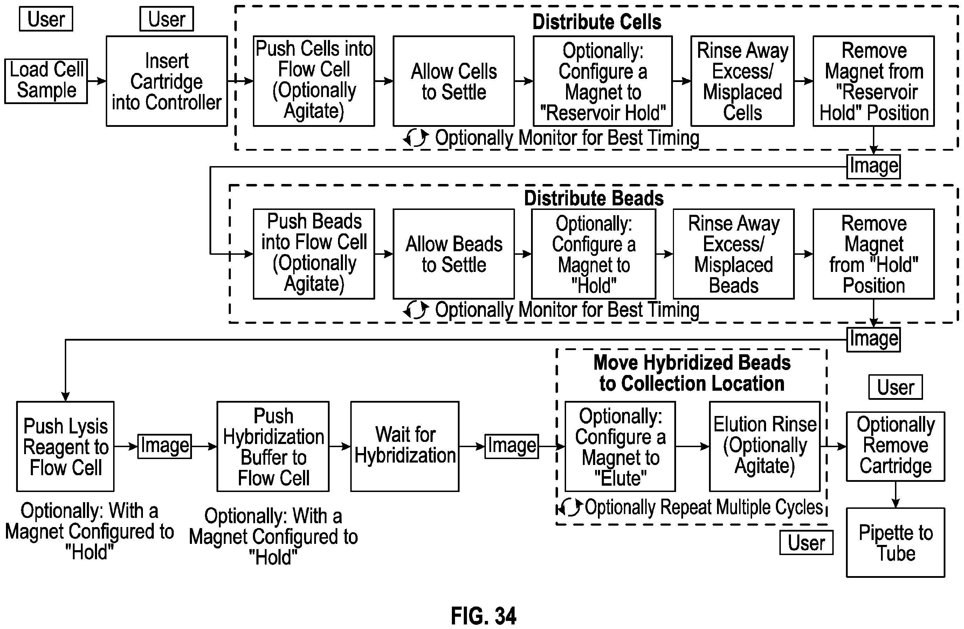

FIG. 34 illustrates another embodiment of the automated assay workflow in which a consumable assay cartridge is supplied to the user with beads and reagents pre-loaded in reagent reservoirs within the cartridge. In some embodiments, the beads and reagents may be contained within the instrument, *e.g.* within replaceable bottles or reagent cartridges, rather than in the assay cartridge. As described above, in some embodiments, the cells may be distributed into microwells first, followed by distribution of beads. In other embodiments, the beads may be distributed first, followed by distribution of cells.

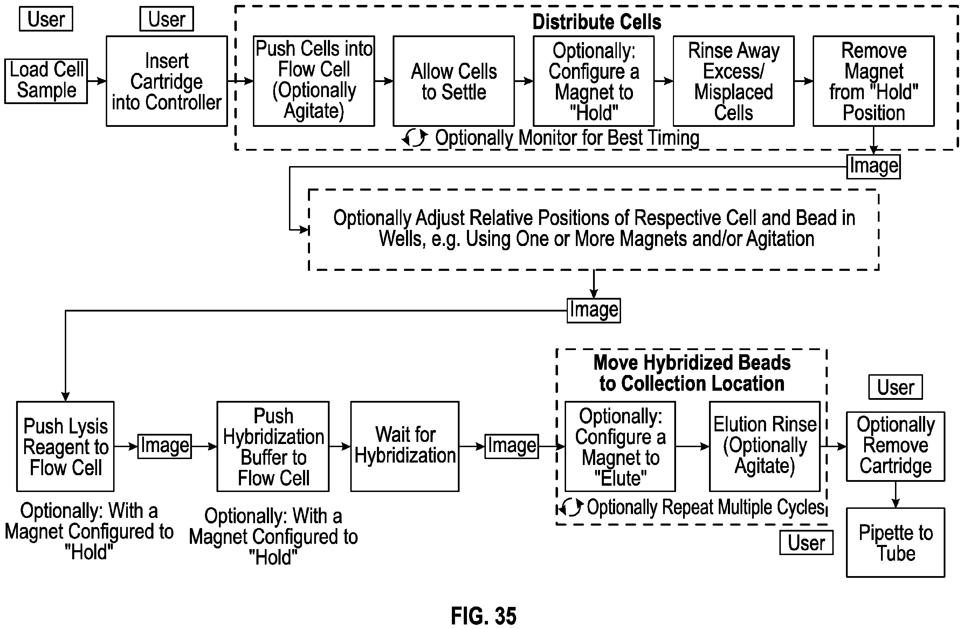

FIG. 35 illustrates yet another embodiment of the automated assay process in which a consumable assay cartridge is supplied to the user which has beads preloaded in the microwells and other assay reagents preloaded in the reagent reservoirs of the cartridge. Following the distribution of cells into the microwells (where again, the imaging system and real-time image processing is used to monitor the cell distribution process, and feedback is used to adjust process steps accordingly to achieve a pre-specified distribution), the relative positions of cells and beads may be adjusted, for example by using one or more magnets to manipulate local magnetic fields and/or using agitation.

*Fluidics:* In general, the instrument system will provide fluidics capability for delivering samples or reagents to the one or more microwell chamber(s) or flow cell(s) within one or more assay cartridge(s) connected to the system. Assay reagents and buffers may be stored in bottles, reagent and buffer cartridges, or other suitable containers that are connected to the cartridge inlets. In some embodiments, assay reagents and buffers may be pre-loaded and stored in reservoirs located within the cartridge itself. The system may also include processed sample and waste reservoirs in the form of bottles, cartridges, or other suitable containers for collecting fluids downstream of the assay cartridge(s). In some embodiments, processed samples and waste fluids may be collected in reservoirs located within the cartridge itself. In some embodiments, the fluidics module may provide switching of flow between different sources, *e.g.* sample or reagent reservoirs located on the cartridge, or reagent bottles located in the instrument, and the microwell chamber inlet(s). In some embodiments, the fluidics module may provide for contacting the cells in the array with an activating agent, chemical stimulus, or test compound at a specified, adjustable time prior to performing cell lysis and downstream assay steps. In some embodiments, the fluidics module may provide switching of flow between the microwell chamber outlet(s) and different collection points, *e.g.* processed sample reservoirs located within the cartridge, waste reservoirs located within the cartridge, or waste bottles located within the instrument.

*Flow control using pumps & valves:* Control of fluid flow through the system will typically be performed through the use of pumps (or other fluid actuation mechanisms) and valves. Examples of suitable pumps include, but are not limited to, syringe pumps, programmable syringe pumps, peristaltic pumps, diaphragm pumps, and the like. In some embodiments, fluid flow through the system may be controlled by means of applying positive pneumatic pressure at the one or more inlets of the reagent and buffer containers, or at the inlets of the assay cartridge(s). In some embodiments, fluid flow through the system may be controlled by means of drawing a vacuum at the one or more outlets of the waste reservoirs, or at the outlets of the assay cartridge(s). Examples of suitable valves include, but are not limited to, check valves, electromechanical two-way or three-way valves, pneumatic two-way and three-way valves, and the like.

*Fluid flow modes:* Different modes of fluid flow control may be utilized at different points in the assay procedure, *e.g.* forward flow (relative to the inlet and outlet for a given microwell chamber), reverse flow, oscillating or pulsatile flow, or combinations thereof, may all be used. In some embodiments, oscillating or pulsatile flow may be used, for example, during microwell loading steps to facilitate uniform distribution of cells and beads. In some embodiments, oscillating or pulsatile flow may be applied during assay wash/rinse steps to facilitate complete and efficient exchange of fluids within the one or more microwell flow cell(s) or chamber(s).

Different fluid flow rates may be utilized at different points in the assay process workflow, for example, in some embodiments of the disclosed instrument modules and system, the volumetric flow rate may vary from -100 ml/sec to +100 ml/sec. In some embodiment, the absolute value of the volumetric flow rate may be at least 0.001 ml/sec, at least 0.01 ml/sec, at least 0.1 ml/sec, at least 1 ml/sec, at least 10 ml/sec, or at least 100 ml/sec. In some embodiments, the absolute value of the volumetric flow rate may be at most 100 ml/sec, at most 10 ml/sec, at most 1 ml/sec, at most 0.1 ml/sec, at most 0.01 ml/sec, or at most 0.001 ml/sec. The volumetric flow rate at a given point in time may have any value within this range, *e.g.* a forward flow rate of 2.5 ml/sec, a reverse flow rate of -0.05 ml/sec, or a value of 0 ml/sec (*i.e.* stopped flow).

*Air injection:* In some embodiments of the fluidics system, it may be advantageous to insert injections of air between injections of solution when changing from one solution to another, *e.g.* between priming of the flow cell and injection of a cell suspension, or between a rinse buffer step and injection of a bead suspension. Potential advantages of this approach include reduced dispersion (by eliminating liquid/liquid interfaces), and reduced sample and reagent consumption (less fluid volume required to fill or empty the flow cell).

In some embodiments, air can be injected into the flow cell itself (*e.g.,* comprising the fluidic layer). In some embodiments, air can be injected into the space in the flow cell above the microwell array (*e.g.,* a microwell chamber). In some embodiments, injection of air may not substantially remove the contents of the microwells in the microwell array. In some embodiments, injection of air may remove at most 1, 2, 3, 4, 5, 6, 7, 8, 9, or 10% or more of the contents of the microwells of the microwell array.

Injection of air may be used to create a uniform environment for subsequent injections (*e.g.,* loading) of liquids (*e.g.,* comprising a cell or bead suspension). In some embodiments, loading of liquids after air injection can be at least 10, 20, 30, 40, 50, 60, 70, 80, 90 or 100% more uniformly dispersed than loading without prior air injection. In some embodiments, loading of liquids after air injection can be at most 10, 20, 30, 40, 50, 60, 70, 80, 90 or 100% more uniformly dispersed than loading without prior air injection.

Injection of air can reduce the dead volume (or dead space) in the flow cell and/or microwell array. In some embodiments, dead volume can be reduced with injection or air by at least 10, 20, 30, 40, 50, 60, 70, 80, 90 or 100%. In some embodiments, dead volume can be reduced with injection or air by at most 10, 20, 30, 40, 50, 60, 70, 80, 90 or 100%.

In some embodiments, injection of air may be performed using an automated pipette, a syringe pump, or the like. In some embodiments, injection of air may be performed at a rate ranging between 0.08 ml per second to 1.8 ml per second. In some embodiments, the rate of air injection is at least 0.08 ml per second, at least 0.1 ml per second, at least 0.2 ml per second, at least 0.3 ml per second, at least 0.4 ml per second, at least 0.5 ml per second, at least 0.6 ml per second, at least 0.7 ml per second, at least 0.8 ml per second, at least 0.9 ml per second, at least 1.0 ml per second, at least 1.2 ml per second, at least 1.4 ml per second, at least 1.6 ml per second, or at least 1.8 ml per second. In some embodiments, the rate of air injection is at most 1.8 ml per second, at most 1.6 ml per second, at most 1.4 ml per second, at most 1.2 ml per second, at most 1.0 ml per second, at most 0.8 ml per second, at most 0.6 ml per second, at most 0.4 ml per second, at most 0.2 ml per second, at most 0.1 ml per second, or at most 0.08 ml per second. Those of skill in the art will recognize that the rate of air injection may have any value within this range, *e.g.* about 1.25 ml per second. In some instances, the injection rate is about 0.36 ml per second.

In some embodiments, the pressure of injection of air may be between 0.01 and 0.25 atm. In some embodiments, the rate of air injection is at least 0.01 atm, at least 0.05 atm, at least 0.10 atm, at least 0.15 atm, at least 0.2 atm, or at least 0.25 atm. In some embodiments, the rate of air injection is at most 0.25 atm, at most 0.2 atm, at most 0.15 atm, at most 0.1 atm, at most 0.05 atm, or at most 0.01 atm. Those of skill in the art will recognize that the pressure of air injection may have any value within this range, *e.g.* about 0.11 atm.

*Cell and bead distribution mechanisms:* As indicated above, in some embodiments the instrument system may include mechanisms for distributing and further facilitating the uniform distribution of cells and beads over the plurality of microwells. Examples of such mechanisms include, but are not limited to, magnetic transport, rocking, shaking, swirling, recirculating flow, oscillatory or pulsatile flow, low frequency agitation (for example, through pulsing of a flexible (*e.g.* silicone) membrane that forms a wall of the chamber or nearby fluid channel), or high frequency agitation (for example, through the use of piezoelectric transducers). In some embodiments, one or more of these mechanisms is utilized in combination with physical structures or features on the interior walls of the flow cell or microwell chamber, *e.g.* mezzanine/top hat structures, chevrons, or ridge arrays, to facilitate mixing or to help prevent pooling of cells or beads within the array chamber. Flow-enhancing ribs on upper or lower surfaces of the flow cell or microwell chamber may be used to control flow velocity profiles and reduce shear across the microwell openings (*i.e.* to prevent cells or beads from being pulled out of the microwells during reagent exchange and rinse steps.

*Cell and bead distribution targets:* When distributing beads amongst a plurality of microwells, any of a variety of pre-determined levels may be targeted. For example, in some embodiments, the percentage of microwells that contain a single bead may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single bead.

When distributing beads amongst a plurality of microwells, in some embodiments, the percentage of microwells that contain two beads may be at least 1%, at least 2%, at least 3%, at least 4%, at least 5%, at least 6%, at least 7%, at least 8%, at least 9%, or at least 10% or more. In other embodiments, the percentage of microwells that contain two beads may be at most 10%, at most 9%, at most 8%, at most 7%, at most 6%, at most 5%, at most 4%, at most 3%, at most 2%, or at most 1%.

When distributing cells amongst a plurality of microwells, any of a variety of pre-determined levels may be targeted. For example, in some embodiments, the percentage of microwells that contain a single cell may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single cell. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single cell.

When distributing cells amongst a plurality of microwells, in some embodiments, the percentage of microwells that contain two cells may be at least 1%, at least 2%, at least 3%, at least 4%, at least 5%, at least 6%, at least 7%, at least 8%, at least 9%, or at least 10% or more. In other embodiments, the percentage of microwells that contain two cells may be at most 10%, at most 9%, at most 8%, at most 7%, at most 6%, at most 5%, at most 4%, at most 3%, at most 2%, or at most 1%.

When distributing both cells and beads amongst a plurality of microwells, any of a variety of pre-determined levels may be targeted. For example, in some embodiments, the percentage of microwells that contain both a single cell and a single bead may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain both a single cell and a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain both a single cell and a single bead.

Bead and/or cell distribution may be dependent on size and/or density. For example, larger beads and/or cells may settle (*i.e.,* into wells) at a faster rate than smaller beads and/or cells. For example, beads that are 33 micron in diameter may settle about 0.5, 1, 1.5, 2, 2.5, or 3 or more times faster than beads that are 22 microns in diameter (assuming equal or similar density). In some instances, beads that are 33 microns in diameter settle about 2.25 times faster than beads that are 22 micron in diameter.

Cells of different sizes and/or densities may settle at different rates into the wells of the substrate. For example, red blood cells may settle at least 0.5, 1, 1.5, 2, 2.5, or 3 or more times faster than white blood cells. Red blood cells may settle faster than white blood cells due to their higher density in spite of their smaller size.

In some embodiments, a buffer may be flowed over the microwell array before and/or after cells or beads have been loaded. The buffer can be a lysis buffer and/or a wash buffer. In many embodiments, flow of the buffer will not substantially remove the contents of the microwells. In some embodiments, flow of the buffer may remove the contents of at most 1%, at most 2%, at most 3%, at most 4%, at most 5%, at most 6%, at most 7%, at most 8%, at most 9%, or at most 10% or more microwells.

In some embodiments, the viscosity and/or density of a buffer may be adjusted to optimize the uniform loading of beads and/or cells into microwells. Varying the viscosity or density of the loading buffer may, for example, help protect cells from shear forces or provide positive or negative buoyancy to cells and/or beads to facilitate uniform loading. For example, in some embodiments of the disclosed methods the viscosity of a buffer used for loading beads and/or cells may range from about 1x to about 10x that of water. In some embodiments, the viscosity of the buffer may be at least 1x, at least 1.1x, at least 1.2x, at least 1.3x, at least 1.4x, at least 1.5x, at least 1.6x, at least 1.7x, at least 1.8x, at least 1.9x, at least 2x, at least 3x, at least 4x, at least 5x, at least 6x, at least 7x, at least 8x, at least 9x, or at least 10x or more times the viscosity of water. In some embodiments, the viscosity of the buffer may be at most 10x, at most 9x, at most 8x, at most 7x, at most 6x, at most 5x, at most 4x, at most 3x, at most 2x, at most 1.9x, at most 1.8x, at most 1.7x, at most 1.6x, at most 1.5x, at most 1.4x, at most 1.3x, at most 1.2x, at most 1.1x, or at most 1x that of water. Those of skill in the art will recognize that the buffer viscosity may have any value within this range, for example, about 1.75x that of water.

Similarly, in some embodiments, the density of a buffer used for loading beads and/or cells may range from about 0.8x to about 1.25x that of the density of the beads and/or cells to be loaded. In some embodiments, the density of a buffer used for loading beads and/or cells may be at least 0.8x, at least 0.9x, at least 1.0x, at least 1.1x, at least 1.2x, or at least 1.25x that of the beads and/or cells, or higher. In some embodiments, the density of a buffer used for loading beads and/or cells may be at most 1.25x, at most 1.2x, at most 1.1x, at most 1.0x, at most 0.9x, or at most 0.8x that of the beads and/or cells, or lower. Those of skill in the art will recognize that the density of the buffer used for loading beads and/or cells may have any value within this range, for example, about 0.85x that of the beads and/or cells.

In some embodiments, the viscosity and/or density of a buffer may be adjusted to optimize the efficiency of retrieving beads from microwells. Varying the viscosity or density of the bead retrieval buffer may, for example, provide viscous drag forces or provide positive or less negative buoyancy to beads to facilitate efficient bead retrieval. For example, in some embodiments of the disclosed methods the viscosity of a buffer used for bead retrieval may range from about 1x to about 10x that of water. In some embodiments, the viscosity of the buffer may be at least 1x, at least 1.1x, at least 1.2x, at least 1.3x, at least 1.4x, at least 1.5x, at least 1.6x, at least 1.7x, at least 1.8x, at least 1.9x, at least 2x, at least 3x, at least 4x, at least 5x, at least 6x, at least 7x, at least 8x, at least 9x, or at least 10x or more times the viscosity of water. In some embodiments, the viscosity of the buffer may be at most 10x, at most 9x, at most 8x, at most 7x, at most 6x, at most 5x, at most 4x, at most 3x, at most 2x, at most 1.9x, at most 1.8x, at most 1.7x, at most 1.6x, at most 1.5x, at most 1.4x, at most 1.3x, at most 1.2x, at most 1.1x, or at most 1x that of water. Those of skill in the art will recognize that the buffer viscosity may have any value within this range, for example, about 2.3x that of water.

Similarly, in some embodiments, the density of a buffer used for bead retrieval may range from about 0.8x to about 1.25x that of the density of the beads. In some embodiments, the density of a buffer used for bead retrieval may be at least 0.8x, at least 0.9x, at least 1.0x, at least 1.1x, at least 1.2x, or at least 1.25x that of the beads and/or cells, or higher. In some embodiments, the density of a buffer used for bead retrieval may be at most 1.25x, at most 1.2x, at most 1.1x, at most 1.0x, at most 0.9x, or at most 0.8x that of the beads and/or cells, or lower. Those of skill in the art will recognize that the density of the buffer used for bead retrieval may have any value within this range, for example, about 1.1x that of the beads.

Examples of buffer additives that may be used to adjust buffer viscosity and/or density include, but are not limited to sucrose, polyethylene glycol (PEG), Ficoll, glycerin, glycerol, dextran sulfate, histopaque, bovine serum albumin, and the like.

*Magnetic field-assisted bead transport & manipulation:* In some embodiments, cells or beads may be distributed among the microwells, removed from the microwells, or otherwise transported through a flow cell or cartridge of an instrument system by using magnetic beads (*e.g.* conjugated to antibodies directed against cell surface markers, or as solid supports for libraries of stochastic labels) and externally-applied magnetic field gradients. In some embodiments, for example when using magnetic fields to trap magnetic beads in microwells or to elute magnetic beads from microwells, an externally-applied magnetic field gradient may be applied to the entire microwell pattern simultaneously. In some embodiments, an externally-applied magnetic field gradient may be applied to a selected area of the microwell pattern. In some embodiments, an externally-applied magnetic field gradient may be applied to a single microwell. In some embodiments, permanent magnets may be used to apply time-varying magnetic field gradients by moving the position of one or more permanent magnets relative to the microwell array or vice versa. In these embodiments, the velocity of the relative motion may be adjusted to so that the time-dependence of the magnetic field gradient is matched to the timescale on which magnetic beads undergo magnetophoresis into or out of microwells. In some embodiment, time-varying magnetic fields may be provided by varying the current applied to one or more electromagnets. In some embodiments, a combination of one or more permanent magnets and one or more electromagnets may be used to provide magnetic field gradients for transporting magnetic beads into microwells, out of microwells, or through the device. In some embodiments, cells or beads may be distributed among the microwells, removed from the microwells, or otherwise transported through a flow cell or cartridge of an instrument system by means of centrifugation or other non-magnetic means.

In some embodiments, beads (solid supports) may be removed from the microwells using one or more magnetic fields. In some embodiments, beads may be removed after lysis of cells in the microwells and/or attachment of nucleic acids to the pluralities of oligonucleotides immobilized on the individual beads. A magnet can be place on top of the cartridge and beads may be removed from the wells using the resultant magnetic field. In some embodiments, at least 70%, at least 75%, at least 80%, at least 85%, at least 90%, at least 95%, or at least 100% of the beads may be removed. In some embodiments, at most 70%, at most 75%, at most 80%, at most 85%, at most 90%, at most 95%, or at most 100% of the beads may be removed.

*Real-time imaging & feedback to improve cell and/or bead distribution:* In some embodiments, an imaging system and real-time image processing and analysis is used to monitor the cell and bead distribution processes (*i.e.* the distribution of cells and/or beads within the plurality of microwells) and feedback is used to adjust process steps accordingly, *e.g.* by prolonging or repeating some steps, by activating alternative cell or bead distribution mechanisms, and the like, in order to improve cell and/or bead distributions, or to achieve pre-specified target distributions.

*Real-time imaging & feedback to select sub-populations of cells:* In some embodiments, real-time image processing and analysis may be used to identify wells containing cells exhibiting one or more specified characteristics (as described in more detail below), followed by selection or exclusion of a subset of cells from further analysis. In some embodiments, real-time image processing and analysis is used to identify wells containing two or more cells, followed by the exclusion of the cells in those wells from further analysis. Examples of mechanisms that may be used to select or exclude a subset of cells from further analysis (*e.g.* selection mechanisms) include, but are not limited to, (i) physical removal of selected cells or beads from the array, (ii) physical entrapment of selected cells or beads within the array, (iii) physical destruction of selected cells or beads within the array, or (iv) use of dual-encoding schemes whereby the sequence data that is generated for a given cell is selected for or excluded from further analysis.

One non-limiting example of a selection mechanism for physical removal of selected beads (wherein the beads are magnetic beads) from microwells (thereby preventing downstream sequence analysis of nucleic acid target molecules from the corresponding cell(s)) is the use of miniaturized magnetic probes (*e.g.* modified computer hard drive write heads) to pluck beads from wells containing cells that have been identified to match a pre-specified set of cellular characteristics. Modern hard drives can reach bit densities of over 800 Gbit/in$^2$, which corresponds to a bit area of 8.1 x $10^{-4}$ $\mu$m$^2$. Hard drive technology dating from the 1980s had bit areas of approximately 50 $\mu$m$^2$ or less, hence magnetic write head technology may be adapted in some embodiments of the disclosed devices and systems for the purpose of removing beads from specified wells.

Another example of a selection mechanism for physical removal of selected cells or beads from microwells is the use of micropipettes and micromanipulators. In embodiments where the microwells are accessible, *e.g.* through the use of a removable chamber wall or optical window, a micromanipulator may be used to position one or more micropipettes, extract selected cells or beads from microwells (*e.g.* by applying gentle suction to the one or more micropipettes), and moving them to a position in the cartridge or instrument (*e.g.* a reservoir) where they may be sequestered for subsequent disposal or subsequent processing and analysis. Commercially-available micromanipulators provide sub-micron step resolution for precise positioning, while commercially-available micropipette pullers permit fabrication of tip diameters of about 5 $\mu$m and smaller.

Another example of a selection mechanism for physical removal of selected cells or beads from microwells is the use of single light beam gradient force traps (*e.g.* "optical tweezers"). These apparatus use a highly focused laser beam to create an optical trap (*e.g.* by generating attractive or repulsive forces, depending on the mismatch in refractive index between an object and the surrounding medium) to physically hold and move microscopic dielectric objects. In some embodiments, optical tweezers may be used to extract selected cells or beads from microwells and move them, either directly using the optical tweezers or through the simultaneous control of fluid flow through the flow cell or microwell chamber, to a position in the cartridge (*e.g.* a reservoir) where they may be sequestered for subsequent disposal or subsequent processing and analysis.

Another example of a selection mechanism for physical removal of selected cells or beads from microwells may be the use of acoustic droplet ejection. Acoustic droplet ejection has been used for precision dispensing of small droplets of liquid (ranging in volume from several hundred picoliters to several hundred nanoliters), and has also been used to dispense cells, beads, and protein microcrystals. Focused acoustic energy generated by a microfabricated ultrasonic transducer might be used to eject and capture selected cells or beads from microwells in order to exclude them from downstream assay and analysis steps. Typically the size of the droplets may be controlled by adjusting ultrasound parameters such as pulse frequency and amplitude. In some embodiments, the ejected cells or beads might be retained for selective use in downstream assay and analysis steps.

A related example of a selection mechanism for ensuring that data for selected cells is eliminated from downstream processing, without requiring physical removal of cells or beads from microwells, would be the use of photocleavable linkers for the attachment of stochastic labels to beads. Beads co-localized with specified cells would be illuminated with a focused light beam (typically UV light) to release the attached labels and allow them to be rinsed away and eliminated from further assay steps. This approach will likely require that suitable conditions be identified for achieving efficient photolysis while leaving the remaining cells in the microwell pattern intact.

An example of a selection mechanism for physical destruction of selected cells or beads in microwells is the use of laser photoablation, in which focused laser light, *e.g.* focused $CO_2$ or excimer laser pulses, is used to selectively break bonds and remove material while causing little or no damage to surrounding materials.

One non-limiting example of a selection mechanism for physical entrapment of magnetic beads within microwells is the use of miniaturized magnetic probes, *e.g.* magnetic write head technology originally developed for computer hard disks may be adapted in some embodiments of the disclosed devices and systems for the purpose of trapping beads in specified wells. One or more modified microfabricated magnetic write heads could be moved into proximity with one or more specified microwells and activated to hold the corresponding beads in place, thereby preventing them from elution and downstream assay steps. In some embodiments, an array of microfabricated electromagnets may be fabricated on one surface of a microwell array substrate (or within the substrate itself) to create an addressable array of magnetic probes that may be used to trap selected beads.

Other examples of selection mechanisms for physical entrapment of cells or beads include the use of bead or microwell substrate materials that shrink or swell upon exposure to a localized physical or chemical stimulus. In some embodiments, for example, beads are fabricated from a suitable material such that selected beads, *i.e.* those associated with a specified subset of cells in the microwells, are subjected to a local stimulus and swell such that they may not be removed from the microwells within which they are located, thereby effectively removing them from further assay process steps. Alternatively, in some embodiments, microwells are fabricated from a suitable material such that selected wells, *i.e.* those wells containing a specified subset of cells, are subjected to a local stimulus and shrink such that the beads contained within may not be removed, thereby effectively removing them from further assay process steps. Examples of suitable swellable or shrinkable materials may include thermoresponsive polymer gels (which exhibit a discontinuous change in degree of swelling with temperature), pH-sensitive polymers (which shrink or swell depending on local pH), electro-responsive polymers (which shrink or swell in response to local electric fields), and light-responsive polymers (which shrink or swell in response to exposure to UV or visible light).

*Distribution of more than one cell type:* In some embodiments, the system may include functionality for distributing more than one cell type over the microwell array. For example, the system may load the microwell array with a first cell type A, followed by rinsing and subsequent loading with a second cell type B, such that a plurality of microwells contain a single cell of type A and a single cell of type B. Such system functionality may be useful in studying cell-cell interactions and other applications. In general, the system may be configured to distribute at least one cell type, at least two cell types, at least three cell types, at least four cell types, or at least five cell types over the microwell array. In some embodiments, the system maybe configured to distribute at most five cell types, at most four cell types, at most three cell types, at most two cell types, or at most one cell type over the microwell array. In some embodiments, the system may be configured to distribute complex mixtures of cells over the microwell array. In all of these configurations, the system may be set up to optimize the distribution of cells in microwells, and to identify wells having a greater or lesser number of cells than a specified number of cells, using cell distribution, real-time imaging, and feedback mechanisms as described above. In general, the percentage of microwells that contain more than one cell type, *e.g.* one cell each of types A and B, or one cell each from types A, B, and C, may range from about 1% to about 100%. In some embodiments, the percentage of microwells that contain more than one cell type may be at least 1%, at least 5%, at least 10%, at least 20%, at least 40%, at least 60%, at least 80%, or at least 90%. In other embodiments, the percentage of microwells that contain more than one cell type may be at most 100%, at most 90%, at most 80%, at most 60%, at most 40%, at most 20%, at most 10%, at most 5%, or at most 1%. In specific embodiment, the percentage of microwells that contain more than one cell type may have a value that falls anywhere within this range, *e.g.* about 8.5%.

*Cell lysis mechanisms:* In some embodiments of the disclosed methods, devices, and systems, cell lysis may be accomplished by chemical or biochemical means, by osmotic shock, or by means of thermal lysis, mechanical lysis, or optical lysis. In some embodiments, for example, cells may be lysed by addition of a cell lysis buffer comprising a detergent *(e.g.* SDS, Li dodecyl sulfate, Triton X-100, Tween-20, or NP-40), an organic solvent (*e.g.* methanol or acetone), or digestive enzymes (*e.g.* proteinase K, pepsin, or trypsin), or combinations thereof. In some embodiments of the instrument system, one or more of these reagents (for example, bead suspensions) may be stored in bottles or containers that are connected to the microwell flow cell or cartridge when inserted into the instrument. In some embodiments, the reagents (for example, bead suspensions) may be pre-loaded into the cartridge.

In some embodiments, the instrument system may include mechanical cell lysis capability as an alternative to the use of detergents or other reagents. Sonication using a high frequency piezoelectric transducer is one example of a suitable technique.

*Magnetic field control:* As indicated elsewhere in this disclosure, many embodiments of the disclosed methods utilize magnetic fields for removing beads from the microwells upon completion of the assay. In some embodiments, the instrument system may further comprise use of magnetic fields for transporting beads into or out of the microwell flow cell or chamber, or through other parts of the instrument system, or for retaining or trapping beads in particular locations after they have been loaded or distributed prior to the assay or during the assay. Examples of suitable means for providing control of magnetic fields include, but are not limited to, use of electromagnets in fixed position(s) relative to the cartridge, or the use of permanent magnets that are mechanically repositioned as necessary. In some embodiments of the instrument system, the strength of the applied magnetic field(s) will be varied by varying the amount of current applied to one or more electromagnets. In some embodiments of the instrument system, the strength of the applied magnetic fields will be varied by changing the position of one or more permanent magnets relative to the position of the microwell chamber(s) using, for example, stepper motor-driven linear actuators, servo motor-driven linear actuators, or cam shaft mechanisms. In other embodiments, the positions of magnets may be controlled in a linear (or non-linear) fashion, with speeds chosen to maximize bead collection efficiency, as opposed to performing transitions between just two fixed positions. In some embodiments of the instrument system, the use of pulsed magnetic fields may be advantageous, for example, to prevent clustering of magnetic beads.

In addition to consideration of the strength and location of magnetic fields for manipulating beads and other materials, it is important to design the system such that the magnetic field gradient is suitable for the task being performed. It is spatial gradients in magnetic field which exert translational force on magnetic materials and particles. Suitable gradients in fields can be achieved by the use of multiple magnets, the use of magnets or magnetized materials with particular edge and face geometries, and by designing magnets with appropriate spatial scale. Here, the term "magnets" refers to permanent magnets or electromagnets. Magnet assemblies comprising multiple magnetic domains, formed intrinsically or by design, may be used to generate magnetic fields with desirable field strengths and spatial variations. For example, patterns of small magnets with parallel or antiparallel field axes, or other relative angles, may be placed adjacent to the pattern of wells and fluidics, to achieve optimal trapping or manipulation of beads during the loading and operation of the device. In some embodiments of the disclosed systems, for example, when using magnetic fields to trap magnetic beads in microwells or to elute magnetic beads from microwells, an externally-applied magnetic field gradient may be applied to the entire microwell pattern simultaneously. In some embodiments, externally-applied magnetic field gradients may be applied to a selected area of the microwell pattern. In some embodiments, an externally-applied magnetic field gradient may be applied to a single microwell. In some embodiments, the magnetic field lines for an externally-applied magnetic field may lie at an angle relative to the plane of the microwell substrate of between about 30 degrees and 89 degrees. In some embodiments, the angle of the magnetic field lines relative to the plane of the microwell substrate may be between about 45 degrees and 80 degrees. In some embodiments, the angle of the magnetic field lines relative to the plane of the microwell substrate may be at least 45 degrees, at least 50 degrees, at least 55 degrees, at least 60 degrees, at least 65 degrees, at least 70 degrees, at least 75 degrees, or at least 80 degrees, or higher. In some embodiments, the angle of the magnetic field lines relative to the plane of the microwell substrate may be at most 80 degrees, at most 75 degrees, at most 70 degrees, at most 65 degrees, at most 60 degrees, at most 55 degrees, at most 50 degrees, or at most 45 degrees, or smaller. Those of skill in the art will recognize that the angle of the magnetic field lines relative to the plane of the microwell substrate may have any value within this range, for example, about 52 degrees.

*Temperature control:* In some embodiments, the instrument system will include temperature control functionality for the purpose of facilitating the accuracy and reproducibility of assay results, for example, cooling of the microwell flow cell or chamber may be advantageous for minimizing molecular diffusion between microwells. Examples of temperature control components that may be incorporated into the instrument system (or cartridge) design include, but are not limited to, resistive heating elements, infrared light sources, Peltier heating or cooling devices, heat sinks, thermistors, thermocouples, and the like. In some embodiments of the system, the temperature controller may provide for a programmable temperature change at a specified, adjustable time prior to performing cell lysis and downstream assay steps. In some embodiments of the system, the temperature controller may provide for programmable changes in temperature over specified time intervals. In some embodiments, the temperature controller may further provide for cycling of temperatures between two or more set temperatures with specified frequency and ramp rates so that thermal cycling for amplification reactions may be performed.

*Imaging capability:* As indicated above, in many embodiments the instrument system will include optical imaging or other spectroscopic capabilities. Such functionality may be useful, for example, for inspection of the microwell substrate to determine whether or not the microwell pattern has been uniformly and optimally populated with cells or beads. Any of a variety of imaging modes may be utilized, including but not limited to, bright-field, dark-field, fluorescence, luminescence, or phosphorescence imaging. The choice of imaging mode will impact the design of microwell arrays, flow cells, and cartridge chambers in that the microwell substrate or opposing wall of the flow cell or microwell chamber will necessarily need to be transparent over the spectral range of interest. In some embodiments, partially-coherent illumination light may be used to improve the contrast of unstained cells in bright-field images.

In some embodiments, quantitative phase imaging may be used to improve the performance of automated image processing and analysis software in determining the number of cells located in each microwell. Unstained cells typically absorb very little light, but cause measureable phase delays in transmitted light. Quantitative phase imaging can refer to any of several methods for calculating phase information from a series of two or more images (which capture intensity data) collected using coherent or partially-coherent light. A series of suitable intensity images may be captured, for example, by capturing images at different defocus distances. The images are then processed to recover phase information using, for example, using the "Transport of Intensity" algorithm or iterative techniques based on the Gerchberg-Saxton approach, to create a shape and density map of the cells in the field of view.

In some embodiments, each plurality of microwells may be imaged in its entirety within a single image. In some embodiments, a series of images may be "tiled" to create a high resolution image of the entire microwell pattern. In some embodiment, a single image that represents a subsection of the pattern may be used to evaluate properties, *e.g.* cell or bead distributions, for the pattern as a whole.

In some embodiments, dual wavelength excitation and emission (or multi-wavelength excitation or emission) imaging may be performed.

*Light sources:* Any of a variety of light sources may be used to provide the imaging or excitation light, including but not limited to, tungsten lamps, tungsten-halogen lamps, arc lamps, lasers, light emitting diodes (LEDs), or laser diodes. In many embodiments, a combination of one or more light sources, and additional optical components, *e.g.* lenses, filters, apertures, diaphragms, mirrors, and the like, will comprise an illumination system (or sub-system).

*Detectors:* Any of a variety of image sensors may be used for imaging purposes, including but not limited to, photodiode arrays, charge-coupled device (CCD) cameras, or CMOS image sensors. Imaging sensors may be one-dimensional (linear) or two-dimensional array sensors. In many embodiments, a combination of one or more image sensors, and additional optical components, *e.g.* lenses, filters, apertures, diaphragms, mirrors, and the like, will comprise an imaging system (or sub-system).

*Other optical components:* The optical system will typically include a variety of optical components for steering, shaping, filtering, or focusing light beams through the system. Examples of suitable optical components include, but are not limited to, lenses, mirrors, prisms, diffraction gratings, colored glass filters, narrowband interference filters, broadband interference filters, dichroic reflectors, optical fibers, optical waveguides, and the like. In some embodiments, the imaging system will further comprise one or more translation stages or other motion control mechanisms for the purpose of moving the microwell substrate(s) relative to the illumination and/or imaging systems, or vice versa. In some embodiments, the instrument system may use an optically transparent microarray substrate as a waveguide for delivering excitation light to the microwells.

*Complementary assay techniques:* The choice of imaging mode may also enable the use of other types of assays to be run in parallel with stochastic labeling and molecular indexing assays, for example, the use of trypan blue live cell / dead cell assays with bright field imaging, the use of fluorescence-based live cell / dead cell assays with fluorescence imaging, *etc.* Correlation of viability data for individual cells with the cell tag associated with each bead in the associated microwell may provide an additional level of discrimination in analyzing the data from multiplexed, single cell assays.

*Additional system capabilities:* In some embodiments, the system may comprise non-imaging or non-optical capabilities for probing the microwell array. Examples of non-imaging or non-optical techniques for detecting trapped air bubbles, determining the cell or bead distribution over the array, *etc.,* include but are not limited to measurements of light scattering, ultraviolet/visible/infrared absorption measurements (*e.g.* using stained cells or beads that incorporate dyes), coherent Raman scattering, and conductance measurements (*e.g.* using microfabricated arrays of electrodes in register with microwell arrays). In some embodiments, information obtained about the condition or contents of particular wells may be used to determine that those wells must be sequestered, excised, or otherwise prevented from contributing to the assay results. For example, electrical heating elements may be used to form a bubble or denature the well contents, or optical energy may be applied to deform the walls of the well and thereby trap the contents, or a local magnetic field could be applied such that the bead to be eliminated is trapped in the substrate instead of eluted for analysis.

*Interfaces with PCR thermocyclers, sequencers, & FACS instruments:* In some embodiments, the instrument systems of the present disclosure may further comprise interfaces with PCR thermocyclers, sequencers, cell sorters, fluorescence-activated cell sorter (FACS) instruments, or other types of lab automation equipment.

In some embodiments, an interface for PCR thermocyclers is provided such that instrument system outputs labeled oligonucleotide libraries directly into tubes, strips, or plates that are compatible with commercially-available PCR instruments, for example, the Roche LightCycler® series of real-time PCR instruments, and the like.

In some embodiments, an interface is provided for cell sorters or FACS instruments such that sorted cells are deposited directly into a microwell array or cartridge. The interface for FACS instruments may, for example, include both hardware and software components, where the software provides the capability for simultaneous control of the FACS instrument and the single cell, stochastic labeling or molecular barcoding system. In some embodiments, the software may provide analysis capability for identifying correlations between the FACS data (*e.g.* the presence or absence of specified cell surface markers) and the copy numbers for one or more genes in a specified sub-population of cells. FACS machines can be used to sort single cells directly into the microwell array of the disclosure.

In some embodiments, an interface with lab automation equipment in general is provided, for example, cartridges for use with the disclosed instrument systems may be configured to have inlet ports of the proper dimension and spacing such that samples and reagents may be dispensed directly into the cartridge using commercially-available pipetting stations and liquid-handling robotics. Similarly, in some embodiments, cartridges for use with the disclosed instrument systems may be configured to have dimensions that are compatible with commercially-available plate-handling robotics for automated storage, retrieval, or movement between other laboratory workstations.

*System processor and software:*

In general, instrument systems designed to support the automation of multiplexed, single cell stochastic labeling and molecular barcoding assays will include a processor or computer, along with software to provide (i) instrument control functionality, (ii) image processing and analysis capability, and (iii) data storage, analysis, and display functionality.

*System processor and control software:* In many embodiments, the instrument system will comprise a computer (or processor) and computer-readable media that includes code for providing a user interface as well as manual, semi-automated, or fully-automated control of all system functions, *e.g.* control of the fluidics system, the temperature control system, cell or bead distribution functions, magnetic bead manipulation functions, and the imaging system. In some embodiments, the system computer or processor may be an integrated component of the instrument system (*e.g.* a microprocessor or mother board embedded within the instrument). In some embodiments, the system computer or processor may be a stand-alone module, for example, a personal computer or laptop computer. Examples of fluid control functions provided by the instrument control software include, but are not limited to, volumetric fluid flow rates, fluid flow velocities, the timing and duration for sample and bead addition, reagent addition, and rinse steps. Examples of temperature control functions provided by the instrument control software include, but are not limited to, specifying temperature set point(s) and control of the timing, duration, and ramp rates for temperature changes. Examples of cell or bead distribution functions provided by the instrument control software include, but are not limited to, control of agitation parameters such as amplitude, frequency, and duration. Examples of magnetic field functions provided by the instrument control software include, but are not limited to, the timing and duration the applied magnetic field(s), and in the case of electromagnets, the strength of the magnetic field as well. Examples of imaging system control functions provided by the instrument control software include, but are not limited to, autofocus capability, control of illumination or excitation light exposure times and intensities, control of image acquisition rate, exposure time, and data storage options.

*Image processing software:* In some embodiments of the instrument system, the system will further comprise computer-readable media that includes code for providing image processing and analysis capability. Examples of image processing and analysis capability provided by the software include, but are not limited to, manual, semi-automated, or fully-automated image exposure adjustment (*e.g.* white balance, contrast adjustment, signal-averaging and other noise reduction capability, *etc.*), automated edge detection and object identification (*i.e.* for identifying cells and beads in the image), automated statistical analysis (*i.e.* for determining the number of cells or beads identified per microwell or per unit area of the microwell substrate, or for identifying wells that contain more than one cell or more than one bead), and manual measurement capabilities (*e.g.* for measuring distances between objects, *etc.*). In some embodiments, the instrument control and image processing/analysis software will be written as separate software modules. In some embodiments, the instrument control and image processing/analysis software will be incorporated into an integrated package.

In some embodiments, the system software may provide integrated real-time image analysis and instrument control, so that cell and bead sample loading steps may be prolonged, modified, or repeated until optimal cell and bead distributions (*e.g.* uniformly distributed across the microwell pattern at a pre-determined level for the number of wells containing a single cell, the number of wells containing a single bead, or the number of wells containing both a single cell and a single bead) are achieved. Any of a number of image processing and analysis algorithms known to those of skill in the art may be used to implement real-time or post-processing image analysis capability. Examples include, but are not limited to, the Canny edge detection method, the Canny-Deriche edge detection method, first-order gradient edge detection methods (*e.g.* the Sobel operator), second order differential edge detection methods, phase congruency (phase coherence) edge detection methods, other image segmentation algorithms (*e.g.* intensity thresholding, intensity clustering methods, intensity histogram-based methods, *etc.*), feature and pattern recognition algorithms (*e.g.* the generalized Hough transform for detecting arbitrary shapes, the circular Hough transform, *etc.*), and mathematical analysis algorithms (*e.g.* Fourier transform, fast Fourier transform, wavelet analysis, auto-correlation, *etc.*), or combinations thereof. As outlined above, examples of mechanisms for facilitating cell and bead distribution which may be controlled through feedback from real-time image analysis include, but are not limited to, rocking, shaking, swirling, recirculating flow, oscillatory or pulsatile flow, low frequency agitation (for example, through pulsing of a flexible (*e.g.* silicone) membrane that forms a wall of the chamber or nearby fluid channel), or high frequency agitation (for example, through the use of piezoelectric transducers). In some embodiments, the instrument system may monitor the total number of cells captured in the microwells, as determined by image processing and analysis, and turn off the supply of cells when a pre-determined number of cells is reached in order to avoid loading an excess number of wells with two or more cells. In some embodiments, the instrument system may monitor the number of wells containing single cells, and turn off the supply of cells when a pre-determined number of wells are reached in order to avoid loading an excess number of wells with two or more cells. In some embodiments, the instrument system may monitor the number of wells containing single beads, and turn off the supply of beads when a predetermined number of wells is reached in order to avoid loading an excess number of wells with two or more beads. In some embodiments, the instrument system may monitor the number of wells containing both a single cell and a single bead, and turn off the supply of cells or beads (or both) in order to avoid loading an excess number of wells with two or more cells or beads.

When using integrated real-time image analysis and instrument control to achieve optimal bead distributions, any of a variety of pre-determined levels may be targeted. For example, in some embodiments, the percentage of microwells that contain a single bead may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single bead.

When using integrated real-time image analysis and instrument control to achieve optimal cell distributions, any of a variety of pre-determined levels may be targeted. For example, in some embodiments, the percentage of microwells that contain a single cell may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain a single cell. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain a single cell.

Real-time image analysis can comprise monitoring cell capture efficiency in the microwells (*i.e.,* determining the number of wells that have a cell in them and/or determining the percentage of cells that are in between wells). Cell capture can be improved by methods such as agitation, washing (*i.e.,* flushing) fluid, and/or magnetic methods. For example, the percentage of cells that may be between microwells may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the cells may be between microwells (*e.g.,* on the surface between microwells). In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the cells may be between microwells (e.g., on the surface between microwells).

When using integrated real-time image analysis and instrument control to achieve optimal cell and bead distributions, *e.g.* to maximize the percentage of microwells containing both a single cell and a single bead, any of a variety of pre-determined levels may be targeted. For example, in some embodiments, the percentage of microwells that contain both a single cell and a single bead may be between 1% and 100%. In some embodiments, at least 1%, at least 5%, at least 10%, at least 20%, at least 30%, at least 40%, at least 50%, at least 60%, at least 70%, at least 80%, at least 90%, at least 95%, or at least 99% of the microwells in the plurality of microwells may contain both a single cell and a single bead. In some embodiments, at most 100%, at most 99%, at most 95%, at most 90%, at most 80%, at most 70%, at most 60%, at most 50%, at most 40%, at most 30%, at most 20%, at most 10%, at most 5%, or at most 1% of the microwells in the plurality of microwells may contain both a single cell and a single bead.

In some embodiments, the system software may provide integrated real-time image analysis and instrument control, so that cells may be optically monitored and classified according to a pre-determined set of characteristics, and subsequently included or excluded from the downstream sequence data analysis. Examples of cellular characteristics that may be optically monitored and used for classification purposes include, but are not limited to, cell size, cell shape, live cell / dead cell determination (*e.g.* using selectively absorbed chromophores such as Trypan blue, or fluorescent dyes such as calcein AM, ethidium homodimer-1, $DiOC_2(3)$, $DiOC_5(3)$, $DiOC_6(3)$, $DiSC_3(5)$, $DiIC_1(5)$, $DiOC_{18}(3)$, propidium iodide, SYBR® 14, SYTOX® Green, *etc.*), cells exhibiting a specified range of intracellular pH (*e.g.* using intracellular pH-sensitive fluorescent probes such as 2',7'-Bis-(2-carboxyethyl)-5-(and-6-)carboxyfluorescein (BCECF), 2',7'-bis-(2-carboxypropyl)-5-(and-6-)-carboxyfluorescein (BCPCF), *etc.),* cells exhibiting a specified range of membrane potential (*e.g.* using membrane potential-sensitive fluorophores such as FluoVolt™, di-3-ANEPPDHQ, Bis-(1,3-Dibutyl-barbituric Acid) Trimethine Oxonol ($DiBAC_4(3)$), $DiBAC_4(5)$, $DiSBAC_2(3)$, Merocyanine 540, JC-1, JC-9, Oxonol V, Oxonol VI, Tetramethylrhodamine methyl and ethyl esters, Rhodamine 123, Di-4-ANEPPS, Di-8-ANEPPS, Di-2-ANEPEQ, Di-3-ANEPPDHQ, Di-4-ANEPPDHQ, *etc.*), cells exhibiting a specified level of intracellular calcium (*e.g.* using $Ca^{2+}$-sensitive fluorescent dyes such as fura-2, indo-1, fluo-3, fluo-4, Calcium Green-1, Quin 2, *etc.*), cells exhibiting one or more specified cell surface markers (*e.g.* using fluorescently-labeled antibodies directed towards the cell surface markers), cells expressing fluorescent proteins (*e.g.* GFP, bilirubin-inducible fluorescent protein, UnaG, dsRed, eqFP611, Dronpa, TagRFPs, KFP, EosFP, Dendra, IrisFP, *etc.*), and the like. In many embodiments, two or more dyes, fluorophores, or other optical probes having non-overlapping spectral properties (*e.g.* non-overlapping excitation peaks, non-overlapping absorption or emission peaks, *etc.*) can be selected so that cells may be simultaneously characterized with respect to two or more properties. In some embodiments, real-time image processing and analysis is used to identify wells containing cells exhibiting one or more specified characteristics, followed by selection or exclusion of a subset of cells on the array from further analysis. In some embodiments, real-time image processing and analysis is used to identify wells containing two or more cells, followed by the exclusion of the cells in those wells from further analysis. As described above in more detail, examples of mechanisms that may be used to select or exclude a subset of cells from further analysis include, but are not limited to, (i) physical removal of selected cells or beads from microwells by means of miniaturized magnetic probes, optical tweezer apparatus, micromanipulators, photoablation, *etc.,* (ii) physical entrapment of selected cells or beads within microwells by means of miniaturized magnetic probes, swellable beads, shrinkable wells, or (iii) use of dual-encoding schemes whereby the sequence data that is generated for a given cell is selected for or excluded from further analysis.

In some embodiments, the system software (or a stand-alone software module) may provide image analysis capability for automated cell counting using a hemocytometer, thereby allowing users to determine how much cell suspension to load onto the microwell array substrate. In some embodiments, the automated cell counting capability may be coupled with optional fluorescence image analysis so that cells may be characterized with respect to viability or other properties (using, for example, the optical probed described above) at the same time that counting is performed.

*Data analysis & display software:* In some embodiments of the instrument system, the system will comprise computer-readable media that includes code for providing data analysis for the sequence datasets generated by performing single cell, stochastic labeling or molecular barcoding assays. Examples of data analysis functionality that may be provided by the data analysis software include, but are not limited to, (i) algorithms for decoding/demultiplexing of the sample barcode, cell barcode, molecular barcode, and target sequence data provided by sequencing the oligonucleotide library created in running the assay, (ii) algorithms for determining the number of reads per gene per cell, and the number of unique transcript molecules per gene per cell, based on the data, and creating summary tables, (iii) statistical analysis of the sequence data, *e.g.* for clustering of cells by gene expression data, or for predicting confidence intervals for determinations of the number of transcript molecules per gene per cell, *etc.,* (iv) algorithms for identifying sub-populations of rare cells, for example, using principal component analysis, hierarchical clustering, k-mean clustering, self-organizing maps, neural networks *etc.,* , (v) sequence alignment capabilities for alignment of gene sequence data with known reference sequences and detection of mutation, polymorphic markers and splice variants, and (vi) automated clustering of molecular labels to compensate for amplification or sequencing errors. In some embodiments, commercially-available software may be used to perform all or a portion of the data analysis, for example, the Seven Bridges () software may be used to compile tables of the number of copies of one or more genes occurring in each cell for the entire collection of cells. In some embodiments, the data analysis software may include options for outputting the sequencing results in useful graphical formats, *e.g.* heatmaps that indicate the number of copies of one or more genes occurring in each cell of a collection of cells. In some embodiments, the data analysis software may further comprise algorithms for extracting biological meaning from the sequencing results, for example, by correlating the number of copies of one or more genes occurring in each cell of a collection of cells with a type of cell, a type of rare cell, or a cell derived from a subject having a specific disease or condition. In some embodiment, the data analysis software may further comprise algorithms for comparing populations of cells across different biological samples.

In some embodiments all of the data analysis functionality may be packaged within a single software package. In some embodiments, the complete set of data analysis capabilities may comprise a suite of software packages. In some embodiments, the data analysis software may be a standalone package that is made available to users independently of the assay instrument system. In some embodiments, the software may be web-based, and may allow users to share data.

*System processors & networks:* In general, the computer or processor included in the presently disclosed instrument systems, as illustrated in FIG. 35, may be further understood as a logical apparatus that can read instructions from media **511** or a network port **505,** which can optionally be connected to server **509** having fixed media **512.** The system **500,** such as shown in FIG. 35 can include a CPU **501,** disk drives **503,** optional input devices such as keyboard **515** or mouse **516** and optional monitor **507.** Data communication can be achieved through the indicated communication medium to a server at a local or a remote location. The communication medium can include any means of transmitting or receiving data. For example, the communication medium can be a network connection, a wireless connection or an internet connection. Such a connection can provide for communication over the World Wide Web. It is envisioned that data relating to the present disclosure can be transmitted over such networks or connections for reception or review by a party **522** as illustrated in FIG. 35.

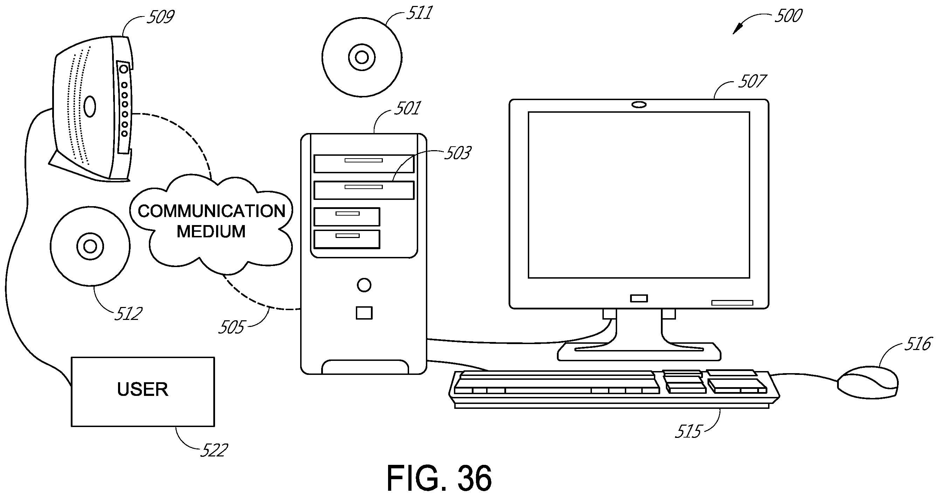

FIG. 36 is a block diagram illustrating a first example architecture of a computer system **100** that can be used in connection with example embodiments of the present disclosure. As depicted in FIG. 36, the example computer system can include a processor **102** for processing instructions. Non-limiting examples of processors include: Intel Xeon™ processor, AMD Opteron™ processor, Samsung 32-bit RISC ARM 1176JZ(F)-S v1.0™ processor, ARM Cortex-A8 Samsung S5PC100™ processor, ARM Cortex-A8 Apple A4™ processor, Marvell PXA 930™ processor, or a functionally-equivalent processor. Multiple threads of execution can be used for parallel processing. In some embodiments, multiple processors or processors with multiple cores can also be used, whether in a single computer system, in a cluster, or distributed across systems over a network comprising a plurality of computers, cell phones, or personal data assistant devices.

As illustrated in FIG. 36, a high speed cache **104** can be connected to, or incorporated in, the processor **102** to provide a high speed memory for instructions or data that have been recently, or are frequently, used by processor **102.** The processor **102** is connected to a north bridge **106** by a processor bus **108.** The north bridge **106** is connected to random access memory (RAM) **110** by a memory bus **112** and manages access to the RAM **110** by the processor **102.** The north bridge **106** is also connected to a south bridge **114** by a chipset bus **116.** The south bridge **114** is, in turn, connected to a peripheral bus **118.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **118.** In some alternative architectures, the functionality of the north bridge can be incorporated into the processor instead of using a separate north bridge chip.

In some embodiments, system **100** can include an accelerator card **122** attached to the peripheral bus **118.** The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage **124** and can be loaded into RAM **110** or cache **104** for use by the processor. The system **100** includes an operating system for managing system resources; non-limiting examples of operating systems include: Linux, Windows™, MacOS™, BlackBerry OS™, iOS™, and other functionally-equivalent operating systems, as well as application software running on top of the operating system for managing data storage and optimization in accordance with example embodiments of the present invention.

In this example, system **100** also includes network interface cards (NICs) **120** and **121** connected to the peripheral bus for providing network interfaces to external storage, such as Network Attached Storage (NAS) and other computer systems that can be used for distributed parallel processing.

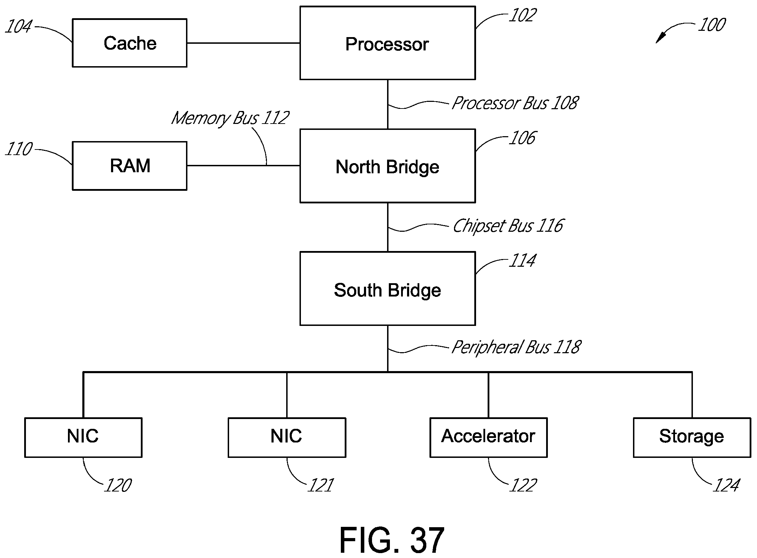

FIG. 37 is a diagram showing a network 200 with a plurality of computer systems 202a, and **202b,** a plurality of cell phones and personal data assistants **202c**, and Network Attached Storage (NAS) **204a,** and **204b.** In example embodiments, systems **212a, 212b,** and **212c** can manage data storage and optimize data access for data stored in Network Attached Storage (NAS) **214a** and **214b.** A mathematical model can be used for the data and be evaluated using distributed parallel processing across computer systems **212a,** and **212b,** and cell phone and personal data assistant systems **212c.** Computer systems **212a,** and **212b,** and cell phone and personal data assistant systems **212c** can also provide parallel processing for adaptive data restructuring of the data stored in Network Attached Storage (NAS) **214a** and **214b.** FIG. 37 illustrates an example only, and a wide variety of other computer architectures and systems can be used in conjunction with the various embodiments of the present invention. For example, a blade server can be used to provide parallel processing. Processor blades can be connected through a back plane to provide parallel processing. Storage can also be connected to the back plane or as Network Attached Storage (NAS) through a separate network interface.

In some example embodiments, processors can maintain separate memory spaces and transmit data through network interfaces, back plane or other connectors for parallel processing by other processors. In other embodiments, some or all of the processors can use a shared virtual address memory space.

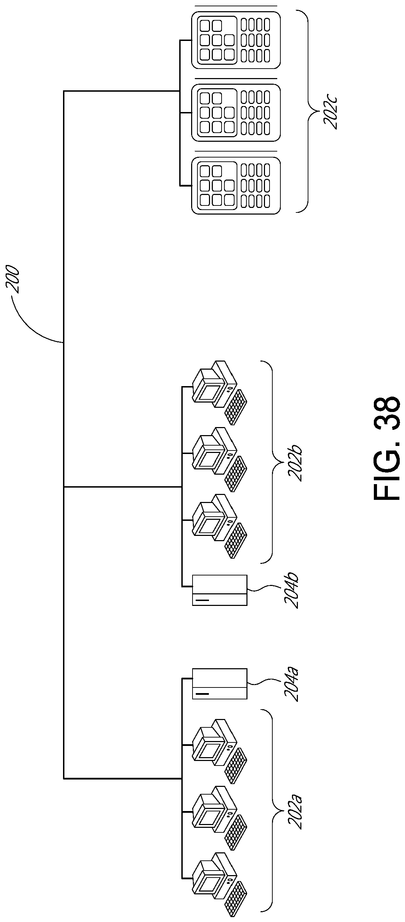

FIG. 38 is a block diagram of a multiprocessor computer system **300** using a shared virtual address memory space in accordance with an example embodiment. The system includes a plurality of processors **302a-f** that can access a shared memory subsystem **304.** The system incorporates a plurality of programmable hardware memory algorithm processors (MAPs) **306a-f** in the memory subsystem **304.** Each MAP **306a-f** can comprise a memory **308a-f** and one or more field programmable gate arrays (FPGAs) **310a-f.** The MAP provides a configurable functional unit and particular algorithms or portions of algorithms can be provided to the FPGAs **310a-f** for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory **308a-f,** allowing it to execute tasks independently of, and asynchronously from, the respective microprocessor **302a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some embodiments, all or part of the computer system can be implemented in software or hardware. Any variety of data storage media can be used in connection with example embodiments, including random access memory, hard drives, flash memory, tape drives, disk arrays, Network Attached Storage (NAS) and other local or distributed data storage devices and systems.

In example embodiments, the computer subsystem of the present disclosure can be implemented using software modules executing on any of the above or other computer architectures and systems. In other embodiments, the functions of the system can be implemented partially or completely in firmware, programmable logic devices such as field programmable gate arrays (FPGAs) as referenced in FIG. 38, system on chips (SOCs), application specific integrated circuits (ASICs), or other processing and logic elements. For example, the Set Processor and Optimizer can be implemented with hardware acceleration through the use of a hardware accelerator card, such as accelerator card **122** illustrated in FIG 36.

*Kits:*

Also disclosed herein are kits for performing single cell, stochastic labeling or molecular barcoding assays. In some embodiments, the kit may comprise one or more microwell substrates (either as a free-standing substrate (or chip) comprising one or more microwell patterns, or packaged within one or more flow-cells or cartridges) and one or more bead suspensions, wherein the individual beads within a suspension comprise a plurality of attached stochastic labels. In some embodiments, the kit may further comprise a mechanical fixture for mounting a free-standing microwell substrate in order to create reaction wells that facilitate the pipetting of samples and reagents into the microwells. In some embodiments, the kit may further comprise reagents, *e.g.* lysis buffers, rinse buffers, or hybridization buffers, for performing the stochastic labeling assay. In some embodiments, the kit may further comprise reagents (*e.g.* enzymes, primers, or buffers) for performing nucleic acid extension reactions, for example, reverse transcription reactions. In some embodiments, the kit may further comprise reagents (*e.g.* enzymes, universal primers, sequencing primers, target-specific primers, or buffers) for performing amplification reactions to prepare sequencing libraries. In some embodiments, the kit may comprise one or more molds, for example, molds comprising a pattern of micropillars, for casting microwell patterns, and one or more bead suspensions, wherein the individual beads within a suspension comprise a plurality of attached stochastic labels. In some embodiments, the kit may further comprise a material for use in casting microwells, *e.g.* agarose, a hydrogel, PDMS, and the like.

In some embodiments of the disclosed kits, the kit may comprise one or more microwell substrates that are pre-loaded with beads comprising a plurality of attached stochastic labels, wherein there is at most one bead per microwell. In some embodiments, the plurality of stochastic labels may be attached directly to a surface of the microwell, rather than to a bead. In any of these embodiments, the one or more microwell substrates may be provided in the form of free-standing substrates (or chips), or they may be packed in flow-cells or cartridges.

In some embodiments of the disclosed kits, the kit may comprise one or more cartridges that incorporate one or more microwell substrates. In some embodiments, the one or more cartridges may further comprise one or more pre-loaded bead suspensions, wherein the individual beads within a suspension comprise a plurality of attached stochastic labels. In some embodiments, the beads may be pre-distributed into the one or more microwell substrates of the cartridge. In some embodiments, the beads, in the form of suspensions, may be pre-loaded and stored within reagent wells of the cartridge. In some embodiments, the one or more cartridges may further comprise other assay reagents that are pre-loaded and stored within reagent reservoirs of the cartridges.

*Applications:*

The methods, devices, and systems disclosed herein may be used for a variety of applications in basic research, biomedical research, environmental testing, and clinical diagnostics. Examples of potential applications for the disclosed technologies include, but are not limited to, genotyping, gene expression profiling, detection and identification of rare cells, diagnosis of a disease or condition, determining prognosis for a disease or condition, determining a course of treatment for a disease (*e.g.,* determining if a patient may respond to a therapy) or condition, and monitoring the response to treatment for a disease or condition, and understanding biological development processes.

*Circulating tumor cells (CTCs):* In some embodiments, the methods, devices, and systems of the disclosure can be used for characterizing circulating tumor cells (CTCs). The methods, devices, and systems of the disclosure can be used for detecting the expression profile in circulating tumor cells from an enriched blood sample. The method can comprise obtaining a biological sample containing a mixed population of cells from an individual suspected of having target rare cells, contacting said sample (which may be enriched) to the devices and systems of the disclosure such that a single cell is in a single well. The sample can be subjected to methods for removal of red blood cells and/or dead cells from blood. The sample can be contacted with beads of the disclosure such that a single bead is in a single well with a single cell. The cell can be lysed. The bead can comprise a stochastic label that can bind to a specific location genes in the cell and/or mRNAs of the cell. The molecules from the CTC associated with solid support can be subjected to the molecular biology methods of the disclosure, including reverse transcription, amplification, and sequencing. Cells can be clustered based on the gene expression profile of the cells, thereby identifying rare cells (*e.g.,* CTCs) and subpopulations of rare cells (*e.g.,* CTCs) based on their genotypes.

The methods can be used for identifying and characterizing subpopulations (subgroups) of circulating tumor cells (CTCs) in a population of CTCs or a sample, quantifying subgroups of CTCs in the population of CTCs or the sample, diagnosing and monitoring of cancer, treatment and prognosis, in particular for solid tumors, and using the identified CTC subgroups as biomarkers for diagnosis, prognosis and therapeutic treatment.

*T-cell receptor chain pairing:* T-cell receptors (TCRs) are recognition molecules present on the surface of T lymphocytes. The T-cell receptors found on the surface of T-cells can be comprised of two glycoprotein subunits which are referred to as the α and β chains. Both chains can comprise a molecular weight of about 40 kDa and possess a variable and a constant domain. The genes which encode the α and β chains can be organized in libraries of V, D and J regions from which the genes are formed by genetic rearrangement. TCRs can recognize antigen which is presented by an antigen presenting cell as a part of a complex with a specific self-molecule encoded by a histocompatibility gene. The most potent histocompatibility genes are known as the major histocompatibility complex (MHC). The complex which is recognized by T-cell receptors, therefore, consists of and MHC/peptide ligand.

In some embodiments, the methods, devices, and systems of the disclosure can be used for T cell receptor sequencing and pairing. The methods, devices, and systems of the disclosure can be used for sequencing T-cell receptor alpha and beta chains, pairing alpha and beta chains, and/or determining the functional copy of T-cell receptor alpha chains. A single cell can be contained in a single well with a single bead. The cell can be lysed. The bead can comprise a stochastic label that can bind to a specific location within an alpha and/or beta chain of a TCR. The TCR alpha and beta molecules associated with solid support can be subjected to the molecular biology methods of the disclosure, including reverse transcription, amplification, and sequencing. TCR alpha and beta chains that comprise the same cellular label can be considered to be from the same single cell, thereby pairing alpha and beta chains of the TCR.

*Heavy and light chain pairing in antibody repertoires:* The methods devices and systems of the disclosure can be used for heavy and light chain pairing in antibodies. The methods of the present disclosure allow for the repertoire of immune receptors and antibodies in an individual organism or population of cells to be determined The methods of the present disclosure may aid in determining pairs of polypeptide chains that make up immune receptors. B cells and T cells each express immune receptors; B cells express immunoglobulins, and T cells express T cell receptors (TCRs). Both types of immune receptors can comprise two polypeptide chains. Immunoglobulins can comprise variable heavy (VH) and variable light (VL) chains. There can be two types of TCRs: one consisting of an $\alpha$ and a $\beta$ chain, and one consisting of a $\gamma$ and a $\delta$ chain. Polypeptides in an immune receptor can comprise constant region and a variable region. Variable regions can result from recombination and end joint rearrangement of gene fragments on the chromosome of a B or T cell. In B cells additional diversification of variable regions can occur by somatic hypermutation. The immune system has a large repertoire of receptors, and any given receptor pair expressed by a lymphocyte can be encoded by a pair of separate, unique transcripts. Knowing the sequences of pairs of immune receptor chains expressed in a single cell can be used to ascertain the immune repertoire of a given individual or population of cells.

In some embodiments, the methods, devices, and systems of the disclosure can be used for antibody sequencing and pairing. The methods, devices, and systems of the disclosure can be used for sequencing antibody heavy and light chains (e.g., in B cells), and/or pairing the heavy and light chains. A single cell can be contained in a single well with a single bead. The cell can be lysed. The bead can comprise a stochastic label that can bind to a specific location within a heavy and/or light chain of an antibody (e.g., in a B cell). The heavy and light chain molecules associated with solid support can be subjected to the molecular biology methods of the disclosure, including reverse transcription, amplification, and sequencing. Antibody heavy and light chains that comprise the same cellular label can be considered to be from the same single cell, thereby pairing heavy and light chains of the antibody.

*Immuno-oncology:* Immune-mediated treatments based on antigens, antibodies, peptides, DNA and the like can be used to treat cancer. The treatments can stimulate a patient's own immune system into fighting the cancer (*e.g.,* by increasing the immune response, and/or by removing the brakes of the immune response). Exemplary immunotherapies can include an antibody (Ab), ipilimumab (YERVOY®) that binds to and inhibits Cytotoxic T-Lymphocyte Antigen-4 (CTLA-4) for the treatment of patients with advanced melanoma and the development of Abs that block the inhibitory PD-1 pathway. Programmed Death-1 (PD-1) is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to downregulate T cell activation and cytokine secretion upon binding to PD-1. Unlike CTLA-4, PD-1 primarily functions in peripheral tissues where activated T-cells may encounter the immunosuppressive PD-L1 (B7-H1) and PD-L2 (B7-DC) ligands expressed by tumor and/or stromal cells. Inhibition of the PD-1/PD-L1 interaction can mediate potent antitumor activity in clinical samples.

Not all patients with cancer respond to immunotherapies. There have not been reliable methods for establishing if patients will respond to treatment. The devices, methods, and systems of the disclosure can be used for predicting a response to and/or benefit from immunotherapy in a patient suffering from cancer involving, for example, determining in a tumor sample the expression of at least one marker gene indicative of a response to immunotherapy for the tumor, and depending on the gene expression, predicting the response and/or benefit. In some instances, the devices methods and systems of the disclosure can be used for predicting an effect of an immunotherapy in a cancer patient, comprising for example, determining the level of at least one marker of cancer in a sample from a cancer patient, wherein a higher (or increased) level of the marker compared to the median of a given cancer patient population is indicative for a beneficial effect of an immunotherapy for the patient.

In some embodiments of the disclosed methods, devices, and systems, a first cell sample is obtained from a person not having a disease or condition, and a second cell sample is obtained from a person having the disease or condition. In some embodiments, the persons are different. In some embodiments, the persons are the same but cell samples are taken at different time points. In some embodiments, the persons are patients, and the cell samples are patient samples. In some embodiments, the disease or condition is a cancer, a bacterial infection, a viral infection, an inflammatory disease, a neurodegenerative disease, a fungal disease, a parasitic disease, a genetic disorder, or any combination thereof.

In some embodiments, the cells are cancer cells excised from a cancerous tissue, for example, breast cancer, lung cancer, colon cancer, prostate cancer, ovarian cancer, pancreatic cancer, brain cancer, melanoma and non-melanoma skin cancers, and the like. In some instances, the cells are derived from a cancer but collected from a bodily fluid (*e.g.* circulating tumor cells). Non-limiting examples of cancers may include, adenoma, adenocarcinoma, squamous cell carcinoma, basal cell carcinoma, small cell carcinoma, large cell undifferentiated carcinoma, chondrosarcoma, and fibrosarcoma.

In some embodiments, the cells are cells that have been infected with virus and contain viral oligonucleotides. In some embodiments, the viral infection may be caused by a virus selected from the group consisting of double-stranded DNA viruses (*e.g.* adenoviruses, herpes viruses, pox viruses), single-stranded (+ strand or "sense") DNA viruses (*e.g.* parvoviruses), double-stranded RNA viruses (*e.g.* reoviruses), single-stranded (+ strand or sense) RNA viruses (*e.g.* picornaviruses, togaviruses), single-stranded (- strand or antisense) RNA viruses (*e.g.* orthomyxoviruses, rhabdoviruses), single-stranded ((+ strand or sense) RNA viruses with a DNA intermediate in their life-cycle) RNA-RT viruses (*e.g.* retroviruses), and double-stranded DNA-RT viruses (*e.g.* hepadnaviruses).

In some embodiments, the cells are bacteria. These may include either gram-positive or gram-negative bacteria. Examples of bacteria that may be analyzed using the disclosed methods, devices, and systems include, but are not limited to, Actinomedurae, Actinomyces israelii, Bacillus anthracis, Bacillus cereus, Clostridium botulinum, Clostridium difficile, Clostridium perfringens, Clostridium tetani, Corynebacterium, Enterococcus faecalis, Listeria monocytogenes, Nocardia, Propionibacterium acnes, Staphylococcus aureus, Staphylococcus epiderm, Streptococcus mutans, Streptococcus pneumoniae and the like. Gram negative bacteria include, but are not limited to, Afipia felis, Bacteriodes, Bartonella bacilliformis, Bortadella pertussis, Borrelia burgdorferi, Borrelia recurrentis, Brucella, Calymmatobacterium granulomatis, Campylobacter, Escherichia coli, Francisella tularensis, Gardnerella vaginalis, Haemophilius aegyptius, Haemophilius ducreyi, Haemophilius influenziae, Heliobacter pylori, Legionella pneumophila, Leptospira interrogans, Neisseria meningitidia, Porphyromonas gingivalis, Providencia sturti, Pseudomonas aeruginosa, Salmonella enteridis, Salmonella typhi, Serratia marcescens, Shigella boydii, Streptobacillus moniliformis, Streptococcus pyogenes, Treponema pallidum, Vibrio cholerae, Yersinia enterocolitica, Yersinia pestis and the like. Other bacteria may include Myobacterium avium, Myobacterium leprae, Myobacterium tuberculosis, Bartonella henseiae, Chlamydia psittaci, Chlamydia trachomatis, Coxiella burnetii, Mycoplasma pneumoniae, Rickettsia akari, Rickettsia prowazekii, Rickettsia rickettsii, Rickettsia tsutsugamushi, Rickettsia typhi, Ureaplasma urealyticum, Diplococcus pneumoniae, Ehrlichia chafensis, Enterococcus faecium, Meningococci and the like.

In some embodiments, the cells are fungi. Non-limiting examples of fungi that may be analyzed using the disclosed methods, devices, and systems include, but are not limited to, Aspergilli, Candidae, Candida albicans, Coccidioides immitis, Cryptococci, and combinations thereof.

In some embodiments, the cells are protozoans or other parasites. Examples of parasites to be analyzed using the methods, devices, and systems of the present disclosure include, but are not limited to, Balantidium coli, Cryptosporidium parvum, Cyclospora cayatanensis, Encephalitozoa, Entamoeba histolytica, Enterocytozoon bieneusi, Giardia lamblia, Leishmaniae, Plasmodii, Toxoplasma gondii, Trypanosomae, trapezoidal amoeba, worms (*e.g.*, helminthes), particularly parasitic worms including, but not limited to, Nematoda (roundworms, *e.g.*, whipworms, hookworms, pinworms, ascarids, filarids and the like), Cestoda (*e.g.*, tapeworms).

*Example 1 - Cell Capture, Labeling, and Sequencing*

*Fabrication of microwell arrays:* Microwell arrays were fabricated using standard photolithography. An array (~35mm x 15mm) containing ~150,000 micropillars were patterned with SU-8 on a silicon wafer. PDMS was poured onto the wafer to create arrays of microwells. Replicas of the wafer were made with NOA63 optical adhesive using the PDMS microwell array as template. Agarose (5%, type IX-A, Sigma) microwell arrays were cast from the NOA63 replica before each experiment. For experiments described here, a subsection of the full array was cut and used, ranging from ~25,000 to 100,000 wells (Table 1). The size of the microwell array can be increased simply by increasing the total area of the microwell array pattern on the lithography mask and fabricated with the same steps above. For instance, a 3" x 2" microscope slide can hold ~1.4 million wells, for capturing ~140,000 single cells.

*Table 1.* Microwell array size, number of cells loaded, and cell capture efficiency on the array. Capture efficiency is defined as the number of unique cell barcode retained after data filtering as compared to the amount of cells loaded onto the microwell array based on hemocytometer counting. Note that in the current microwell array design, the total well area constituted ~23% of the total surface area. Cells that did not settle in the wells were washed away. Additionally, stringent filtering criteria (see Methods) tend to underestimate the total number of cells assayed, especially for samples with relatively shallow sequencing.

| | microwell array size | number of cell loaded based on hemacytometer counting | number of unique cell barcodes retained after filtering | special note | % captured |
|---|---|---|---|---|---|
| K562+Ramos | ~25000 | ~6250 | 765 | | ~12.2% |
| Primary B + Ramos | ~25000 | ~8000 | 1198 | | ~15.0% |
| PBMC | ~32500 | ~4000 | 632 | | ~15.8% |

(continued)

| | microwell array size | number of cell loaded based on hemacytometer counting | number of unique cell barcodes retained after filtering | special note | % captured |
|---|---|---|---|---|---|
| PBMC replicate | ~37500 | ~7000 | 731 | | ~10.4% |
| Donor 1 antiCD3/antiCD28 stimulated | ~100000 | ~8350 | 3517 | | ~42.1% |
| Donor 1 antiCD3/antiCD28 negative control | ~100000 | ~7680 | 1478 | | ~19.2% |
| Donor 2 antiCD3/antiCD28 stimulated | ~100000 | ~4800 | 669 | relative shallow sequencing | ~13.9% |
| Donor 2 antiCD3/antiCD28 negative control | ~100000 | ~18000 | 595 | relative shallow sequencing | ~3.3% |
| Donor 1 CMV stimulated | ~100000 | ~10000 | 581 | relative shallow sequencing | ~5.8% |
| Donor 1 CMV negative control | ~100000 | ~10000 | 253 | A portion of beads was lost after bead washing | ~2.5% |
| Donor 2 CMV stimulated | ~100000 | ~12000 | 2274 | | ~19.0% |
| Donor 2 CMV negative control | ~100000 | ~24000 | 2337 | | ~9.7% |
| | | | | | |
| **average** | | | | | **14.1%** |

*Synthesis of bead library:* Beads were manufactured using a split-pool combinatorial approach. Briefly, twenty-micron diameter magnetic beads functionalized with carboxyl groups (Spherotech) were distributed into 96 tubes. Using carbodiimide chemistry, a 5' amine modified oligonucleotide bearing a universal PCR priming sequence, a unique 8 nucleotide cell label and a common linker was coupled to the beads in each tube. Conjugated beads from all tubes were then pooled and split into a second set of 96 tubes for annealing to template oligonucleotides bearing the complement to the common linker, another 8 nucleotide cell label and a new common linker. Following enzymatic polymerization, the beads were again pooled and split into a third set of 96 tubes for annealing to oligonucleotides bearing oligo(dA)17 on the 5' end, followed by a randomly synthesized 8 nucleotide sequence that serves as the molecular index, a third 8 nucleotide cell label, and a complementary sequence to the second linker. After enzymatic polymerization, the beads were pooled to derive the final library. Each resulting bead is coated with tens to hundreds of millions of oligo-dT oligonucleotides of the same clonally represented cell label (884,736 or 96x96x96 possible barcodes), and a molecular indexing diversity of 65,536 ($4^8$). The library size is increased exponentially by linearly increasing the diversity at each step of synthesis.

*Sample preparation:* K562 and Ramos cells were cultured in RPMI-1640 with 10% FBS and 1x antibioticantimycotic. Primary B cells from a healthy donor were purchased from Sanguine Biosciences. PBMCs from a healthy donor were isolated from fresh whole blood in sodium heparin tube acquired from the Stanford Blood Center using Lymphoprep solution (StemCell).

*Single cell capture:* Cell density was measured by hemocytometer counting (SI Table 3) and adjusted to achieve ~1 captured cell per 10 or more microwells. The cell suspension was pipetted onto the microwell array and allowed to settle by gravity. Cell filling of microwells was confirmed by microscopy. Cells that settled on the surface in-between wells (~77% of the total surface area under current design) were removed and could be saved for future use. The bead library was then loaded at a density of ~5 beads per well to saturate all wells. Excess beads were washed away and cold lysis buffer (0.1M Tris-HCl pH 7.5, 0.5M LiCl, 1% LiSDS, 10mM EDTA, 5mM DTT) was pipetted over the surface of the microwell array. After 10 minutes of incubation on a slide magnet, the lysis buffer covering the array was removed and replaced by fresh lysis buffer. Beads with captured mRNAs were retrieved by placing the magnet on top of the microwell array. Beads in solution were collected into a microcentrifuge tube by pipetting, and washed twice in the tube with wash A buffer (0.1M Tris-HCl, 0.5M LiCl, 1mM EDTA) and once with wash B buffer (20mM Tris-HCl pH 7.5, 50mM KCl, 3mM MgCl$_2$). Under current implementation without the use of automation, this process takes up to 1.5 hours with two samples processed in parallel.

*cDNA synthesis:* Washed beads were resuspended in 40μl RT mix (Life Technologies,1x First Strand buffer, 20 units SuperaseIN RNase Inhibitor, 200 units SuperScript II or SuperScript III, 3mM additional MgCl$_2$, 1mM dNTP, 0.2μg/ml BSA) in a microcentrifuge tube rotated at 16rpm in an oven at 50°C for 50 minutes (when using SuperScript III for the early experiment with K562 and Ramos cells) or 42°C for 90 minutes (when using Superscript II for all other experiments). After cDNA synthesis, excess oligonucleotides on the beads were removed by treatment with 20units of ExoI (NEB) in 40μl of 1x ExoI buffer at 37°C for 30 minutes, and then inactivated at 80°C for 15 minutes.

*Multiplex PCR and sequencing:* Gene sequences were retrieved from RefSeq. Each marker panel consists of two sets of gene specific primers designed using Primer3. MATLAB was used to select PCR primers with minimal 3' end complementarity within each set (Table 2). The amplification scheme is shown in FIG. 5. PCR was performed on the beads with the KAPA Fast Multiplex Kit, using 50nM of each gene specific primer in the first primer set and 400nM universal primer in 50μl (for K562 and Ramos cell mixture), 100μl (for PBMC and B cell experiments) or 200μl (for T cell experiments), with the following cycling protocol: 3min at 95°C; 15 cycles of 15s at 95°C, 60s at 60°C, 90s at 72°C; 5min at 72°C. The increase in PCR volume was to mitigate the inhibitory effect of the iron on the magnetic beads. The beads were recovered using a magnet, and PCR products were purified with 0.7x Ampure XP (Beckman Coulter). Half of the purified products were used for the next round of nested PCR with the second primer set using the same KAPA kit and cycling protocol. After clean up with 0.7x Ampure XP, 1/10th of the product was input into a final PCR reaction whereby the full length Illumina adaptors were appended (1x KAPA HiFi Ready Mix, 200nM of primer P5, 200nM of primer P7. 95°C 5min; 8 cycles of 98°C 15s, 60°C 30s, 72°C 30s; 72°C 5min). Sequencing was performed on the Illumina MiSeq instrument with 150x2 bp chemistry at a median depth of 1.6 million reads per sample (Table 3).

Table 2A. Gene panel for K562 and Ramos cell mixture.

| Gene | Outer Primer | Nested Primer with Common 5' Flanking Sequence |
|---|---|---|
| CD41 | CCCCTGGAAGAAGATGATGA | CAGACGTGTGCTCTTCCGATCTTTCTCCAACAAGTTGCCTCC |
| GYPD | GAGGAAATGAAGCCAAACACA | CAGACGTGTGCTCTTCCGATCTAATCGTGACCTTAAAGGCCC |
| GATA1 | TTAGCCACCTCATGCCTTTC | CAGACGTGTGCTCTTCCGATCTCTACTGTGGTGGCTCCGCT |
| GATA2 | GGAGGAGGATTGTGCTGATG | CAGACGTGTGCTCTTCCGATCTGTGTCCGCATAAGAAAAGAATC |
| HBG1 | GCAAGAAGGTGCTGACTTCC | CAGACGTGTGCTCTTCCGATCTCTGCATGTGGATCCTGAGAA |
| CD27 | CTGCAGTCCCATCCTCTTGT | CAGACGTGTGCTCTTCCGATCTGATGAGGTGGAGAGTGGGAA |
| IGJ | GGACATAACAGACTTGGAAGCA | CAGACGTGTGCTCTTCCGATCTCAATCCATTTTGTAACTGAACCTT |
| TCL1A | AAGCCTCTGGGTCAGTGGT | CAGACGTGTGCTCTTCCGATCTTGGAAAAGGGATAGAGGTTGG |
| CD74 | TAGACAGATCCCCGTTCCTG | CAGACGTGTGCTCTTCCGATCTACAGGGAGAAGGGATAACCC |
| SEPT9 | CAGCATCCCAGCCTTGAG | CAGACGTGTGCTCTTCCGATCTCCTCAATGGCCTTTTGCTAC |
| CD79a | CCTCTAAACTGCCCCACCTC | CAGACGTGTGCTCTTCCGATCTCCTTAATCGCTGCCTCTAGG |
| GAPDH | CACATGGCCUCCAAGGAGUAA | CAGACGTGTGCTCTTCCGATCTCAGCAAGAGCACAAGAGGAA |

*Table 2B.* B cell gene panel.

| Gene | Outer Primer | Nested Primer with Common 5' Flanking Sequence |
|---|---|---|
| CD19 | GCAGGGGTCCCAGTCCTATG | CAGACGTGTGCTCTTCCGATCTCCAATCATGAGGAAGATGCA |
| CD27 | TCCAGGAGGATTACCGAAAA | CAGACGTGTGCTCTTCCGATCTCCATCCAAGGGAGAGTGAGA |
| CD138 | AATGGCAAAGGAAGGTGGAT | CAGACGTGTGCTCTTCCGATCTGCAGACACCTTGGACATCCT |
| CD38 | AGATCTGAGCCAGTCGCTGT | CAGACGTGTGCTCTTCCGATCTTGGTGCAGAGCTGAAGATTTT |
| CD24 | AAAAGTGGGCTTGATTCTGC | CAGACGTGTGCTCTTCCGATCTTTTTGTTCGCATGGTCACAC |
| CD10 | ATATTCCTTTGGGCCTCTGC | CAGACGTGTGCTCTTCCGATCTTCAAGTTTGGGTCTGTGCTG |
| CD95 | CCCCCGAAAATGTTCAATAA | CAGACGTGTGCTCTTCCGATCTTGCTCTTGTCATACCCCCA |
| CD21 | TAGCTTCCTCCTCTGGTGGT | CAGACGTGTGCTCTTCCGATCTTTTGCCTTTCCATAATCACTCA |
| CXCR3 | CTGGCTCTCCCCAATATCCT | CAGACGTGTGCTCTTCCGATCTGCTCTGAGGACTGCACCATT |
| CD40 | GTGGTGTTGGGGTATGGTTT | CAGACGTGTGCTCTTCCGATCTATACACAGATGCCCATTGCA |
| CD69 | AGACAGGTCCTTTTCGATGG | CAGACGTGTGCTCTTCCGATCTTGTGCAATATGTGATGTGGC |
| CD1c | TTGAGACAGGCACATACAGCTT | CAGACGTGTGCTCTTCCGATCTTTGCTTCCTCAATCTGTCCA |
| IL10 | CCCCAACCACTTCATTCTTG | CAGACGTGTGCTCTTCCGATCTTTCAATTCCTCTGGGAATGTT |
| IL4R | TGCCTAGAGGTGCTCATTCA | CAGACGTGTGCTCTTCCGATCTGTTGATGCTGGAGGCAGAAT |
| IL21R | AGCCTGGGTCACAGATCAAG | CAGACGTGTGCTCTTCCGATCTAGGTAGGAGGGTGGATGGAG |
| IL6R | CCAGCACCAGGGAGTTTCTA | CAGACGTGTGCTCTTCCGATCTAGGAAAGGATTGGAACAGCA |
| CXCL12 | GGGTTTCAGGTTCCAATCAG | CAGACGTGTGCTCTTCCGATCTTTTGTAACTTTTTGCAAGGCA |
| CCL3 | GTGAGGAGTGGGGTCCAGAAA | CAGACGTGTGCTCTTCCGATCTAGTGGGGAGGAGCAGGAG |
| CCL14 | CCATTCCCTTCTTCCTCCTC | CAGACGTGTGCTCTTCCGATCTTACCTACAAGATCCCGCGTC |
| CCL20 | TTGGACATAGCCCAAGAACA | CAGACGTGTGCTCTTCCGATCTTGTGCCTCACTGGACTTGTC |
| CCL18 | ACCTGAAGCTGAATGCCTGA | CAGACGTGTGCTCTTCCGATCTCTGGAGGCCACCTCTTCTAA |
| TCL1A | GGTAAACACGCCTGCAAAC | CAGACGTGTGCTCTTCCGATCTCAGGACTCAGAAGCCTCTGG |
| TACI | CAACAAAGCACAGTGTTAAATGAA | CAGACGTGTGCTCTTCCGATCTTGTGTCAGCTACTGCGGAAA |
| AICDA | TGAGCAGATCCACAGGAAAA | CAGACGTGTGCTCTTCCGATCTGAAATGGAGTCTCAAAGCTTCA |
| FCRL4 | TCCCAACTACGCTGATTTGA | CAGACGTGTGCTCTTCCGATCTGACCAAAGGAATGTGTGGG |

| Gene | Outer Primer | Nested Primer with Common 5' Flanking Sequence |
|---|---|---|
| BCL2 | TGCAAGAGTGACAGTGGATTG | CAGACGTGTGCTCTTCCGATCTTCAACCAAGGTTTGCTTTTGT |
| FASLG | AGAGGCTGAAAGAGGCCAAT | CAGACGTGTGCTCTTCCGATCTAATATGGGTTGCATTTGGTCA |
| BCL6 | AAATCTGCAGAAGGAAAAATGTG | CAGACGTGTGCTCTTCCGATCTAGTTTTCAATGATGGGCGAG |
| AURKB | GCTCAAGGGAGAGCTGAAGA | CAGACGTGTGCTCTTCCGATCTGACTACCTGCCCCCAGAGAT |
| CD81 | GTGGCGTGTATGAGTGGAGA | CAGACGTGTGCTCTTCCGATCTCACTCGCCCAGAGACTCAG |
| CD80 | GCACATCTCATGGCAGCTAA | CAGACGTGTGCTCTTCCGATCTGCTTCACAAACCTTGCTCCT |
| CD23a | ACATTTTCTGCCACCCAAAC | CAGACGTGTGCTCTTCCGATCTAACAGCACCCTCTCCAGATG |
| CD44 | GCCTGGTAGAATTGGCTTTTC | CAGACGTGTGCTCTTCCGATCTTTTTGTAGCCAACATTCATTCAA |
| LEF1 | CAATTGGCAGCCCTATTTCA | CAGACGTGTGCTCTTCCGATCTGTTCAGCAGACTGGTTTGCA |
| CXCR5 | CCGTGAGGATGTCACTCAGA | CAGACGTGTGCTCTTCCGATCTACGAGGAAGCCCTAAGACGT |
| PRKCB | TTGAGCCTGGGGTGTAAGAC | CAGACGTGTGCTCTTCCGATCTGTCTTCCAGGATTCACGGTG |
| PRKCD | GAGCACCTCCTGGAAGATTG | CAGACGTGTGCTCTTCCGATCTTAAGCACCAGTGGGACTGTG |
| CD20 | TAGGAGCAGGCCTGAGAAAA | CAGACGTGTGCTCTTCCGATCTGATTCCTCTCCAAACCCATG |
| CD30 | TGTTTTGGGGAAAGTTGGAG | CAGACGTGTGCTCTTCCGATCTCTGTTTGCCCAGTGTTTGTG |
| CD30L | TGCAACCCAACTGTGTGTTA | CAGACGTGTGCTCTTCCGATCTTTTTCACCAACTGTTCTCTGAGC |
| BAFFR | GCCCTGAGCAACAATAGCAG | CAGACGTGTGCTCTTCCGATCTTTTCAGCTCTTCACTCCAGCA |
| CMRF-35H | AGGAAAAGATGTGGCTCACG | CAGACGTGTGCTCTTCCGATCTGGAGTTGGGGAGAACTGTCA |
| PRDM1 | TCGAATAATCCAGGGAAACC | CAGACGTGTGCTCTTCCGATCTACCAAAGCATCACGTTGACA |
| HLA-DRA | GGCTTTACAAAGCTGGCAAT | CAGACGTGTGCTCTTCCGATCTTATGCCTCTTCGATTGCTCC |
| GNAI2 | CCTTGAGTGTGTCTGCGTGT | CAGACGTGTGCTCTTCCGATCTCCACAGAATTGGGTTCCAAG |
| RGS1 | AACTGGGAAGGCCAGGTAAC | CAGACGTGTGCTCTTCCGATCTTGTTTTCAAATTGCCATTGC |
| CD5 | CTTTCTCCACGCCATTTGAT | CAGACGTGTGCTCTTCCGATCTACTAGGATATGGGGTGGGCT |
| CD22 | GGGATCTGCTCGTCATCATT | CAGACGTGTGCTCTTCCGATCTGTTTCTGCCTCTGAGGGAAA |
| PIK3CD | GCGTGCGCGTTATTTATTTA | CAGACGTGTGCTCTTCCGATCTTGTCTGGGGAAGGCAAGTTA |
| DOCK8 | GCAGTCAGCCAGAAATCACA | CAGACGTGTGCTCTTCCGATCTTTTTTCTCCTCTCTGGGACCA |

(continued)

| Gene | Outer Primer | Nested Primer with Common 5' Flanking Sequence |
|---|---|---|
| CD11b | TGAAAAGTCTCCCTTTCCAGA | CAGACGTGTGCTCTTCCGATCTCCTTCAGACAGATTCCAGGC |
| FCGR2B | GGAGAGGAGAGATGGGGATT | CAGACGTGTGCTCTTCCGATCTGAGTGAGTGCCCCTTTTCTT |
| CD72 | CTCATGCCAACAAGAACCTG | CAGACGTGTGCTCTTCCGATCTTGACCCACACCTGACACTTC |
| BCL11B | TCGTGGAACACAGGCAAAC | CAGACGTGTGCTCTTCCGATCTTTGCATTTGTACTGGCAAGG |
| CD86 | TCAAGGCAACCAGAGGAAAC | CAGACGTGTGCTCTTCCGATCTACTAAGGGATGGGGCAGTCT |
| TBX21 | ACCTTTTCGTTGGCATGTGT | CAGACGTGTGCTCTTCCGATCTTCAGGGAAAGGACTCACCTG |
| FOXP1 | ATGCTGAAGGCATTTCTTGG | CAGACGTGTGCTCTTCCGATCTCTGTGAGCATGGTGCTTCAT |
| MCL1 | GAGGGGAGTGGTGGGTTTAT | CAGACGTGTGCTCTTCCGATCTCAAAAGGGAAAGGGAGGATT |
| IFNB1 | AGGGGAAAACTCATGAGCAG | CAGACGTGTGCTCTTCCGATCTTCACTGTGCCTGGACCATAG |
| BLNK | TTGGGCAGAAAGAAAAATGG | CAGACGTGTGCTCTTCCGATCTCAAAAGATTCCACCAGACTGAA |
| CD40LG | CCTCCCCCAGTCTCTCTTCT | CAGACGTGTGCTCTTCCGATCTGAGTCAGGCCGTTGCTAGTC |
| IGBP1 | GGCTGATCTTCCCACAACAC | CAGACGTGTGCTCTTCCGATCTACGAGGGCAAAGATGCTAAA |
| IRF4 | ATTCCCGTGTTGCTTCAAAC | CAGACGTGTGCTCTTCCGATCTAGAACTGCCAGCAGGTAGGA |
| CD79a | CACTTCCCTGGGACATTCTC | CAGACGTGTGCTCTTCCGATCTCTCACTCTTCTCCAGGCCAG |
| LTA | TGATGTCTGTCTGGCTGAGG | CAGACGTGTGCTCTTCCGATCTCCACACACAGAGGAAGAGCA |
| HDAC5 | CCAGCCTGTAGGAAACCAAA | CAGACGTGTGCTCTTCCGATCTCTCCTTCTATCTCCAGGGCC |
| RAG1 | GGATGCAGGTGGTTTTTGAT | CAGACGTGTGCTCTTCCGATCTCATTGTACCCATTTTACATTTTCTT |
| RAG2 | CAAACCTTAAACACCCAGAAGC | CAGACGTGTGCTCTTCCGATCTATAACAATTCGGCAGTTGGC |
| CD1d | GAACCAGTTTCCTCCTGTGC | CAGACGTGTGCTCTTCCGATCTAAGATGTGGAGGCTGTTGCT |
| TGFB1 | GACTGCGGATCTCTGTGTCA | CAGACGTGTGCTCTTCCGATCTTCTGCACTATTCCTTTGCCC |
| CD9 | TCAGTATGATCTTGTGCTGTGCT | CAGACGTGTGCTCTTCCGATCTTACCCATGAAGATTGGTGGG |
| CD11c | CACAGCATGAGAGGCTCTGT | CAGACGTGTGCTCTTCCGATCTTCTCAGTTCCGATTTCCCAG |
| FOXP3 | TCAGGATCTGAGGTCCCAAC | CAGACGTGTGCTCTTCCGATCTTCACCTGTGTATCTCACGCA |
| LAG3 | AGAGCTGTCTAGCCCAGGTG | CAGACGTGTGCTCTTCCGATCTTGGTGTCCTTTCTCTGCTCC |
| CD73 | CTTAACGTGGGAGTGGAACC | CAGACGTGTGCTCTTCCGATCTGTGTGCAAATGGCAGCTAGA |

(continued)

| Gene | Outer Primer | Nested Primer with Common 5' Flanking Sequence |
|---|---|---|
| CD70 | TCTCAGCTTCCACCAAGGTT | CAGACGTGTGCTCTTCCGATCTTCACTGGGACACTTTTGCCT |
| CCR7 | CAGGGGAGAGTGTGGTGTTT | CAGACGTGTGCTCTTCCGATCTGACATGCACTCAGCTCTTGG |
| CD45RA | TGCATAGTTCCCATGTTAAATCC | CAGACGTGTGCTCTTCCGATCTTACCAGGAATGGATGTCGCT |
| PDCD1 | ACATCCTACGGTCCCAAGGT | CAGACGTGTGCTCTTCCGATCTGCAGAAGTGCAGGCACCTA |
| MYC | TGCATGATCAAATGCAACCT | CAGACGTGTGCTCTTCCGATCTTTGGACTTTGGGCATAAAAGA |
| CD25 | AAATCACGGCAGTTTTCAGC | CAGACGTGTGCTCTTCCGATCTCTCATCTGTGCACTCTCCCC |
| FCAMR | GTGGGAAGAGAAGCTGATGC | CAGACGTGTGCTCTTCCGATCTTCAAGCATTATCCACGTCCA |
| CCND2 | TGTGATGCCATATCAAGTCCA | CAGACGTGTGCTCTTCCGATCTTCAGTGTATGCGAAAAGGTTTT |
| MKI67 | AGCCTCTCTTGGGCTTTCTT | CAGACGTGTGCTCTTCCGATCTGTTTTCCCTGCCTGGAACTT |
| CCND3 | CTTTGCTGCTGAAGGCTCAT | CAGACGTGTGCTCTTCCGATCTACAAGTGGTGGTAACCCTGG |
| IL12A | TGCTTCCTAAAAAGCGAGGT | CAGACGTGTGCTCTTCCGATCTGAACTAGGGAGGGGGAAAGA |
| IFNG | GCAGCCAACCTAAGCAAGAT | CAGACGTGTGCTCTTCCGATCTATCCAGTTACTGCCGGTTTG |
| TNFA | GAATGCTGCAGGACTTGAGA | CAGACGTGTGCTCTTCCGATCTACTTCCTTGAGACACGGAGC |
| IL2 | ACCCAGGGACTTAATCAGCA | CAGACGTGTGCTCTTCCGATCTGCTGATGAGACAGCAACCATT |
| IL4 | GACATCTTTGCTGCCTCCA | CAGACGTGTGCTCTTCCGATCTATGAGAAGGACACTCGCTGC |
| IL6 | TTAAGGAGTTCCTGCAGTCCA | CAGACGTGTGCTCTTCCGATCTTCCACTGGGCACAGAACTTA |
| BAFF | TCCTTCGCTTTGCTTGTCTT | CAGACGTGTGCTCTTCCGATCTAGGTGGAAAAATAGATGCCAGTC |
| IGHE | CCCGGAAGTCTATGCGTTT | CAGACGTGTGCTCTTCCGATCTAGGACATCTCGGTGCAGTG |
| IGHD | TGTGTGAGGTGTCTGGCTTC | CAGACGTGTGCTCTTCCGATCTAGGAGCACCACGTTCTGG |
| IGHM | CCCGGAGAAGTATGTGACCA | CAGACGTGTGCTCTTCCGATCTGTACTTCGCCCACAGCATC |
| IGHA | CTGAACGAGCTGGTGACG | CAGACGTGTGCTCTTCCGATCTAGTACCTGACTTGGGCATCC |
| IGHG1 | CAAGGGCCCATCGGTCTT | CAGACGTGTGCTCTTCCGATCTTTGTGACAAAACTCACACATGC |
| IGHG4 | CAAGGGCCCATCGGTCTT | CAGACGTGTGCTCTTCCGATCTCAAATATGGTCCCCCATGC |
| IGHG2 | CAAGGGCCCATCGGTCTT | CAGACGTGTGCTCTTCCGATCTGCAAATGTTGTGTCGAGTGC |
| IGHG3 | CAAGGGCCCATCGGTCTT | CAGACGTGTGCTCTTCCGATCTACCCCACTTGGTGACACAAC |

(continued)

| Gene | Outer Primer | Nested Primer with Common 5' Flanking Sequence |
|---|---|---|
| TLR1 | CCATTCCGCAGTACTCCATT | CAGACGTGTGCTCTTCCGATCTAAGGAAAAGAGCAAACGTGG |
| TLR2 | TTGGTTGACTTCATGGATGC | CAGACGTGTGCTCTTCCGATCTGGAAACAGCACAAATGAACTTAA |
| TLR3 | CATCATGCAGTTCAACAAGC | CAGACGTGTGCTCTTCCGATCTATGCACTCTGTTTGCGAAGA |
| TLR4 | GGGTGTGTTTCCATGTCTCA | CAGACGTGTGCTCTTCCGATCTTTGAAAGTGTGTGTGTCCGC |
| TLR5 | TCAGGCTGTTGCATGAAGAA | CAGACGTGTGCTCTTCCGATCTGTATGCCCTTGCTGGACCTA |
| TLR6 | ATGCGCAGTAAAAACTCGTG | CAGACGTGTGCTCTTCCGATCTTACAGTTCCACGCTGAGCTG |
| TLR7 | GCCTGTACTTTCAGCTGGGTA | CAGACGTGTGCTCTTCCGATCTAAGGTGTTTGTGCCATTTGG |
| TLR8 | GGTGAGCTCTGATTGCTTCA | CAGACGTGTGCTCTTCCGATCTTATCAGGAGGCAGGGATCAC |
| TLR9 | GACCGGGTCAGTGGTCTCT | CAGACGTGTGCTCTTCCGATCTGGTGATCCTGAGCCCTGAC |
| TLR10 | TGCAGTGAGCTGAGATCGAG | CAGACGTGTGCTCTTCCGATCTATGGAAAACATCCTCATGGC |
| GAPDH | CACATGGCCUCCAAGGAGUAA | CAGACGTGTGCTCTTCCGATCTCAGCAAGAGCACAAGAGGAA |

Table 3. Sequencing and alignment statistics.

| experiment | total number of reads | number of reads with exactly 1 match to gene in panel | % reads aligned to one gene in the panel | number of reads with exact match to a cell barcode and alignment to one gene | % read after gene and barcode alignment | number of unique cell barcodes that satisfy filtering criteria | number of reads associated with those cell barcodes |
|---|---|---|---|---|---|---|---|
| K562 + Ramos | 2399025 | 2154454 | 90% | 1175715 | 49% | 765 | 913642 |
| Primary B+ Ramos | 5711013 | 5203308 | 91% | 3495392 | 61% | 1198 | 2868577 |
| PBMC | 1270214 | 1105687 | 87% | 803151 | 63% | 632 | 670576 |
| PBMC replicate | 3927672 | 3468538 | 88% | 2459367 | 63% | 731 | 1920956 |
| Donor 1 antiCD3/antiCD28 stimulated | 3529898 | 3249998 | 92% | 2122416 | 60% | 3517 | 1466000 |
| Donor 1 antiCD3/antiCD28 negative control | 1557996 | 1292211 | 83% | 939094 | 60% | 1478 | 719351 |
| Donor 2 antiCD3/antiCD28 stimulated | 606865 | 552877 | 91% | 403943 | 67% | 669 | 246234 |
| Donor 2 antiCD3/antiCD28 negative control | 332951 | 283723 | 85% | 205762 | 62% | 595 | 86866 |
| Donor 1 CMV stimulated | 1064648 | 958410 | 90% | 697057 | 65% | 581 | 401629 |
| Donor 1 CMV negative control | 619957 | 547259 | 88% | 406801 | 66% | 253 | 192605 |
| Donor 2 CMV stimulated | 1902977 | 1692734 | 89% | 1229667 | 65% | 2274 | 688296 |
| Donor 2 CMV negative control | 1671419 | 1346637 | 81% | 977344 | 58% | 2337 | 715453 |

*Data analysis:* The cell label, the molecular index, and the gene identity were detected on each sequenced read (FIG. 5). Gene assignment for the second paired read (read 2) was performed using the alignment software 'bowtie2' (44) with default settings. Cell labels and molecular indices on the first paired read (read 1) were analyzed using custom MATLAB scripts. Only reads perfectly matching the combinatorial cell barcodes were retained, but this requirement may be further relaxed since the cell barcodes were designed to enable error correction (M. Hamady, et al. (2008), Nat. Methods 5, 235-237). Reads were grouped first by cell label, then by gene identity and molecular index. To calculate the number of unique molecules per gene per cell, the molecular indices of reads of the same gene transcript from the same cell were clustered. Edit distance greater than 1 nucleotide was considered as a unique cluster, and thus a unique transcript molecule. A table containing the digital gene expression profile of each cell was constructed for each sample - each row in the table represents a unique cell, each column represents a gene, and each entry in the table represents the count of unique transcript molecules for that gene in any given cell. The table was filtered to remove unique molecules that were sequenced only once (*i.e.* redundancy = 1). For the experiment with mixture of K562 and Ramos cells, cells with 30 or more total unique molecules were retained for clustering. For the rest of the experiments, cells with a sum of 10 or more unique molecules or with co-expression of 4 or more genes in the panel were retained for clustering. The filtered table was then used for clustering analysis. Principal component analysis and hierarchical clustering was performed on natural log-transformed transcript count (with pseudo-count of 1 added) with built-in functions in MATLAB.

*Measurement of GAPDH copy number in single Ramos cells using alternative methods:* In the first method, total RNA from Ramos cells was extracted by RNeasy Mini Kit (Qiagen) and quantified by Nanodrop. Serial dilutions down to 7 pg were prepared and loaded onto the 12.765 Digital Array (Fluidigm) with 1x EXPRESS SuperScript qPCR mix (Life Technologies), 1x EXPRESS SuperScript enzyme mix (Life Technologies), 1x GAPDH FAM assay (ABI), 1x Loading Reagent (Fluidigm), and 1x ROX dye. The array was analyzed on the BioMark (Fluidigm) with the following protocol: 50°C for 15min, 95°C for 2min, and 35 cycles of 95°C for 15s and 60°C for 60min. GAPDH was measured to be 34 copies per pg of total RNA. In the second method, a Ramos cell suspension was diluted in PBS to about 1 cell per 10 microliters. A microliter of suspension was pipetted into multiple 0.2 ml tubes. The presence of a single cell in a tube was confirmed by microscopy. GAPDH counts in the single cells were determined using the method outlined in Fu, et al. (2014), Analytical Chemistry 86, 2867-2870. Measurements were obtained from 8 cells. An average of 214 +/- 36 (S.E.) copies (range 113 to 433 copies) was obtained per cell. GAPDH counts per spiked in Ramos cells (18 single cells) were compared to these two methods to evaluate RNA detection efficiency.

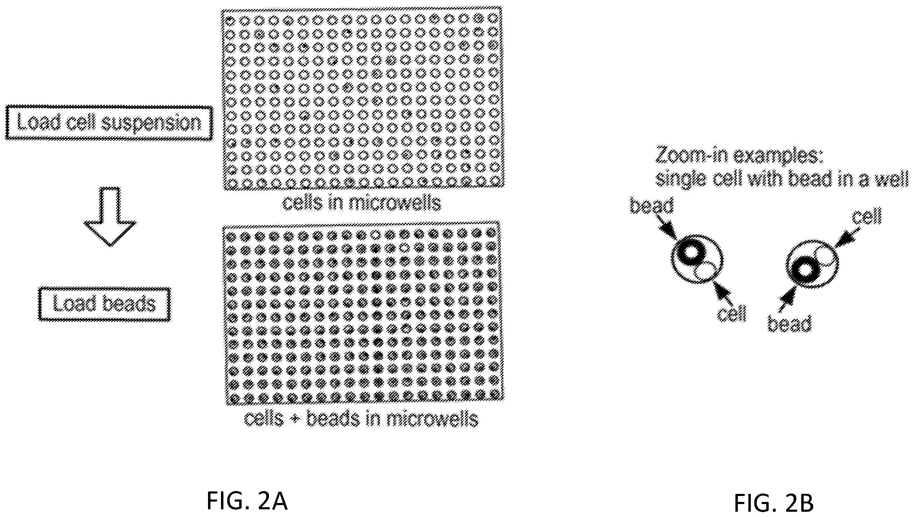

*Results:* The single cell, stochastic labeling and molecular barcoding procedure is outlined in FIGS. 1 and 2. First, a cell suspension is loaded onto a microfabricated surface with up to 100,000 microwells. Each 30 micron diameter microwell contains a volume of -20 picoliters. The number of cells in the suspension is adjusted so that only about one out of ten wells receives a cell. Cells simply settle into wells by gravity.

Next, the bead library is loaded onto the microwell array to saturation, so that most wells become filled. The dimensions of the beads and wells have been optimized to prevent double occupancy of beads. Each 20 micron bead has been functionalized with tens to hundreds of millions of oligonucleotide primers of the structure outlined in FIG. 1. Oligonucleotides consist of a universal PCR priming site, followed by a combinatorial cell label, a molecular index, and an mRNA capture sequence of oligo(dT). All primers on each bead share the same cell label but incorporate a diversity of molecular indices. A combinatorial split-pool synthesis method was devised to generate the bead library attaining a cell labeling diversity of close to one million. Since only ~1% of the total available cell label diversity is used, the probability of having two single cells tagged with the same label is low (on the order of $10^{-4}$). Similarly, the diversity of the molecular labels on a single bead is on the order of $10^5$, so that the likelihood of two transcript molecules of the same gene from the same cell tagged with the same molecular index is low. Once single cells and beads are co-localized in the microwells, a lysis buffer is applied onto the surface and allowed to diffuse inside. The high local concentration of released mRNAs (tens of nanomolar) effectively drives their hybridization onto the beads.

Following mRNA capture, all beads are magnetically retrieved from the microwell array. From this point on, all reactions are carried out in a single tube. After reverse transcription, cDNA molecules synthesized on each bead become encoded with cell and molecular barcodes and serve as amplification templates (FIGS. 1 and 5A-D).

Sequencing of amplification products reveals the cell label, the molecular index, and the gene identity (FIGS. land 5A-D). Computational analysis groups the reads based on the cell label, and collapses the reads with the same molecular index and gene sequence into a single entry to correct for amplification bias, allowing the determination of absolute transcript numbers for each gene in each cell.

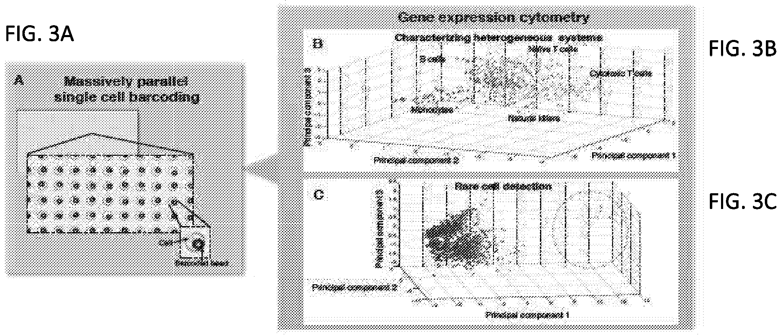

FIG. 3A depicts single cells trapped in microwells along with beads comprising libraries of tethered stochastic labels (one cell and one bead per well). FIG. 3B depicts an example of principal component analysis of stochastic barcoding data for human peripheral blood mononuclear cells. FIG. 3C depicts an example of principal component analysis of stochastic barcoding data for a rare cell population.

*Example 2 - Identifying Cell Types in Cell Mixtures*

*Methods:* The methods used for this example were the same as described for Example 1.

*Results:* To demonstrate the ability of single cell stochastic labeling or molecular barcoding to identify individual cells among a population of two cell types, a ~1:1 mixture of K562 (myelogenous leukemia) and Ramos (Burkitt's lymphoma) cells was loaded onto a partial array of ~25,000 microwells. A panel of 12 genes was selected and amplified from the cDNA beads and sequenced. The panel consisted of 5 genes specific for K562 cells, 6 genes specific for Ramos cells, and the common housekeeping gene GAPDH (Table 2A). The majority of the sequencing reads (~78%) were associated with 765 unique cell labels.

The gene expression profile of each cell was clustered using principal component analysis (PCA) (FIG. 4A). The first principal component (PC) separated the cells into two major clusters based on cell type. The genes that contribute to the positive side of the first PC were specific to Ramos, while the genes that contributed to the negative side of the same PC were specific to K562. The second PC highlighted the high degree of variability in fetal hemoglobin (HBG1) expression within the K562 cells, which has been observed previously (G. Fu, et al. (2014), Analytical Chemistry 86, 2867-2870, R. D. Smith, et al. (2000), Nucleic Acids Res. 28, 4998-5004).

Next, we spiked in a small number of Ramos cells into primary B cells from a healthy individual. A panel of 111 genes (Table 2B) known to be involved in B cell function (D. A. Kaminski, et al. (2012), Frontiers in Immunology 3:302;

Y. Shen et al. (2004), BMC Immunol. 5:20; J. A. Weinstein, et al. (2013), PloS One 8:e67624) was analyzed across 1,198 cells. Eighteen cells (-1.5% of the population) were found to have a distinct gene expression pattern (FIG. 4B). Genes preferentially expressed by this group are known to be associated with Burkitt's lymphoma, and include MYC and IgM, and markers (CD10, CD20, CD22, BCL6) associated with follicular B cells from which Burkitt's lymphoma originates (22) (FIG. 4C). In addition, this group of cells contained higher levels of CCND3 and GAPDH, as well as an overall higher mRNA content (FIG. 4B). This finding is consistent with the fact that lymphoma cells are physically larger than primary B cells in normal individuals, and that they are rapidly proliferating and transcriptionally more active.

Detected copies of GAPDH were used to estimate the RNA capture efficiency of the single cell, stochastic labeling or molecular barcoding assay. GAPDH in 10pg of Ramos total RNA was measured to be -343 copies using digital RT-PCR. Additionally, we tested individual Ramos cells using a sensitive molecular indexing technique (G. Fu, et al. (2014) and determined an average of 214 +/- 36 (S.E.) GAPDH transcripts per cell, comparable to the 152 +/- 10 (S.E.) copies yielded by single cell, stochastic labeling or molecular barcoding.

*Example 3 - Fabrication of Microwell Arrays Having Domed Ridges*

In some embodiments of the disclosed methods and systems, it may be desirable to fabricate microwell arrays having domed ridges (or other features) between the wells in order to minimize the number of cells or beads that settle between wells. FIG. 7A shows a micrograph of a microwell array having domed surfaces or ridges between wells. FIG. 7B shows a side view of the micropillar array used to mold the microwells, in which the top surface of each pillar was rounded off using a reflow process (the arrow indicates the curved edge of one micropillar). Micropillar arrays were fabricated using optical lithography techniques and a positive photoresist (MicroChemicals AZ® 40 XT). Following development of the resist, the micropillar array was subjected to heat (110 °C to 130 °C) for time intervals ranging from 30 seconds to 4 minutes. In general, higher heat and/or longer exposure times resulted in more curvature. The micropillar array shown in FIG. 7B was heated at 130 °C for 30 seconds. The resulting micropillar arrays were used to cast polydimethylsiloxane (PDMS) intermediate structures, which were then used to micromold the microwell arrays in agarose, Norland optical adhesive, or other polymers. The microwell array shown in FIG. 7A has wells of approximately 30 $\mu$m in diameter and 30 to 40 $\mu$m deep. The micropillars used to cast the microwells were approximately 45 $\mu$m in diameter prior to being subjected to the reflow process. As can be seen in the micrograph of FIG. 7A, there were relatively few cells or beads that settled on the substrate surface between wells. Microwell arrays having a domed ridge between the wells generally exhibited significantly fewer cells or beads settling between wells than microwell arrays having flat surfaces between the wells.

FIGS. 8A-B shows micrographs of microwell arrays molded with a micropillar master before and after subjecting the micropillar array to a reflow process. FIG. 8A shows a microwell array having flat substrate surfaces between wells, as fabricated using a micropillar master before subjecting it to a reflow process. Beads (22 $\mu$m diameter) were loaded onto the array and allowed to settle without futher agitation of the fluid. As can be seen in the micrograph, a significant number of beads settled on the flat substrate surfaces between wells. FIG. 8B shows a microwell array having domed ridges between wells, as fabricated with a micropillar master that had been subjected to a reflow process comprising heating at 110 °C for 1 minute. Again, 22 $\mu$m diameter beads were loaded onto the array and allowed to settle without further agitation of the fluid. As can be seen in the micrograph, essentially no beads settled on the substrate surfaces between wells.

*Example 4 - Imaging System Design*

In some embodiments, the instrument is a bright-field microscope using trans-illumination. The illumination system and imaging system are coaxial to each other and located on opposite sides of the sample. The instrument is used to count cells and beads so that the number of cells (usually 0, 1, or 2) and beads (usually 0 or 1) in each well can be determined. High-resolution images of individual cells are not required.

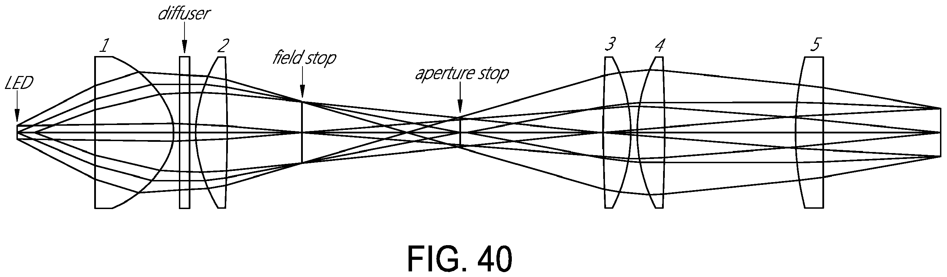

One embodiment of the illumination system is shown in FIG. 40. The light source (LED Engin LZ4 20MA00) is a 4-color source consisting of a 2 x 2 array of single-color LEDs (blue, green, yellow, red) mounted in close proximity to each other on a small circuit board. The LEDs can be controlled individually. In many embodiments of the instrument, only one LED is used at a time. In some embodiments, more than one LED may be used simultaneously. Lens **1** (Thorlabs ACL2520-A) is an aspheric lens with a focal length of 20 mm. Lens **2** (Edmund Optics 66017) is an aspheric lens with a focal length of 40 mm. Lenses **3** and **4** (Edmund Optics 66018) are aspheric lenses with focal lengths of 50 mm. Lens **5** (Edmund Optics 47350) is a spherical lens with a focal length of 100 mm. The aperture stop and field stop (Thorlabs SM1D12C) are iris diaphragms with maximum iris diameters of 12 mm. The diffuser (Thorlabs ED1-C20) produces radially symmetric uniform scattering at angles from 0 to 10 degrees and very little scattering at larger angles.

Light emitted by the light source is collimated by lens **1** and focused onto the aperture stop by lens **2.** Because none of the LEDs are centered on the optical axis, a diffuser is needed to ensure that the emitted light uniformly fills the aperture stop. In some embodiments, in which an adequately large 1-color LED is centered on the optical axis, the diffuser may not be necessary. The field stop is located between lens 2 and the aperture stop. Lenses **3** and **4** image the field stop onto the sample plane. Each point in the sample plane is illuminated by a bundle of rays having a numerical aperture set by the aperture stop. In the absence of lens **5** the illuminator is telecentric, meaning at each point in the field the chief ray is perpendicular to the sample plane. For each point in the field, the chief ray is the ray that passes through the center of the aperture stop. A telecentric illuminator is appropriate if the imaging lens is telecentric. In some embodiments of the instrument, the imaging lens is not telecentric. The purpose of lens **5** is to bend the bundles of rays so that at each point in the field the chief ray is aimed at the center of the aperture stop of the imaging lens. Except for the addition of the diffuser and lens **5**, the illuminator is similar to Kohler illuminators that have been used for microscopy since 1893.

Many variations of the illuminator design are possible. For example, in some embodiments, lenses **1** and **2** may be replaced by a single lens. In some embodiments, lenses **3**, **4**, and **5** may be replaced by a single lens. Achromatic lenses containing two or more elements may be used instead of one-element lenses that are not achromatic. In some embodiments, the iris diaphragms may be replaced by apertures of fixed diameter. In some embodiments, the circular field stop may be replaced by a square or rectangular field stop (*e.g.* to match the shape of the sensor used in the imaging system) or may be omitted entirely. In some embodiments, Abbe illumination may be used instead of Kohler illumination.

One embodiment of the imaging system includes an imaging lens (Edmund Optics 45760) that is a symmetric 10-element relay lens specifically designed for use at a magnification of 1, and produces an inverted image. Symmetric in this case means that the lens contains a plane of symmetry perpendicular to the optical axis. It is well known (see, for example, Warren Smith, Modern Optical Engineering, 3rd edition, p. 401) that when a symmetric lens is used at a magnification of 1, coma, distortion, and lateral color are zero. The numerical aperture of the imaging lens is 0.12 in object space and 0.12 in image space. The sensor, in this embodiment of the imaging system design, is a 10-megapixel monochrome CMOS sensor (Aptina MT9J003) having 3856 x 2764 pixels. Pixel size is 1.67 microns. Because the imaging lens is used at a magnification of 1, the effective pixel size at the sample plane is 1.67 microns. The sensor is contained in a camera (IDS Imaging UI-1490LE-M-GL or Basler acA3800-14um) having 8- or 12-bit digital output and a USB 2.0 or USB 3.0 interface.

Many variations of the imaging system are possible. For example, in some embodiments the magnification may be greater than 1 or less than 1. In some embodiment, two separate lenses (for example, a microscope objective and a microscope tube lens) may be used instead of a single multi-element relay lens. In some embodiments, the sensor may have more or less than 10 million pixels. In some embodiments, the size of the sensor's pixels may be more or less than 1.67 microns. The sensor used may be a linear array instead of a 2-dimensional array (if a linear array is used, a translation stage that has an axis of motion parallel to the sample but perpendicular to the long axis of the sensor will be used in scanning mode instead of stepping mode). In some embodiments, the sensor may be a CCD instead of a CMOS device.

In bright-field microscopy, image quality is often best if the numerical aperture of the illumination system is chosen to be equal to the numerical aperture of the imaging system. However, under these conditions images of unstained cells immersed in buffer solution have poor contrast. We have found that a combination of partially coherent illumination and defocus improves contrast. Partially coherent illumination occurs when the illumination numerical aperture is less than the imaging numerical aperture. We have found that contrast is best when the partial coherence factor (fill factor, sigma, S) is approximately 0.5. The partial coherence factor is the illumination numerical aperture divided by the imaging numerical aperture. In our case, a partial coherence factor of 0.5 means an illumination numerical aperture of 0.06. Under these conditions, with a defocus of a few tens of microns, an image of a cell immersed in buffer solution appears as a bright spot surrounded by a dark annulus. This is apparently due to the fact that the refractive index of the cell is higher than the refractive index of the buffer solution, and therefore each cell is in effect acting as a miniature ball lens. For example, if the cell diameter is 5 microns, the refractive index of the cell is 1.376, and the refractive index of the buffer solution is 1.336, then the illumination light transmitted through the cell comes to a focus approximately 35 microns beyond the center of the cell. Since cells are not perfect spheres, and are not internally homogeneous, this model is of course a rough approximation. With a defocus of a few tens of microns in the opposite direction, cells immersed in buffer solution appear as dark spots. To improve the accuracy of cell counting, in some embodiments of the imaging system it may be advantageous to capture and compare two images of each area - one image in which cells appear as bright spots surrounded by dark rings, and one image in which cells appear as dark spots. Image processing software could be used to subtract one image from the other and then count the bright spots in the difference image. Alternatively it may be advantageous to use image processing software to analyze the two images separately, and then compare the results. Because the magnetic beads used are opaque and relatively large, images of beads immersed in buffer solution have good contrast under a wide range of illumination conditions. An image of a bead appears as a large dark spot, usually containing a small central bright spot. The bright spot is probably an Arago spot caused by Fresnel diffraction. Beads show up clearly in images whether the Arago spot is present or absent.

*Example 5 - Cell and Bead Detection Using Automated Image Analysis*

*Experimental methods:* An agarose microwell array comprising about 150,000 wells (30 $\mu$m diameter) was cast and placed in an enclosed flow cell having a volume of approximately 1.5 ml (FIG. 12). The flow cell height was 2mm. The diameter of the Ramos cells ranged from 5-10 microns with an average of 8 microns. Ramos cells were suspended in PBS at a concentration of approximately 53,000 cells/ml (as determined by a Muse Cell Analyzer), injected through the inlet onto the agarose microwell array in order to fill the flow cell, and allowed to settle into wells under the influence of gravity. Cells that were not captured in the wells were subsequently displaced and washed away by injecting PBS into the chamber. Next, a magnetic bead suspension (20 $\mu$m diameter beads; approximately 33,000 beads/ml as determined by a Muse Cell Analyzer) was injected through the inlet and beads were allowed to settle into wells under the influence of gravity. To examine cell and bead settling and distribution in the array as a function of time, a series of brightfield images of the microwell array were collected. A custom image analysis program was written in Matlab to detect the presence of cells and beads in the microwell array. The program identified the location of each well in the array and determined the presence of beads and cells in each well. In addition, the number of cells present in each well and cell radius were also calculated and recorded.

*Analysis algorithm:* To detect wells, the program first detects edges in the image using the Canny method, which determines the local maxima of intensity gradients in the image. Following edge detection, the program then detects wells by identifying circles having a radius within a specified range using a circular Hough transform. To make the detection of wells more robust, some embodiments of the analysis algorithm may also utilize detection of patterns in the image, e.g. patterns corresponding to the known regular grid of wells on the array. This approach can be used to help eliminate false positive results, and can also improve the speed of analysis. Examples of algorithms suitable for implementing such approaches include, but are not limited to Fourier transforms, wavelet analysis, and autocorrelation functions.

Once the wells have been detected, the program examines the interior of the wells to count beads and/or cells. To detect beads, the image is divided into smaller sub-images containing individual wells, converted to binary data by applying an intensity threshold calculated using Otsu's Method, and the presence of beads in each well determined based on the known sizes of the beads and wells, and the fraction of pixels located within the well having a value of zero. If the presence of beads is detected within the well, the program then uses a circular Hough transform to determine the location and size of the beads. To detect cells, the program first detects edges within each well using the Canny method, and cells are then identified by detecting circles having a radius within a specified range using a circular Hough transform.

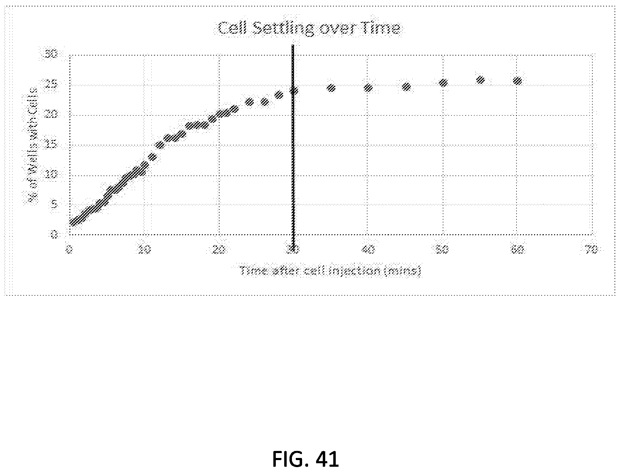

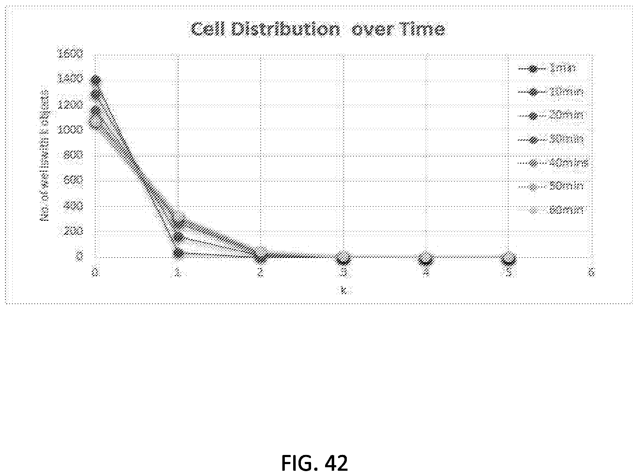

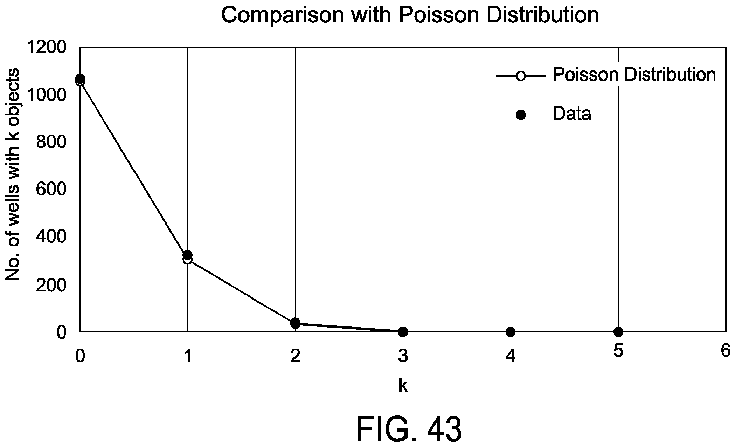

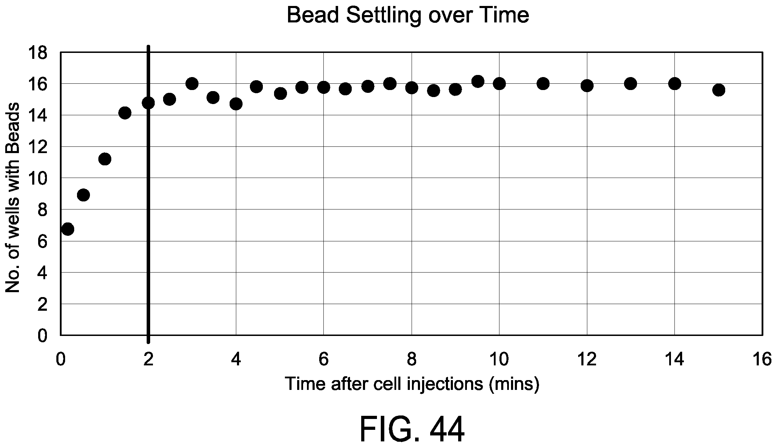

*Results:* Images of the microwell array (corresponding to a portion of the total array that comprised about 1,500 wells) were processed using the algorithm(s) described above. Following injection into the flow cell, the number of wells containing cells increased gradually over time and eventually reached a plateau (FIG. 41). The number of wells containing cells reached over 90% of the saturated value at 30 minutes after cell injection. As the number of cells settling into wells increased, the number of wells containing two or more cells also increased (FIG. 42). Cell distribution in the microwells followed the predictions of the Poisson distribution, as demonstrated by comparison with experimental data collected at 60 minutes (FIG. 43). Beads settled into the microwells more rapidly than cells. A saturation value was reached 2 minutes following bead injection into the flow cell (FIG. 44).

*Example 6 - Automated Cell Counting with a Hemocytometer*

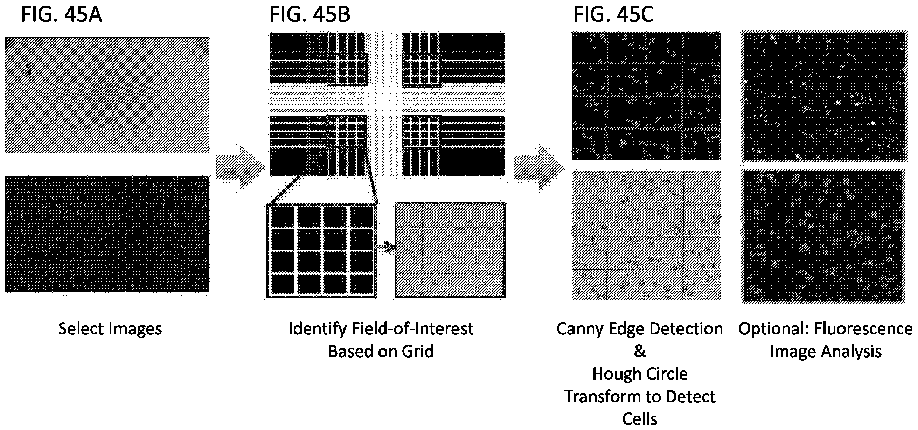

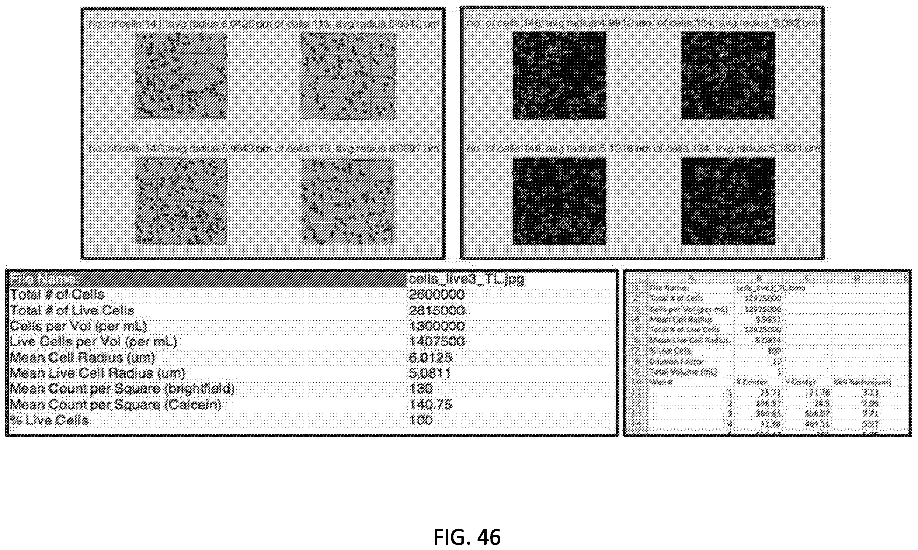

An example of automated cell counting using the image processing and analysis software described above and a hemocytometer is illustrated in FIGS. 45-46. Cell counting is useful in terms of determining how much of a cell suspension to load into the flow cell containing the microwell array. In some embodiments, cell counting may be coupled with a simultaneous determination of cell viability, for example, using a fluorescence-based live cell / dead cell assay. FIG. 45A (upper) depicts a bright field image of a cells distributed in a hemocytometer. FIG. 45A (lower) depicts a corresponding fluorescence image of the same field-of-view where the cells have been preloaded with calcein, a fluorescent indicator (excitation and emission wavelengths of 495 nm and 515 nm, respectively) of calcium concentration and cell viability. The workflow for image processing and automated cell counting comprises several steps (FIGS. 45A-C), including (i) selection of the bright field image to be analyzed, (ii) selection of a corresponding calcein-stained image (or an image acquired using other fluorescent indicators) if desired, (iii) input of a name for the output text file (which will contain basic statistics on total cell number, live cell %, cell radii, *etc.*), (iv) input of the dilution factor and total volume of cell suspension used, (v) algorithm-based identification of four 1 mm x 1 mm grids at the corners of the hemocytometer, and (vi) image processing to identify and count cells in each grid based on edge detection and the Hough circle transform. The image processing and analysis process takes approximately 30 seconds to perform. An example of the output results is shown in FIG. 46. The number of cells identified in each of the four quadrants and their average radius is listed, along with statistics on cell concentration, average cell radius, live cell percentage, *etc.*

*Example 7 - Microwell, Cell, and Bead Detection Using Automated Image Analysis*

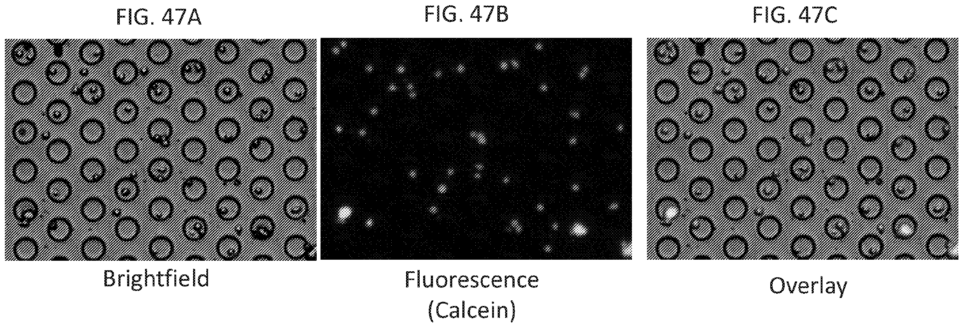

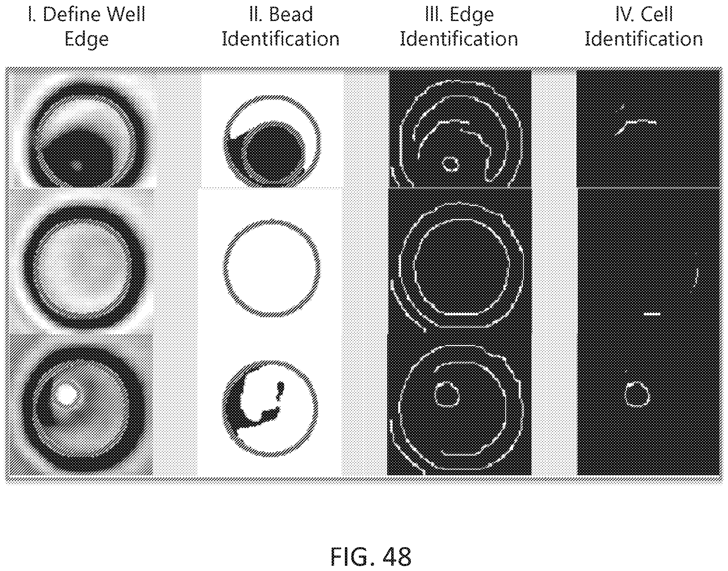

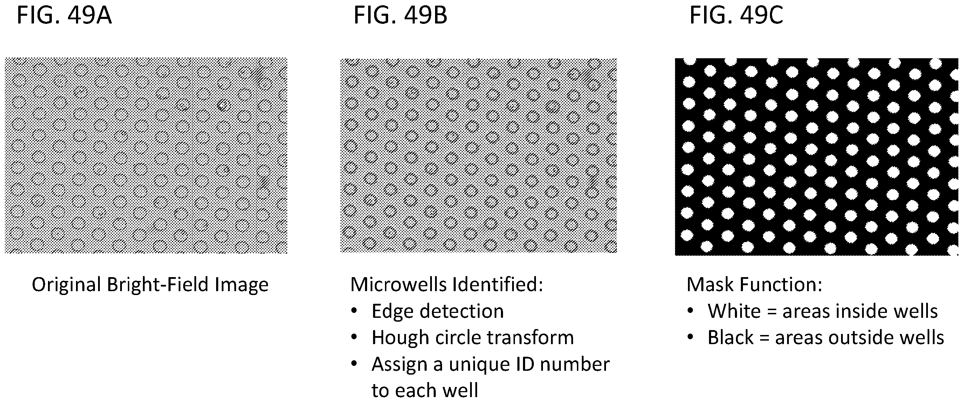

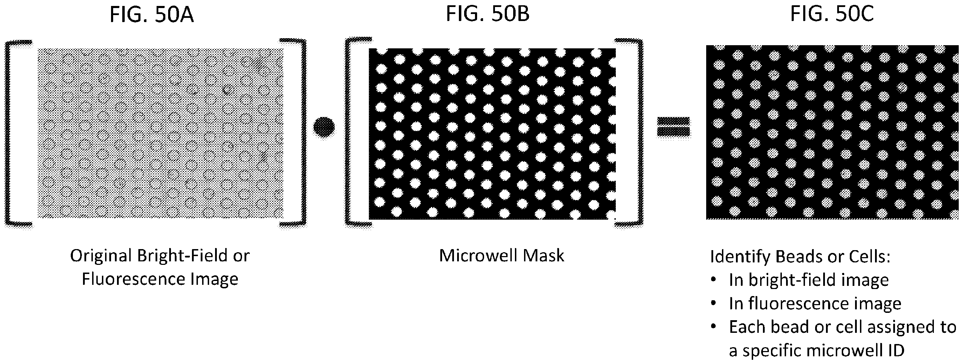

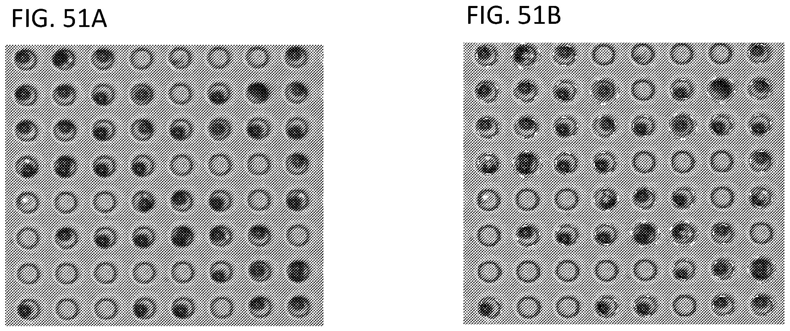

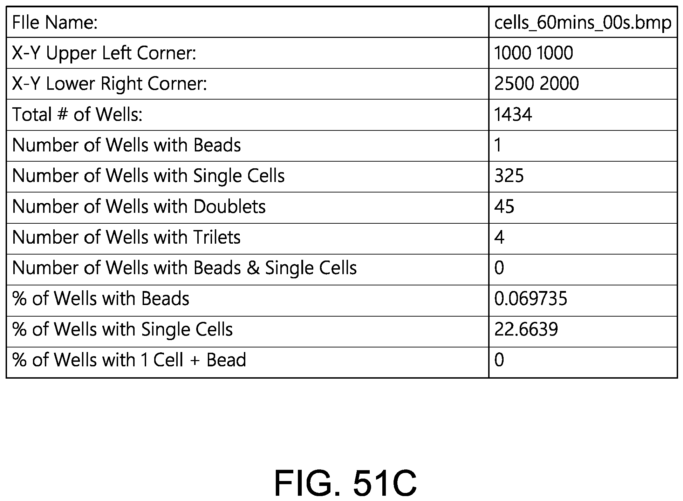

Additional examples of automated microwell detection, cell detection, and bead detection using the image processing and analysis software described above are shown in FIGS. 47-51. The workflow for image processing and analysis comprises several steps, including (i) selection of the bright field image(s) to be analyzed, (ii) selection of a corresponding calcein-stained image(s) (or image(s) acquired using other fluorescent indicators) if desired, (iii) input of a name for the output text file (which will contain basic statistics on total cell number, live cell %, cell radii, *etc.*), (iv) selection of whether to detect beads, cells, or both (with the additional option of selecting small, medium, or large cells), (v) input of the region-of-interest (ROI) within the image(s) to be analyzed, and (vi) image processing and analysis to identify the microwells in the image and the cells and/or beads contained within the microwells. FIGS. 47A-C illustrate a bright-filed image of a microwell array containing cells (FIG. 47A), the corresponding fluorescence image where the cells have been pre-loaded with calcein (FIG. 47B), and an overlay of the two images (FIG. 47C). The image processing and analysis proceeds in four steps (FIG. 48): (1) identification and numbering of microwells through the use of edge detection, (2) bead identification, (3) edge detection within the microwells, and (4) cell identification. The edges of microwells are identified by magnifying the image (at 4X magnification, the wells encompass an area of approximately 20 x 20 pixels), performing edge detection, and applying the Hough circle transform (FIGS. 49A-B). The results of the microwell detection step may be used to create a mask to exclude image features lying outside of the microwells from subsequent analysis (FIG. 49C; FIGS. 50A-C). Beads are identified within individual wells by applying a binary intensity threshold to the image, and determining whether or not a bead is present based on the percentage of dark pixels within each well. If present, the bead's location within the well may be determined using the Hough circle transform (HCT) (FIG. 48). Once beads have been detected, additional edge detection is performed within the area of each well (*i.e.* outside the region of the well associated with a detected bead) to identify the presence of cells and determine the cell's location using the HCT (FIG. 48). A typical image (~6mm x 4mm in area, ~10,000 wells) takes approximately 2 minutes to process and analyze. An example of the output results is shown in FIGS. 51A-C. The software provides a graphical display of the results (FIGS. 51A-B) as well as a summary table (FIG. 51C). The detailed results are stored in a text file (including the number of wells, data on cell and bead distributions, *etc.*). In some embodiments, the graphical display of results comprises the original bright-field image overlaid with graphics, *e.g.* circles of different colors, to indicate wells containing no cells or beads, wells containing a single bead, wells containing a single cell, wells containing both a single bead and single cell, wells containing cells exhibiting selected properties, *e.g.* as indicated by a fluorescent indicator, *etc.* Automated microwell, bead, and cell detection using image processing and analysis may be useful for quantitative analysis of bead and cell distribution efficiencies, bead retrieval efficiencies, *etc.,* as a function of device and system design parameters.

*Example 8 - Cell & Bead Distribution and Retrieval vs. Flow Cell Design, Substrate Material, and Well Diameter*

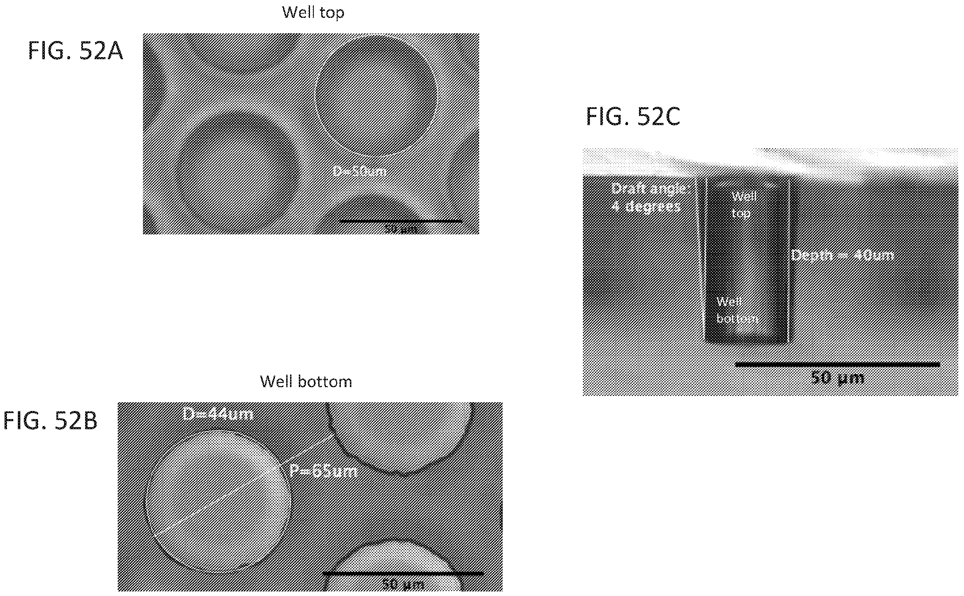

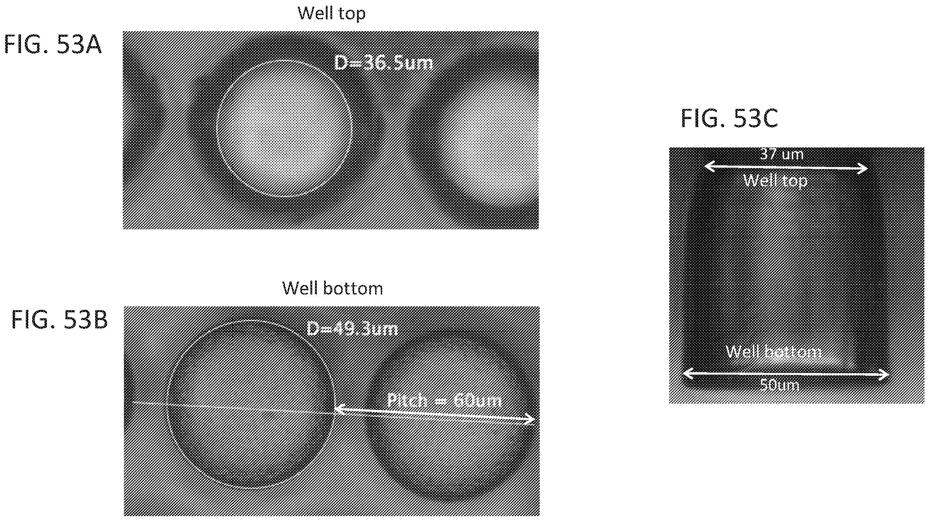

*Fabrication of microwell arrays:* Microwell arrays having a range of diameters were fabricated from several different substrate materials. FIGS. 52A-C shows micrographs of microwells fabricated from Norland Optical Adhesive 63 (NOA63) .using a UV activated molding process to replicate microwell substrates from polydimethylsiloxane (PDMS) stamps (See Example 10). Microwells ranged from 27.5 $\mu$m to 80 $\mu$m in diameter, with a 40 $\mu$m depth and variable pitch (minimum center-to-center separation = 15 $\mu$m). FIGS. 53A-C shows micrographs of microwells fabricated from cyclic olefin copolymer (COC) using a soft embossing process and an optical adhesive structure as illustrated in FIGS. 52A-C as a master. The COC microwells shown in FIGS. 53A-C had a 50 $\mu$m diameter, with a depth of 50 $\mu$m and a 60 $\mu$m pitch. Microwell arrays having other diameters, depths, and pitches were also fabricated from COC and tested, as were microwell arrays fabricated from cyclo-olefin polymer (COP). A preferred embodiment comprised microwells of 55 $\mu$m diameter, 40 $\mu$m depth, and 70 $\mu$m pitch.

*Microwell array substrate treatment:* Microwell arrays fabricated from NOA63, COC, or COP were typically pre-treated with Pluronic F108 (0.02% in phosphate buffered saline (PBS)) for at least one half hour prior to loading cells. Cells were suspended in Pluronic F68 (1% in PBS) for use in loading the microwell arrays.

In studies of bead retrieval efficiencies for microwell arrays having pre-loaded beads, some microwell array substrates were pre-treated with poly-HEMA (10 - 20 mg/ml in ethanol or lysis buffer (0.1M Tris HCl (pH 7.5), 0.5M LiCl, 1% Lithium dodecyl sulfate (LiDS), 10 mM EDTA, 5mM DTT)) prior to loading the beads.

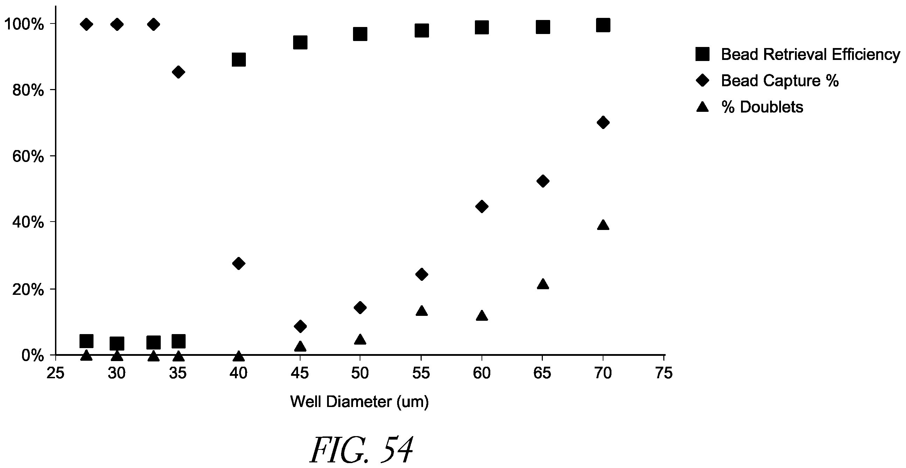

*Bead capture and retrieval efficiency vs. microwell diameter:* FIG. 54 shows examples of data for bead capture and retrieval efficiency for microwells of different diameter (50 $\mu$m depth, 15 $\mu$m minimum well separation) fabricated from NOA63. Beads (33 $\mu$m diameter) were suspended in PBS for loading onto the array. As one would expect, bead capture efficiency jumped dramatically for wells having a diameter larger than that of the bead. As the well diameters increased, the percentage of doublets (wells containing two beads) also increased. The bead retrieval efficiency exhibited a minimum for wells of approximately the same size as the beads, and then increased as the well diameters increased.

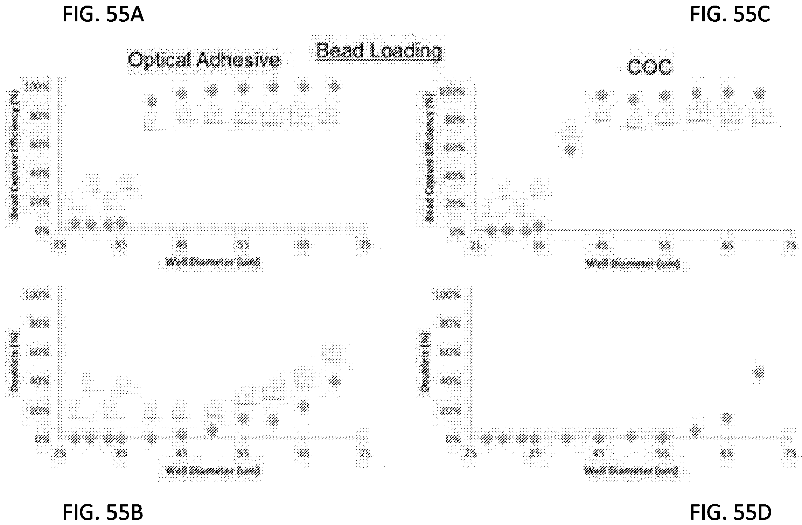

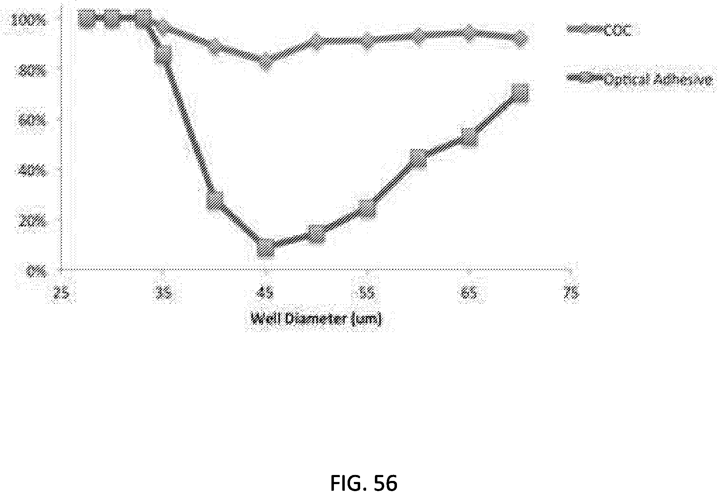

FIGS. 55A-D and 56 shows examples of data for bead capture and retrieval from microwells of different diameter fabricated from NOA63 or COC (well depth = 40 μm for the optical adhesive and COC wells). The bead loading properties were similar for the two materials (FIG. 55A-D). Bead retrieval efficiency was improved for microwells fabricated from COC (and for COP) relative to that for microwells fabricated from the optical adhesive (FIG. 56).

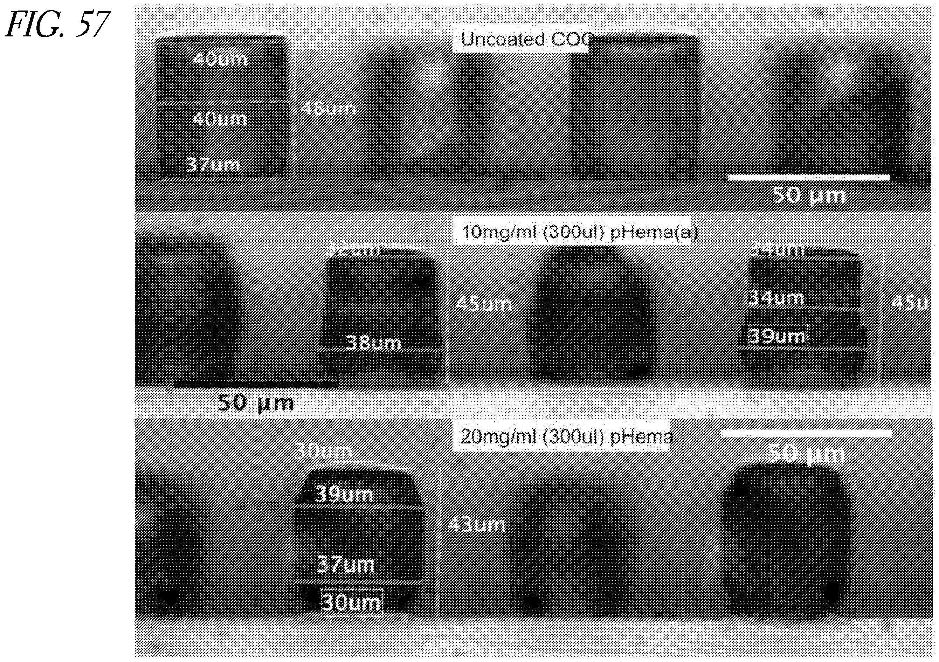

*Bead retrieval efficiency for pre-loaded beads:* FIG. 57 shows micrographs of COC microwells pre-treated with poly-HEMA as described above. Microwells were preloaded with 35 μm diameter beads suspended in PBS. After loading, the microwell substrate was rinsed 3x in deionized water to remove salts, and dried under vacuum overnight. Beads loaded into non-treated COC microwell arrays could not be magnetically retrieved from the wells. Beads loaded into the COC microwells that had been pre-treated with poly-HEMA could be magnetically retrieved with essentially 100% efficiency.

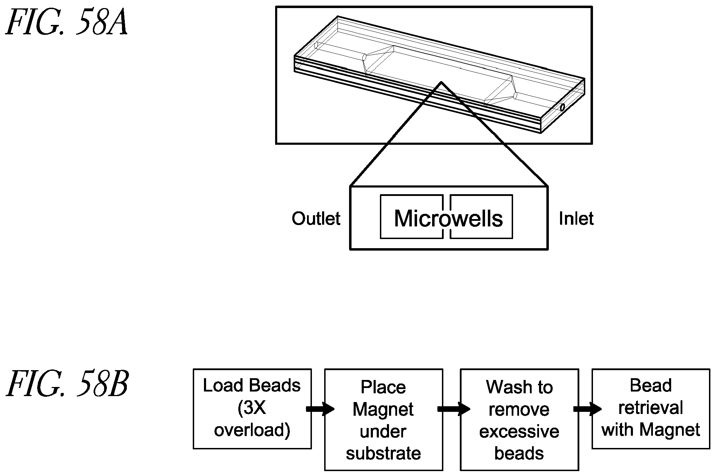

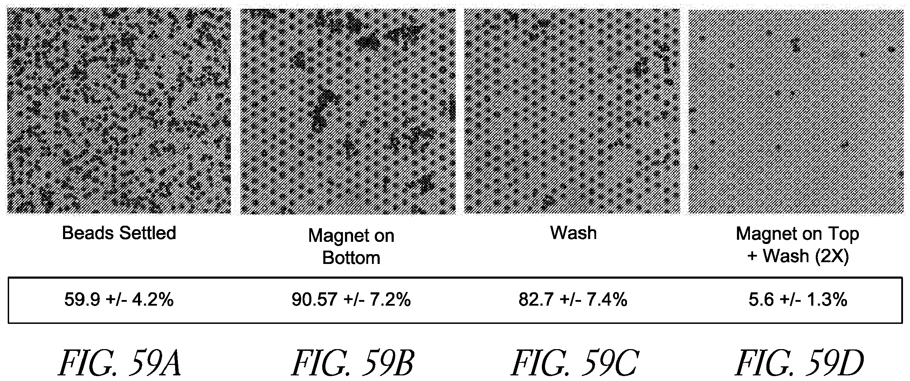

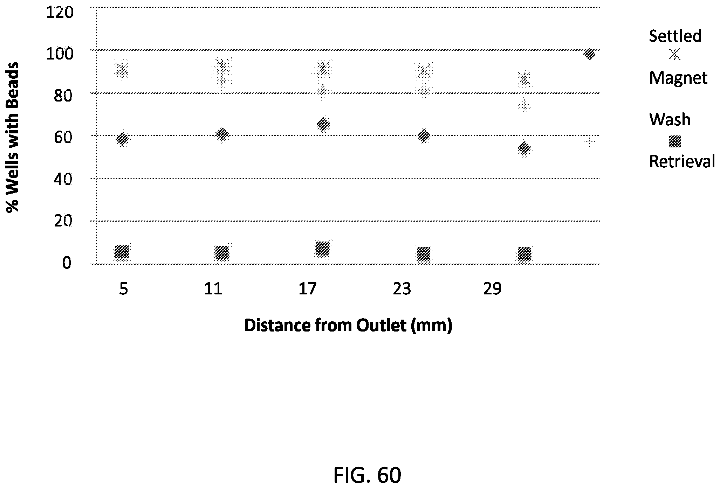

*Bead loading* & *retrieval efficiency vs. flow cell design* & *position on substrate:* FIGS. 58-60 show examples of data for studies of bead loading and retrieval after different process steps and as a function of position on the microwell array substrate. FIG. 58A illustrates a flow cell that encloses the microwell array and includes a tapered inlet and outlet. FIG. 58B illustrates the steps of the loading and retrieval process: (i) loading, (ii) magnetic field-enhanced entrapment, (iii) wash, and (iv) magnetic field-based retrieval. FIGS. 59A-D shows micrographs of a microwell array after each of the steps outlined in FIG. 58B. The percentage of wells containing a bead are indicated below each image. FIG. 60 shows examples of data generated using automated image processing and analysis as described previously to quantify the number of wells containing beads after each process step as a function of position on the substrate, *e.g.* as specified by the distance from the flow cell outlet. The data indicate that the efficiency of magnetic field-assisted loading and retrieval is quite high. The flow cell design used in this example (FIG. 58A; tapered inlet and outlet) provided for a uniform distribution of beads along the length of the microwell array substrate.

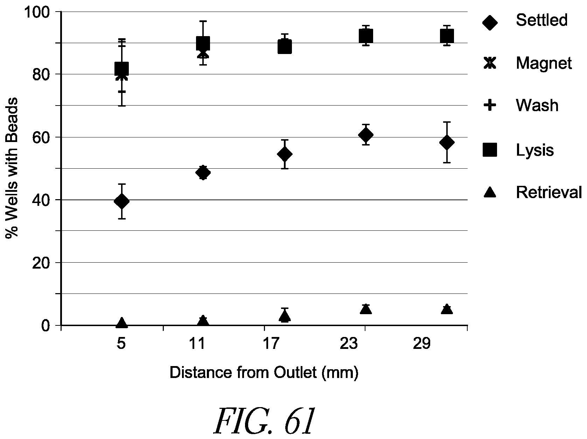

FIG. 61 shows another example of data for studies of bead loading and retrieval after different process steps, including the lysis step, as a function of position on the microwell substrate. An NOA63 microwell array substrate (33 μm diameter wells) was loaded with Ramos cells, followed by loading with 22 μm diameter blank beads. Automated image processing and analysis as described previously to quantify the number of wells containing beads after each process step as a function of position on the substrate. Again, the efficiency of magnetic field-assisted loading and retrieval is quite high, with a fairly uniform distribution of beads along the length of the microwell array substrate.

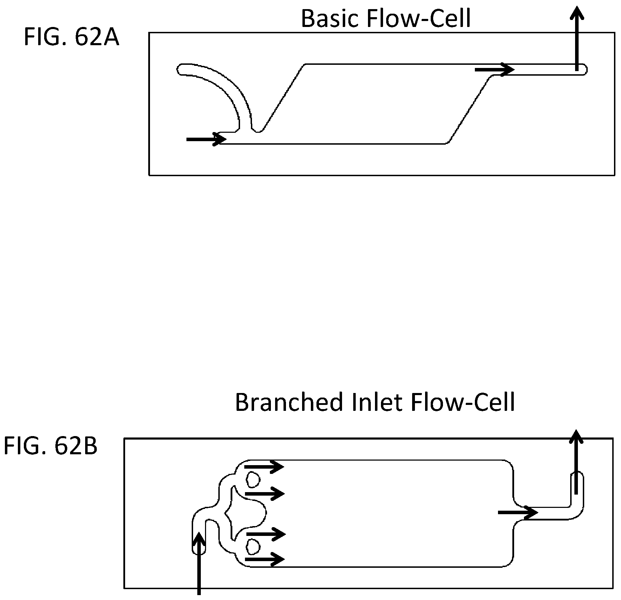

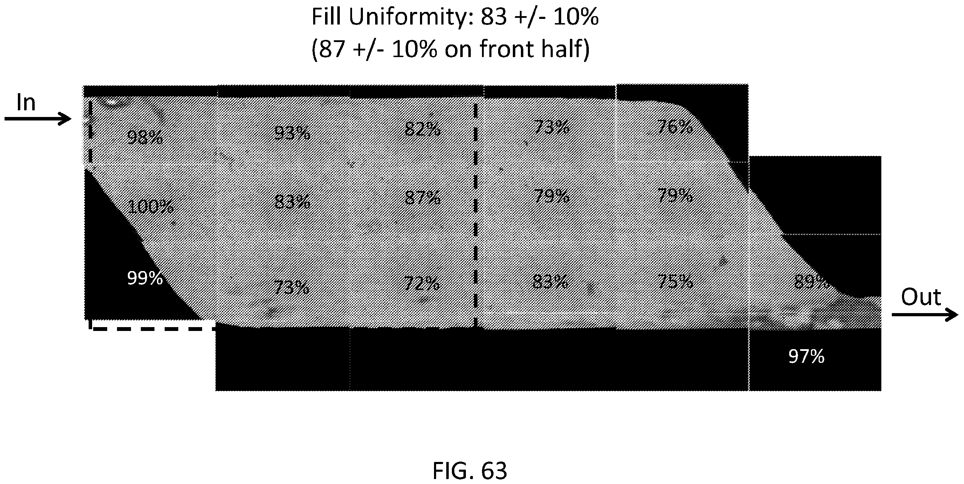

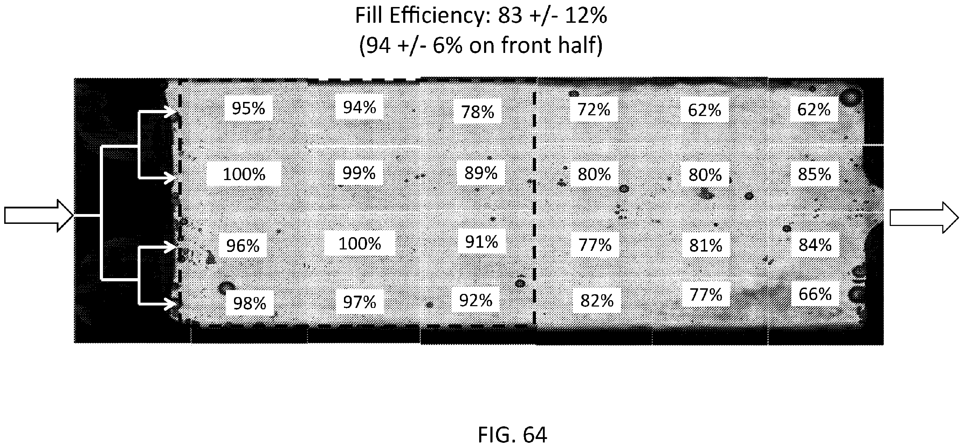

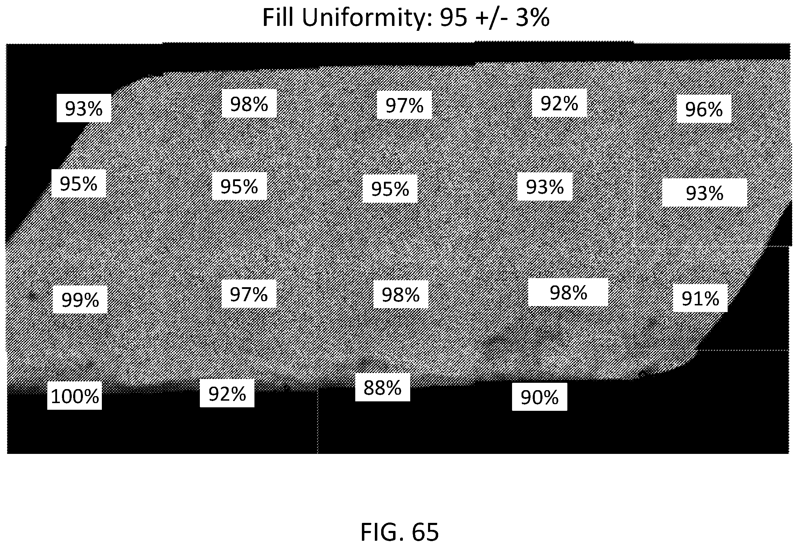

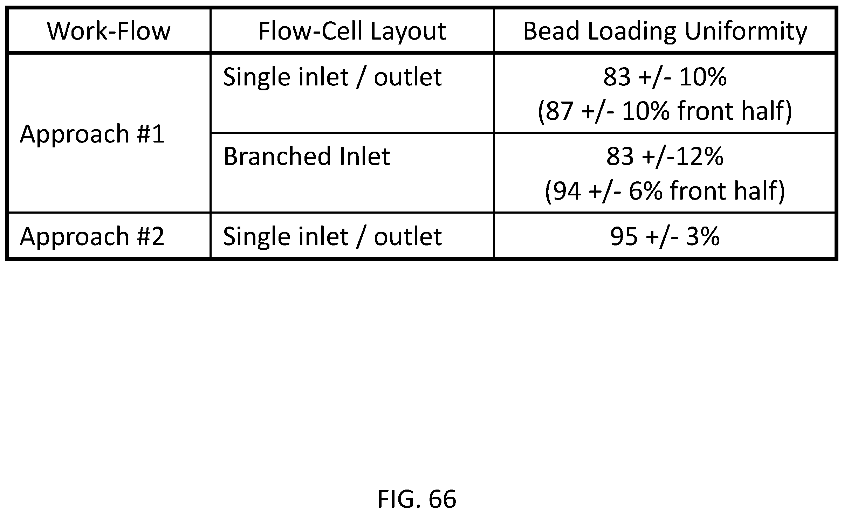

FIGS. 62-66 illustrate the results of studies to determine the uniformity of bead distribution across the microwell substrate as a function of flow cell design and the optional use of an air injection step between liquid injections to help minimize dispersion at liquid-liquid interfaces. FIGS. 62A-B depicts the two different flow cell designs tested: a single inlet - single outlet design (FIG. 62A) and a branched inlet design (FIG. 62B). Two different approaches to fluid exchange within the flow cell were also tested. Approach #1 consisted of performing the steps of priming the flow cell, loading the cells, and loading the beads, where all steps were performed using PBS and each solution was directly displaced by the next. Approach #2 consisted of performing the steps of priming the flow cell, displacing the priming buffer with an air injection, loading the cells, displacing the cell suspension with an air injection, and loading the beads. Bead loading uniformity was then assessed using the automated image processing and analysis software described above. FIG. 63 shows an image overlaid with bead fill efficiency data for the single inlet-single outlet flow cell using approach #1 (12 second dispense duration). The numbers indicate the local fill percentage (*i.e.* the number of wells with a bead present / total number of wells imaged). The overall fill percentage was 83 ± 10% (87 ± 10% over the front half of the substrate). FIG. 64 shows an image overlaid with bead fill efficiency data for the branched inlet flow cell using approach #1 (12 second dispense duration). The overall fill percentage was 83 ± 12% (94 ± 6% over the front half of the substrate). FIG. 65 shows an image overlaid with bead fill efficiency data for the single inlet-single outlet flow cell using approach #2 (*i.e.* using air injections between liquid dispensing). The overall fill percentage was 95 ± 3%. The results of the study are summarized in FIG. 66. The use of the air injections between liquid dispense steps induces a plug flow fluid velocity profile throughout the flowcell, and improves the uniformity of bead distribution across the microwell substrate. In a similar fashion, the use of air injections between liquid dispense steps also improves the uniformity of the cell distribution within the flowcell.

It was observed over the course of performing these studies that the beads may sometimes become trapped in semi-solid deposits, *e.g.* genomic DNA and/or cross-linked proteins, created during cell lysis. These deposits may be broken up or dislodged using 'mechanical' methods, *e.g.* injected air bubbles, vigorous flow, and/or sonication. Alternatively, the deposits may be prevented from forming, or dissolved, through the use of buffers having lower salt concentration, *e.g.* PBS (molality ~ 150 mM) instead of lysis buffer (molality > 500 mM), different concentrations of surfactant, different pH ranges, or treatment with DNAses and/or proteases.

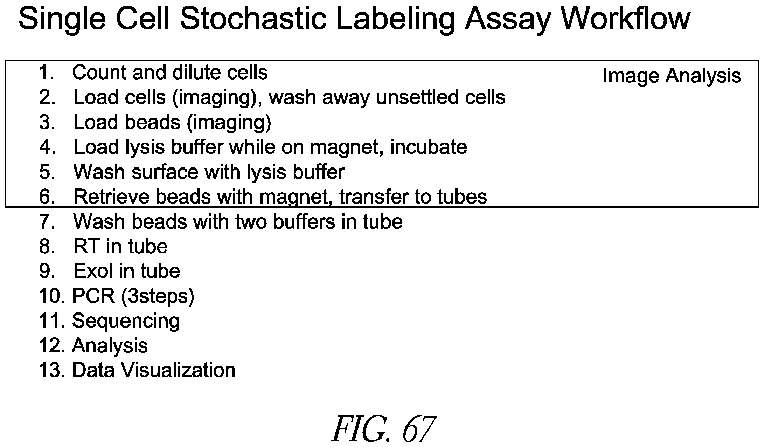

*Flow cell proof-of-concept experiment:* FIG. 67 provides a high level overview of the assay workflow for single cell, stochastic labeling. The assay comprises 13 steps, including: (1) counting and diluting the cells to be loaded in the microwells, (2) loading the cells, and washing away any cells that haven't settled in microwells, (3) loading the beads comprising the stochastic label library using an externally-applied magnetic field, (4) loading lysis buffer while the beads continue to be retained by the magnetic field, and incubating, (5) wash the substrate surface with lysis buffer, (6) retrieving the beads with an externally-applied magnet field, and transferring the beads to tubes, (7) washing the beads to exchange buffers, (8) performing reverse transcriptase (RT) in the tubes, (9) treating with Exonuclease 1, (10) performing three rounds of PCR, (11) sequencing the amplified product, (12) performing data analysis, and (13) providing data visualization. Image processing and analysis facilitates the first six of these process steps.

An end-to-end flow cell proof-of-concept study was performed using COC microwell substrates having 50 $\mu$m diameter wells of 50 $\mu$m depth and 60 $\mu$m center-to-center spacing. The study was performed using Ramos and K562 cell lines, and 33 $\mu$m diameter, oligonucleotide-functionalized magnetic beads. The COC substrate was packaged in a flow cell having a total area of 225 mm$^2$ which contained approximately 72,000 exposed microwells. The flow-cell was primed with 99% ethanol, and then flushed with PBS. Cells were loaded in PBS (3,500 K562 cells and 3,500 Ramos cells) and allowed to settle for 20 minutes. The oligo-bearing beads (100,000 beads) were then loaded in PBS and allowed to settle for 5 minutes, following which lysis buffer was introduced to lyse the cells. A magnet was placed below the flow-cell during lysis to avoid having beads pushed out of the microwells due to cell expansion during lysis. Beads were then retrieved from the microwells by placing a magnet above the flow-cell and flushing with additional lysis buffer. Cell and bead loading and retrieval efficiencies following different process steps were determined using the automated image processing and analysis software described above. The remaining assay process steps were then performed outside of the flowcell.

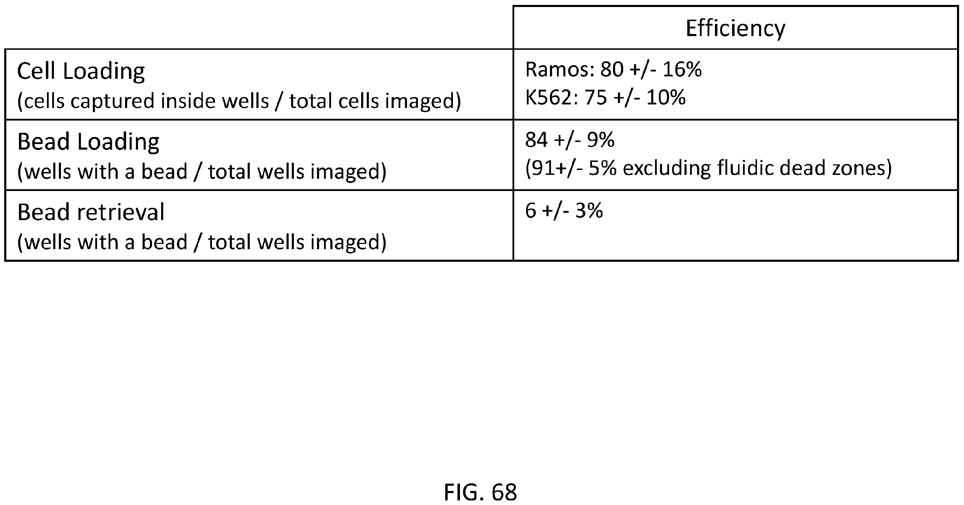

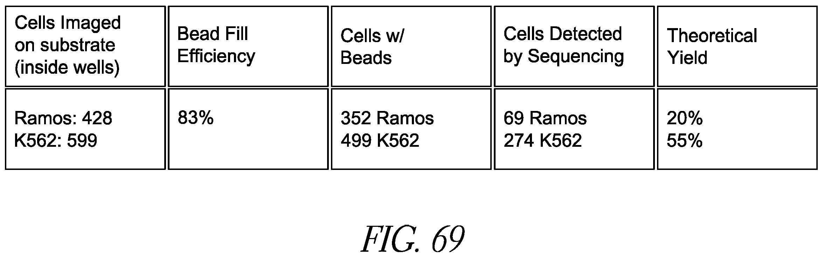

The results of the proof-of-concept study are shown in FIGS 68-69. The cell loading efficiency (*i.e.* the number of cells captured inside wells / total cells imaged) was about 80% for both cell types, which was somewhat higher than expected considering that 44% of the surface area of the microwell substrate used in this study was dead space. The higher loading efficiency may be due to tumbling of cells on the substrate surface during cell loading. The bead loading efficiency (*i.e.* the number of wells containing a bead / total wells imaged) was about 84% (about 90% excluding the fluidic dead zones within the flow cell). After retrieval of the beads, approximately 6% of the wells still contained a bead, implying a retrieval efficiency of about 93%. The number of cells identified by retrieving the beads and processing the corresponding oligonucleotide labels (*i.e.* performing the reverse transcription, PCR amplification, and sequencing reactions) is shown in FIG. 69. At present, counting efficiencies of approximately 20-50% are achievable. It was unclear in this study why the Ramos cell population is under-represented by molecular counting.

*Example 9 - Cell & Bead Distribution and Retrieval vs. Flow Cell Thickness, Automated Magnetic Retrieval, and Lysis Buffer Composition*

A series of experiments were performed to examine cell and bead distribution and retrieval efficiencies as a function of flow cell thickness (*i.e.* the depth of the flow cell chamber) and flow rate, lysis buffer composition, and use of automated magnetic bead retrieval. The automated magnetic bead retrieval mechanism comprised a small, fixed magnet held in a stationary position as the flow cell was translated past the magnet using a linear translation stage (alternatively, the flow cell may be held in a stationary position as the magnet is translated).

*Cell and bead distribution and retrieval vs. flow cell depth and flow rate:* Experiments were performed using COP microwell substrates with 50 $\mu$m diameter x 50 $\mu$m deep wells spaced on a 60 $\mu$m pitch. The microwell substrates were packaged in a flow cell having a single inlet-single outlet diagonal channel design having either: (a) a 1mm thick, 5mm wide channel, or (b) a 2mm thick, 3mm wide channel. Functionalized beads of 33 $\mu$m diameter were used for bead loading. K562 cells were used for cell loading. Bead and cell loading efficiencies were determined as a function of position along the flow cell using the automated image processing and analysis software described above.

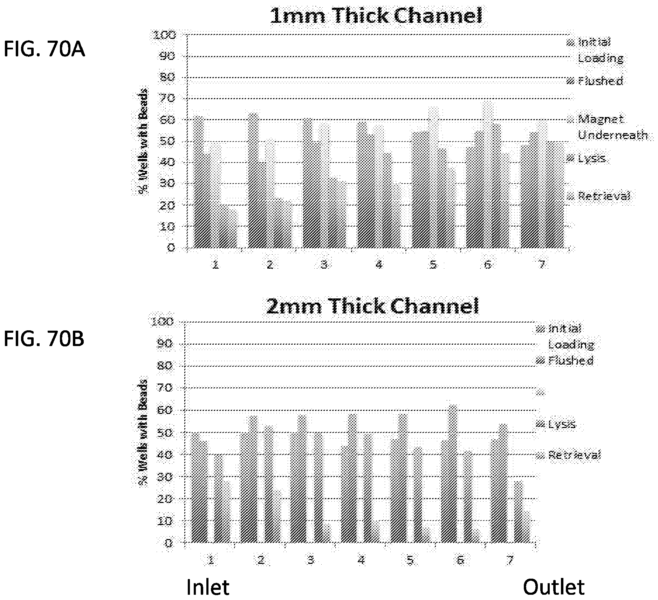

FIGS. 70A-B show examples of bead loading data as a function of position along the flow cell at different steps in the assay procedure. FIG. 70A shows data for a 1 mm thick flow cell. The percentage of wells containing beads was fairly uniform along the length of the flow cell following initial loading, after rinsing out the flow cell, and after using a magnet to pull beads down into wells. The percentage of wells containing beads was lower at the inlet end of the flow cell than at the outlet following the cell lysis and bead retrieval steps. FIG. 70B shows data for a 2 mm thick flow cell. Again, the percentage of wells containing beads was fairly uniform along the length of the flow cell following initial loading and after rinsing out the flow cell. The percentage of wells containing beads was somewhat higher at the inlet end of the flow cell and lower at the outlet end following the cell lysis and bead retrieval steps, indicating that the bead recovery process may be influenced by flow cell geometry, fluid channel dimensions, and the corresponding changes in flow velocities and flow profiles within the microwell array chamber.

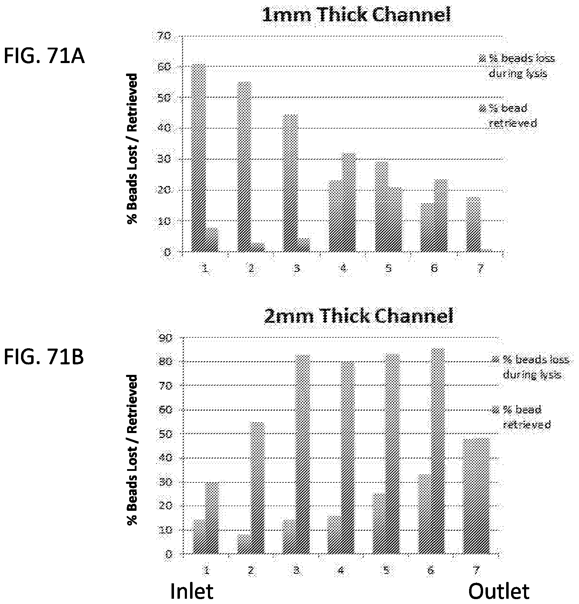

FIGS. 71A-B show examples of data for the percentage of beads lost during the lysis step or retrieved at the end of the process as a function of position along the flow cell. The data shown in FIG. 71A indicate that for the 1 mm thick flow cell, there is a significant positional-dependent loss of beads observed during the lysis step under the conditions used in this experiment. The overall percentage of beads recovered was correspondingly quite low. FIG. 71B shows the corresponding data for the 2 mm thick flow cell. The percentage of beads lost during the lysis step was generally lower, and the corresponding percentage of beads retrieved was generally higher, than that for the 1 mm flow cell.

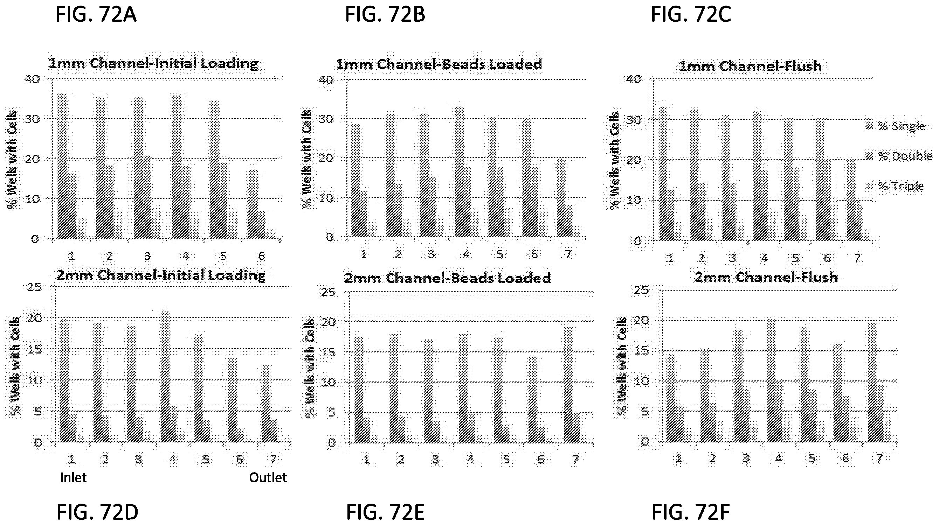

FIGS. 72A-F show examples of data for cell loading as a function of position along the flow cell at different steps in the assay procedure. FIGS. 72A-C show data for the 1 mm thick flow cell. FIGS. 72D-F show data for the 2 mm thick flow cell. The percentage of wells containing two or three cells was generally lower for the 2 mm thick flow cell, and fairly independent of position along the length of the flow cell.

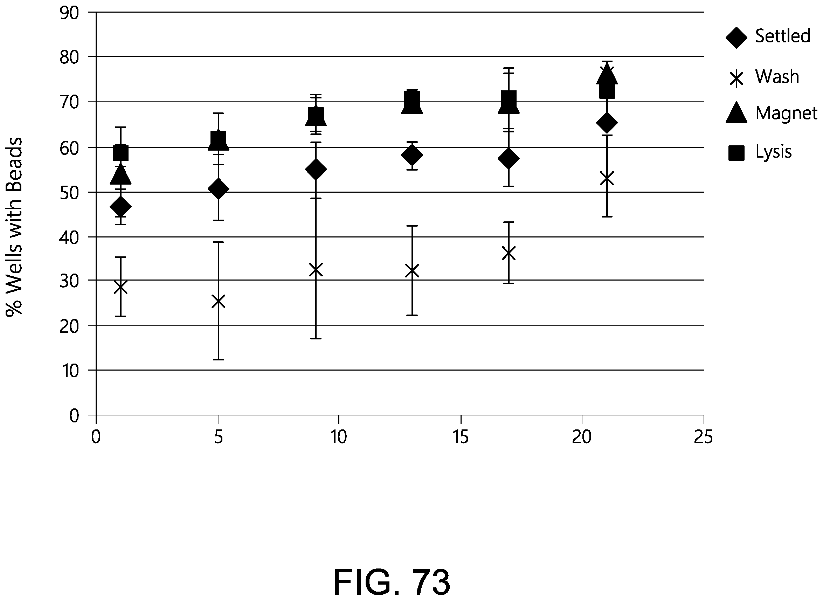

*Automated magnetic bead retrieval:* FIGS. 73-76 illustrate results of cell and bead distribution and retrieval studies performed using an automated magnetic bead retrieval system. Using a motion-control mechanism, *e.g.*, a linear translation stage, for moving the magnet relative to the flow cell (or vice versa) offers the advantage of being able to execute a repeatable, slow relative motion that may be optimized to better match the timescale of moving beads in and out of wells. Experiments were performed using COC substrates having microwells of 50 $\mu$m diameter. 3000 Ramos cells were loaded in 1% F68. Beads were 33 microns in diameter and 200,000 beads were loaded on to the system. Bead and cell loading efficiencies were determined as a function of position along the flow cell using the automated image processing and analysis software described above.

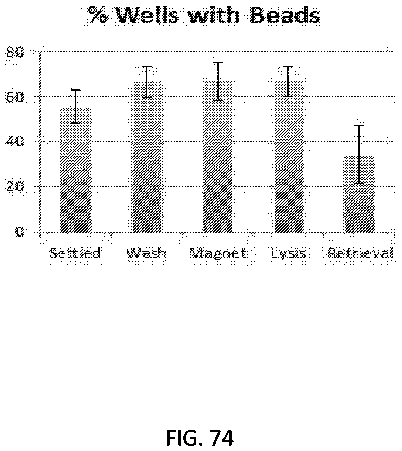

FIG. 73 shows data for the percentage of wells containing beads as a function of position along the length of the flow cell at various stages of the assay process, *i.e.* after initial loading and settling of the beads, after flushing the flow cell with a rinse buffer, after pulling down the beads with the external magnet, and after introducing the lysis buffer. The percentage of wells containing beads (averaged over the entire set of microwells) after each process step is shown in FIG. 74. The overall bead retrieval efficiency in these studies was approximately 48.8%.

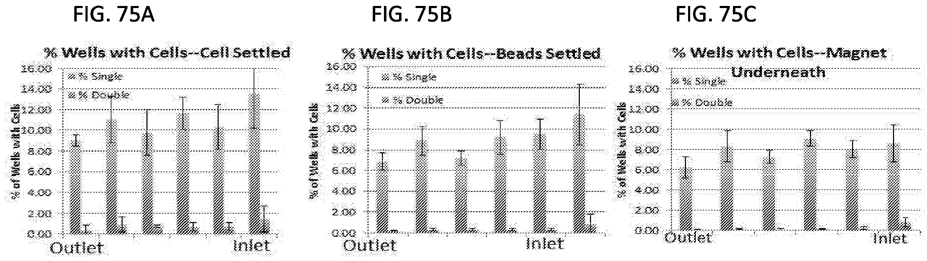

FIGS. 75A-C show data for cell loading after the initial cell loading and settling step (FIG. 75A), after the initial bead loading and settling step (FIG. 75B), and after the beads are subsequently pulled down with the magnet (FIG. 75C). Some loss of cells can be observed in FIG. 71C, perhaps due to displacement of the cells as the beads are pulled deeper into the microwells.

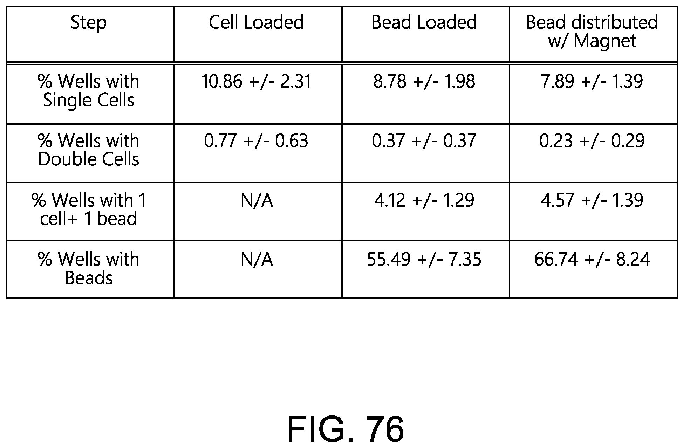

FIG. 76 provides a summary of cell loading and bead loading data at different steps in the process. The percentage of wells containing single cells ranged from about 8% to about 11%. The percentage of wells containing two cells ranged from about 0.2% to about 0.8%. The percentage of wells containing both a single cell and a single bead ranged from about 41.% to about 4.6%. The percentage of wells that contained beads only ranged from about 55.5% to about 66.7%. The use of the magnet to distribute beads had little effect on the number of wells containing single cells, two cells, or both a single cell and single bead, although there was a slight increase in the percentage of cells containing beads.

*Bead retrieval vs. lysis buffer composition:* A set of experiments was performed to determine whether or not modification of the lysis buffer, *e.g.* by addition of dithiothreitol (DTT) to reduce disulfide bonds in thiolated genomic DNA, in combination with magnetic-field assisted bead distribution would improve bead retrieval efficiencies. Studies were performed using COC microwell array substrates having 50 $\mu$m diameter x 50 $\mu$m deep microwells on a 60 $\mu$m pitch. The flow cell was primed using 100% ethanol, and rinsed 3x with PBS. Ramos cells (30,000 total) were loaded using 1% Pluronic F68 in PBS, followed by loading of 33 $\mu$m diameter, functionalized beads in PBS. After loading of the beads, the flow cell was flushed with PBS, and a magnet placed underneath the substrate was used to pull remaining beads down into the microwells. Lysis buffer (with DTT, 1:20 dilution) was injected twice, followed by a 20 minute incubation period (with the magnet positioned beneath the substrate), followed by magnetic retrieval of the beads. Bead loading efficiencies were determined as a function of position along the flow cell using the automated image processing and analysis software described above.

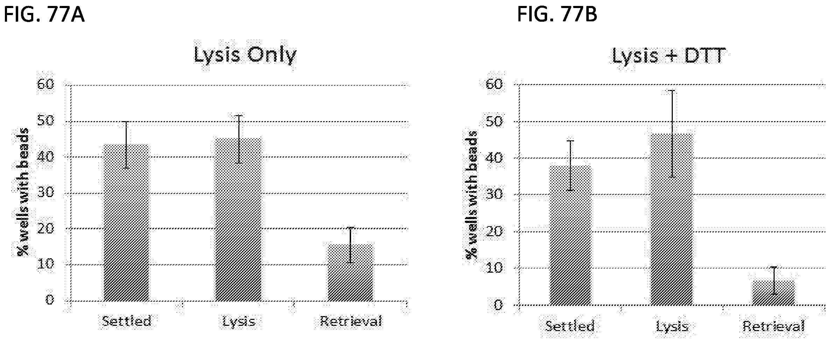

FIGS. 77A-B show examples of data comparing bead loading at different steps in the assay procedure when using lysis buffer with and without DTT added. In the absence of DTT, the bead retrieval rate (retrieved beads/loaded beads * 100%) was about 65.5% (FIG. 77A). When DTT was added to the lysis buffer, the bead retrieval rate increased to about 85.8% (FIG. 77B).

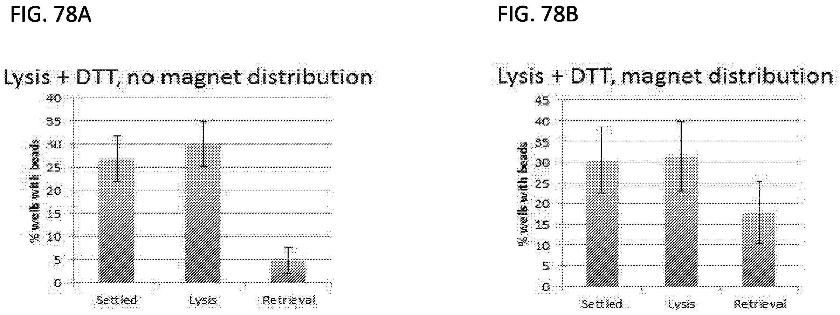

FIGS. 78A-B show examples of data comparing the impact of magnetic field-assisted bead distribution on bead loading at different steps in the assay procedure when the lysis buffer includes DTT. The bead retrieval rate was about 84.6% without the use of magnetic field-assisted bead distribution (FIG. 78A). When the magnetic field-assisted bead distribution was employed, the bead retrieval rate dropped to about 43.6% (FIG. 78B).

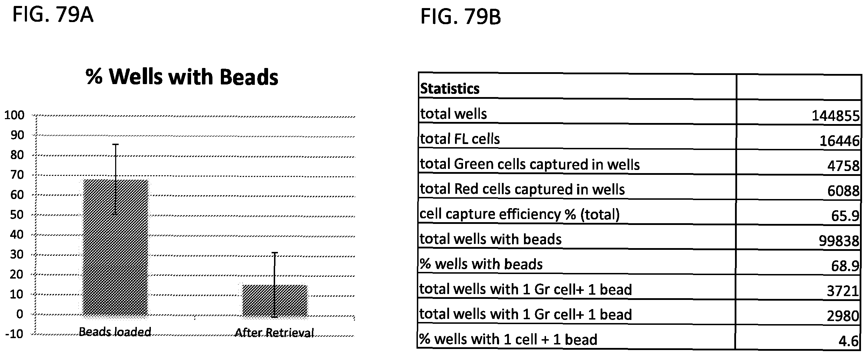

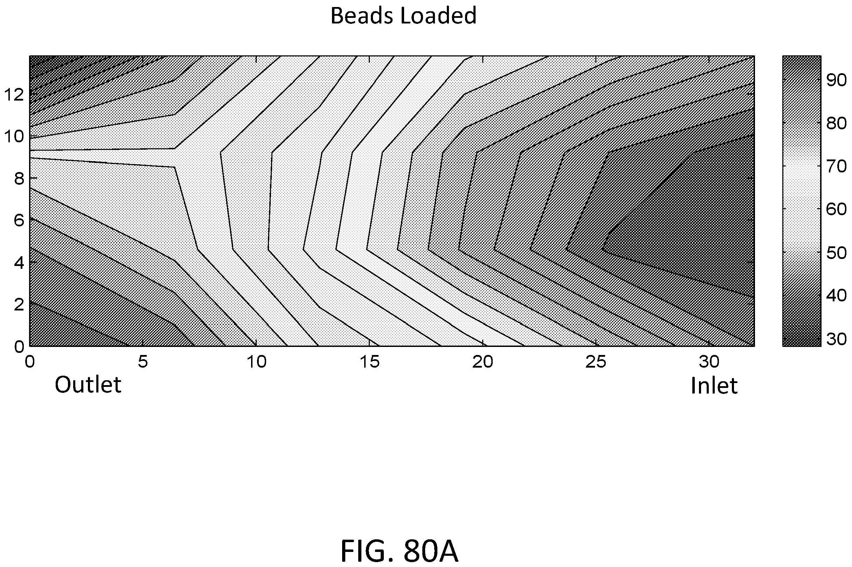

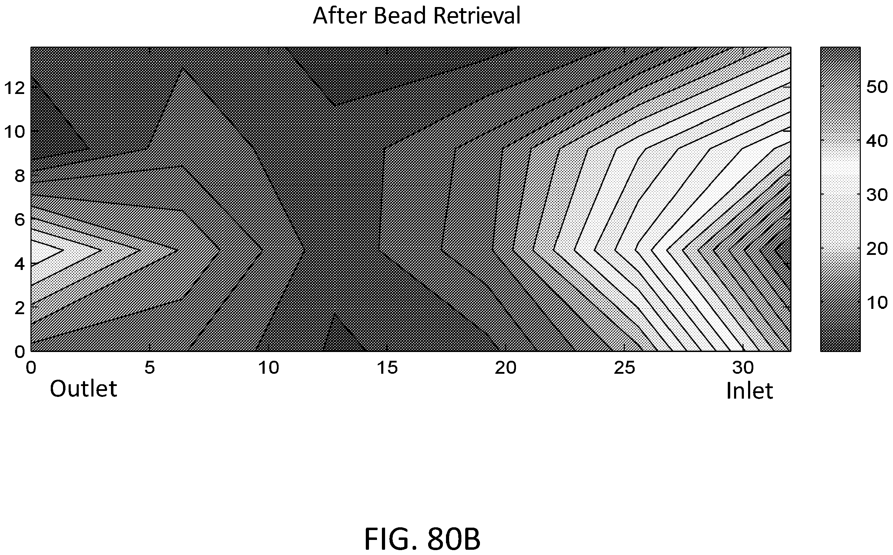

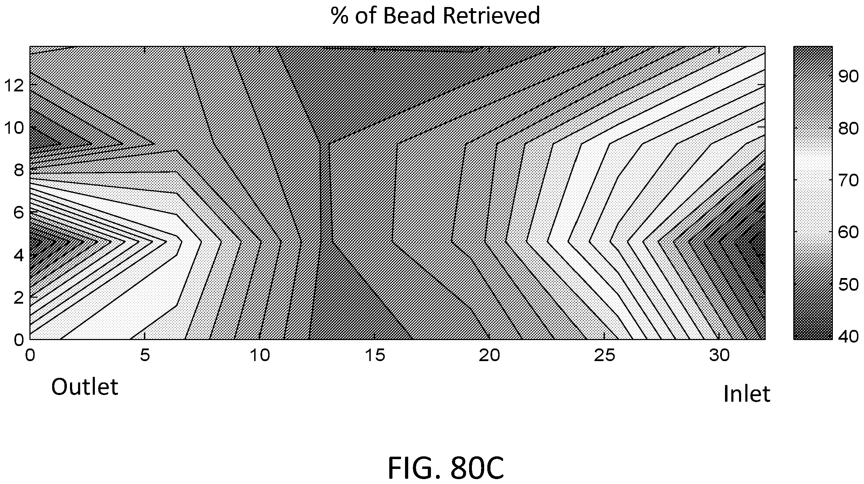

*Flow cell studies of bead loading & retrieval:* FIGS. 79 and 80 show examples of data for bead loading and retrieval studies performed using COC microarray substrates having 50 $\mu$m diameter x 50 $\mu$m deep microwells (60 $\mu$m pitch) packaged in flow cells. Microwells were loaded with a mixture of cells (1:1:1:1 K562, THP1, Ramos, Jurkat cells; 20,000 cells total) and 50 $\mu$m diameter beads functionalized with oligonucleotide barcoding libraries (100,000 beads total).

FIG. 79A shows the percentage of wells containing beads after the bead loading step and after bead retrieval. FIG. 79B provides a table of analysis statistics for an individual experiment. FIGS. 80A and 80B show heat maps for the bead fill rate (% of wells with beads) as a function of position within the flow cell after the bead loading and after bead retrieval steps. FIG. 80C shows a heat map of the bead retrieval percentage (1 - beads remaining/beads loaded)*100%) as a function of position with the flow cell after the bead retrieval step. The bead retrieval rate ranged from 77.2% to 93.3% over three independent experiments.

*Example 10 - Optical Adhesive on Glass Substrate Fabrication*

This example illustrates the procedures required to fabricate a patterned substrate with optical adhesive on a glass microscope slide. Using 3M tape, fix the microscope slide to the inside of a petri dish. Dispense approximately 300- 350 μl of NOA63 onto the microscope slide and use a P1000 pipette tip to evenly distribute the NOA63 on the slide and eliminate bubbles. Slowly lay the PDMS mold onto microscope slide such that the patterned side of the PDMS contacts the NOA63, and taking care to avoid trapping of bubbles between the PDMS and NOA63. Place the slide-NOA63-PDMS in the base of the aluminum jig and place a clean microscope slide on top of the PDMS mold. Complete the jig assembly by placing the top piece of the jig on the glass slide and *lightly* advancing the screws until there is resistance. Then, tighten the screws in increments of no more than 1/8 turn at a time. Repeat until there is no slack before each screw is tightened. Wait for 5 minutes to allow even distribution of NOA63 throughout and around the perimeter of the patterned portions of the PDMS mold. Lightly tighten the screws one more time, using the same tightening procedure as above. Place the assembled aluminum jig onto the Maestro trans-illuminator and cure for a minimum of 1 hour. Set the UV source to high and 365 nm. Record the "1st cure" start time. Record the "1st cure" finish time. Disassemble the custom jig. De-mold the PDMS mold from the molded NOA63/Microscope slide. Using the scalpel and razor blade, remove protruding NOA63 from the perimeter of the substrate. Cure the substrate for at least an additional 6 hours with the NOA63 side of the substrate facing the UV source, placing the substrate on a microscope slide at either end. Set the UV source to high and 365 nm. Record the "2nd cure" start time. Record the "2nd cure" finish time.

*Example 11 - Fabrication of Microwells Using a Photolithographically-Patterned Master & Injection Molding*

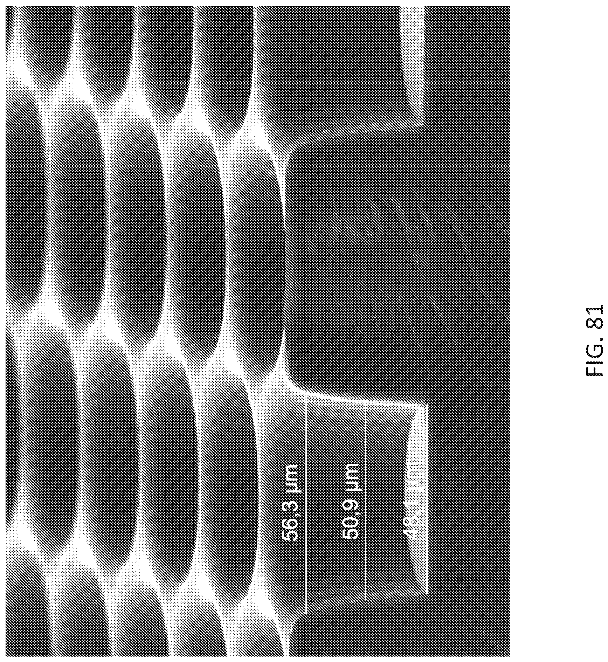

Several approaches to fabrication of microwell arrays have been evaluated. In this example, microwell arrays were fabricated using a photolithographically-patterned master and injection molding. The method comprised: (i) fabricating a master pattern using UV optical lithography and a suitable substrate (*e.g.* glass or silicon); the UV dose was chosen so as to only partially expose the substrate walls between the wells, such that the wall angle is the desired draft angle and the substrate surface between wells has the desired rounded profile; (ii) coating the patterned master with a durable material such as nickel; and (iii) injection molding using the coated master and COC or other suitable polymers to fabricate the microwell arrays. FIG. 81 shows a micrograph of a microwell array fabricated using this approach.

*Example 12 - Alternative Assay Workflows*

An alternate assay workflow for loading beads into microwells prior to loading of cells was examined. Potential advantages for this alternate workflow (*i.e.* beads loaded first rather than cells loaded first) are: (a) simplification of the workflow (reduced number of steps) and ease of user operation, (b) reduced complexity, cost, and development time for automation of the assay workflow, and (c) possible enablement of pre-loading of beads into the microwell substrate prior to shipping of a consumable cartridge to a customer.

In the standard workflow for loading cells and micro-beads into the microwell array, cells are loaded prior to loading of the beads. This study examined an alternate workflow where beads are loaded to the micro-well array prior to loading of the cells. This approach was explored due to expected benefits such as simplification of the workflow resulting from the reduction of process steps, potential ease of automation, and the potential for enabling pre-loading of beads to the cartridge during fabrication of the consumable. In addition to these potential benefits we also found some unexpected advantages to the approach, *e.g.* the efficiency of bead loading was improved (*i.e.* the frequency of observing bead doublets was reduced) and the cell capture efficiency, which was expected to drop with this alternate approach, appeared to be similar to the cell capture efficiency achieved with the standard workflow of loading the cells first.

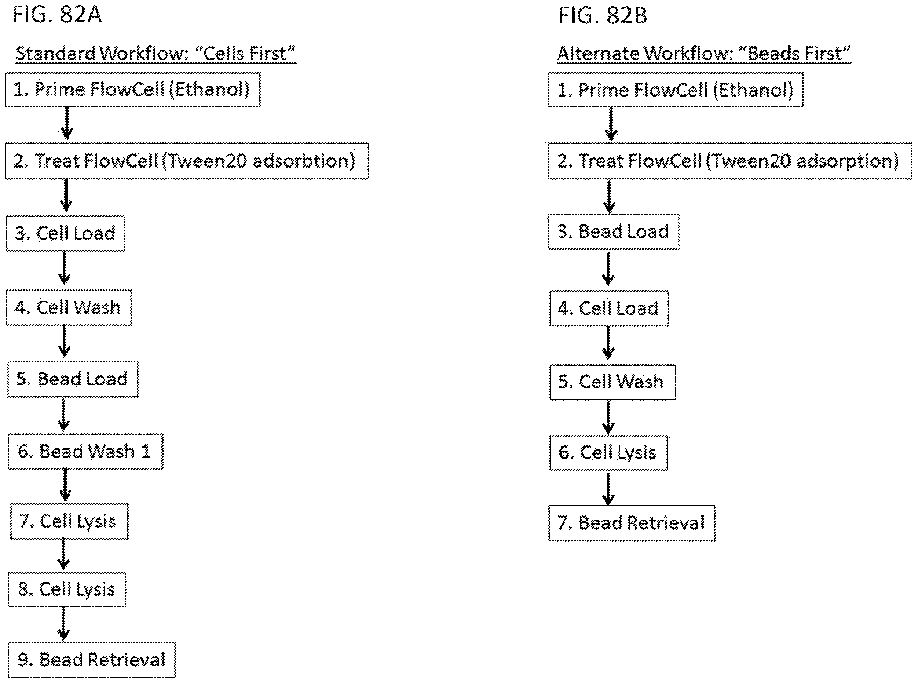

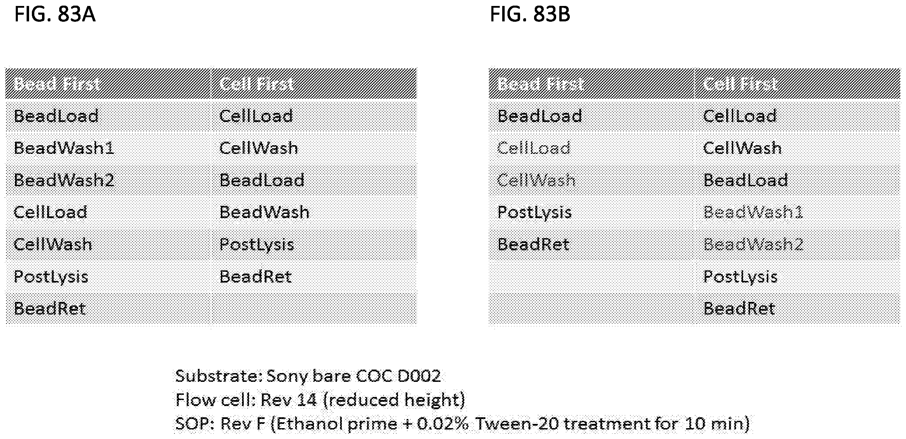

FIGS. 82A-B compare the standard ("cells first") and alternate ("beads first") workflows. The alternate workflow had the benefit of reducing the number of assay steps to be performed. Several variations on the "cell first" and "bead first" workflows were examined (FIGS. 83A-B) using a microwell substrate fabricated from COC and flow cell design (Rev. 14) having a reduced height (depth). Both procedures included an ethanol priming step and treatment of the flow cell with 0.02% Tween-20 for 10 minutes prior to loading beads or cells. Experiments were repeated twice.

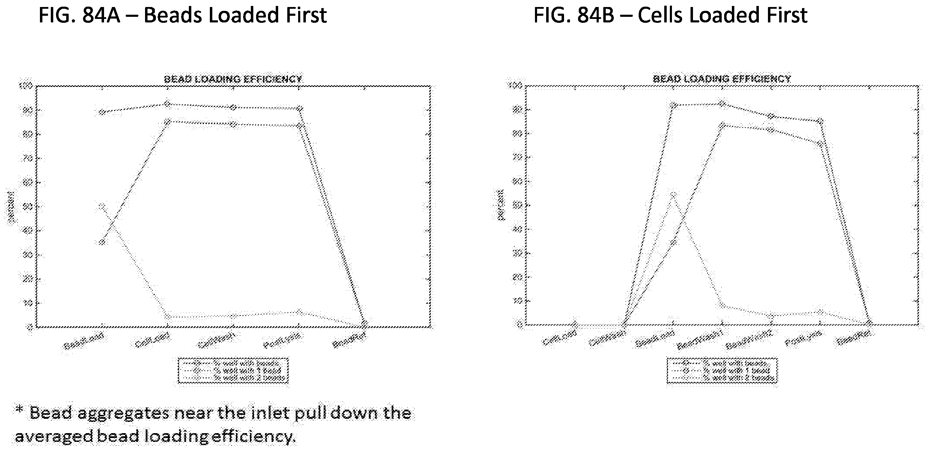

FIGS. 84A-B show a comparison of bead loading efficiency for each step in the "beads first" and "cells first" assay workflows, respectively. The observed frequency of doublets in the "cells first" approach varies, but it is usually in the range of 5% - 15%. At the step prior to cell lysis the observed frequency of bead doublets was roughly 5% in both the "beads first" and the "cells first" approaches used for this experiment.

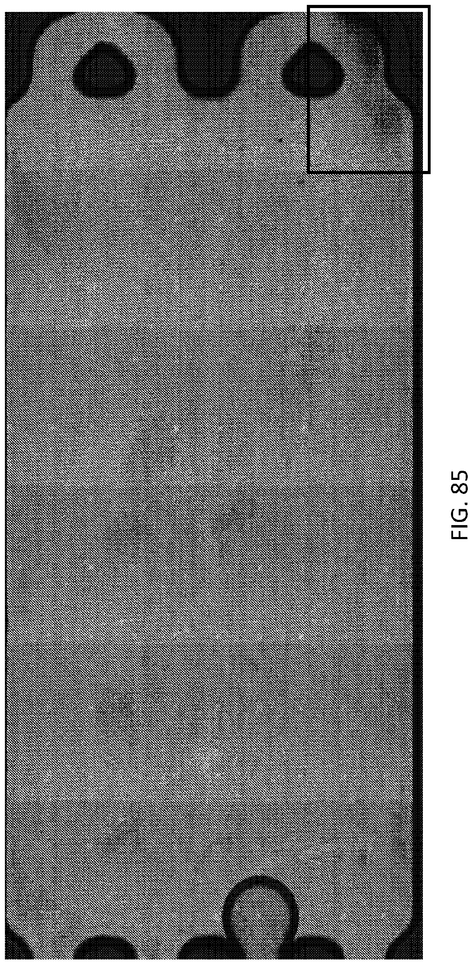

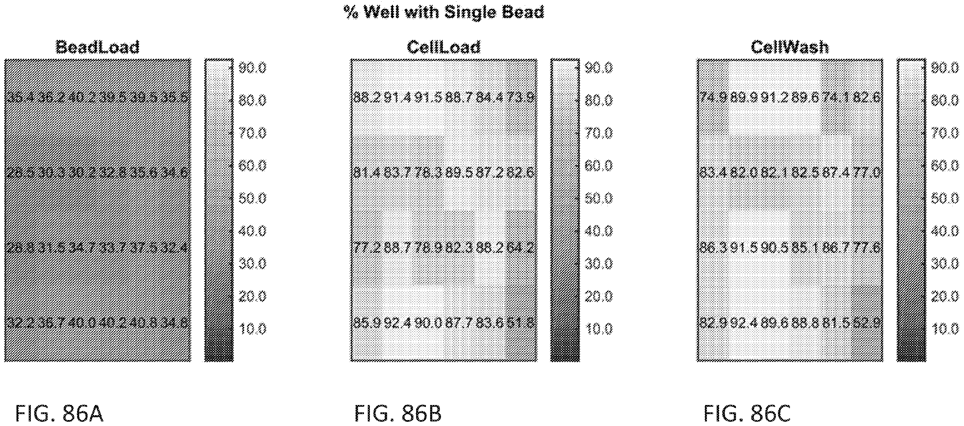

FIG. 85 shows a composite image of the flow cell comprising the microwell array used in these studies. FIGS. 86A-C show heat maps of single bead loading efficiency as a function of position within the flow cell for different steps of the "beads first" assay workflow. "Hotspots" of bead doublet frequency were observed due to an increased flow impedance in an outlet branch of the flow-cell (outlined in the lower right-hand corner of the composite image in FIG. 85). This reliability issue is independent of the workflow, and the observed doublet frequency is expected to improve (*i.e.* be reduced) as such reliability concerns are resolved. With all fields-of-view (FOVs) in the composite image included, the observed doublet frequency was ~ 5%. Excluding the wells in the lower right-hand corner of the composite image yielded a bead doublet frequency for the "beads first" assay workflow of ~ 4%.

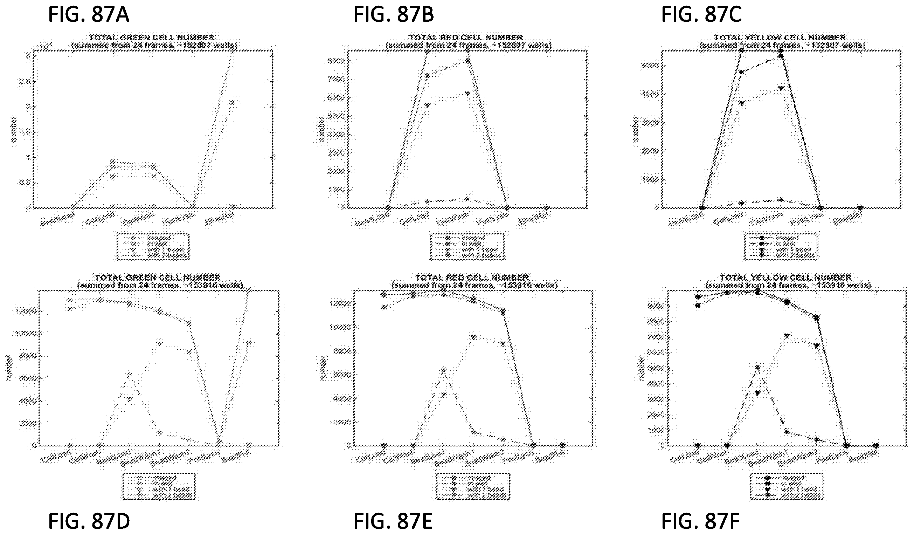

FIGS 87A-F show a comparison of the cell capture efficiency for the "beads first" (FIGS. 87A-C) and "cells first" (FIGS. 87D-F) assay workflows. A key performance metric is the number of cells captured in the step prior to cell lysis versus the number of cells imaged in the "cell load" step. For the "beads first" approach the cells captured during the "cell wash" step (*i.e.* the step prior to cell lysis) is nearly equivalent to the number of cells imaged during the "cell load" step. In the "cells first" approach the number of cells captured during the "bead wash" step (*i.e.* the step prior to cell lysis) is lower than the number of cells imaged during the "cell load" step. The net loss of cells ("bead wash" step versus "cell load" step) in the "cells first" approach is not always high, but it is consistent, and this loss was not observed in the "beads first" approach.

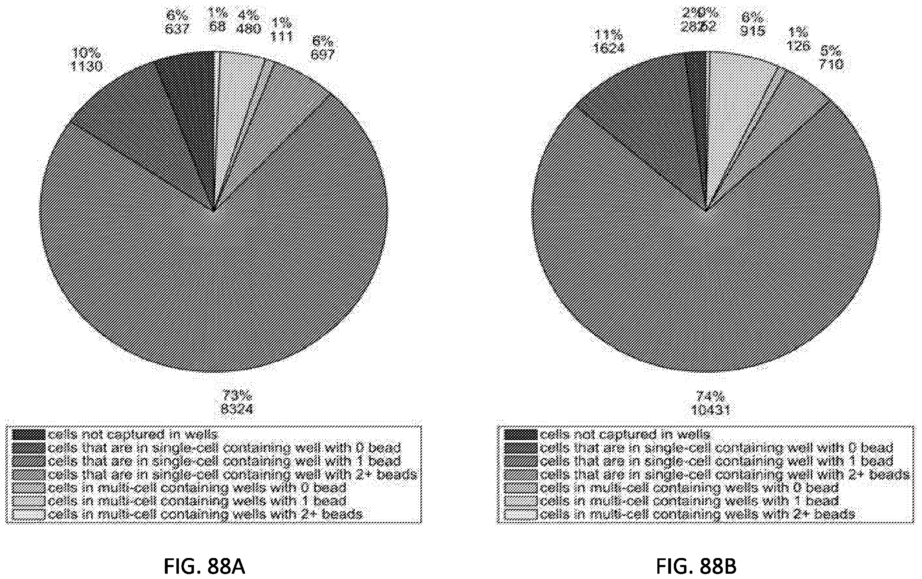

FIGS. 88A-B show the frequency of another important performance metric, *i.e.* the percentage of cells associated with a single bead. Both approaches exhibited similar performance with respect to this parameter.

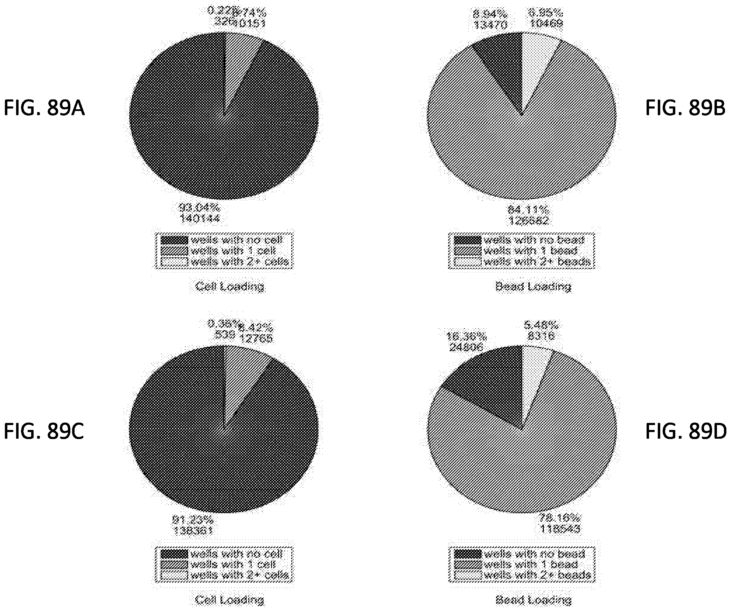

FIGS. 89A-D show data for another important performance metric, *i.e.* the percentage of wells containing a single bead prior to the cell lysis step. For this metric, the "beads first" approach (FIGS. 89A-B) shows an improvement over the "cells first" approach (FIGS. 89C-D).

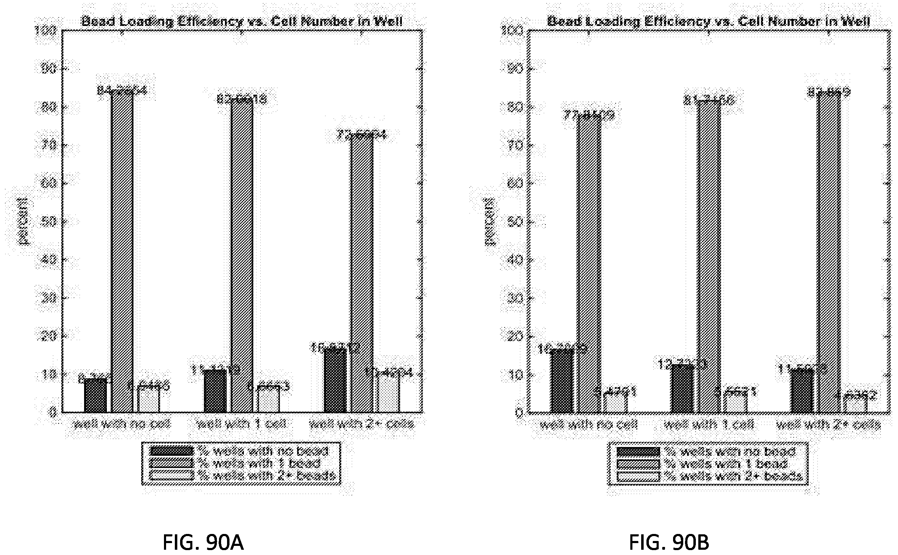

FIGS. 90A-B show plots of bead loading efficiency versus the number of cells in the microwell for the "bead first" (FIG. 90A) and "cells first" (FIG. 90B) assay workflows, respectively.

*Example 13 - Modified Pipette Tip Interface*

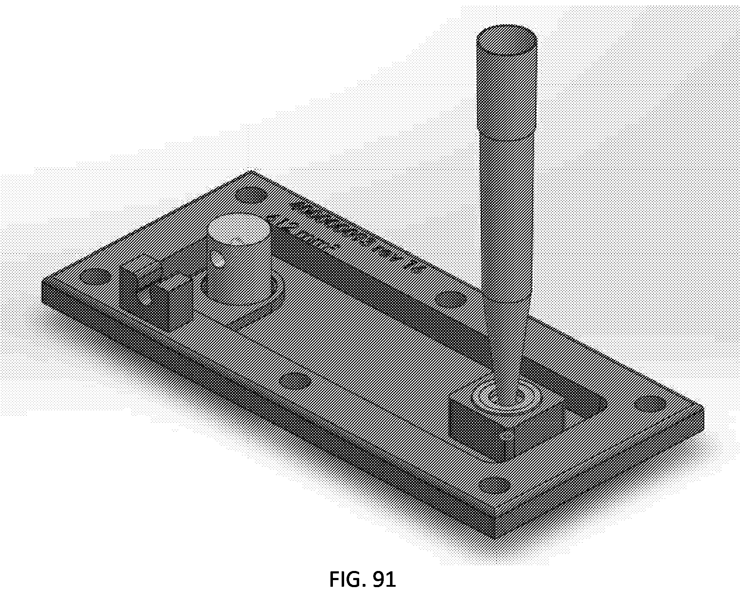

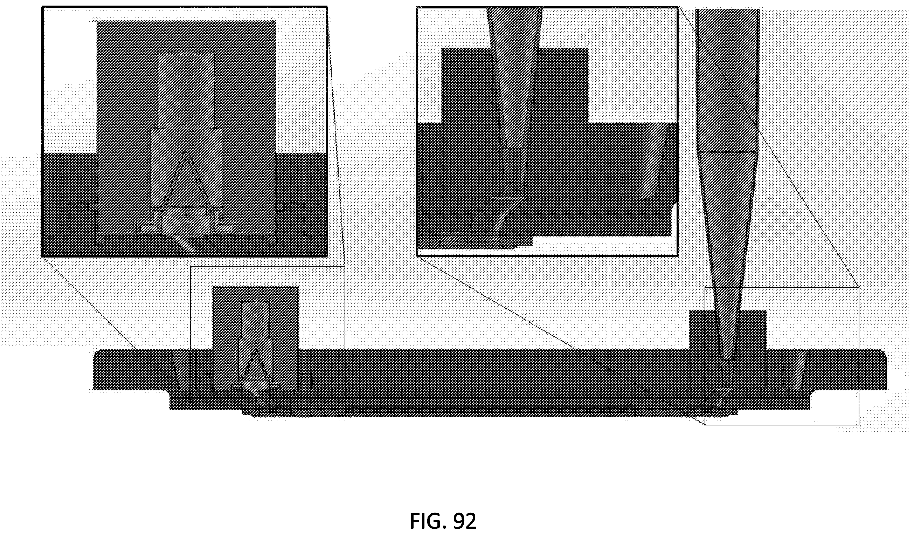

The previous pipette tip interface design utilized a one-way duckbill valve at the outlet to eliminate back-flow into the cartridge from an outlet port reservoir (FIGS. 91-92; showing the duckbill valve on the cartridge outlet). The duckbill valve was found to be incompatible with assay workflow because the use of 33um beads restricts the valve from closing completely, thereby resulting in backflow of buffer from an outlet (*e.g.* waste reservoir) reservoir to the cartridge. The modified design eliminates the outlet valve and uses an x-fragm dispense valve (miniValve Inc.) at the inlet instead. The x-fragm inlet valve is cracked open and then penetrated by the pipette tip. Fluid (such as a bead loading buffer) flows through the pipette tip rather than directly through the valve, so the issue of incomplete closure of the valve observed for the previous design are avoided.

The modified pipette tip interface design also includes changes in the design of the seal formed around the pipette tip. The previous design utilized a friction fit seal between the pipette tip and the cartridge (FIGS. 91-92; showing the friction fit pipette interface on the cartridge inlet). The deficiencies observed for the friction fit seal included: (i) deformation / pinching of the pipette tip when the pipette tip was pressed into the cartridge with too much force, thereby resulting in increased fluidic impedance and reduced flow rates to the cartridge; this was especially problematic for manual cartridge operation; (ii) the friction fit did not smoothly release the pipette tip from the cartridge when the pipette tip was retracted, thereby resulting in lifting of the cartridge or release of the pipette tip from the pipette; this was especially problematic for automated operation of the cartridge with a robotic pipette; and (iii) the fully-seated position for the pipette tip is not well defined for the friction fit and varies with the force applied to the pipette tip; if an air gap is present inside the pipette interface after a seal between the pipette tip and the cartridge is formed, then a bubble may be injected into the cartridge.

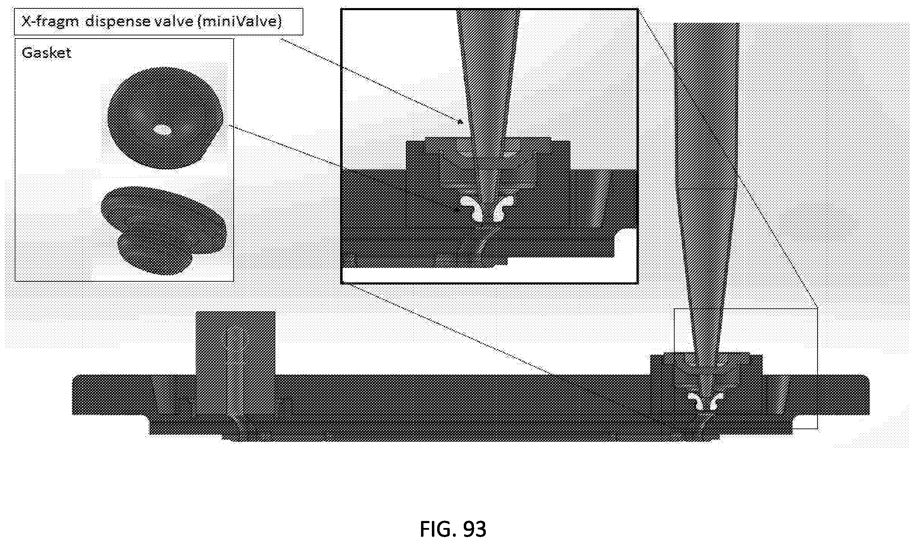

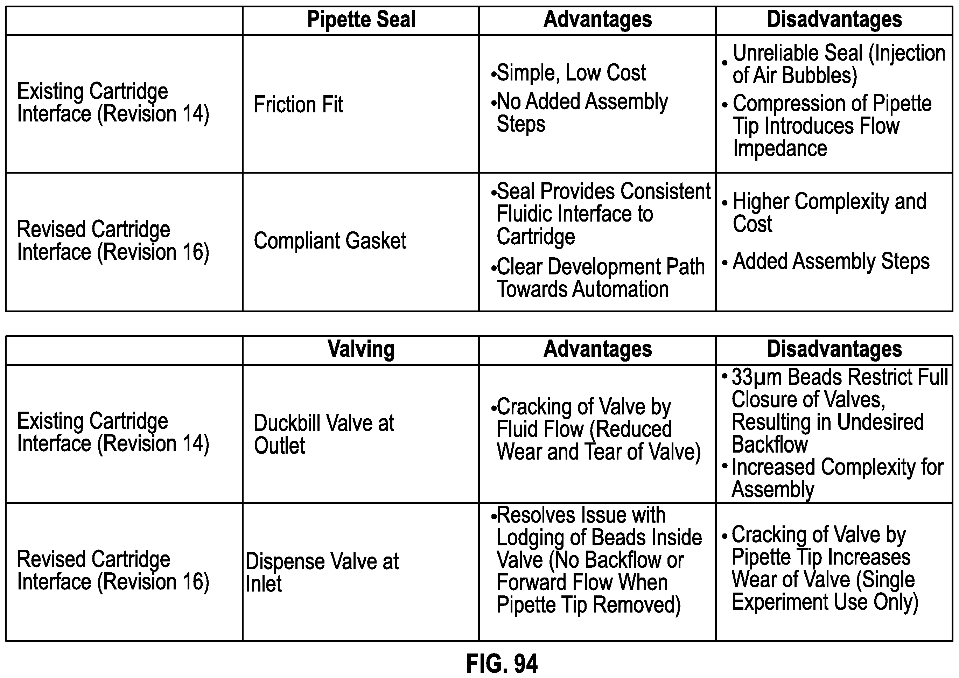

The updated design utilizes a custom molded gasket made of a compliant material, e.g. polydimethylsiloxane (PDMS), polyisoprene, polybutadiene, or polyurethane. By replacing the friction fit interface with a gasket interface, the size of the air gap between the pipette tip and the cartridge is significantly reduced. Provided that a small drop of liquid is present on the pipette tip before a seal is made (such a drop can be introduced for automated pipetting), a fluidic interface is formed between the gasket and the pipette tip without injection of bubbles to the cartridge. FIG. 93 shows the updated cartridge interface with the molded compliant gasket and the x-fragm dispense valve. FIG. 94 shows a summary comparison of the two pipette tip interface designs.

*Example 14 - Modified "Beads First" Assay Workflow with Ficoll-Based Bead Washing and Cell Lysis Steps*

A modified assay workflow using Ficoll-based bead washing and cell lysis steps was evaluated, wherein magnetic microbeads were loaded into a consumable cartridge comprising a microwell array prior to loading single cells (*i.e.* a "beads first" assay workflow). Potential advantages for this modified assay workflow are: (a) elimination of air displacement steps performed on a tilt plate that were used to fully displace liquids from the flow cell, (b) elimination of fluid displacement steps performed on a tilt plate that were used to fully displace air bubbles from the flow cell (thereby enabling a fully-automated workflow), and (c) reduced RNA diffusion and potential crosstalk between microwells.

In the existing "beads first" assay workflow, beads are loaded prior to cells and washed out with air displacement to reduce the percentage of bead doublets in microwells. The air displacement step causes significant bead loss with

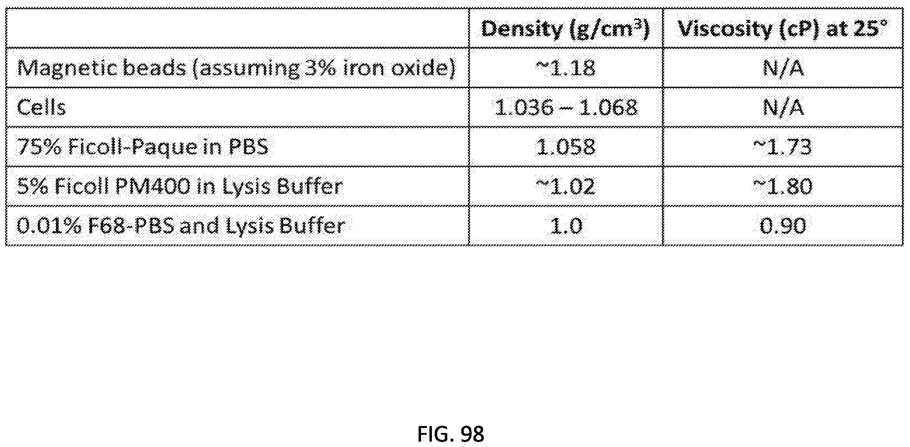

SiO$_2$-coated substrates and requires that the operation be performed on a tilt plate. A modified workflow was evaluated where beads were washed out using 75% Ficoll-Paque in PBS and 0.01% Pluronic F68-PBS with no air displacement. The use of 75% Ficoll-Paque was expected to effectively reduce the frequency of bead doublets due to its higher viscosity (~1.73 cP compared to 0.90 cP of PBS, see FIG. 98). The subsequent wash with 0.01% F68-PBS helps to flush away floating beads generated during the Ficoll wash. This Ficoll-based bead washing approach was examined due to expected benefits such as reduced frequency of bead doublets, reduced bead loss, reduced risk of bubble generation, and the potential ease of automation provided by a tilt-free assay workflow. In addition to the above modifications to the bead washing buffer, 5% Ficoll PM400 was also added to the lysis buffer to increase the density and viscosity of the buffer (FIG. 98). This was expected to slow RNA diffusion after cell lysis and reduce potential crosstalk between microwells.

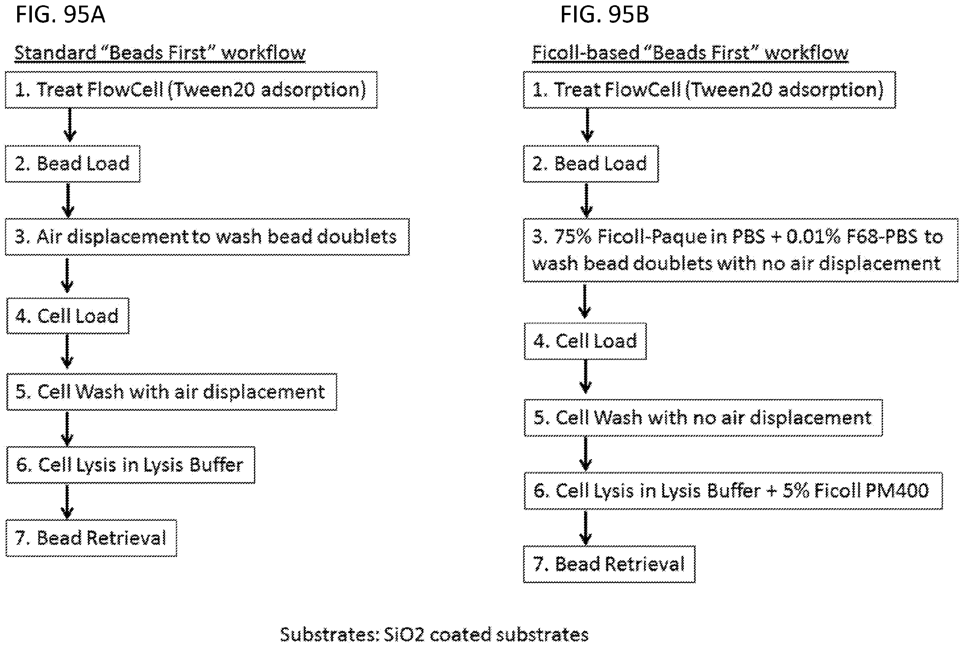

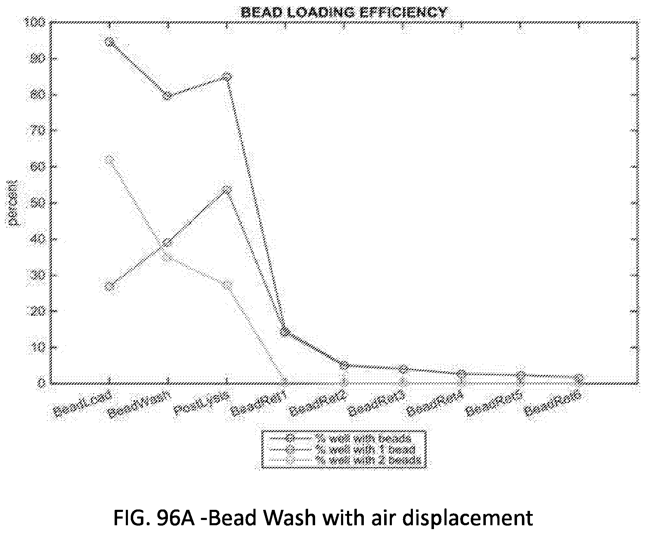

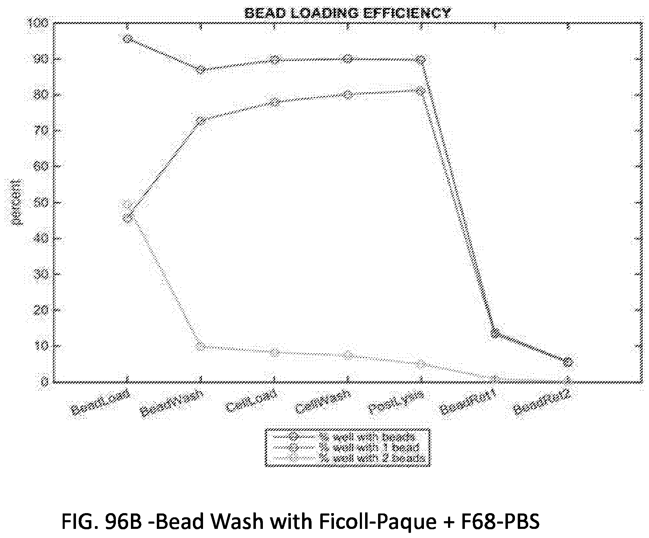

FIGS 95A-B show the standard "beads first" assay workflow (FIG. 95A) and the modified, Ficoll-based assay workflow (FIG. 95B). FIGS. 96A-B show examples of data illustrating the improved bead loading efficiency achieved using Ficoll-based bead washing steps with SiO$_2$-coated microwell substrates. When air displacement was used, we observed a high percentage of empty wells and a high percentage of wells with 2 beads. When air displacement was replaced by 75% Ficoll-Paque in PBS and 0.01% F68-PBS during the bead washing steps, the percentages of both empty wells and wells with 2 beads decreased. Increasing the volume of 75% Ficoll-Paque in PBS during the bead washing steps is expected to further improve bead loading efficiency, which will be a target for further optimization.

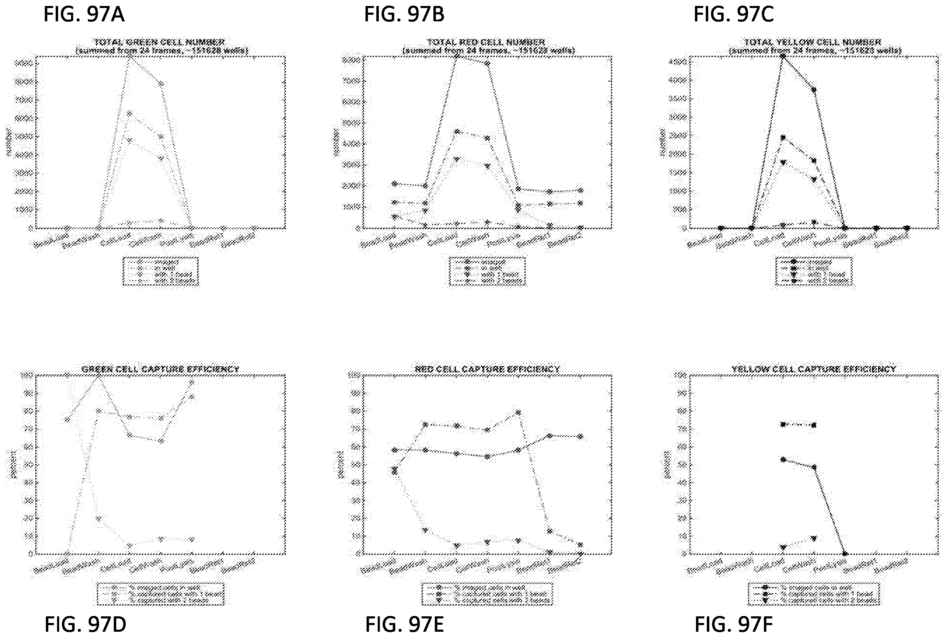

FIGS. 97A-F show examples of data for the cell capture efficiency achieved using the Ficoll-based assay workflow. Cell viability was well maintained with the introduction of Ficoll-Paque. Cell capture efficiency within the microwells decreased due to the higher density of 75% Ficoll-Paque in PBS filling the microwells. Further optimization is needed to increase the cell capture efficiency of the Ficoll-based assay workflow.

FIG. 98 summarizes the estimated density and viscosity values of magnetic beads, cells, and solutions used in the Ficoll-based assay workflow.

**Claims**

1. A device comprising:

   a substrate comprising at least 100 microwells, wherein each microwell has a volume ranging from about 1,000 $\mu$m$^3$ to about 786,000 $\mu$m$^3$, and
   a plurality of beads, wherein a plurality of the at least 100 microwells each contain a single bead, and wherein the ratio of the average diameter of the microwells to the diameter of the beads ranges from about 1.2 to about 1.8; and
   a flow cell in fluid communication with the substrate; and
   a pipette tip interface for loading or removing samples, assay reagents, bead suspensions, or waste from the device;
   and a valve that prevents fluid flow within the device unless a pipette tip is inserted into the conical feature of the pipette tip interface.

2. The device of claim 1, wherein a surface of the at least 100 microwells is coated with a surface coating to improve wettability.

3. The device of any one of claim 1 or claim 2, wherein

   (i) the coefficient of variation for microwell volume is less than 5%; or
   (ii) each microwell has a volume ranging from about 21,000 $\mu$m$^3$ to about 170,000 $\mu$m$^3$; or
   (iii) the aspect ratio of average diameter to depth for the at least 100 microwells ranges from about 0.1 to 2; or
   (iv) the ratio of the average diameter of the microwells to the diameter of the beads is about 1.5; or
   (v) the side walls of the microwells have a positive draft angle of about 1 to 15 degrees; or
   (vi) any combination of (i) to (v).

4. The device of any one of claims 1 to 3, wherein the substrate is fabricated from a material selected from the group consisting of silicon, fused-silica, glass, a polymer, a metal, an elastomer, polydimethylsiloxane, agarose, and a hydrogel, or any combination thereof.

5. The device of any one of claims 1 to 4, wherein the flow cell comprises the substrate or wherein the flow cell can be detached from the substrate.

**6.** The device of any one of claims 1 to 5, wherein the conical feature mates to a pipette tip to form a fluid connection with the inlet port or outlet port, and optionally wherein the conical feature is comprised of a compliant material that forms a substantially leak-proof seal with the pipette tip.

**7.** The device of any one of claims 1 to 6, further comprising a molded gasket made of a compliant material, wherein the gasket is configured to form a releasable seal with a pipette tip inserted into the pipette tip interface.

**8.** A system comprising:

the device of any one of claims 1 to 7; and
a flow controller; wherein the flow controller is configured to control the delivery of fluids, and optionally wherein the flow controller is configured to intersperse fluid injections into the flow cell with air injections.

**9.** The system of claim 8, further comprising fluids wherein the fluids comprise cell samples, bead suspensions, assay reagents, or any combination thereof.

**10.** The system of claim 8 or claim 9, further comprising

(i) a distribution mechanism for enhancing the uniform distribution of cells and beads across the at least 100 microwells, wherein the distribution mechanism performs an action selected from the group consisting of rocking, shaking, swirling, recirculating flow, low frequency agitation, and high frequency agitation, or any combination thereof; or
(ii) a cell lysis mechanism that uses a high frequency piezoelectric transducer for sonicating the cells; or
(iii) a temperature controller for maintaining a user-specified temperature, or for ramping temperature between two or more specified temperatures over two or more specified time intervals; or
(iv) a magnetic field controller for creating magnetic field gradients used in eluting beads from the at least 100 microwells or for transporting beads through the device; or
(v) an imaging system configured to capture and process images of all or a portion of the at least 100 microwells, wherein the imaging system further comprises an illumination subsystem, an imaging subsystem, and a processor; or
(vi) a selection mechanism, wherein information derived from the processed images is used to identify a subset of cells exhibiting one or more specified characteristics, and the selection mechanism is configured to either include or exclude the subset of cells from subsequent data analysis; or
(vii) any combination of (i) to (vi).

**11.** A kit comprising the device of any one of claim 1 to 7.

**12.** The kit of claim 11, further comprising

(i) reagents for performing a reverse transcription reaction; or
(ii) reagents for performing a nucleic acid amplification reaction; or
(iii) reagents for performing one or more target-specific nucleic acid amplification reactions; or
(iv) a cell lysis buffer or hybridization buffer; or
(v) any combination of (i) to (iv).

**13.** A method for loading one or more cell samples into the microwells of the device of any one of claims 1 to 7, the method comprising:

a) injecting air into the flow cell in fluid communication with the substrate comprising at least 100 microwells;
b) injecting a cell sample into the flow cell; and
c) injecting air into the flow cell,

and optionally injecting a buffer or bead suspension into the flow cell after the air is injected into the flow cell.

**Patentansprüche**

**1.** Vorrichtung, umfassend:

ein Substrat, das mindestens 100 Mikrovertiefungen umfasst, wobei jede Mikrovertiefung ein Volumen im Bereich von etwa 1.000 $\mu$m bis etwa 786.000 $\mu$m$^3$ aufweist und

eine Vielzahl von Kügelchen, wobei eine Vielzahl der mindestens 100 Mikrovertiefungen jeweils ein einzelnes Kügelchen enthält und wobei das Verhältnis des durchschnittlichen Durchmessers der Mikrovertiefungen zum Durchmesser der Kügelchen im Bereich von etwa 1,2 bis etwa 1,8 liegt; und

eine Durchflusszelle in Fluidverbindung mit dem Substrat; und

eine Pipettenspitzenschnittstelle zum Laden oder Entfernen von Proben, Testreagenzien, Kügelchensuspensionen oder Abfällen aus der Vorrichtung;

und ein Ventil, das den Flüssigkeitsfluss innerhalb der Vorrichtung verhindert, es sei denn, eine Pipettenspitze wird in das konische Merkmal der Pipettenspitzenschnittstelle eingeführt.

2. Vorrichtung nach Anspruch 1, wobei eine Oberfläche der mindestens 100 Mikrovertiefungen mit einer Oberflächenbeschichtung beschichtet ist, um die Benetzbarkeit zu verbessern.

3. Vorrichtung nach einem von Anspruch 1 oder Anspruch 2, wobei

(i) der Variationskoeffizient für das Mikrovertiefungsvolumen weniger als 5% beträgt; oder

(ii) jede Mikrovertiefung ein Volumen im Bereich von etwa 21.000 $\mu$m bis etwa 170.000 $\mu$m$^3$ aufweist; oder

(iii) das Aspektverhältnis von durchschnittlichem Durchmesser zu Tiefe für die mindestens 100 Mikrovertiefungen im Bereich von etwa 0,1 bis 2 liegt; oder

(iv) das Verhältnis des durchschnittlichen Durchmessers der Mikrovertiefungen zum Durchmesser der Kügelchen etwa 1,5 beträgt; oder

(v) die Seitenwände der Mikrovertiefungen einen positiven Schrägenwinkel von etwa 1 bis 15 Grad aufweisen; oder

(vi) irgendeine Kombination von (i) bis (v).

4. Vorrichtung nach einem der Ansprüche 1 bis 3, wobei das Substrat aus einem Material gefertigt ist, das ausgewählt ist aus der Gruppe bestehend aus Silicium, Quarzglas, Glas, einem Polymer, einem Metall, einem Elastomer, Polydimethylsiloxan, Agarose und einem Hydrogel oder einer beliebigen Kombination davon.

5. Vorrichtung nach einem der Ansprüche 1 bis 4, wobei die Durchflusszelle das Substrat umfasst oder wobei die Durchflusszelle vom Substrat lösbar ist.

6. Vorrichtung nach einem der Ansprüche 1 bis 5, wobei das konische Merkmal mit einer Pipettenspitze zusammenpasst, um eine Fluidverbindung mit dem Einlassanschluss oder dem Auslassanschluss zu bilden, und wobei das konische Merkmal wahlweise aus einem nachgiebigen Material besteht, das im Wesentlichen eine auslaufsichere Abdichtung mit der Pipettenspitze bildet.

7. Vorrichtung nach einem der Ansprüche 1 bis 6, ferner umfassend einen geformten Dichtungsring aus einem nachgiebigen Material, wobei die Dichtung konfiguriert ist, um eine lösbare Dichtung mit einer in die Pipettenspitzenschnittstelle eingesetzten Pipettenspitze zu bilden.

8. System, umfassend:

die Vorrichtung nach einem der Ansprüche 1 bis 7; und

eine Durchflusssteuerung;

wobei die Durchflusssteuerung konfiguriert ist, um die Abgabe von Fluiden zu steuern, und wobei die Durchflusssteuerung wahlweise konfiguriert ist, um Fluidinjektionen in die Durchflusszelle mit Luftinjektionen zu verteilen.

9. System nach Anspruch 8, ferner umfassend Fluide, wobei die Fluide Zellproben, Kügelchensuspensionen, Testreagenzien oder irgendeine Kombination davon umfassen.

10. System nach Anspruch 8 oder Anspruch 9, ferner umfassend

(i) einen Verteilungsmechanismus zum Verbessern der gleichmäßigen Verteilung von Zellen und Kügelchen über die mindestens 100 Mikrovertiefungen, wobei der Verteilungsmechanismus eine Aktion durchführt, die ausgewählt ist aus der Gruppe, bestehend aus Schwenken, Schütteln, Verwirbeln, Umwälzen, niederfrequentes Rühren und hochfrequentes Rühren oder irgendeine Kombination davon; oder

(ii) einen Zelllysemechanismus, der einen piezoelektrischen Hochfrequenzwandler zum Beschallen der Zellen verwendet; oder

(iii) eine Temperatursteuerung zum Aufrechterhalten einer vom Benutzer festgelegten Temperatur oder zum Erhöhen der Temperatur zwischen zwei oder mehr festgelegten Temperaturen über zwei oder mehr festgelegten Zeitintervallen; oder

(iv) eine Magnetfeldsteuerung zum Erzeugen von Magnetfeldgradienten, die zum Eluieren von Kügelchen aus den mindestens 100 Mikrovertiefungen oder zum Transportieren von Kügelchen durch die Vorrichtung verwendet werden; oder

(v) ein Abbildungssystem, das zum Erfassen und Verarbeiten von Bildern aller oder eines Anteils der mindestens 100 Mikrovertiefungen konfiguriert ist, wobei das Abbildungssystem ferner ein Beleuchtungssubsystem, ein Abbildungssubsystem und einen Prozessor umfasst; oder

(vi) einen Auswahlmechanismus, wobei aus den verarbeiteten Bildern abgeleitete Informationen verwendet werden, um eine Teilmenge von Zellen zu identifizieren, die eine oder mehrere festgelegte Eigenschaften aufzeigen, und der Auswahlmechanismus konfiguriert ist, um die Teilmenge von Zellen von der nachfolgenden Datenanalyse entweder einzuschließen oder auszuschließen; oder

(vii) irgendeine Kombination von (i) bis (vi).

11. Kit, umfassend die Vorrichtung nach einem der Ansprüche 1 bis 7.

12. Kit nach Anspruch 11, ferner umfassend

(i) Reagenzien zur Durchführung einer umgekehrten Transkriptionsreaktion; oder

(ii) Reagenzien zum Durchführen einer Nukleinsäureamplifikationsreaktion; oder

(iii) Reagenzien zum Durchführen einer oder mehrerer zielspezifischer Nukleinsäureamplifikationsreaktionen; oder

(iv) einen Zelllysepuffer oder Hybridisierungspuffer; oder

(v) irgendeine Kombination von (i) bis (iv).

13. Verfahren zum Laden einer oder mehrerer Zellproben in die Mikrovertiefungen der Vorrichtung nach einem der Ansprüche 1 bis 7, wobei das Verfahren umfasst:

a) Einspritzen von Luft in die Durchflusszelle in Fluidverbindung mit dem Substrat, das mindestens 100 Mikrovertiefungen umfasst;

b) Einspritzen einer Zellprobe in die Durchflusszelle; und

c) Einspritzen von Luft in die Durchflusszelle, und wahlweise Einspritzen einer Puffer- oder Kügelchensuspension in die Durchflusszelle, nachdem die Luft in die Durchflusszelle eingespritzt wurde.

## Revendications

1. Dispositif comprenant :

un substrat comprenant au moins 100 micropuits, chaque micropuits ayant un volume allant d'environ 1000 $\mu m^3$ à environ 786 000 $\mu m^3$, et

une pluralité de billes, une pluralité des au moins 100 micropuits contenant chacun une seule bille, et le rapport entre le diamètre moyen des micropuits et le diamètre des billes allant d'environ 1,2 à environ 1,8 ; et

une cellule d'écoulement en communication fluidique avec le substrat ; et

une interface de bout de pipette pour charger ou retirer des échantillons, des réactifs de dosage, des suspensions de billes, ou un déchet du dispositif ;

et une vanne qui empêche un écoulement de fluide à l'intérieur du dispositif sauf si un bout de pipette est inséré dans l'élément conique de l'interface de bout de pipette.

2. Dispositif de la revendication 1, dans lequel une surface des au moins 100 micropuits est recouverte d'un revêtement de surface pour améliorer la mouillabilité.

3. Dispositif de l'une quelconque des revendications 1 et 2, dans lequel

(i) le coefficient de variation de volume de micropuits est inférieur à 5 % ; ou

(ii) chaque micropuits a un volume allant d'environ 21 000 $\mu m^3$ à environ 170 000 $\mu m^3$; ou

(iii) le rapport de forme entre le diamètre moyen et la profondeur pour les au moins 100 micropuits va d'environ 0, 1 à 2 ; ou

(iv) le rapport entre le diamètre moyen des micropuits et le diamètre des billes est d'environ 1,5 ; ou

(v) les parois latérales des micropuits ont un angle de dépouille positif d'environ 1 à 15 degrés ; ou

(vi) toute combinaison de (i) à (v).

**4.** Dispositif de l'une quelconque des revendications 1 à 3, dans lequel le substrat est fabriqué à partir d'un matériau choisi dans le groupe constitué par le silicium, la silice fondue, le verre, un polymère, un métal, un élastomère, le polydiméthylsiloxane, l'agarose, et un hydrogel, ou toute combinaison de ceux-ci.

**5.** Dispositif de l'une quelconque des revendications 1 à 4, dans lequel la cellule d'écoulement comprend le substrat ou dans lequel la cellule d'écoulement peut être détachée du substrat.

**6.** Dispositif de l'une quelconque des revendications 1 à 5, dans lequel l'élément conique s'accouple à un bout de pipette pour former un raccord fluidique avec l'orifice d'entrée et/ou l'orifice de sortie, et éventuellement dans lequel l'élément conique est composé d'un matériau souple qui forme un joint sensiblement étanche avec le bout de pipette.

**7.** Dispositif de l'une quelconque des revendications 1 à 6, comprenant en outre un joint statique moulé constitué d'un matériau souple, le joint statique étant configuré pour former un joint amovible avec un bout de pipette inséré dans l'interface de bout de pipette.

**8.** Système comprenant :

le dispositif de l'une quelconque des revendications 1 à 7 ; et

un régulateur de débit ; le régulateur de débit étant configuré pour réguler la délivrance de fluides, et le régulateur de débit étant éventuellement configuré pour parsemer les injections de fluide dans la cellule d'écoulement d'injections d'air.

**9.** Système de la revendication 8, comprenant en outre des fluides, les fluides comprenant des échantillons cellulaires, des suspensions de billes, des réactifs de dosage, ou toute combinaison de ceux-ci.

**10.** Système de la revendication 8 ou la revendication 9, comprenant en outre

(i) un mécanisme de distribution pour améliorer la distribution uniforme de cellules et de billes entre les au moins 100 micropuits, le mécanisme de distribution effectuant une action choisie dans le groupe constitué par un balancement, un secouement, un tourbillon, un écoulement de recirculation, une agitation à basse fréquence, et une agitation à haute fréquence, ou toute combinaison de ceux-ci ; ou

(ii) un mécanisme de lyse cellulaire qui utilise un transducteur piézoélectrique à haute fréquence pour soumettre les cellules à des ultrasons ; ou

(iii) un régulateur de température pour maintenir une température spécifiée par l'utilisateur, ou pour faire varier la température entre au moins deux températures spécifiées sur au moins deux intervalles de temps spécifiés ; ou

(iv) un contrôleur de champ magnétique pour créer des gradients de champ magnétique utilisés dans l'émission de billes à partir des au moins 100 micropuits ou pour transporter des billes à travers le dispositif ; ou

(v) un système d'imagerie configuré pour capturer et traiter des images de tout ou partie des au moins 100 micropuits, le système d'imagerie comprenant en outre un sous-système d'éclairage, un sous-système d'imagerie, et un processeur ; ou

(vi) un mécanisme de sélection, des informations déduites des images traitées étant utilisées pour identifier un sous-ensemble de cellules présentant une ou plusieurs caractéristiques spécifiées, et le mécanisme de sélection étant configuré pour inclure ou exclure le sous-ensemble de cellules d'une analyse ultérieure de données ; ou

(vii) toute combinaison de (i) à (vi).

**11.** Trousse comprenant le dispositif de l'une quelconque des revendications 1 à 7.

**12.** Trousse de la revendication 11, comprenant en outre

(i) des réactifs pour effectuer une réaction de transcription inverse ; ou

(ii) des réactifs pour effectuer une réaction d'amplification d'acides nucléiques ; ou

(iii) des réactifs pour effectuer une ou plusieurs réactions d'amplification d'acides nucléiques spécifiques d'une cible ; ou

(iv) un tampon de lyse cellulaire ou un tampon d'hybridation ; ou

(v) toute combinaison de (i) à (iv).

13. Procédé de chargement d'un ou plusieurs échantillons cellulaires dans les micropuits du dispositif de l'une quelconque des revendications 1 à 7, le procédé comprenant :

a) l'injection d'air dans la cellule d'écoulement en communication fluidique avec le substrat comprenant au moins 100 micropuits ;

b) l'injection d'un échantillon cellulaire dans la cellule d'écoulement ; et

c) l'injection d'air dans la cellule d'écoulement,

et éventuellement l'injection d'un tampon ou d'une suspension de billes dans la cellule d'écoulement après que l'air a été injecté dans la cellule d'écoulement.

FIG. 1

cells in microwells

Load cell suspension

Load beads

cells + beads in microwells

FIG. 2A

Zoom-in examples:
single cell with bead in a well

bead

cell

cell

bead

FIG. 2B

FIG. 3A

FIG. 3B

FIG. 3C

FIG. 4A

FIG. 4B

FIG. 4C

First PCR (multiplex) of genes of interest (15 cycles):

FIG. 5A

| bead | Illumina seq primer 1 | Cell Label | Molecular label | oligodT | GENE |

⇩

Second PCR (multiplex, nested) of genes of interest (15 cycles):

FIG. 5B

| Illumina seq primer 1 | Cell Label | Molecular label | oligodT | GENE |

Illumina seq primer 2

⇩

Third PCR (universal) to add full length sequencing adaptor (8 cycles):

FIG. 5C

| Illumina seq primer 1 | Cell Label | Molecular label | oligodT | GENE | Illumina seq primer 2 |

PS

Sample index +P7

⇩

Paired-end sequencing:

Read1 (150bp)

Index1 (6bp)

FIG. 5D

| PS | Illumina seq primer 1 | Cell Label | Molecular label | oligodT | GENE | seq primer 2 | Sample index +P7 |

Read2 (150bp)

FIG. 6

FIG. 7B

501

FIG. 7A

FIG. 8A

FIG. 8B

FIG. 9A

FIG. 9B

FIG. 10

FIG. 11

FIG. 12

FIG. 13A

FIG. 13B

FIG. 13C

FIG. 14A

FIG. 14B

Check valve inside machined housing

Single 3D-printed part
(2 materials)

Flowcell (layout with branched inlet
and outlet)

Baseplate (microtiter footprint)

FIG. 15

EP 3 248 018 B1

Machined housing for check valve

Check valve (Minivalve DU 039)

Single 3D-printed part, 2 materials
- Rigid portions Top plate
  - Pipettor interface port
  - Fluidic layer for flowcell layout
- Compliant elastomer
  - Gasket which traces outline of flowcell on fluidic layer

Substrate

Baseplate

FIG. 16

EP 3 248 018 B1

Retention feature for valve housing

Single 3D-printed part, 2 materials
- Rigid portions (material "VeroClear" transparent ABS)
  - Top plate
  - Pipettor interface port
  - Fluidic layer for flowcell layout
- Compliant elastomer "TangoPlus"
  - Gasket which traces outline of flowcell on fluidic layer

FIG. 17

EP 3 248 018 B1

Compliant material is inset in trench within rigid part, for added strength

0.5 mm compliance thickness is achieved using a 1 mm thick printed feature

Rounded profile reduces compression force required to achieve seal

FIG. 18

FIG. 19A

FIG. 19B

FIG. 20A

FIG. 20B

FIG. 20C

Clamping only        Hybrid clamp/consumable    Pre-assembled consumable

Cartridge

Instrument

EP 3 248 018 B1

FIG. 21A  FIG. 21B  FIG. 21C

2105  2106  2107

2101  2102

2103

2104

2108  2109  2110

EP 3 248 018 B1

FIG. 22A

FIG. 22B

2203    2204

2201    2202

2205    2206    2207

EP 3 248 018 B1

FIG. 23B

Loads via P1000 micropipettor interface

FIG. 23A

4 fluid ports

Loads from top surface
via tubing ports

FIG. 24A

FIG. 24B

FIG. 24C

FIG. 25A

FIG. 25B

Modified pipette tip interface

Nanoport

Rail for O-ring (8mm diameter, 1mm wide)

Collection Tube

Pipette tip interface

Connect to syringe

Alternative: check valve

FIG. 26

EP 3 248 018 B1

FIG. 27

Outlet: connect to waste &
collection tube

Pipette tip inlet: connect with
duckbill valve & tubing

Nanoports

Connect to check valve & syringe

FIG. 28

FIG. 29A

FIG. 29B

FIG. 30

Integrated System

Imaging Module

Optical Imaging System

Microwell Array Flow Cell (fixed or removable)

or

Microwell Array Cartridge (removable)

Processor / Controller

Flow Controller

Fluidics Control System

Cell/Bead Distribution System

Cell Lysis System

Magnetic Field System

Temperature Control System

**Instrument**

**Assay Automation**

- Distribute Cells
- Wash and Lyse
- Hybridize RNA/DNA
- Magnet-Assisted Bead Retrieval

**Imaging and Analysis**

- Number of Cells
- Option: Live/Dead Evaluation
- Quality Statistics Including Bead-Cell Correlations

Embedded PC Running Custom Resolve Software

| Imaging | Motion Control | Magnet Control | Pressure/ Vacuum |

Sample In

Catridge

Catridge

EP 3 248 018 B1

**FIG. 31**

**FIG. 32**

EP 3 248 018 B1

FIG. 33

**FIG. 34**

**FIG. 35**

EP 3 248 018 B1

FIG. 36

EP 3 248 018 B1

**FIG. 37**

EP 3 248 018 B1

FIG. 38

FIG. 39

FIG. 40

FIG. 41

FIG. 42

Comparison with Poisson Distribution

FIG. 43

**Bead Settling over Time**

FIG. 44

FIG. 45A

FIG. 45B

FIG. 45C

Select Images

Identify Field-of-Interest
Based on Grid

Canny Edge Detection
&
Hough Circle
Transform to Detect
Cells

Optional: Fluorescence
Image Analysis

EP 3 248 018 B1

FIG. 46

FIG. 47A — Brightfield

FIG. 47B — Fluorescence (Calcein)

FIG. 47C — Overlay

| I. Define Well Edge | II. Bead Identification | III. Edge Identification | IV. Cell Identification |

FIG. 48

## FIG. 49A

Original Bright-Field Image

## FIG. 49B

Microwells Identified:
- Edge detection
- Hough circle transform
- Assign a unique ID number to each well

## FIG. 49C

Mask Function:
- White = areas inside wells
- Black = areas outside wells

# FIG. 50A

Original Bright-Field or
Fluorescence Image

# FIG. 50B

Microwell Mask

# FIG. 50C

Identify Beads or Cells:
• In bright-field image
• In fluorescence image
• Each bead or cell assigned to
  a specific microwell ID

EP 3 248 018 B1

FIG. 51B

FIG. 51A

| FIle Name: | cells_60mins_00s.bmp |
|---|---|
| X-Y Upper Left Corner: | 1000 1000 |
| X-Y Lower Right Corner: | 2500 2000 |
| Total # of Wells: | 1434 |
| Number of Wells with Beads | 1 |
| Number of Wells with Single Cells | 325 |
| Number of Wells with Doublets | 45 |
| Number of Wells with Trilets | 4 |
| Number of Wells with Beads & Single Cells | 0 |
| % of Wells with Beads | 0.069735 |
| % of Wells with Single Cells | 22.6639 |
| % of Wells with 1 Cell + Bead | 0 |

# FIG. 51C

FIG. 52C

FIG. 52A

Well top

FIG. 52B

Well bottom

Well top

FIG. 53A

D=36.5um

FIG. 53C

37 um
Well top

Well bottom

FIG. 53B

D=49.3um

Pitch = 60um

Well bottom
50um

EP 3 248 018 B1

*FIG. 54*

EP 3 248 018 B1

**FIG. 55A**

**FIG. 55B**

**FIG. 55C**

**FIG. 55D**

FIG. 56

*FIG. 57*

EP 3 248 018 B1

*FIG. 58A*

*FIG. 58B*

| | | | |
|---|---|---|---|
| Beads Settled | Magnet on Bottom | Wash | Magnet on Top + Wash (2X) |
| 59.9 +/- 4.2% | 90.57 +/- 7.2% | 82.7 +/- 7.4% | 5.6 +/- 1.3% |

*FIG. 59A*      *FIG. 59B*      *FIG. 59C*      *FIG. 59D*

FIG. 60

EP 3 248 018 B1

FIG. 61

Basic Flow-Cell

FIG. 62A

Branched Inlet Flow-Cell

FIG. 62B

Fill Uniformity: 83 +/- 10%
(87 +/- 10% on front half)

FIG. 63

Fill Efficiency: 83 +/- 12%
(94 +/- 6% on front half)

FIG. 64

Fill Uniformity: 95 +/- 3%

FIG. 65

| Work-Flow | Flow-Cell Layout | Bead Loading Uniformity |
|---|---|---|
| Approach #1 | Single inlet / outlet | 83 +/- 10%<br>(87 +/- 10% front half) |
| | Branched Inlet | 83 +/-12%<br>(94 +/- 6% front half) |
| Approach #2 | Single inlet / outlet | 95 +/- 3% |

FIG. 66

# Single Cell Stochastic Labeling Assay Workflow

| | |
|---|---|
| 1. Count and dilute cells<br>2. Load cells (imaging), wash away unsettled cells<br>3. Load beads (imaging)<br>4. Load lysis buffer while on magnet, incubate<br>5. Wash surface with lysis buffer<br>6. Retrieve beads with magnet, transfer to tubes | Image Analysis |

7. Wash beads with two buffers in tube
8. RT in tube
9. Exol in tube
10. PCR (3steps)
11. Sequencing
12. Analysis
13. Data Visualization

*FIG. 67*

|  | Efficiency |
|---|---|
| Cell Loading<br>(cells captured inside wells / total cells imaged) | Ramos: 80 +/- 16%<br>K562: 75 +/- 10% |
| Bead Loading<br>(wells with a bead / total wells imaged) | 84 +/- 9%<br>(91+/- 5% excluding fluidic dead zones) |
| Bead retrieval<br>(wells with a bead / total wells imaged) | 6 +/- 3% |

FIG. 68

| Cells Imaged on substrate (inside wells) | Bead Fill Efficiency | Cells w/ Beads | Cells Detected by Sequencing | Theoretical Yield |
|---|---|---|---|---|
| Ramos: 428<br>K562: 599 | 83% | 352 Ramos<br>499 K562 | 69 Ramos<br>274 K562 | 20%<br>55% |

*FIG. 69*

EP 3 248 018 B1

FIG. 70A

FIG. 70B

Inlet                                   Outlet

FIG. 71A

**1mm Thick Channel**

FIG. 71B

**2mm Thick Channel**

Inlet                                              Outlet

FIG. 72D          FIG. 72E          FIG. 72F

EP 3 248 018 B1

FIG. 73

**% Wells with Beads**

FIG. 74

FIG. 75A

FIG. 75B

FIG. 75C

% Wells with Cells--Cell Settled

% Wells with Cells--Beads Settled

% Wells with Cells--Magnet Underneath

| Step | Cell Loaded | Bead Loaded | Bead distributed w/ Magnet |
|---|---|---|---|
| % Wells with Single Cells | 10.86 +/- 2.31 | 8.78 +/- 1.98 | 7.89 +/- 1.39 |
| % Wells with Double Cells | 0.77 +/- 0.63 | 0.37 +/- 0.37 | 0.23 +/- 0.29 |
| % Wells with 1 cell+ 1 bead | N/A | 4.12 +/- 1.29 | 4.57 +/- 1.39 |
| % Wells with Beads | N/A | 55.49 +/- 7.35 | 66.74 +/- 8.24 |

FIG. 76

FIG. 77A

FIG. 77B

Lysis Only

Lysis + DTT

FIG. 78A

FIG. 78B

Lysis + DTT, no magnet distribution

Lysis + DTT, magnet distribution

## FIG. 79A

**% Wells with Beads**

## FIG. 79B

| Statistics | |
|---|---|
| total wells | 144855 |
| total FL cells | 16446 |
| total Green cells captured in wells | 4758 |
| total Red cells captured in wells | 6088 |
| cell capture efficiency % (total) | 65.9 |
| total wells with beads | 99838 |
| % wells with beads | 68.9 |
| total wells with 1 Gr cell+ 1 bead | 3721 |
| total wells with 1 Gr cell+ 1 bead | 2980 |
| % wells with 1 cell + 1 bead | 4.6 |

EP 3 248 018 B1

FIG. 80A

After Bead Retrieval

FIG. 80B

% of Bead Retrieved

Outlet

Inlet

FIG. 80C

FIG. 81

FIG. 82B

Alternate Workflow: "Beads First"

1. Prime FlowCell (Ethanol) → 2. Treat FlowCell (Tween20 adsorption) → 3. Bead Load → 4. Cell Load → 5. Cell Wash → 6. Cell Lysis → 7. Bead Retrieval

FIG. 82A

Standard Workflow: "Cells First"

1. Prime FlowCell (Ethanol) → 2. Treat FlowCell (Tween20 adsorbtion) → 3. Cell Load → 4. Cell Wash → 5. Bead Load → 6. Bead Wash 1 → 7. Cell Lysis → 8. Cell Lysis → 9. Bead Retrieval

## FIG. 83A

| Bead First | Cell First |
|---|---|
| BeadLoad | CellLoad |
| BeadWash1 | CellWash |
| BeadWash2 | BeadLoad |
| CellLoad | BeadWash |
| CellWash | PostLysis |
| PostLysis | BeadRet |
| BeadRet | |

## FIG. 83B

| Bead First | Cell First |
|---|---|
| BeadLoad | CellLoad |
| CellLoad | CellWash |
| CellWash | BeadLoad |
| PostLysis | BeadWash1 |
| BeadRet | BeadWash2 |
| | PostLysis |
| | BeadRet |

Substrate: Sony bare COC D002
Flow cell: Rev 14 (reduced height)
SOP: Rev F (Ethanol prime + 0.02% Tween-20 treatment for 10 min)

FIG. 84B – Cells Loaded First

FIG. 84A – Beads Loaded First

* Bead aggregates near the inlet pull down the averaged bead loading efficiency.

FIG. 85

% Well with Single Bead

**BeadLoad**

|  |  |  |  |  |  |
|---|---|---|---|---|---|
| 35.4 | 36.2 | 40.2 | 39.5 | 39.5 | 35.5 |
| 28.5 | 30.3 | 30.3 | 32.8 | 35.6 | 34.6 |
| 28.5 | 31.5 | 34.7 | 33.7 | 37.5 | 32.4 |
| 32.2 | 36.7 | 40.0 | 40.2 | 40.8 | 34.8 |

**CellLoad**

|  |  |  |  |  |  |
|---|---|---|---|---|---|
| 88.2 | 91.4 | 91.5 | 88.7 | 84.4 | 73.9 |
| 81.4 | 83.7 | 78.3 | 89.5 | 87.2 | 82.6 |
| 77.2 | 88.7 | 78.9 | 82.3 | 88.2 | 64.2 |
| 85.9 | 92.4 | 90.0 | 87.7 | 83.6 | 51.8 |

**CellWash**

|  |  |  |  |  |  |
|---|---|---|---|---|---|
| 74.9 | 89.9 | 91.2 | 89.6 | 74.1 | 82.6 |
| 83.4 | 82.0 | 82.1 | 82.5 | 87.4 | 77.0 |
| 86.3 | 91.5 | 90.5 | 85.1 | 86.7 | 77.6 |
| 82.9 | 92.4 | 89.6 | 88.8 | 81.5 | 52.9 |

FIG. 86A

FIG. 86B

FIG. 86C

EP 3 248 018 B1

FIG. 87A

TOTAL GREEN CELL NUMBER
(summed from 24 frames, ~152807 wells)

FIG. 87B

TOTAL RED CELL NUMBER
(summed from 24 frames, ~152807 wells)

FIG. 87C

TOTAL YELLOW CELL NUMBER
(summed from 24 frames, ~152807 wells)

TOTAL GREEN CELL NUMBER
(summed from 24 frames, ~153916 wells)

TOTAL RED CELL NUMBER
(summed from 24 frames, ~153916 wells)

TOTAL YELLOW CELL NUMBER
(summed from 24 frames, ~153916 wells)

FIG. 87D

FIG. 87E

FIG. 87F

EP 3 248 018 B1

FIG. 88B

FIG. 88A

FIG. 89B

FIG. 89D

FIG. 89A

FIG. 89C

FIG. 90B

FIG. 90A

FIG. 91

FIG. 92

FIG. 93

|  | Pipette Seal | Advantages | Disadvantages |
|---|---|---|---|
| Existing Cartridge Interface (Revision 14) | Friction Fit | •Simple, Low Cost<br>•No Added Assembly Steps | • Unreliable Seal (Injection of Air Bubbles)<br>• Compression of Pipette Tip Introduces Flow Impedance |
| Revised Cartridge Interface (Revision 16) | Compliant Gasket | •Seal Provides Consistent Fluidic Interface to Cartridge<br>•Clear Development Path Towards Automation | • Higher Complexity and Cost<br>• Added Assembly Steps |

|  | Valving | Advantages | Disadvantages |
|---|---|---|---|
| Existing Cartridge Interface (Revision 14) | Duckbill Valve at Outlet | •Cracking of Valve by Fluid Flow (Reduced Wear and Tear of Valve) | •33μm Beads Restrict Full Closure of Valves, Resulting in Undesired Backflow<br>• Increased Complexity for Assembly |
| Revised Cartridge Interface (Revision 16) | Dispense Valve at Inlet | •Resolves Issue with Lodging of Beads Inside Valve (No Backflow or Forward Flow When Pipette Tip Removed) | • Cracking of Valve by Pipette Tip Increases Wear of Valve (Single Experiment Use Only) |

**FIG. 94**

EP 3 248 018 B1

FIG. 95B

Ficoll-based "Beads First" workflow

1. Treat FlowCell (Tween20 adsorption) → 2. Bead Load → 3. 75% Ficoll-Paque in PBS + 0.01% F68-PBS to wash bead doublets with no air displacement → 4. Cell Load → 5. Cell Wash with no air displacement → 6. Cell Lysis in Lysis Buffer + 5% Ficoll PM400 → 7. Bead Retrieval

FIG. 95A

Standard "Beads First" workflow

1. Treat FlowCell (Tween20 adsorption) → 2. Bead Load → 3. Air displacement to wash bead doublets → 4. Cell Load → 5. Cell Wash with air displacement → 6. Cell Lysis in Lysis Buffer → 7. Bead Retrieval

Substrates: SiO2 coated substrates

FIG. 96A -Bead Wash with air displacement

FIG. 96B -Bead Wash with Ficoll-Paque + F68-PBS

FIG. 97A

FIG. 97B

FIG. 97C

FIG. 97D

FIG. 97E

FIG. 97F

|  | Density (g/cm³) | Viscosity (cP) at 25° |
|---|---|---|
| Magnetic beads (assuming 3% iron oxide) | ~1.18 | N/A |
| Cells | 1.036 – 1.068 | N/A |
| 75% Ficoll-Paque in PBS | 1.058 | ~1.73 |
| 5% Ficoll PM400 in Lysis Buffer | ~1.02 | ~1.80 |
| 0.01% F68-PBS and Lysis Buffer | 1.0 | 0.90 |

FIG. 98

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100300895 A
- US 20060040297 A
- WO 2008147428 A
- WO 2013148525 A

**Non-patent literature cited in the description**

- **FAN et al.** Combinatorial Labeling of Single Cells for Gene Expression Cytometry. *Science,* vol. 347 (6222), 628
- *Science,* vol. 347 (6222), 1258367
- **M. HAMADY et al.** *Nat. Methods,* 2008, vol. 5, 235-237
- **FU et al.** *Analytical Chemistry,* 2014, vol. 86, 2867-2870
- **G. FU et al.** *Analytical Chemistry,* 2014, vol. 86, 2867-2870
- **R. D. SMITH et al.** *Nucleic Acids Res.,* 2000, vol. 28, 4998-5004
- **D. A. KAMINSKI et al.** *Frontiers in Immunology,* 2012, vol. 3, 302
- **Y. SHEN et al.** *BMC Immunol.,* 2004, vol. 5, 20
- **J. A. WEINSTEIN et al.** *PloS One,* 2013, vol. 8, e67624
- **WARREN SMITH.** Modern Optical Engineering. 401